(19) Europäisches Patentamt
European Patent Office
Office européen des brevets

(11) Publication number: **0 166 516 B1**

# (12) EUROPEAN PATENT SPECIFICATION

(45) Date of publication of patent specification: **08.04.92**

(51) Int. Cl.5: **C07D 417/12**, C07D 417/14, C07D 403/12, C07F 7/10, A01N 47/36, A01N 55/02

(21) Application number: **85303231.6**

(22) Date of filing: **07.05.85**

The file contains technical information submitted after the application was filed and not included in this specification

## (54) Novel phenyl-subsituted sulfonamides.

(30) Priority: **07.05.84 US 607989**
**22.10.84 US 663555**
**29.03.85 US 716351**

(43) Date of publication of application:
**02.01.86 Bulletin 86/01**

(45) Publication of the grant of the patent:
**08.04.92 Bulletin 92/15**

(84) Designated Contracting States:
**BE CH DE FR GB IT LI LU NL SE**

(56) References cited:
**EP-A- 0 099 339**
**EP-A- 0 107 979**
**EP-A- 0 165 653**

(73) Proprietor: **E.I. DU PONT DE NEMOURS AND COMPANY**
**1007 Market Street**
**Wilmington Delaware 19898(US)**

(72) Inventor: **Pasteris, Robert James**
**305 Plymouth Road**
**Wilmington Delaware 19803(US)**

(74) Representative: **Hildyard, Edward Martin et al**
**Frank B. Dehn & Co. Imperial House 15-19 Kingsway**
**London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 166 516 B1

## Description

This invention relates to novel condensed ring sulfonylureas and their use as herbicides and growth regulants.

U.S. Patent 4,127,405 teaches compounds which are useful for controlling weeds in wheat having the formula:

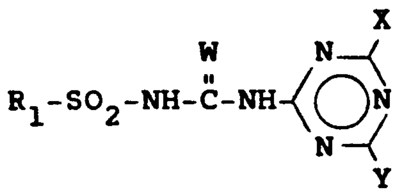

(I)

wherein

$R_1$ is

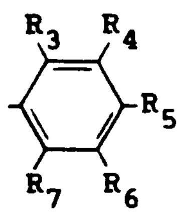

,

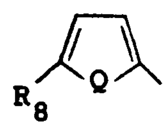

,

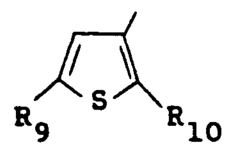

or

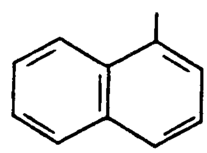

;

$R_3$ and $R_6$ are independently hydrogen, fluorine, chlorine, bromine, iodine, alkyl of 1-4 carbon atoms, alkoxy of 1-4 carbon atoms, nitro, trifluoromethyl, cyano, $CH_3S(O)_n$- or $CH_3CH_2S$-$(O)_n$-;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1-2 carbon atoms or alkoxy of 1-2 carbon atoms;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1-3 carbon atoms, trifluoromethyl, $CH_3S$- or $CH_3OCH_2$-;

and

Y is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

U.S. Patent 4,169,719 discloses herbicidal benzenesulfonylureas.

Herbicidal indanesulfonylureas are taught in U.S. Patent 4,465,506.

Herbicidal quinolinesulfonylureas are described in U.S. Patent 4,369,329, issued January 18, 1983.

Herbicidal benzofuran, benzothiophene, benzopyran and benzothiopyran sulfonylureas are disclosed in

EP-A-79,683, published May 25, 1983.

EP-A-82,681, published June 29, 1983, discloses herbicidal 1,3-benzodioxole and 1,4-benzodioxanesulfonylureas.

South African Patent Application 83/5165 discloses herbicidal sulfonylureas of the general structure shown below:

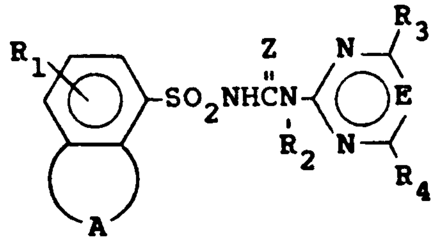

wherein

A is an unsubstituted or substituted bridge of 3 or 4 atoms which contains 1 or 2 oxygen, sulfur or nitrogen atoms and, together with the linking carbon atom, forms a non-aromatic 5- or 6-membered heterocyclic ring system, with the proviso that two oxygen atoms are separated by at least one carbon atom and that oxygen and sulfur atoms are only linked to each other if the sulfur atom takes the form of the -SO- or $SO_2$- group.

South African Patent Application 83/7434 discloses herbicidal sulfonamides of formula

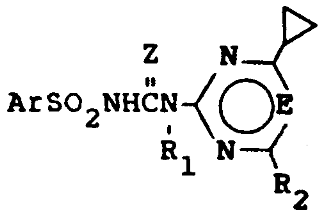

where Ar is

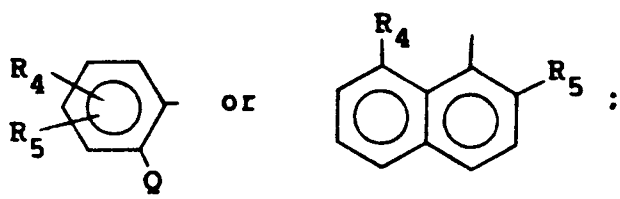

and

$R_2$ is halogen, $C_1$-$C_3$ alkyl, $C_1$-$C_3$ haloalkyl, $C_1$-$C_3$ alkoxy, $C_1$-$C_3$ haloalkoxy, amino, $C_1$-$C_3$ alkylamino, di($C_1$-$C_3$ alkyl)amino, $C_3$-$C_6$ cycloalkyl or $C_2$-$C_6$ alkoxyalkyl.

The presence of undesired vegetation causes substantial damage to useful crops, especially agricultural products that satisfy man's basic food needs, such as soybeans, barley, wheat, and the like. The current population explosion and concomitant world food shortage demand improvements in the efficiency of producing these crops. Prevention or minimizing the loss of a portion of valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. The need exists, however, for still more effective herbicides that destroy or retard weeds without causing significant damage to useful crops.

## Summary of the Invention

This invention relates to novel compounds of Formula I, agriculturally suitable compositions containing

them, and their method-of-use as preemergent or postemergent herbicides or as plant growth regulants.

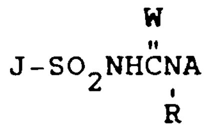

I

wherein

J is

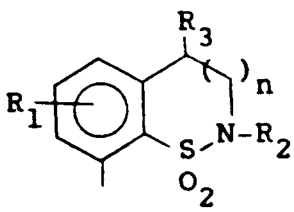
,
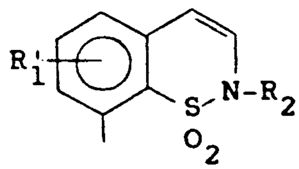
,
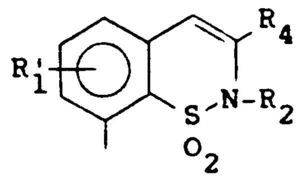
,

J-1 J-2 J-3

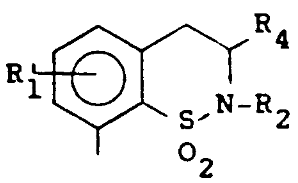
,

J-4

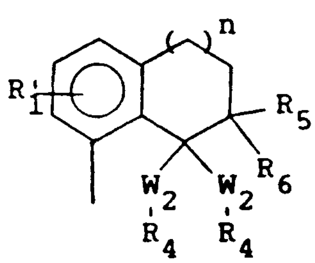

J-11

or

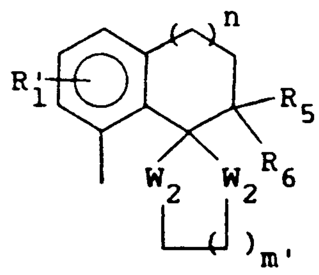

J-12

n is 0 or 1;
W is O or S;
$W_1$ is S;
$W_2$ is O or S;
R is H or $CH_3$;
$R_1$ is H, $C_1$-$C_6$ alkyl, $C_1$-$C_6$ haloalkyl, halogen, nitro, $C_1$-$C_6$ alkoxy, $SO_2NR_aR_b$, $C_1$-$C_6$ alkylthio, $C_1$-$C_6$ alkylsulfinyl, $C_1$-$C_6$ alkylsulfonyl, CN, $CO_2R_c$, $C_1$-$C_6$ haloalkoxy, $C_1$-$C_6$ haloalkylthio, $NH_2$, $C_1$-$C_6$ alkylamino, di($C_1$-$C_6$ alkyl)amino, $Si(CH_3)_2$($C_1$-$C_4$ alkyl), $Si(CH_3)_2$phenyl or $C_1$-$C_3$ alkyl substituted with $C_1$-$C_3$ alkoxy, $C_1$-$C_3$ alkylthio, $C_1$-$C_3$ alkylsulfinyl, $C_1$-$C_3$ alkylsulfonyl, $SO_2NR_dR_e$, $NO_2$, CN, $CO_2R_f$, $C_1$-$C_3$ haloalkoxy or $C_1$-$C_3$ haloalkylthio;
$R_a$ is H, $C_1$-$C_4$ alkyl, $C_1$-$C_3$ cyanoalkyl, methoxy or ethoxy;
$R_b$ is H, $C_1$-$C_4$ alkyl or $C_3$-$C_4$ alkenyl; or
$R_a$ and $R_b$ may be taken together as $-(CH_2)_3-$, $-(CH_2)_4-$, $-(CH_2)_5-$ or $-CH_2CH_2OCH_2CH_2-$;
$R_c$ is $C_1$-$C_4$ alkyl, $C_3$-$C_4$ alkenyl, $C_3$-$C_4$ alkynyl, $C_2$-$C_4$ haloalkyl, $C_2$-$C_3$ cyanoalkyl, $C_5$-$C_6$ cycloalkyl, $C_4$-$C_7$ cycloalkylalkyl or $C_2$-$C_4$ alkoxyalkyl;
$R_d$ is $C_1$-$C_3$ alkyl;
$R_e$ is H or $C_1$-$C_3$ alkyl;
$R_f$ is $C_1$-$C_3$ alkyl;
$R_i$ is H, $C_1$-$C_3$ alkyl, $C_1$-$C_3$ alkoxy, $C_1$-$C_3$ haloalkoxy, $C_1$-$C_3$ haloalkyl, $C_1$-$C_3$ alkylthio, $C_1$-$C_3$ haloalkylthio, amino, $C_1$-$C_3$ alkylamino, di($C_1$-$C_3$ alkyl)amino, halogen or $NO_2$;
$R_2$ is H, $R_{11}$, $SR_{11}$, $SO_2R_{11}$, $OR_{11}$, $C(O)R_{11}$, $C(O)OR_{11}$, $(C(O))_2OR_{11}$, $(CO)_2R_{11}$, $C(O)NR_{12}R_{18}$, C-(O)NRA, $C(S)SR_{11}$, $NH_2$, $NR_{12}R_{18}$, OH, CN, $P(O)R_{13}R_{14}$, $P(S)R_{13}R_{14}$, $Si(CH_3)_2R_{15}$, L or C(O)L;
$R_3$ is H or $CH_3$;
$R_4$ is $C_1$-$C_4$ alkyl;
$R_5$ is H or $C_1$-$C_4$ alkyl;
$R_6$ is H or $CH_3$;

A is 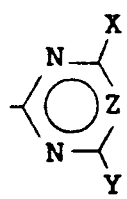 , 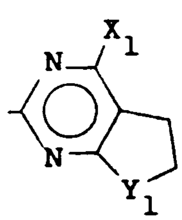 , 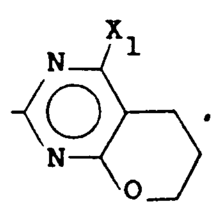 ,

A-1 A-2 A-3

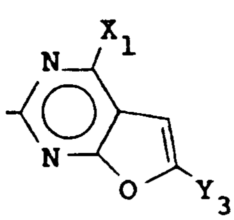 , 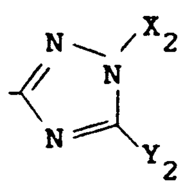 or 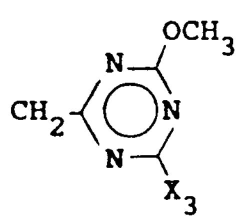 ;

A-4 A-5 A-6

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, F, Cl, Br, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, F, Cl, Br, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_1$–$C_4$ haloalkyl, azido, cyano,

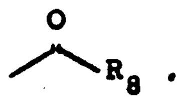

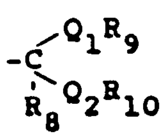 , 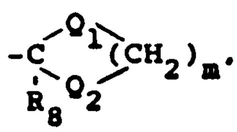 , 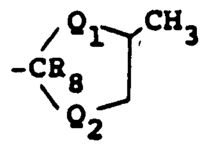 or

or $N(OCH_3)CH_3$;

m is 2 or 3;

$Q_1$ and $Q_2$ are independently O or S;

$R_8$ is H or $C_1$-$C_3$ alkyl;

$R_9$ and $R_{10}$ are independently $C_1$-$C_3$ alkyl;

Z is CH, N, $CCH_3$, $CC_2H_5$, CCl or CBr;

$Y_1$ is O or $CH_2$;

$X_1$ is $CH_3$, $OCH_3$, $OC_2H_5$ or $OCF_2H$;

$X_2$ is $CH_3$, $C_2H_5$ or $CH_2CF_3$;

$Y_2$ is $OCH_3$, $OC_2H_5$, $SCH_3$, $SC_2H_5$, $CH_3$ or $CH_2CH_3$;

$X_3$ is $CH_3$ or $OCH_3$;

$Y_3$ is H or $CH_3$;

$R_{11}$ is $C_1$-$C_{10}$ alkyl, $C_1$-$C_{10}$ alkoxyalkoxyalkyl, $C_2$-$C_{10}$ alkenyl, up to $C_{10}$ alkenylalkenyl, $C_2$-$C_{10}$ epoxyalkyl, $C_2$-$C_{10}$ alkynyl, up to $C_{10}$ alkynylalkynyl, up to $C_{10}$ alkynylalkenyl, $C_3$-$C_6$ cycloalkyl,$C_4$-$C_7$ cycloalkylalkyl or

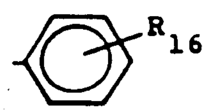 ;

when $R_{11}$ is $C_3$-$C_6$ cycloalkyl or $C_4$-$C_7$ cycloalkylalkyl it may optionally be substituted by $C_1$-$C_4$ alkyl, 1 to 3 atoms of Cl or F or 1 Br; when $R_{11}$ is $C_1$-$C_{10}$ alkyl, $C_2$-$C_{10}$ alkenyl or $C_2$-$C_{10}$ alkynyl it may optionally be substituted by one or more halogens and/or by $(R_{17})_{m'}$, where when m' is 2, the values of $R_{17}$ may be identical or different;

m' is 1 or 2;

$R_{12}$ is H or $C_1$-$C_4$ alkyl;

$R_{13}$ and $R_{14}$ are independently $C_1$-$C_4$ alkyl, $C_1$-$C_4$ alkoxy or $C_1$-$C_4$ alkylthio;

$R_{15}$ is $C_1$-$C_{10}$ alkyl, benzyl or

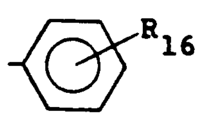 ;

$R_{16}$ is H, F, Cl, Br, $CH_3$, $OCH_3$, $NO_2$, CN, $SCH_3$, $SO_2CH_3$ or $CF_3$;

$R_{17}$ is $OR_{18}$, $OC(O)R_{18}$, $P^+R_9R_{10}R_{15}$, $P^+(C_6H_5)_3$, $OC(O)NR_{12}R_{18}$, $OSO_2R_{18}$, $OP(O)R_{13}R_{14}$, P(O)-$R_{13}R_{14}$ $OP(S)R_{13}R_{14}$, $P(S)R_{13}R_{14}$, $OSi(CH_3)_2R_{15}$, $Si(CH_3)_2R_{15}$, $SR_{18}$, $SOR_{18}$, $SO_2R_{18}$, SCN,

CN, $SP(O)R_{13}R_{14}$, $SP(S)R_{13}R_{14}$, $N^{+}R_{12}R_{15}R_{18}$, $NR_{12}R_{18}$, $NR_{12}C(O)R_{18}$, $NR_{12}C(O)OR_{18}$, $NR_{12}C(O)NR_{12}R_{18}$, $NR_{12}SO_2R_{18}$, $NR_{12}P(O)R_{13}R_{14}$, $NR_{12}P(S)R_{13}R_{14}$, $NO_2$, $C(O)R_{18}$, $C(O)OR_{18}$, $C(O)NR_{12}R_{18}$, $SeR_{18}$, naphthyl, L,

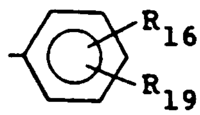
,
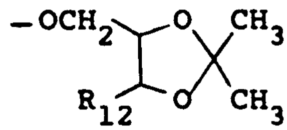
,

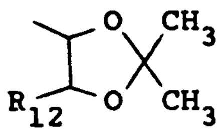
,
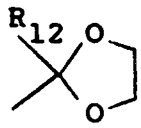
,
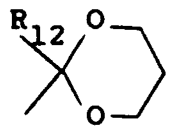

or
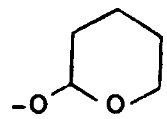

$R_{18}$ is H, $C_1$-$C_{10}$ alkyl, $C_1$-$C_{10}$ haloalkyl, $C_2$-$C_{10}$ alkenyl, $C_2$-$C_{10}$ alkynyl, $C_3$–$C_6$ cycloalkyl or

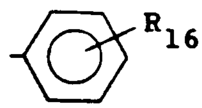
;

$R_{19}$ is H, F, Cl, Br, $CH_3$,

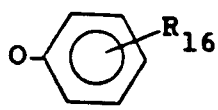

or

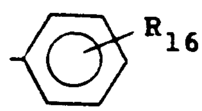
;

and

L is a 5- or 6-membered aromatic heterocycle, a 5- or 6-membered dihydroaromatic heterocycle or a 5- or 6-membered tetrahydroaromatic heterocycle which contains 1-4 heteroatoms selected from 0-1 oxygen atoms, 0-1 sulfur atoms, wherein sulfur may take the form of S, SO or $SO_2$, and/or 0-4 nitrogen atoms, with the proviso that oxygen and sulfur are only linked to each other if the sulfur is in the form of SO or $SO_2$, and these heterocycles may optionally be substituted by 1-4 $CH_3$, 1-2 $OCH_3$, $SCH_3$, Cl, $N(CH_3)_2$ or CN or L is a 5- or 6-membered lactone, lactam or cycloalkanone which may optionally be substituted by 1-4 $CH_3$ groups;

provided that

a) when W is S, then R is H, J is $J_1$, $J_2$, $J_3$ or $J_4$; A is A-1, Z is CH or N, and Y is $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CF_3$, $OCH_2CH_2OCH_3$, $CH(OCH_3)_2$ or

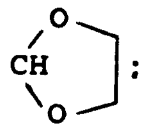

b) when X is F, Cl, Br, then Z is CH and Y is $OCH_3$, $OC_2H_5$, $NH_2$, $NHCH_3$, $N(CH_3)_2$ or $OCF_2H$;

c) when $R_3$ is $CH_3$, then n is O;

d) when J is J-1 or J-2 and $R_2$ is H or $C_1$-$C_4$ alkyl, then $R_1$ and $R_1'$ are other than H, F, Cl, Br, $CH_3$, $OCH_3$, $CF_3$, $OCF_2H$, or $SCH_3$ or X is other than $CH_3$, $OCH_3$, $OCH_2CH_3$, F, Cl, Br, $OCF_2H$, $CH_2Cl$, $CH_2Br$, $CH_2F$, cyclopropyl or $CF_3$ or Y is $C_3$-$C_4$ alkyl, $C_3$-$C_4$ alkoxy, $C_4$ haloalkoxy, $C_4$ haloalkylthio, $C_3$-$C_5$ alkoxyalkyl, $C_4$-$C_5$ alkoxyalkoxy, $C_2$-$C_3$ alkylamino, di($C_2$-$C_3$ alkyl)amino, $C_4$ alkenyloxy, $C_4$ alkynyloxy, $C_3$-$C_5$ alkylthioalkyl, $C_2$-$C_4$ haloalkyl, $C_2$-$C_4$ alkynyl, $C(O)R_8$ or $N(OCH_3)CH_3$;

e) the total number of carbon atoms in $R_2$ does not exceed 13;

f) when X is $C_3$-$C_5$ cycloalkyl, then Y is $CH_3$, $CH_2CH_3$, $OCH_3$, $OCH_2CH_3$, $CH_2OCH_3$, $OCF_2H$, $SCF_2H$, $OCH_2CF_3$, $CF_3$, $OCH_2CH=CH_2$, $OCH_2C{\equiv}CH$, $NHCH_3$, $N(CH_3)_2$ or $CH(OCH_3)_2$;

g) when $R_{18}$ is H, $R_{17}$ is other than $SOR_{18}$, $SO_2R_{18}$, $OSO_2R_{18}$ or $NR_{12}C(O)OR_{18}$;

h) when $R_1$ or $R_1'$ is para to the sulfonylurea bridge then $R_1$ or $R_1'$ are H, $CH_3$, F, Cl, Br or $OCH_3$; and

i) when X or Y is $OCH_2CH_2F$ or $OCH_2CHF_2$ then $R_2$ is other than H or $C_1$-$C_5$ alkyl, $CH_3OCH_2CH_2$, $C_2H_5OCH_2CH_2$ or $C_1$-$C_4$ alkyl substituted with 1-3 atoms of F, Cl or Br;

j) when X or Y is $OCH_2CH_2F$, $OCH_2CHF_2$ or $OCH_2CF_3$ then the other is not di($C_1$-$C_3$ alkyl)amino, $C_1$-$C_3$ alkylamino or $N(OCH_3)CH_3$;

k) when X or Y is $OCF_2H$, then Z is CH; and their agriculturally suitable salts.

Preferred for reasons of their higher herbicidal activity, greater plant growth regulant activity or more favorable ease of synthesis are:

1) compounds of Formula I wherein J is J-1 or J-4;

2) Compounds of Formula I wherein J is J-2 or J-3;

3) Compounds of Formula I wherein J is J-11 or J-12;

4) Compounds of Preferred 1 wherein W is O; R is H;

X is $CH_3$, $OCH_3$, $OCH_2CH_3$, Cl, F, Br, $OCF_2H$, $CH_2F$, $CF_3$ or cyclopropyl;

Y is H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C{\equiv}CH$, $OCH_2CF_3$, CN, $N_3$, $OCH_2CH_2OCH_3$, $CH_2SCH_3$,

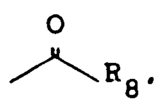

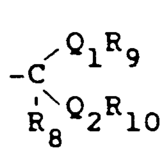
,
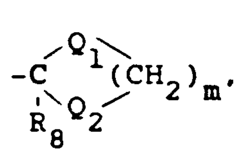

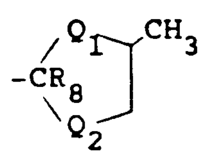

or $QCF_2T$;

Q is O or S;

T is H, CHClF, CHBrF or $CHFCF_3$;

5) Compounds of Preferred 4 wherein $R_1$ or $R_1'$ is H, Cl, $C_1$-$C_3$ alkyl, $C_1$-$C_3$ alkoxy, $C_1$-$C_3$ haloalkoxy, $C_1$-$C_3$ haloalkyl, $C_1$-$C_3$ alkylthio, $C_1$-$C_3$ haloalkylthio, amino, $C_1$-$C_3$ alkylsulfinyl, $C_1$-$C_3$ alkylsulfonyl, CN, $NH_2$, $C_1$-$C_3$ alkylamino, di($C_1$-$C_3$ alkylamino) or $C_1$-$C_2$ alkyl substituted with $C_1$-$C_2$ alkoxy, $C_1$-$C_2$ alkylthio, $C_1$-$C_2$ haloalkoxy, $C_1$-$C_2$ haloalkylthio, CN or $NO_2$;

6) Compounds of Preferred 5 wherein $R_2$ is $C_1$-$C_6$ alkyl, $C_1$-$C_6$ alkylcarbonyl, $C_1$-$C_6$ alkoxycarbonyl, $C_1$-$C_6$ alkyl substituted by 1-3 atoms of F, Cl or 1 Br, or by 1 or 2 groups selected from $C_1$-$C_2$ alkoxy, CN, $C_1$-$C_2$ alkoxycarbonyl, $C_1$-$C_2$ alkylcarbonyl, OH, $C_1$-$C_2$alkylthio, $C_1$-$C_2$ alkylsulfonyl, $C_1$-$C_2$ alkylsulfonyloxy or $C_1$-$C_2$ alkylcarbonyloxy, $C_3$-$C_4$ alkenyl, $C_3$-$C_4$ haloalkenyl, $C_3$-$C_4$ alkynyl, $C_4$ alkynyl substituted by 1 atom of F or Cl, phenyl or phenyl substituted with Cl, $CF_3$, $NO_2$, CN or $SO_2CH_3$; and $R_4$ is $CH_3$;

7) Compounds of Preferred 6 wherein A is A-1 and Y is $CH_3$, $C_2H_5$, $OCH_3$, $CH_2OCH_3$, $OCF_2H$ or $CH(OCH_3)_2$;

8) Compounds of Preferred 7 wherein $R_1$ and $R_1'$ are H, Cl, $C_1$-$C_2$ alkyl, $C_1$-$C_2$ alkoxy or $C_1$-$C_2$ alkylthio;

9) Compounds of Preferred 8 wherein J is J-1;

10) Compounds of Preferred 9 wherein $R_1$ is H and Z is CH;

11) Compounds of Preferred 10 wherein $R_2$ is $C_1$-$C_4$ haloalkyl, $C_1$-$C_3$ alkyl substituted with $C_1$-$C_2$ alkoxy, $C_1$-$C_2$ alkylthio, $C_1$-$C_2$ alkylsulfonyl or CN, $C_3$-$C_4$ alkenyl, $C_3$-$C_4$ haloalkenyl, $C_3$-$C_4$ alkynyl, $C_1$-$C_3$ alkoxycarbonyl or $C_1$-$C_3$ alkylcarbonyl;

12) Compounds of Preferred 9 wherein $R_1$ is H; Z is N; and $R_2$ is $C_1$-$C_3$ alkylcarbonyl or $C_1$-$C_3$ alkoxycarbonyl.

13) Compounds of Preferred 2 wherein W is O; $R_1$ is H; X is $CH_3$, $OCH_3$, $OCH_2CH_3$, Cl, F, Br, $OCF_2H$, $CH_2F$, $CF_3$ or cyclopropyl; Y is H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C{\equiv}CH$, $OCH_2CF_3$, CN,

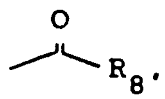

$N_3$, $OCH_2CH_2OCH_3$, $CH_2SCH_3$,

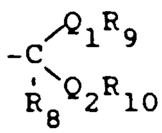 , 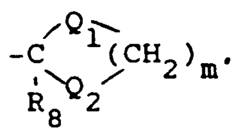 , 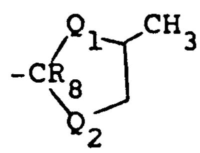

or $QCF_2T$; $R_2$ is $C_1$-$C_6$ alkyl, $C_1$-$C_6$ alkylcarbonyl, $C_1$-$C_6$ alkoxycarbonyl, $C_1$-$C_6$ alkyl substituted by 1-3 atoms of F, Cl or 1 Br, or by 1 or 2 groups selected from $C_1$-$C_2$ alkoxy, CN, $C_1$-$C_2$ alkoxycarbonyl, $C_1$-$C_2$ alkylcarbonyl, OH, $C_1$-$C_2$ alkylthio $C_1$-$C_2$ alkylsulfonyl, $C_1$-$C_2$ alkylsulfonyloxy or $C_1$-$C_2$ alkylcarbonyloxy, $C_3$-$C_4$ alkenyl, $C_3$-$C_4$ haloalkenyl, $C_3$-$C_4$ alkynyl, $C_4$ alkynyl substituted by 1 atom of F or Cl, phenyl or phenyl substituted with Cl, $CF_3$, $NO_2$, CN or $SO_2CH_3$; $R_1$' is H, Cl, $C_1$-$C_2$ alkyl, $C_1$-$C_2$ alkoxy or $C_1$-$C_2$ alkylthio;

14) Compounds of Preferred 13 wherein A is A-1; and Y is $CH_3$, $C_2H_5$, $OCH_3$ , $CH_2OCH_3$, $OCF_2H$ or $CH(OCH_3)_2$;

15) Compounds of Preferred 3 wherein W is O, $R_1$ is H, $R_1$' is H, A is A-1, X is $CH_3$, $OCH_3$ , $OCH_2CH_3$, Cl, F, Br, $OCF_2H$, $CH_2F$ or cyclopropyl; and Y is $CH_3$, $C_2H_5$, $OCH_3$ , $CH_2OCH_3$, $OCF_2H$ or $CH(OCH_3)_2$.

Other compounds of interest are those disclosed and claimed in our U.S. Patent Applications Serial Nos. 607,989; 663,555; and 716,351. Copies of these U.S. Patent Applications are laid open on the file of the present Application.

Specifically preferred for reasons of their highest herbicidal activity, greatest plant growth regulant activity and/or most favorable ease of synthesis are:

- 2,3-dihydro-N-[(4,6-dimethoxypyrimidin-2-yl)aminocarbonyl]-2-(2-methoxyethyl)-1,2-benzisothiazole-7-sulfonamide, 1,1-dioxide, m.p. 194-197° C;
- 2,3-dihydro-N-[(4,6-dimethoxypyrimidin-2-yl)aminocarbonyl]-2-(3-methylbutyl)-1,2-benzisothiazole-7-sulfonamide, 1,1-dioxide, m.p. 227-229° C;
- 2,3-dihydro-N-[(4,6-dimethoxypyrimidin-2-yl)aminocarbonyl]-2-(phenylmethyl)-1,2-benzisothiazole-7-sulfonamide, 1,1-dioxide, m.p. 216-218° C;
- 2,3-dihydro-N-[(4,6-dimethoxypyrimidin-2-yl)aminocarbonyl]-2-(2-oxopropyl)-1,2-benzisothiazole-7-sulfonamide, 1,1-dioxide, m.p. 178-182° C;
- 2,3-dihydro-N-[(4-chloro-6-methoxypyrimidin-2-yl)-aminocarbonyl]-2-(2-ethoxyethyl)-1,2-benzisothiazole-7-sulfonamide, 1,1-dioxide, m.p. 193-197° C;
- 2,3-dihydro-N-[(4,6-dimethoxypyrimidin-2-yl)aminocarbonyl]-2-(2-ethoxyethyl)-1,2-benzisothiazole-7-sulfonamide, 1-1-dioxide, m.p. 203-204° C;
- 2,3-dihydro-N-[(4,6-dimethylpyrimidin-2-yl)aminocarbonyl]-2-(2-fluoroethyl)-1,2-benzisothiazole-7-sulfonamide, 1,1-dioxide, m.p. 214-215° C;
- 2,3-dihydro-N-[(4-methoxy-6-methyl-1,3,5-triazin-2-yl)aminocarbonyl]-2-(2-fluoroethyl)-1,2-benzisothiazole-7-sulfonamide, 1,1-dioxide, m.p. 190-192°C;

- 2,3-dihydro-N-[(4,6-dimethoxypyrimidin-2-yl)aminocarbonyl]-2-acetyl-1,2-benzoisothiazole-7-sulfonamide, 1,1-dioxide, m.p. 191-193°C;
- 2,3-dihydro-N-[(4,6-dimethoxypyrimidin-2-yl)aminocarbonyl]-2-(3-chloropropyl)-1,2-benzisothiazole-7-sulfonamide, 1,1-dioxide, m.p. 201-203°C;
- 2,3-dihydro-N-[(chloro-6-methoxypyrimidin-2-yl)-aminocarbonyl]-2-(2-fluoroethyl)-1,2-benzisothiazole-7-sulfonamide, 1,1-dioxide, m.p. 215-217°C;
- 2,3-dihydro-N-[(4,6-dimethoxypyrimidin-2-yl)aminocarbonyl]-2-(3-fluoropropyl)-1,2-benzisothiazole-7-sulfonamide, 1,1-dioxide, m.p. 199-200°C;
- 2,3-dihydro-N-[(4-chloro-6-methoxypyrimidin-2-yl)-aminocarbonyl]-2-(2-chloroethyl)-1,2-benzisothiazole-7-sulfonamide, 1,1-dioxide, m.p. 219-220°C;
- N-[(4-chloro-6-methoxypyrimidin-2-yl)aminocarbonyl]-2-(2-fluoroethyl)-2,3-dihydro-1,2-benzoisothiazole-7-sulfonamide, 1,1-dioxide, m.p. 215-217°C; and
- N-[(4-chloro-6-methoxypyrimidin-2-yl)aminocarbonyl]-2-(3-fluoropropyl)-2,3-dihydro-1,2-benzoisothiazole-7-sulfonamide, 1,1-dioxide, m.p. 222.5-223.5°C.

## DETAILED DESCRIPTION OF THE INVENTION

### Synthesis

The following discussion represents a general outline for the preparation of the compounds of this invention. All of the syntheses described below are multistep with one or more methods being taught for each step. This allows for a wide variety of possible synthetic pathways to prepare a particular compound of Formula I. The proper choice of the synthetic pathway and the best ordering of the reaction sequences for each individual compound will be known to one skilled in the art.

The compounds of Formula I can be prepared by one or more of the methods described below in Equations 1 through 5. Reaction conditions are given by way of example only.

As shown in Equation 1, many of the compounds of Formula I where J is $J_1$, $J_2$, $J_3$ and $J_4$, can be prepared by reacting a sulfonylisocyanate (W = 0) or, a sulfonylisothiocyanate (W = S) of Formula II with an appropriate heterocyclic amine of Formula III. R, A and W are as previously defined.

Equation 1

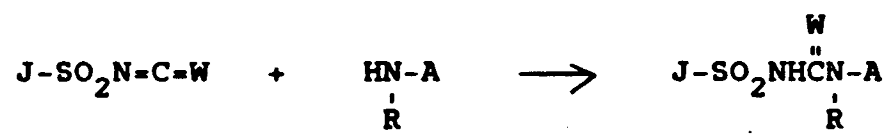

II III I

The reaction is carried out at 25° to 100°C in an inert, aprotic solvent such as methylene chloride or xylene for 0.5 to 24 hours as taught in U.S. Patent 4,127,405.

Many of the compounds of Formula I, where J is $J_1$, $J_2$, $J_3$ and $J_4$, W is S and R is H, (Ia) can be prepared by reacting the appropriate sulfonamide of Formula IV with a heterocyclic isothiocyanate of Formula V, as shown in Equation 2.

Equation 2

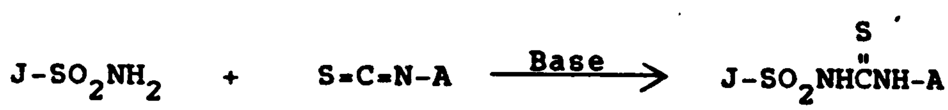


IV V Ia

The reaction is carried out at 25° to 80°C in an inert, aprotic solvent such as acetone or acetonitrile in the presence of a base such as potassium carbonate for 0.5 to 24 hours. The required heterocyclic isothiocyanates V are prepared from the corresponding amines III which would be known to one skilled in the art as taught in EPO Publication 35,893.

Many of the compounds of Formula I, where J is $J_1$, $J_2$, $J_3$ and $J_4$ and W is O (Ib), can be prepared by reacting a sulfonylcarbamate of Formula VI with an appropriate amine of Formula III, as shown in Equation 3.

Equation 3

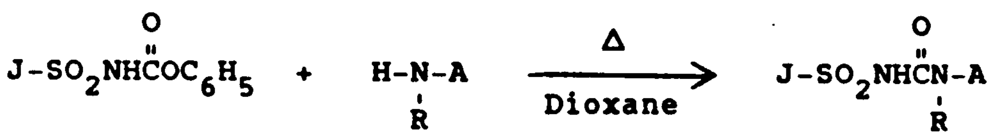


VI III Ib

The reaction is carried out at 50° to 100°C in a solvent such as dioxane for 0.5 to 24 hours. The required carbamates VII are prepared by reacting the corresponding sulfonamides IV with diphenylcarbonate in the presence of a strong base.

Compounds of Formula Ib can also be prepared, as shown in Equation 4, by reacting a heterocyclic carbamate of Formula VII with an appropriate sulfonamide of Formula IV.

Equation 4

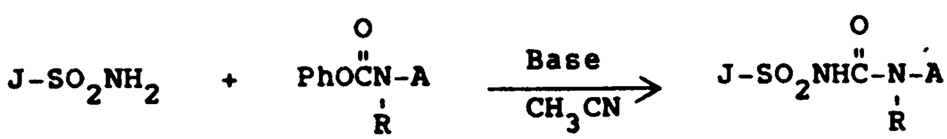


IV VII Ib

The reaction is carried out at 0° to 50°C in a solvent such as acetonitrile or dioxane in the presence of a non-nucleophilic base such as DBU for 0.2 to 24 hours. The required phenylcarbamate VII are prepared by reacting the corresponding heterocyclic amines III with diphenylcarbonate or phenylchloroformate in the presence of a strong base.

Many of the compounds of Formula Ib where J is $J_1$, $J_2$, $J_3$ and $J_4$ can be prepared by reacting the sulfonamides of Formula IV with an appropriate methylcarbamate of Formula VIII in the presence of an equimolar amount of trimethylaluminum, as shown in Equation 5.

Equation 5

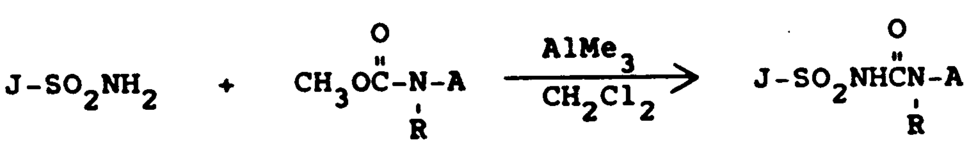


IV VIII Ib

The reaction is carried out at 25° to 40°C in a solvent such as methylene chloride for 10 to 96 hours under on inert atmosphere. The required carbamates VIII are prepared by reacting the corresponding amines III with dimethylcarbonate or methyl chloroformate in the presence of a strong base.

The intermediate sulfonylisocyanates (W = 0) and sulfonylisothiocyanates (W = S) of Formula II from Equation 1 can be prepared as shown in Equations 6 through 8.

As shown in Equation 6, many of the sulfonylisocyanates of Formula IIa where J is $J_1$, $J_2$, $J_3$ and $J_4$ can be prepared by the reaction of sulfonamides of Formula IV with phosgene, in the presence of n-butylisocyanate and a tertiary amine catalyst, at reflux in a solvent such as xylene by the method of U.S. Patent 4,238,621.

Equation 6

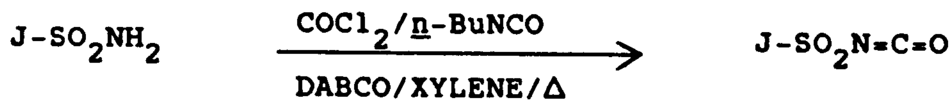


IV IIa

The sulfonylisocyanates can also be prepared from the sulfonamides by a two step procedure involving (a) reacting the sulfonamides with n-butylisocyanate in the presence of a base such as $K_2CO_3$ at reflux in an inert solvent such as 2-butanone forming a n-butylsulfonylurea; and (b) reacting this compound with phosgene and a tertiary amine catalyst at reflux in xylene solvent. The method is similar to a procedure taught by Ulrich and Sayigh, Newer Methods of Preparative Organic Chemistry, Vol. VI. p. 223-241, Academic Press, New York and London, W. Foerst Ed.

Alternatively, as shown in Equation 7, many of the sulfonylisocyanates of Formula IIa where J is $J_1$, $J_2$, $J_3$ and $J_4$ can be prepared by reacting the corresponding sulfonyl chlorides IX with cyanic acid salts.

Equation 7

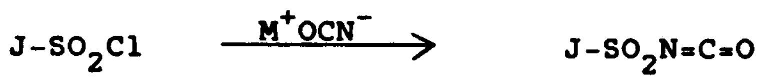


IX IIa

The reaction is carried out at 25° to 100°C in an inert aprotic solvent such as acetonitrile for 0.5-24 hours in the presence of phosphorus pentoxide and an alkali metal salt such as lithium iodide.

Many of the sulfonylisothiocyanates of Formula IIb where J is $J_1$, $J_2$, $J_3$ and $J_4$ can be prepared, as shown in Equation 8, by contacting the sulfonamides of Formula IV with carbon disulfide in the presence of two equivalents of a strong base. The resulting salt is then reacted with phosgene according to the teachings of K. Hartke, Arch. Pharm., 299, 174 (1966).

Equation 8

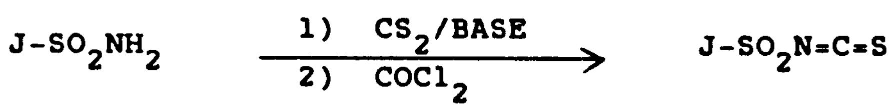


IV IIb

The sulfonamides of Formula IV where J is $J_1$, $J_2$, $J_3$ and $J_4$ of Equations 2, 4, 5, 6 and 8 as well as the other sulfonamides required to prepare the compounds of this invention can be prepared from the corresponding sulfonyl chlorides of Formula IX by contacting with either anhydrous or aqueous ammonia as shown in Equation 9.

Equation 9

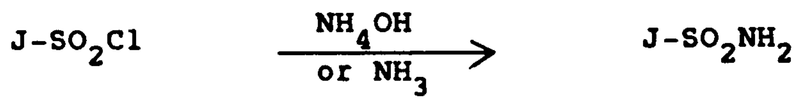


IX IV

The preparation of sulfonamides from sulfonyl chlorides is widely reported in the literature, for reviews see: F. Hawking and J. S. Lawrence, "The Sulfonamides," H. K. Lewis and Co., London, 1950 and E. H. Northey, "The Sulfonamides and Allied compounds," Reinhold Publishing Corp., New York, 1948.

Alternatively, many sulfonamides IV can be prepared by dealkylation of their corresponding N-t-butyl sulfonamides X as shown in Equation 10.

Equation 10

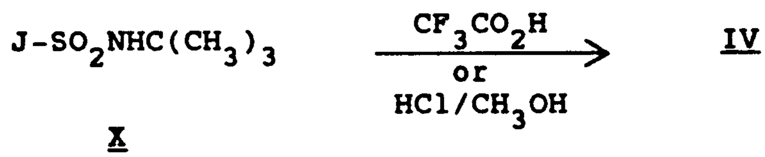


X

The reaction is carried out by contacting the N-t-butyl sulfonamide X with a strong acid such as trifluoroacetic acid or methanolic HCl at 25° to 50°C for 0.5 to 24 hours. The N-t-butyl sulfonamides X are readily prepared by reacting sulfonylchlorides IX with t-butylamine and are useful either as an aid in purification, to enhance solubility for subsequent reactions such as Equation 12 below or to protect the sulfonamide function from competing with reactions at other parts of the molecule.

Many of the sulfonamides of Formula IVa can be prepared by functionalization of the corresponding N-unsubstituted sulfonamides of Formula IVb as shown in Equation 11. $G_1$-$G_2$ is $CHR_3(CHR_4)_n$ and $CH=CR_4$, R' is H or $C(CH_3)_3$ and X is Cl, Br, I or other readily displaceable groups. When $R_3$ is $CH_3$ then n is 0.

Equation 11

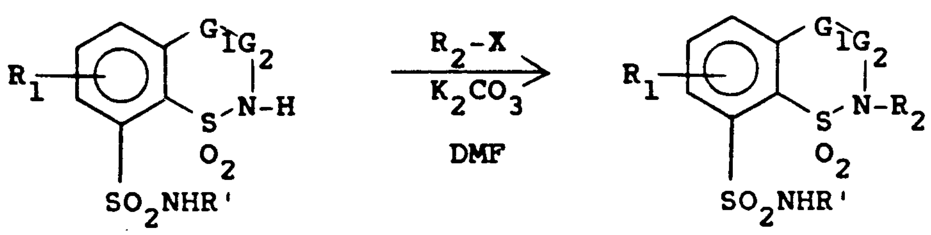


IVb IVa

The reaction is carried out by contacting the sulfonamides IVb with the appropriate electrophile in the presence of a suitable base such as $K_2CO_3$ in an inert, polar solvent such as DMF at 0° to 100°C for 0.5 to 24 hours. In some instances the $R_2$ function can also be introduced by Michael addition of IVb to the appropriate Michael acceptor as known to one skilled in the art.

While many of the $R_2$ groups can be introduced directly, as described above, some of the $R_2$ groups may best be prepared by standard functional group manipulations upon compounds of Formula IVa containing an appropriate $R_2$ group pecursor as will be known to one skilled in the art. Some examples of these manipulations are the preparation of IVa where $R_2$ contains an epoxide by the epoxidation of IVa where $R_2$ contains a carbon-carbon double bond, the preparation of IVa where $R_2$ contains a sulfone by the oxidation of IVa where $R_2$ contains a thioether function, the preparation of IVa where $R_2$ contains $OC(O)CH_3$

by acetylation of IVa where $R_2$ contains OH, or the preparation of IVa where $R_2$ contains $NH_2$ by the reduction of IVa where $R_2$ contains $NO_2$. This is also the case for many of the $R_1$ substituents which can be prepared by an analogous chemical manipulation known to one skilled in the art.

Many of the unsaturated sulfonamides of Formula IVc can also be prepared from the corresponding saturated sulfonamides of Formula IVd by the two-step procedure shown in Equation 12 . $G_1$-$G_2$ is $SO_2$-$NR_2$, CO-$NR_5$ or $NR_5$-CO, R' is H or $C(CH_3)_3$ and R" is H or $R_4$.

Equation 12

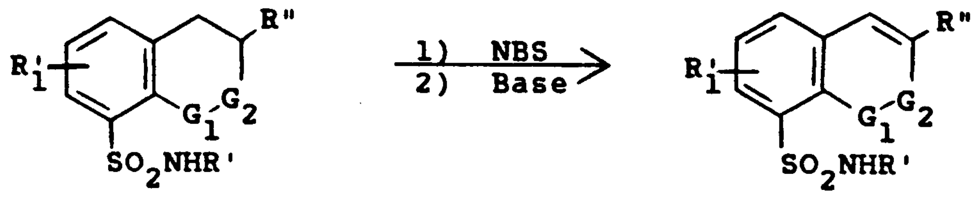


IVd IVc

The first step involves benzylic bromination by N-bromosuccinimide to give a monobromide which is subsequently dehydrobrominated in a second step by reaction with a suitable base such as triethyl amine or potassium-t-butoxide in an inert solvent such as THF. This method has been used to prepare isocoumarins from 3,4-dihydroisocoumarins, see R. Barry, Chem. Rev., 64, 229 (1964). In cases where $R_1$ is an alkyl group, competitive bromination at this site may occur resulting in a mixture. The desired bromide may be separated at this stage, or after treatment with the base, by standard methods.

EP-A-107979 describes the synthesis of indanone and tetralone sulfamides which can be ketalized by standard methods known to one skilled in the art to give sulfonamides of Formula IV where J is J-11 and J-12.

The sulfonyl chlorides of Formula IX of Equations 7 and 9 are important intermediates for the preparation of the compounds of this invention. The syntheses of the required sulfonyl chloride intermediates where J is $J_1$, $J_2$, $J_3$ and $J_4$ are described in Equations 14 through 16.

As shown in Equation 14, many of the sulfonyl chlorides of Formula IX can be prepared from the corresponding amines XI.

Equation 14

$$J\text{-}NH_2 \xrightarrow[2)\ SO_2/CuCl_2/HOAc]{1)\ HONO/HCl} J\text{-}SO_2Cl$$

XI IX

The reaction involves diazotization of the amine XI with sodium nitrite in HCl, followed by reaction of the diazonium salt with sulfur dioxide and cupric chloride in acetic acid analogous to the teachings of Yale and Sowinski, J. Org. Chem., 25, 1824 (1960).

Alternatively, sulfonyl chlorides of Formula IX can be prepared by a modification of the above procedure whereby the diazotization reaction is carried out in dilute sulfuric acid and the resulting diazonium salt is reacted with sulfur dioxide, HCl and cupric chloride in a cosolvent mixture consisting of acetic acid-water (1:1) and an immiscible, inert solvent such as 1-chlorobutane or methylene chloride at 0°-40°C for 1 to 24 hours.

Many of the sulfonyl chlorides of Formula IX can also be prepared by oxidative chlorination of the corresponding thio compounds of Formula XII as shown in Equation 15. R' is H, alkyl, benzyl or carbamoyl, $R_1$ is not $SCH_3$ and J does not contain non-aromatic unsaturation.

Equation 15

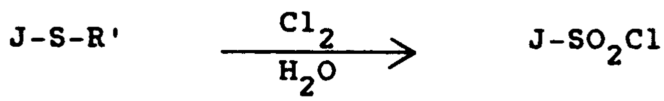


XII IX

The reaction is carried out by addition of molecular chlorine or a chlorine equivalent to the thio compound in the presence of water at 0° to 80°C in an aliphatic carboxylic acid solvent such as acetic acid or an inert organic solvent such as dichloroethane for 1 to 24 hours.

Alternatively, many of the sulfonyl chlorides of Formula IX can be prepared by the two-step sequence shown in Equation 16 starting from the thio compounds XII where R' is H (XIIa) and $R_1$ is not $SCH_3$.

Equation 16

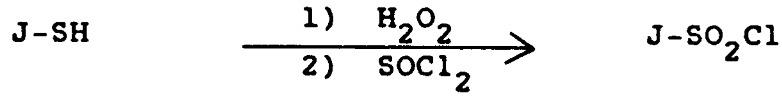


XIIa IX

The thiol XII (R' = H) is contacted with excess hydrogen peroxide in the presence of base to give a sulfonic acid salt which in turn is converted to the desired sulfonyl chloride by contacting with a suitable reagent such as thionyl chloride or phosphorous pentachloride as known to one skilled in the art.

Some of the sulfonyl chlorides of Formula IX may best be prepared by direct chlorosulfonation depending on the substitution pattern on the ring and the nature of the substituent as will be known to one skilled in the art.

Many of the S-arylthiocarbamates of Formula XIIb (XII, R' = $CON(CH_3)_2$) can be prepared by the Newman-Kwart rearrangement starting with the corresponding phenols XIII as shown in Equation 17.

Equation 17

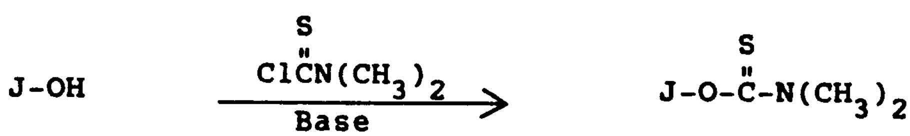


XIII XIV

XIV

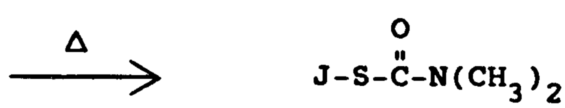


XIIb

The phenol XIII is first reacted with N,N-dimethylthiocarbamoyl chloride in the presence of a base. The resulting O-aryl-N,N-dimethylthiocarbamate XIV is then heated at 150°C to 300°C for 2 to 24 hours as taught by Newman and Karnes J. Org. Chem., 31, 3980 (1966) to give the desired S-aryl-N,N-dimethylthiocarbamate XIIb. The related thiols XIIa can be obtained by hydrolysis of the thiocarbamates XIIb.

Many of the sulfides of Formula XII where R' is alkyl or benzyl can be prepared by reacting a halocompound of Formula XV with an appropriate mercaptan in the presence of a base as shown in Equation 18. R' is alkyl or benzyl and X is F, Cl or Br.

Equation 18

$$\underset{\underline{XV}}{J\text{-}X} \xrightarrow[\text{Base}]{R'\text{-}SH} \underset{\underline{XII}}{J\text{-}S\text{-}R'}$$

The reaction is carried out in a solvent such as DMF at 25° to 150°C for 0.5 to 24 hours. The halocompounds XV must not contain functionality which can be attacked by a mercaptide anion as will be known to one skilled in the art. An example of this would be where J is $J_1$ and $R_2$ was a haloalkyl group. These compounds can be prepared from the corresponding sulfides XII where J is $J_1$, R' is alkyl or benzyl and $R_2$ is H by standard alkylation methods.

Many of the thiocompounds XII of Equation 15 and the chlorocompounds XV (X = Cl) of Equation 18, where J is $J_1$ and n is O, can be prepared by the reaction sequence shown in Equation 19. G is Cl or S-alkyl, $R_1$ does not contain functionality incompatible with BuLi or $BH_3$ and $R_3$ is as previously defined.

Equation 19

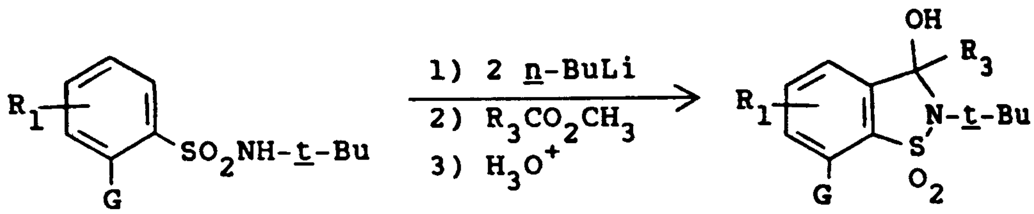


XVI XVII

XVII

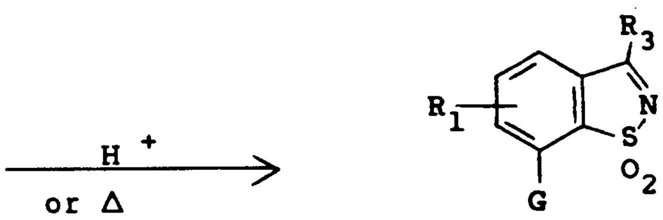


XVIII

XVIII

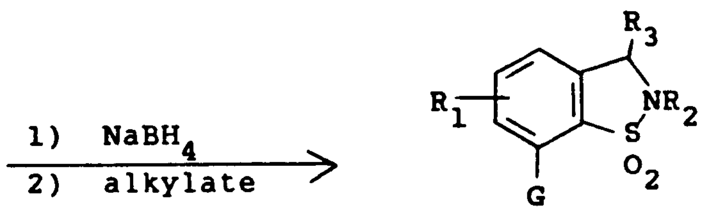


XII ($J=J_1$, R'=alkyl)
XV ($J=J_1$, X=Cl)

XVII

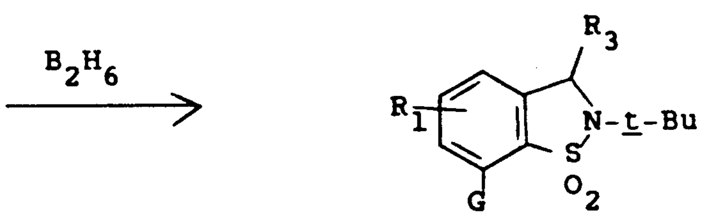


XVIIIa

XXIIIa

1) $H^+$
2) alkylate

XII ($J=J_1$, R'=alkyl)
XV ($J=J_1$, X=Cl)

The sequence begins by contacting an appropriately substituted N-t-butylbenzenesulfonamide XVI with two equivalents of butyl lithium at 0° to 25°C in an inert solvent such as THF for 2 to 10 hours to give a dianion according to the teachings of J. Lombardino, J. Org. Chem., 36, 1843 (1971). The dianion can then be trapped with an ester or, when $R_3$ is H, with dimethylformamide at -78° to 25°C to produce, upon aqueous acid workup, the hemiaminal XVII. The hemiaminal XVII can be de-t-butylated and dehydrated by a catalytic amount of acid such as p-toluene-sulfonic acid in a solvent such as benzene or toluene at reflux. The resulting benzisothiazole XVIII can be reduced with a reagent such as sodium borohydride in a suitable solvent such as ethanol to produce a 2,3-dihydrobenzisothiazole which is N-alkylated as described in

equation II to give either XII ($J = J_1$, R' = alkyl) or XV ($J = J_1$, X = Cl) by standard methods known to one skilled in the art. Alternatively, hemiaminal XVII may be treated with diborane to produce a t-butyl-2,3-dihydrobenzisothiazole XVIIIa. Compounds XII ($J = J_1$, R' = alkyl) or XV ($J = J_1$, X = Cl) are then readily prepared as described previously by treatment of XVIIIa with trifluoroacetic acid followed by N-alkylation. If the t-butyl group in structures XVI and XVII is replaced by the appropriate $R_2$ group, the diborane reduction of the hemiaminal gives XII and XV directly.

Many of the thio compounds XII of Equation 15 and the chloro compounds XV of Equation 18, where J is $J_1$ and $J_4$ and n = 1, can be prepared by the reaction sequence shown in Equation 20. G is Cl or S-alkyl, Y is H or $CH_3$, $R_1$ does not contain functionality incompatible with BuLi. R" is $C(CH_3)_3$ or functionality selected from $R_2$ which is compatible with the lithiation conditions shown as will be known to one skilled in the art and $R_4$ is H or $C_1$-$C_4$ alkyl.

Equation 20

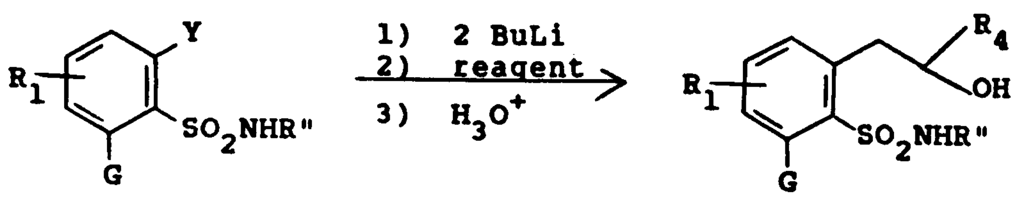


XIX

XX

XX

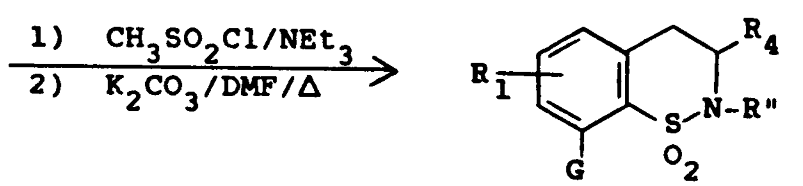


XII ($J = J_1, J_4$, R' = alkyl)
XV ($J = J_1, J_4$, X = Cl)

The reaction is carried out by reacting the N-substituted benzenesulfonamides XIX with two equivalents of n-BuLi at -780 to 250C in a solvent such as THF for 0.5 to 5 hours. When Y is H, an ortho-anion is formed which is trapped with an appropriate epoxide to give the hydroxy sulfonamides XX. When Y is CH , an ortho-methyl anion is produced which is reacted with an aldehyde or DMF to give alcohol XX. The alcohol can be mesylated by reaction of XX with mesyl chloride in the presence of an equivalent of a tertiary amine at 00 to 250C for 1 to 24 hours in a solvent such as methylene chloride. The resulting mesylate is cyclized by heating with a base such as potassium carbonate in a solvent such as DMF to produce the desired compounds XII (J = J±,JΔ, R' = alkyl) or XV (J = J±, JΔ, X = Cl). When R" is C(CH ) , contacting with acid as described in Equation 10 will give the corresponding benzisothiazine where R" is H. This can be alkylated as described previously for the benzisothiazoles of Equation 19.

The amines of Formula XI in Equation 14, can be prepared by reduction of the corresponding nitro compounds of Formula XXI, as shown in Equation 22.

Equation 22

$$J\text{-}NO_2 \xrightarrow{\text{Reduction}} J\text{-}NH_2$$

XXIII XI

The reduction of nitro compounds to amines can be carried out by any of several known methods as described in Preparative Organic Chemistry, 4 Ed., p. 557-563, John Wiley and Sons, New York and London, G. Hilgetag and A. Martini Ed.

Many of the nitro compounds of Formula XXIII in Equation 22 can be prepared by the procedures outlined in Equations 23 through 30. With suitable modifications known to one skilled in the art, the general ring forming reactions outlined below for these nitro compounds XXIII can be adapted to prepare many of the phenols of Formula XIII and halocompounds of Formula XV.

As shown in Equation 23, many of the nitro compounds of Formula XXI, where J is $J_1$ (XXIIIa) can be prepared starting from the appropriately substituted nitrobenzenes of Formula XXII. R' is H or $CH_2Cl$.

Equation 23

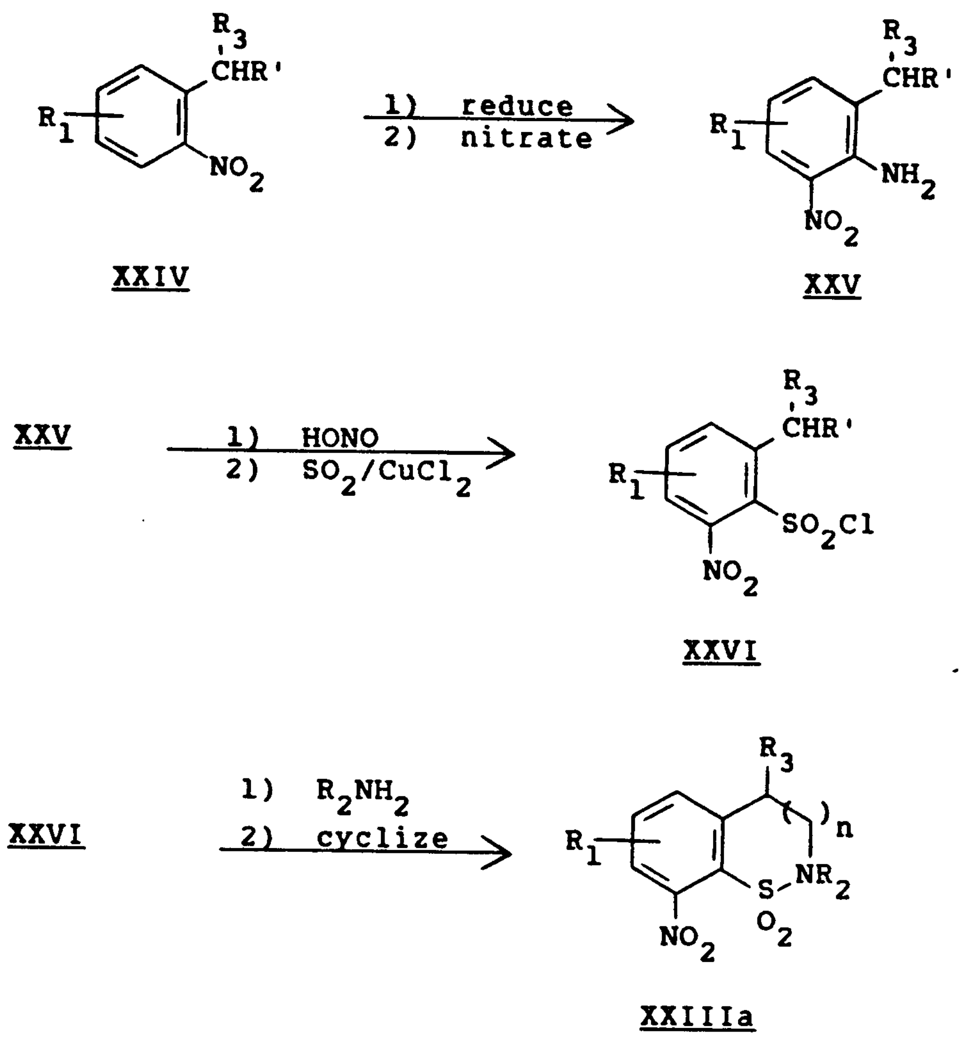


XXIV XXV

XXV XXVI

XXVI XXIIIa

The nitrobenzenes XXIV are first reduced to their corresponding amines and nitrated by standard methods to produce, in part, compounds of Formula XXV. In some instances, it may be desirable to first protect the amino group as its acetate prior to nitration as is known to one skilled in the art. The desired nitro compound XXV can be isolated by either fractional crystallization or chromatographic procedures and converted to sulfonyl chlorides XXVI by the method previously discussed in Equation 14. The intermediate sulfonyl chlorides XXVI can be converted into their corresponding sulfonamides by reaction with an appropriate amine (see Equation 9) and subsequently cyclized: a) when R' is $CH_2Cl$, to the nitro-1,2-benzothiazines XXIIIa, where n is 1, by heating in the presence of a base such as potassium carbonate; or b) when R' is H, by contacting the sulfonamide with NBS in a solvent such as carbon tetrachloride to give sulfonamides where R' is Br, followed by contacting this product with a base to give the nitro benzisothiazoles XXIa where n is 0. Note the discussion of Equation 12 for NBS brominations when $R_1$ is alkyl.

The procedure of Equation 23 is similar to the method taught by E. Sianesi et al., Chem. Ber., 104, 1880 (1971) for the preparation of substituted 1,2-benzothiazine-1,1-dioxides. The starting nitrobenzenes XXII can be prepared by standard methods known to one skilled in the art.

Alternatively, as shown in Equation 24, many of the nitro compounds of Formula XXIIIa where n is 1 can be prepared, in part, by contacting the nitro acetamides XXIII with fuming sulfuric acid according to the method taught by H. Zenno and T. Mizutani, (Chem. Abst.: 72:79122 (1970)) for the preparation of 7-nitro-1,2-benzothiazine-1,1-dioxide. The resulting benzothiazines can be isolated and subsequently alkylated by standard methods known to one skilled in the art.

Equation 24

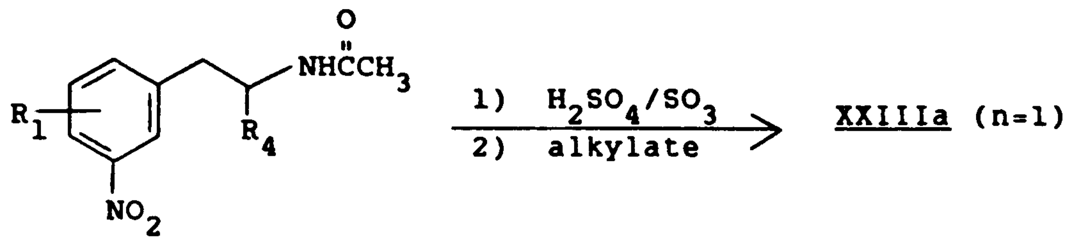


XXVII

Many of the nitro compounds of Formula XXIIIa where n is 0 can also be prepared from the corresponding 1,2-benzisothiazoles XXVI as shown in Equation 25.

Equation 25

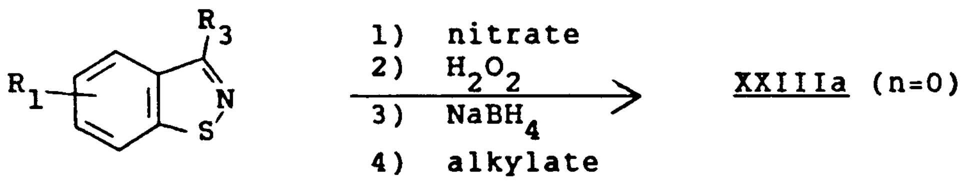


XXVIII

Nitration of XXVIII gives, in part, the 7-nitro derivative which is isolated by standard methods. The derivative is converted to its 1,1-dioxide by treatment with hydrogen peroxide. Reduction of the carbon-nitrogen double bond with $NaBH_4$ followed by alkylation of the resulting sulfonamide gives compounds XXIIIa where n is 0. The above reactions are characteristic of 1,2-benzisothiazoles XXVIII. For reviews of their synthesis and reactions, see L. L. Bambas, "The Chemistry of Heterocyclic Compounds," Vol. 4, part III, 1952, p. 223-378, and M. Davis. Adv. Heterocyclic Chem., Vol. 14 (1972) p. 43-98. 1,2-Benzothiazines are also well known in the literature, for a review of their chemistry and alternate methods of their preparation see J. G. Lombardino, D. E. Kuhla, Adv. Heterocyclic Chem., Vol. 28 (1981) p. 73-126.

Many of the nitro compounds of Formula XXIII, where n = 0 can also be prepared by reduction of the corresponding 3-nitrophthalic anhydrides with either sodium borohydride or lithium aluminum hydride in tetrahydrofuran as taught by M. Kayser and P. Morand, Can. J. Chem., 58, 2848 (1980) for the preparation of 7-nitrophthalide (XXIIId; n = 0, $R_1'$ = H). Phthalides and phthalic anhydrides are well known in the art, for a review of their synthesis and reactions see, S. Wawzonek, Heterocyclic Compounds, Vol. 2, John Wiley and Sons, Inc., New York, 1951.

Many of the nitro compounds of Formula XXIII in Equation 22 where J is $J_2$ or $J_3$ can be prepared from the corresponding nitro compounds of Formula XXIII where J is $J_1$ (n = 1) or $J_4$ respectively, by the bromination-dehydrobromination sequence described above for sulfonamides IV in Equation 12.

The amines of Formula III in Equations 1 and 3 are also important intermediates for the preparation of the compounds of this invention and are described below.

The pyrimidines and triazines of Formula (IIIa) to (IIId) below are either known or can be prepared by

methods obvious to one skilled in the art.

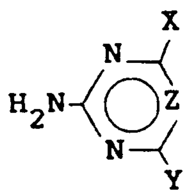

IIIa

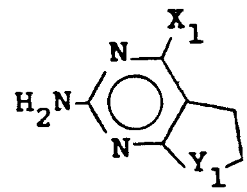

IIIb

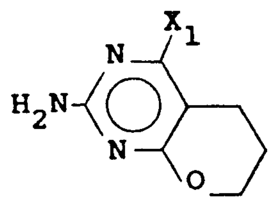

IIIc

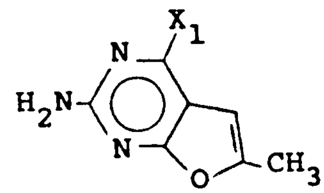

IIId

For a review of the synthesis and reactions of 2-aminopyrimidines (IIIa, Z = CR') see The Chemistry of Heterocyclic Compounds, Vol. 16, John Wiley and Sons, New York (1962). For a review of the synthesis and reactions of 2-amino-s-triazines (IIIa, Z = N) see The Chemistry of Heterocyclic Compounds, Vol. 13, John Wiley, New York (1959), F. C. Schaefer, U.S. Patent 3,154,547 and F. C. Schaefer and K. R. Huffman, J. Org. Chem., 28, 1812 (1963). The synthesis of the bicyclic amines IIIb and IIIc is taught in European Patent Application 15,683. The synthesis of bicyclic amines IIId is taught in European Patent Application 46,677. The synthesis of amines IIIa where X is cyclopropyl is taught in South African Patent Application 83/7434.

The amines of Formula III where X is $OCF_2H$, $OCH_2F$, $OCF_3$ or $CF_3$; or $X_1$ is $OCF_2H$ and/or Y is $OCH_2F$, $OCF_3$, $SCH_2F$, $SCF_3$ or $GCF_2T$ wherein G is O or S and T is H, CHClF, CHBrF or $CHFCF_3$ can be prepared by methods that would be obvious to one skilled in the art.

The pyrimidines of Formula IIIa (Z = CH) where Y is

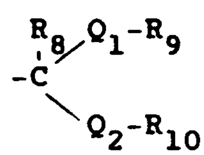 , 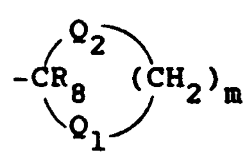 , 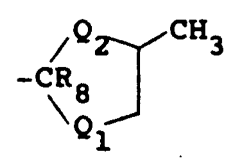

can be prepared according to the methods known to one skilled in the art.

The triazine amines of Formula IIIe where $X_3$ is $CH_3$ or $OCH_3$ and R is H or $CH_3$ can be prepared according to the teachings of European Patent Application No. 94,260.

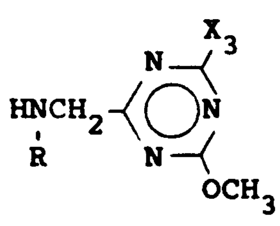 .

IIIe

Preparation of 3-amino-1,2,4-triazoles of Formula IIIf are known in the art and 1,2,4-triazoles are reviewed in The Chemistry of Heterocyclic Compounds "Triazoles 1,2,4" (John Wiley and Sons, New York,

1981). Commonly used starting materials containing nitrogen are N-aminoguanidine, hydrazine, alkylhydrazines, cyanamide, ethyl cyanoacetimidate, dimethyl cyanodithioimidocarbonate, dimethyl cyanoimidocarbonate, ethoxymethylenecyanamide, and acylhydrazines. Some literature syntheses are illustrated below. Using these techniques or suitable modifications that would be apparent to one skilled in the art, the 3-amino-1,2,4-triazole intermediates can be readily prepared.

Heating equimolar amounts of ethyl propionimidate hydrochloride and N-aminoguanidine nitrate in pyridine gives 3-amino-5-ethyltriazole; German Patent 1,073,499 (1960); Berichte, 96, 1064 (1963).

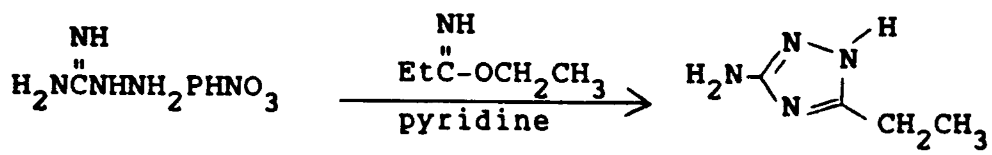


Condensation of hydrazine with ethyl N-cyanoacetimidate yields 3-amino-5-methyltriazole; Journal of Organic Chemistry, 28, 1816 (1963).

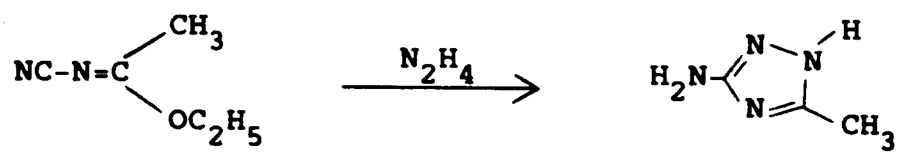


U.S. Patent 2,835,581 (1958) teaches the preparation of 3-amino-5-(hydroxymethyl)triazole from N-aminoguanidine and glycolic acid.

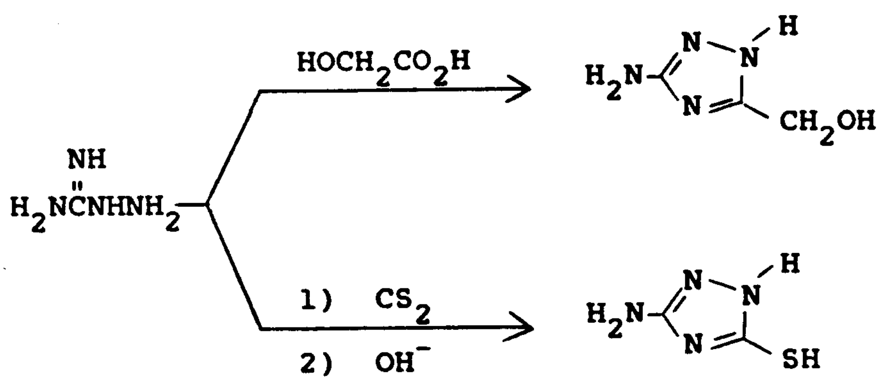


Condensing hydrazine with dimethyl cyanodithioimidocarbonate in acetonitrile gives 3-amino-5-methylthio-1,2,4-triazole while reaction of hydrazine with dimethyl N-cyanoimidocarbonate produces 3-amino-5-methoxy-1,2,4-triazole; Journal of Organic Chemistry, 39, 1522 (1974).

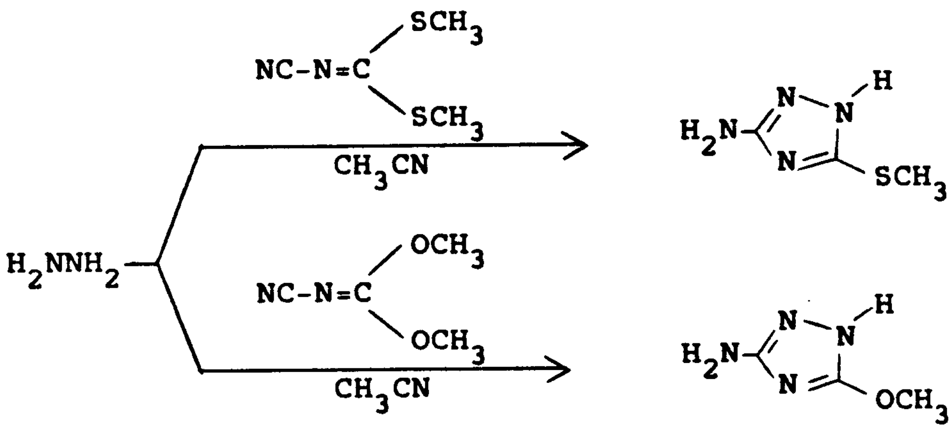


Reaction of substituted hydrazines with N-cyanothioimidocarbonates (prepared according to the procedure given in D. M. Wieland, Ph.D. Thesis, 1971, pp. 123-124) yields disubstituted aminotriazoles as shown below.

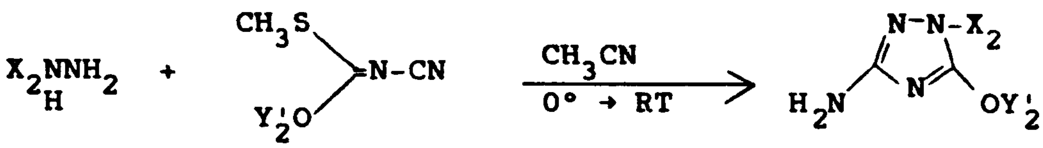


($Y_2' = CH_3$ or $C_2H_5$)

Many of the aminoheterocyclic intermediates of Formula III where R is methyl may be prepared by a two-step procedure as described for IIIg in Equation 31, wherein X, Y and Z are as previously defined.

Equation 31

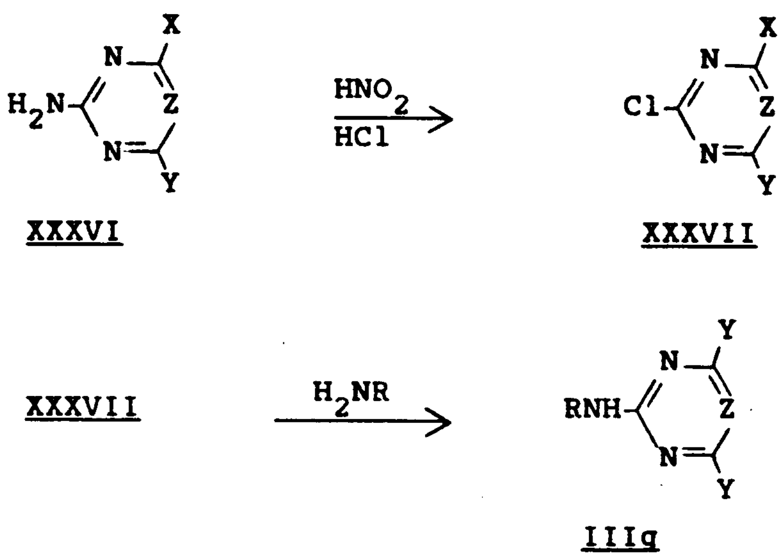


XXXVI

XXXVII

XXXVII

IIIg

A solution of the amine XXXVI in concentrated hydrochloric acid is treated with sodium nitrite solution and the chloro compound XXXVII is isolated in the usual manner by filtration of the acidic solution. A representative procedure is described by Bee and Rose in J. Chem. Soc. C, 2031 (1966), for the case in

which Z = CH, and X = Y = $OCH_3$. Displacement of the chlorine of XXXVII may be accomplished by heating with an excess of methylamine in water to obtain the methylamino heterocycle (IIIg).

Quaternization of amino functionality present in the $R_2$ group of compounds of formula I are also useful as herbicides and can be prepared by standard methods known to one skilled in the art.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct contacting of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I with a suitable acid, e.g., p-toluenesulfonic acid, trichloroacetic acid or the like.

Example 1

**2-t-Butyl-3-hydroxy-7-chloro-2,3-dihydro-1,2-benzisothiazole-1,1-dioxide**

To a solution of 49.5 g of N-t-butyl-2-chlorobenzenesulfonamide in 875 mls of dry THF was added 262 ml of a 1.6M hexane solution of n-butyl lithium at -20° to -5°C under an inert atmosphere. The mixture was stirred at 0°C for 1 hour, room temperature for 2 hours, recooled to -78°C and contacted with 38 mls of dry dimethylformamide. The mixture was allowed to warm to room temperature overnight, poured into water, acidified to a ph of ~3 and ether extracted. The extract was washed with water and brine, dried over $MgSO_4$ and concentrated to give a yellow oil. The oil was dissolved in 50% ether in hexane solution and allowed to stand, giving, after filtration, 38 g of the title compound as colorless crystals, m.p. 139-141°C.

90MHz NMR ($CDCl_3$)δ: 7.7-7.2 (m, 3H, arom);
5.9 (br. d, J = 11Hz, 1H, CH;
4.2 (br. d, J = 11Hz, 1H, OH; and
1.5 (s, 9H, $CH_3$'s).

IR (nujol) 3440 $cm^{-1}$

Example 2

7-Chloro-1,2-benzisothiazole-1,1-dioxide

A solution of 37 g of 2-t-butyl-3-hydroxy-7-chloro-2,3-dihydro-1,2-benzisothiazole-1,1-dioxide and 0.2 g of tosic acid in 370 mls of benzene was refluxed through a Dean-Stark water separator for 16 hours, cooled in ice and filtered to give 18.9 g of the title compound as colorless crystals, m.p. 162-164°C.

90MHz NMR ($CDCl_3$)δ: 9.15 (s, 1H, CH); and
8.0-7.7 (m, 3H, arom).

IR (nujol) 1445, 1320, 1170 cm-1

Example 3

7-Chloro-2,3-dihydro-1,2-benzisothiazole-1,1-dioxide

A suspension of 17.9 g of 7-chloro-1,2-benzisothiazole-1,1-dioxide in 180 ml of absolute ethanol was

cooled to 0°C and treated with 3.33 g of sodium borohydride at such a rate that the temperature remained below 5°C. The mixture was warmed to room temperature for 30 minutes, recooled to 0°C and contacted with glacial acetic acid to destroy excess hydride. The mixture was concentrated to dryness, the resulting solid suspended in water, filtered, washed with water and dried in vacuo at 50°C for 16 hours to give 17.6 g of the title compound as a white powder, m.p. 159-161°C.

90MHz NMR ($CDCl_3$/DMSO-$d_6$)δ: 7.9-7.2 (m, 3H, arom);
6.9 (br, 1H, NH); and
4.4 (s, 2H, $CH_2$).

IR (nujol) 3220 $cm^{-1}$

Example 4

2-Pentyl-7-chloro-2,3-dihydro-1,2-benzisothiazole-1,1-dioxide

A suspension of 10.2 g of 7-chloro-2,3-dihydro-1,2-benzisothiazole-1,1-dioxide, 8.3 g of potassium carbonate and 7.7 ml of 1-iodopentane was refluxed in 50 ml of ethanol for 28 hours, cooled, concentrated and added to 200 ml of water. The mixture was extracted with n-BuCl, the extract washed with water and brine, dried over $MgSO_4$ and concentrated to give 13.3 g of an amber oil which was triturated with hexane to give 11.4 g of the title compound as a tan powder, m.p. 46-48°C.

90MHz NMR ($CDCl_3$)δ: 7.7-7.2 (m, 3H, arom);
4.3 (s, 2H, $CH_2$);
3.3 (t, 2H, $CH_3$); and
2.0-0.7 (m, 9H, alkyl).

IR (nujol) 1573, 1300, 1170, 1158 $cm^{-1}$

Example 5

2-Pentyl-7-(propylthio)-2,3-dihydro-1,2-benzisothiazole-1,1-dioxide

To a solution of 5.0 g of potassium-t-butoxide in 40 ml of dry dimethylformamide was added 4.0 ml of propyl mercaptan at -5° to 0°C under an inert atmosphere followed by 11.0 g of 2-pentyl-7-chloro-2,3-dihydro-1,2-benzisothiazole-1,1-dioxide. The mixture was stirred at room temperature for 16 hours, poured into water and extracted with n-butyl chloride. The extract was washed with water and brine, dried over $MgSO_4$ and concentrated to give 10.3 g of the title compound as a yellow oil.

60MHz NMR ($CDCl_3$)δ: 7.7-7.0 (m, 3H, arom);
4.3 (s, 2H, $CH_2$);
3.4-2.9 (m, 4H, $CH_2$); and
2.0-0.8 (m, 14H, alkyl).

IR (neat) 1582, 1455, 1300, 1170, 1155 $cm^{-1}$

Example 6

2-Pentyl-2,3-dihydro-1,2-benzisothiazole-7-sulfonamide-1,1-dioxide

A solution of 9.5 g of 2-pentyl-7-(propylthio-2,3-dihydro-1,2-benzisothiazole-1,1-dioxide in 40 ml of acetic acid containing 1.3 ml of water was contacted with 7.0 ml of liquified chlorine at 10° to 15°C and allowed to warm to 25°C. The excess chlorine was removed via a stream of nitrogen gas and the solution was poured onto ice. The resulting solid was filtered, washed with water and hexane, dried, dissolved in $CH_2Cl_2$ and contacted with 2.0 ml of liquified ammonia at -78° to 25°C. The mixture was added to 100 ml of 1N HCl and extracted with $CH_2Cl_2$. The extract was washed with water and brine, dried over $MgSO_4$, concentrated and slurried in ether to give 6.35 g of the title compound as a white powder, m.p. 156-160°C.

60MHz NMR ($CDCl_3$/DMSO-$d_6$)δ: 8.2-7.6 (m, 3H, arom);
6.7 (b, 2H, $NH_2$);
4.4 (s, 2H, $CH_2$);
3.3 (t, 2H, $CH_2$); and
2.0-0.8 (m, 9H, alkyl).

IR (nujol) 3370, 3270, 1540, 1285 $cm^{-1}$

Example 7

N-[(4,6-Dimethoxypyrimidin-2-yl)aminocarbonyl]-2-pentyl-2,3-dihydro-1,2-benzisothiazole-7-sulfonamide-1,1-dioxide

A solution of 0.32 g of 2-pentyl-2,3-dihydro-1,2-benzisothiazole-7-sulfonamide-1,1-dioxide and 0.28 g of O-phenyl-N-(4,6-dimethoxypyrimidin-2-yl)carbamate in 5 ml of dry acetonitrile was treated with 0.15 ml of 1,8-diazobicyclo[5.4.0]undec-7-ene. The solution was stirred at 25°C for 10 minutes, diluted with 10 ml of water, cooled to 0°C and acidified with 1N HCl. The resulting precipitate was filtered, washed with water and ether and air dried to give 0.43 g of the title compound as a white powder, m.p. 197-203°C.

200 MHz NMR ($CDCl_3$)δ:
13.0 (b, 1H, NH);
8.34 (d, 1H, arom);
7.77 (t, 1H, arom);
7.64 (d, 1H, arom);
7.12 (b, 1H, NH);
5.77 (s, 1H, CH);
4.34 (s, 2H, $CH_2$);
4.00 (s, 6H, $OCH_3$'s);
3.24 (m, 2H, $CH_2$);
1.75 (m, 2H, $CH_2$);
1.36 (m, 4H, $CH_2$'s); and
0.88 (t, 3H, $CH_3$).

IR (nujol) 1730, 1710, 1610 $cm^{-1}$.

Using the procedures of Examples 1 to 7 and the methods described herein, the following compounds of Tables I-V can be prepared.

## Table Ia

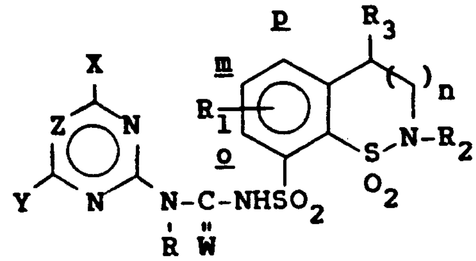

| W | n | R | $R_1$ | $R_2$ | $R_3$ | X | Y | Z | m.p.(°C) |
|---|---|---|---|---|---|---|---|---|---|
| O | 0 | H | H | $CH_2CH_2CH_2CH_2CH_3$ | H | $CH_3$ | $CH_3$ | CH | 189-193 |
| O | 0 | H | H | $CH_2CH_2CH_2CH_2CH_3$ | H | $CH_3$ | $OCH_3$ | CH | 190-197 |
| O | 0 | H | H | $CH_2CH_2CH_2CH_2CH_3$ | H | $OCH_3$ | $OCH_3$ | CH | 197-203 |
| O | 0 | H | H | $CH_2CH_2CH_2CH_2CH_3$ | H | $CH_3$ | $OCH_3$ | N | 172-175 |
| O | 0 | H | H | $CH_2CH_2CH_2CH_2CH_3$ | H | $OCH_3$ | $OCH_3$ | N | 154-158 |
| O | 0 | H | H | $CH_2CH_2CH_2CH_2CH_3$ | H | Cl | $OCH_3$ | CH | 204-206 |
| O | 0 | H | H | $CH_2CH_2CH_2CH_2CH_3$ | H | Br | $OCH_3$ | CH | |
| O | 0 | H | H | $CH_2CH_2CH(CH_3)_2$ | H | $CH_3$ | $CH_3$ | CH | 204-206 |
| O | 0 | H | H | $CH_2CH_2CH(CH_3)_2$ | H | $CH_3$ | $OCH_3$ | CH | 202-205 |
| O | 0 | H | H | $CH_2CH_2CH(CH_3)_2$ | H | $OCH_3$ | $OCH_3$ | CH | 227-229 |
| O | 0 | H | H | $CH_2CH_2CH(CH_3)_2$ | H | $CH_3$ | $OCH_3$ | N | 176-177 |
| O | 0 | H | H | $CH_2CH_2CH(CH_3)_2$ | H | $OCH_3$ | $OCH_3$ | N | 175-178 |
| O | 0 | H | H | $CH_2CH_2CH(CH_3)_2$ | H | Cl | $OCH_3$ | CH | 215-218 |
| O | 0 | H | H | $CH_2CH_2CH(CH_3)_2$ | H | Br | $OCH_3$ | CH | |
| O | 0 | H | H | $CH_2CH(CH_3)CH_2CH_3$ | H | $CH_3$ | $CH_3$ | CH | |
| O | 0 | H | H | $CH_2CH(CH_3)CH_2CH_3$ | H | $CH_3$ | $OCH_3$ | CH | |
| O | 0 | H | H | $CH_2CH(CH_3)CH_2CH_3$ | H | $OCH_3$ | $OCH_3$ | CH | |
| O | 0 | H | H | $CH_2CH(CH_3)CH_2CH_3$ | H | $CH_3$ | $OCH_3$ | N | |
| O | 0 | H | H | $CH_2CH(CH_3)CH_2CH_3$ | H | $OCH_3$ | $OCH_3$ | N | |
| O | 0 | H | H | $CH_2CH(CH_3)CH_2CH_3$ | H | Cl | $OCH_3$ | CH | |
| O | 0 | H | H | $CH_2CH(CH_3)CH_2CH_3$ | H | Br | $OCH_3$ | CH | |
| O | 0 | H | H | $CH(CH_3)CH_2CH_2CH_3$ | H | $CH_3$ | $CH_3$ | CH | |
| O | 0 | H | H | $CH(CH_3)CH_2CH_2CH_3$ | H | $CH_3$ | $OCH_3$ | CH | |
| O | 0 | H | H | $CH(CH_3)CH_2CH_2CH_3$ | H | $OCH_3$ | $OCH_3$ | CH | |

**Table Ia (continued)**

| W | n | R | $R_1$ | $R_2$ | $R_3$ | X | Y | Z | m.p.(°C) |
|---|---|---|---|---|---|---|---|---|---|
| O | O | H | H | $CH(CH_3)CH_2CH_2CH_3$ | H | $CH_3$ | $OCH_3$ | N | |
| O | O | H | H | $CH(CH_3)CH_2CH_2CH_3$ | H | $OCH_3$ | $OCH_3$ | N | |
| O | O | H | H | $CH(CH_3)CH_2CH_2CH_3$ | H | Cl | $OCH_3$ | CH | |
| O | O | H | H | $CH(CH_3)CH_2CH_2CH_3$ | H | Br | $OCH_3$ | CH | |
| O | O | H | H | $CH_2C(CH_3)_3$ | H | $CH_3$ | $OCH_3$ | CH | |
| O | O | H | H | $CH_2C(CH_3)_3$ | H | $OCH_3$ | $OCH_3$ | CH | |
| O | O | H | H | $CH_2Ph$ | H | $CH_3$ | $CH_3$ | CH | 194-195 |
| O | O | H | H | $CH_2Ph$ | H | $CH_3$ | $OCH_3$ | CH | 183-186 |
| O | O | H | H | $CH_2Ph$ | H | $OCH_3$ | $OCH_3$ | CH | 216-218 |
| O | O | H | H | $CH_2Ph$ | H | $CH_3$ | $OCH_3$ | N | 180-184 |
| O | O | H | H | $CH_2Ph$ | H | $OCH_3$ | $OCH_3$ | N | 179-183 |
| O | O | H | H | $CH_2Ph$ | H | Cl | $OCH_3$ | CH | 205-210 |
| O | O | H | H | $CH_2Ph$ | H | Br | $OCH_3$ | CH | |
| O | O | H | H | $OCH_3$ | H | $CH_3$ | $OCH_3$ | CH | |
| O | O | H | H | $OCH_3$ | H | $OCH_3$ | $OCH_3$ | CH | |
| O | O | H | H | $OCH_2CH_3$ | H | $CH_3$ | $OCH_3$ | CH | |
| O | O | H | H | $OCH_2CH_3$ | H | $OCH_3$ | $OCH_3$ | CH | |
| O | O | H | H | $OCH_2CH_2CH_3$ | H | $CH_3$ | $OCH_3$ | CH | |
| O | O | H | H | $OCH_2CH_2CH_3$ | H | $OCH_3$ | $OCH_3$ | CH | |
| O | O | H | H | $OCH(CH_3)_2$ | H | $CH_3$ | $OCH_3$ | CH | |
| O | O | H | H | $OCH(CH_3)_2$ | H | $OCH_3$ | $OCH_3$ | CH | |
| O | O | H | H | $CH_2CH=CH_2$ | H | $CH_3$ | $CH_3$ | CH | 220-221 |
| O | O | H | H | $CH_2CH=CH_2$ | H | $CH_3$ | $OCH_3$ | CH | 223-224 |
| O | O | H | H | $CH_2CH=CH_2$ | H | $OCH_3$ | $OCH_3$ | CH | 210-214 |
| O | O | H | H | $CH_2CH=CH_2$ | H | $CH_3$ | $OCH_3$ | N | 175-177 |
| O | O | H | H | $CH_2CH=CH_2$ | H | $OCH_3$ | $OCH_3$ | N | 193-195 |
| O | O | H | H | $CH_2CH=CH_2$ | H | Cl | $OCH_3$ | CH | 213-215 |
| O | O | H | H | $CH_2CH=CH_2$ | H | Br | $OCH_3$ | CH | |
| O | O | H | H | $CH_2CH=CHCH_3$ | H | $CH_3$ | $CH_3$ | CH | |
| O | O | H | H | $CH_2CH=CHCH_3$ | H | $CH_3$ | $OCH_3$ | CH | 215-217 |
| O | O | H | H | $CH_2CH=CHCH_3$ | H | $OCH_3$ | $OCH_3$ | CH | 219-222 |
| O | O | H | H | $CH_2CH=CHCH_3$ | H | $CH_3$ | $OCH_3$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH=CHCH_3$ | H | $OCH_3$ | $OCH_3$ | N | |
| O | O | H | H | $CH_2CH=CHCH_3$ | H | Cl | $OCH_3$ | CH | 216-218 |
| O | O | H | H | $CH_2CH=CHCH_3$ | H | Br | $OCH_3$ | CH | |
| O | O | H | H | $CH_2C(CH_3)=CH_2$ | H | $CH_3$ | $CH_3$ | CH | |
| O | O | H | H | $CH_2C(CH_3)=CH_2$ | H | $CH_3$ | $OCH_3$ | CH | |
| O | O | H | H | $CH_2C(CH_3)=CH_2$ | H | $OCH_3$ | $OCH_3$ | CH | |
| O | O | H | H | $CH_2C(CH_3)=CH_2$ | H | $CH_3$ | $OCH_3$ | N | |
| O | O | H | H | $CH_2C(CH_3)=CH_2$ | H | $OCH_3$ | $OCH_3$ | N | |
| O | O | H | H | $CH_2C(CH_3)=CH_2$ | H | Cl | $OCH_3$ | CH | |
| O | O | H | H | $CH_2C(CH_3)=CH_2$ | H | Br | $OCH_3$ | CH | |
| O | O | H | H | $CH(CH_3)CH=CH_2$ | H | $CH_3$ | $OCH_3$ | CH | |
| O | O | H | H | $CH(CH_3)CH=CH_2$ | H | $OCH_3$ | $OCH_3$ | CH | |
| O | O | H | H | $CH_2CH_2CH=CH_2$ | H | $CH_3$ | $CH_3$ | CH | 202-204(d) |
| O | O | H | H | $CH_2CH_2CH=CH_2$ | H | $CH_3$ | $OCH_3$ | CH | 198-200(d) |
| O | O | H | H | $CH_2CH_2CH=CH_2$ | H | $OCH_3$ | $OCH_3$ | CH | 201.5-204(d) |
| O | O | H | H | $CH_2CH_2CH=CH_2$ | H | $CH_3$ | $OCH_3$ | N | |
| O | O | H | H | $CH_2CH_2CH=CH_2$ | H | $OCH_3$ | $OCH_3$ | N | |
| O | O | H | H | $CH_2CH_2CH=CH_2$ | H | Cl | $OCH_3$ | CH | 208-210(d) |
| O | O | H | H | $CH_2CH_2CH=CH_2$ | H | Br | $OCH_3$ | CH | |
| O | O | H | H | $CH_2C(Cl)=CH_2$ | H | $CH_3$ | $CH_3$ | CH | |
| O | O | H | H | $CH_2C(Cl)=CH_2$ | H | $CH_3$ | $OCH_3$ | CH | 216-217 |
| O | O | H | H | $CH_2C(Cl)=CH_2$ | H | $OCH_3$ | $OCH_3$ | CH | 215-216 |
| O | O | H | H | $CH_2C(Cl)=CH_2$ | H | Cl | $OCH_3$ | CH | 217-218 |
| O | O | H | H | $CH_2C(Cl)=CH_2$ | H | $CH_3$ | $OCH_3$ | N | |
| O | O | H | H | $CH_2C(Cl)=CH_2$ | H | $OCH_3$ | $OCH_3$ | N | |
| O | O | H | H | $CH_2CH=CH-CH_2Cl$ | H | $CH_3$ | $OCH_3$ | CH | |
| O | O | H | H | $CH_2CH=CH-CH_2Cl$ | H | $OCH_3$ | $OCH_3$ | CH | |
| O | O | H | H | $CH_2C(F)=CHF$ | H | $CH_3$ | $OCH_3$ | CH | |
| O | O | H | H | $CH_2C(F)=CHF$ | H | $OCH_3$ | $OCH_3$ | CH | |
| O | O | H | H | $CH_2C\equiv CH$ | H | $CH_3$ | $OCH_3$ | CH | 211-213 |
| O | O | H | H | $CH_2C\equiv CH$ | H | $OCH_3$ | $OCH_3$ | CH | 194-196 |

Table Ia (continued)

| W | n | R | $R_1$ | $R_2$ | $R_3$ | X | Y | Z | m.p.(°C) |
|---|---|---|---|---|---|---|---|---|---|
| O | 0 | H | H | $CH_2C{\equiv}C-CH_3$ | H | $CH_3$ | $OCH_3$ | CH | |
| O | 0 | H | H | $CH_2C{\equiv}C-CH_3$ | H | $OCH_3$ | $OCH_3$ | CH | |
| O | 0 | H | H | $CH_2CH_2C{\equiv}CH$ | H | $CH_3$ | $OCH_3$ | CH | |
| O | 0 | H | H | $CH_2CH_2C{\equiv}CH$ | H | $OCH_3$ | $OCH_3$ | CH | |
| O | 0 | H | H | $CH(CH_3)C{\equiv}CH$ | H | $CH_3$ | $OCH_3$ | CH | |
| O | 0 | H | H | $CH(CH_3)C{\equiv}CH$ | H | $OCH_3$ | $OCH_3$ | CH | |
| O | 0 | H | H | $CH_2C{\equiv}CCH_2Cl$ | H | $CH_3$ | $OCH_3$ | CH | |
| O | 0 | H | H | $CH_2C{\equiv}CCH_2Cl$ | H | $OCH_3$ | $OCH_3$ | CH | |
| O | 0 | H | H | $CH_2C{\equiv}CCH_2OH$ | H | $CH_3$ | $OCH_3$ | CH | |
| O | 0 | H | H | $CH_2C{\equiv}CCH_2OH$ | H | $OCH_3$ | $OCH_3$ | CH | |
| O | 0 | H | H | $CF_2H$ | H | $CH_3$ | $CH_3$ | CH | |
| O | 0 | H | H | $CF_2H$ | H | $CH_3$ | $OCH_3$ | CH | |
| O | 0 | H | H | $CF_2H$ | H | $OCH_3$ | $OCH_3$ | CH | |
| O | 0 | H | H | $CF_2H$ | H | $CH_3$ | $OCH_3$ | N | |
| O | 0 | H | H | $CF_2H$ | H | $OCH_3$ | $OCH_3$ | N | |
| O | 0 | H | H | $CF_2H$ | H | Cl | $OCH_3$ | CH | |
| O | 0 | H | H | $CF_2H$ | H | Br | $OCH_3$ | CH | |
| O | 0 | H | H | $CF_2CF_2H$ | H | $CH_3$ | $CH_3$ | CH | |
| O | 0 | H | H | $CF_2CF_2H$ | H | $CH_3$ | $OCH_3$ | CH | |
| O | 0 | H | H | $CH_2CF_3$ | H | $CH_3$ | $CH_3$ | CH | 215-216 |
| O | 0 | H | H | $CH_2CF_3$ | H | $CH_3$ | $OCH_3$ | CH | 218-219 |
| O | 0 | H | H | $CH_2CF_3$ | H | $OCH_3$ | $OCH_3$ | CH | 198-199 |
| O | 0 | H | H | $CH_2CF_3$ | H | $CH_3$ | $OCH_3$ | N | 168-170 |
| O | 0 | H | H | $CH_2CF_3$ | H | $OCH_3$ | $OCH_3$ | N | 158-163 |
| O | 0 | H | H | $CH_2CF_3$ | H | Cl | $OCH_3$ | CH | 211-213 |
| O | 0 | H | H | $CH_2CF_3$ | H | Br | $OCH_3$ | CH | |
| O | 0 | H | H | $CH_2CH_2F$ | H | $CH_3$ | $CH_3$ | CH | 214-215 |
| O | 0 | H | H | $CH_2CH_2F$ | H | $CH_3$ | $OCH_3$ | CH | 218-219 |
| O | 0 | H | H | $CH_2CH_2F$ | H | $OCH_3$ | $OCH_3$ | CH | 212-214 |
| O | 0 | H | H | $CH_2CH_2F$ | H | $CH_3$ | $OCH_3$ | N | 190-192 |
| O | 0 | H | H | $CH_2CH_2F$ | H | $OCH_3$ | $OCH_3$ | N | 195-196 |
| O | 0 | H | H | $CH_2CH_2F$ | H | Cl | $OCH_3$ | CH | 215-217 |

Table Ia (continued)

| W | n | R | $R_1$ | $R_2$ | $R_3$ | X | Y | Z | m.p.(°C) |
|---|---|---|---|---|---|---|---|---|---|
| O | 0 | H | H | $CH_2CH_2F$ | H | Br | $OCH_3$ | CH | |
| O | 0 | H | H | $CH_2CH_2Cl$ | H | $CH_3$ | $CH_3$ | CH | 204-205 |
| O | 0 | H | H | $CH_2CH_2Cl$ | H | $CH_3$ | $OCH_3$ | CH | 216-217 |
| O | 0 | H | H | $CH_2CH_2Cl$ | H | $OCH_3$ | $OCH_3$ | CH | 206-208 |
| O | 0 | H | H | $CH_2CH_2Cl$ | H | $CH_3$ | $OCH_3$ | N | 127-128 |
| O | 0 | H | H | $CH_2CH_2Cl$ | H | $OCH_3$ | $OCH_3$ | N | 132-133 |
| O | 0 | H | H | $CH_2CH_2Cl$ | H | Cl | $OCH_3$ | CH | 219-220 |
| O | 0 | H | H | $CH_2CH_2Cl$ | H | Br | $OCH_3$ | CH | |
| O | 0 | H | H | $CH_2CH_2Br$ | H | $CH_3$ | $CH_3$ | CH | 215-216 |
| O | 0 | H | H | $CH_2CH_2Br$ | H | $CH_3$ | $OCH_3$ | CH | 211-212 |
| O | 0 | H | H | $CH_2CH_2Br$ | H | $OCH_3$ | $OCH_3$ | CH | 210-212 |
| O | 0 | H | H | $CH_2CH_2Br$ | H | $CH_3$ | $OCH_3$ | N | 168-171 |
| O | 0 | H | H | $CH_2CH_2Br$ | H | $OCH_3$ | $OCH_3$ | N | 121-123 |
| O | 0 | H | H | $CH_2CH_2Br$ | H | Cl | $OCH_3$ | CH | 222-224 |
| O | 0 | H | H | $CH_2CH_2Br$ | H | Br | $OCH_3$ | CH | |
| O | 0 | H | H | $CH_2CH(Cl)CH_2Cl$ | H | $CH_3$ | $OCH_3$ | CH | |
| O | 0 | H | H | $CH_2CH(Cl)CH_2Cl$ | H | $OCH_3$ | $OCH_3$ | CH | |
| O | 0 | H | H | $CH_2CH(Br)CH_2Br$ | H | $CH_3$ | $OCH_3$ | CH | |
| O | 0 | H | H | $CH_2CH(Br)CH_2Br$ | H | $OCH_3$ | $OCH_3$ | CH | |
| O | 0 | H | H | $CH_2CCl_3$ | H | $CH_3$ | $OCH_3$ | CH | |
| O | 0 | H | H | $CH_2CCl_3$ | H | $OCH_3$ | $OCH_3$ | CH | |
| O | 0 | H | H | $CH_2CH(OH)CH_3$ | H | $CH_3$ | $OCH_3$ | CH | |
| O | 0 | H | H | $CH_2CH(OH)CH_3$ | H | $OCH_3$ | $OCH_3$ | CH | |
| O | 0 | H | H | $CH_2CH_2CH_2OH$ | H | $CH_3$ | $OCH_3$ | CH | |
| O | 0 | H | H | $CH_2CH_2CH_2OH$ | H | $OCH_3$ | $OCH_3$ | CH | |
| O | 0 | H | H | $CH_2CN$ | H | $CH_3$ | $OCH_3$ | CH | 228-230 |
| O | 0 | H | H | $CH_2CN$ | H | $OCH_3$ | $OCH_3$ | CH | 234-236 |
| O | 0 | H | H | $CH_2CH_2CN$ | H | $CH_3$ | $CH_3$ | CH | |
| O | 0 | H | H | $CH_2CH_2CN$ | H | $CH_3$ | $OCH_3$ | CH | 228-230 |
| O | 0 | H | H | $CH_2CH_2CN$ | H | $OCH_3$ | $OCH_3$ | CH | 189-193 |
| O | 0 | H | H | $CH_2CH_2CN$ | H | $CH_3$ | $OCH_3$ | N | 173-176 |
| O | 0 | H | H | $CH_2CH_2CN$ | H | $OCH_3$ | $OCH_3$ | N | 199-203 |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2CN$ | H | Cl | $OCH_3$ | CH | 231-235 |
| O | O | H | H | $CH_2CH_2CN$ | H | Br | $OCH_3$ | CH | |
| O | O | H | H | $CH_2CH(CH_3)CN$ | H | $CH_3$ | $OCH_3$ | CH | |
| O | O | H | H | $CH_2CH(CH_3)CN$ | H | $OCH_3$ | $OCH_3$ | CH | |
| O | O | H | H | $CH_2CH_2CH_2CN$ | H | $CH_3$ | $OCH_3$ | CH | |
| O | O | H | H | $CH_2CH_2CH_2CN$ | H | $OCH_3$ | $OCH_3$ | CH | |
| O | O | H | H | $CH_2OCH_3$ | H | $CH_3$ | $OCH_3$ | CH | 185-187 |
| O | O | H | H | $CH_2OCH_3$ | H | $OCH_3$ | $OCH_3$ | CH | 184-186 |
| O | O | H | H | $CH_2CH_2OCH_3$ | H | $CH_3$ | $CH_3$ | CH | |
| O | O | H | H | $CH_2CH_2OCH_3$ | H | $CH_3$ | $OCH_3$ | CH | 171-175 |
| O | O | H | H | $CH_2CH_2OCH_3$ | H | $OCH_3$ | $OCH_3$ | CH | 194-197 |
| O | O | H | H | $CH_2CH_2OCH_3$ | H | $CH_3$ | $OCH_3$ | N | |
| O | O | H | H | $CH_2CH_2OCH_3$ | H | $OCH_3$ | $OCH_3$ | N | |
| O | O | H | H | $CH_2CH_2OCH_3$ | H | Cl | $OCH_3$ | CH | |
| O | O | H | H | $CH_2CH_2OCH_3$ | H | Br | $OCH_3$ | CH | |
| O | O | H | H | $CH_2CH_2CH_2OCH_3$ | H | $CH_3$ | $OCH_3$ | CH | |
| O | O | H | H | $CH_2CH_2CH_2OCH_3$ | H | $OCH_3$ | $OCH_3$ | CH | |
| O | O | H | H | $CH_2CH_2OCH_2CH_3$ | H | $CH_3$ | $CH_3$ | CH | 195-198 |
| O | O | H | H | $CH_2CH_2OCH_2CH_3$ | H | $CH_3$ | $OCH_3$ | CH | 193-194 |
| O | O | H | H | $CH_2CH_2OCH_2CH_3$ | H | $OCH_3$ | $OCH_3$ | CH | 203-204 |
| O | O | H | H | $CH_2CH_2OCH_2CH_3$ | H | $CH_3$ | $OCH_3$ | N | 193-195 |
| O | O | H | H | $CH_2CH_2OCH_2CH_3$ | H | $OCH_3$ | $OCH_3$ | N | 187-189 |
| O | O | H | H | $CH_2CH_2OCH_2CH_3$ | H | Cl | $OCH_3$ | CH | 193-197 |
| O | O | H | H | $CH_2CH_2OCH_2CH_3$ | H | Br | $OCH_3$ | CH | |
| O | O | H | H | $CH_2CH(OCH_3)_2$ | H | $CH_3$ | $CH_3$ | CH | |
| O | O | H | H | $CH_2CH(OCH_3)_2$ | H | $CH_3$ | $OCH_3$ | CH | |
| O | O | H | H | $CH_2CH(OCH_3)_2$ | H | $OCH_3$ | $OCH_3$ | CH | |
| O | O | H | H | $CH_2CH(OCH_3)_2$ | H | $CH_3$ | $OCH_3$ | N | |
| O | O | H | H | $CH_2CH(OCH_3)_2$ | H | $OCH_3$ | $OCH_3$ | N | |
| O | O | H | H | $CH_2CH(OCH_3)_2$ | H | Cl | $OCH_3$ | CH | |
| O | O | H | H | $CH_2CH(OCH_3)_2$ | H | Br | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | 0 | H | H | $CH_2CH(OCH_2CH_3)_2$ | H | $CH_3$ | $CH_3$ | CH | |
| O | 0 | H | H | $CH_2CH(OCH_2CH_3)_2$ | H | $CH_3$ | $OCH_3$ | CH | |
| O | 0 | H | H | $CH_2CH(OCH_2CH_3)_2$ | H | $OCH_3$ | $OCH_3$ | CH | |
| O | 0 | H | H | $CH_2CH(OCH_2CH_3)_2$ | H | $CH_3$ | $OCH_3$ | N | |
| O | 0 | H | H | $CH_2CH(OCH_2CH_3)_2$ | H | $OCH_3$ | $OCH_3$ | N | |
| O | 0 | H | H | $CH_2CH(OCH_2CH_3)_2$ | H | Cl | $OCH_3$ | CH | |
| O | 0 | H | H | $CH_2CH(OCH_2CH_3)_2$ | H | Br | $OCH_3$ | CH | |
| O | 0 | H | H | $CH_2CH(CH_3)OCH_3$ | H | $CH_3$ | $CH_3$ | CH | |
| O | 0 | H | H | $CH_2CH(CH_3)OCH_3$ | H | $CH_3$ | $OCH_3$ | CH | |
| O | 0 | H | H | $CH_2CH(CH_3)OCH_3$ | H | $OCH_3$ | $OCH_3$ | CH | |
| O | 0 | H | H | $CH_2CH(CH_3)OCH_3$ | H | $CH_3$ | $OCH_3$ | N | |
| O | 0 | H | H | $CH_2CH(CH_3)OCH_3$ | H | $OCH_3$ | $OCH_3$ | N | |
| O | 0 | H | H | $CH_2CH(CH_3)OCH_3$ | H | Cl | $OCH_3$ | CH | |
| O | 0 | H | H | $CH_2CH(CH_3)OCH_3$ | H | Br | $OCH_3$ | CH | |
| O | 0 | H | H | $CH_2CH(CH_3)CH_2OCH_3$ | H | $CH_3$ | $OCH_3$ | CH | |
| O | 0 | H | H | $CH_2CH(CH_3)CH_2OCH_3$ | H | $OCH_3$ | $OCH_3$ | CH | |
| O | 0 | H | H | $CH_2SCH_3$ | H | $CH_3$ | $OCH_3$ | CH | |
| O | 0 | H | H | $CH_2SCH_3$ | H | $OCH_3$ | $OCH_3$ | CH | |
| O | 0 | H | H | $CH_2CH_2SCH_3$ | H | $CH_3$ | $CH_3$ | CH | 189-190 |
| O | 0 | H | H | $CH_2CH_2SCH_3$ | H | $CH_3$ | $OCH_3$ | CH | 192-193 |
| O | 0 | H | H | $CH_2CH_2SCH_3$ | H | $OCH_3$ | $OCH_3$ | CH | 200-201 |
| O | 0 | H | H | $CH_2CH_2SCH_3$ | H | $CH_3$ | $OCH_3$ | N | 163-164 |
| O | 0 | H | H | $CH_2CH_2SCH_3$ | H | $OCH_3$ | $OCH_3$ | N | 168-169 |
| O | 0 | H | H | $CH_2CH_2SCH_3$ | H | Cl | $OCH_3$ | CH | 210-211 |
| O | 0 | H | H | $CH_2CH_2SCH_3$ | H | Br | $OCH_3$ | CH | |
| O | 0 | H | H | $CH_2CH_2SCH_2CH_3$ | H | $CH_3$ | $OCH_3$ | CH | |
| O | 0 | H | H | $CH_2CH_2SCH_2CH_3$ | H | $OCH_3$ | $OCH_3$ | CH | |
| O | 0 | H | H | $CH_2CH_2SO_2CH_2CH_3$ | H | $CH_3$ | $OCH_3$ | CH | |
| O | 0 | H | H | $CH_2CH_2SO_2CH_2CH_3$ | H | $OCH_3$ | $OCH_3$ | CH | |
| O | 0 | H | H | $CH_2CO_2CH_3$ | H | $CH_3$ | $CH_3$ | CH | |
| O | 0 | H | H | $CH_2CO_2CH_3$ | H | $CH_3$ | $OCH_3$ | CH | 175-180 |

Table Ia (continued)

| W | n | R | $R_1$ | $R_2$ | $R_3$ | X | Y | Z | m.p.(°C) |
|---|---|---|---|---|---|---|---|---|---|
| O | 0 | H | H | $CH_2CO_2CH_3$ | H | $OCH_3$ | $OCH_3$ | CH | 178-184 |
| O | 0 | H | H | $CH_2CO_2CH_3$ | H | $CH_3$ | $OCH_3$ | N | 188-192 |
| O | 0 | H | H | $CH_2CO_2CH_3$ | H | $OCH_3$ | $OCH_3$ | N | 161-164 |
| O | 0 | H | H | $CH_2CO_2CH_3$ | H | Cl | $OCH_3$ | CH | 128-130 |
| O | 0 | H | H | $CH_2CO_2CH_3$ | H | Br | $OCH_3$ | CH | |
| O | 0 | H | H | $CH_2CO_2CH_2CH_3$ | H | $CH_3$ | $OCH_3$ | CH | 179-180 |
| O | 0 | H | H | $CH_2CO_2CH_2CH_3$ | H | $OCH_3$ | $OCH_3$ | CH | 173-176 |
| O | 0 | H | H | $CH_2CH_2CO_2CH_3$ | H | $CH_3$ | $OCH_3$ | CH | 214-218 |
| O | 0 | H | H | $CH_2CH_2CO_2CH_3$ | H | $OCH_3$ | $OCH_3$ | CH | 215-217 |
| O | 0 | H | H | $CH_2CH(CH_3)CO_2CH_3$ | H | $CH_3$ | $OCH_3$ | CH | |
| O | 0 | H | H | $CH_2CH(CH_3)CO_2CH_3$ | H | $OCH_3$ | $OCH_3$ | CH | |
| O | 0 | H | H | $CH_2CH_2N(CH_3)_2$ | H | $CH_3$ | $OCH_3$ | CH | |
| O | 0 | H | H | $CH_2CH_2N(CH_3)_2$ | H | $OCH_3$ | $OCH_3$ | CH | |
| O | 0 | H | H | $CH_2N(CH_3)_2$ | H | $CH_3$ | $OCH_3$ | CH | |
| O | 0 | H | H | $CH_2N(CH_3)_2$ | H | $OCH_3$ | $OCH_3$ | CH | |
| O | 0 | H | H | $CH_2CH_2N(CH_3)CH_2CH_3$ | H | $CH_3$ | $OCH_3$ | CH | |
| O | 0 | H | H | $CH_2CH_2N(CH_3)CH_2CH_3$ | H | $OCH_3$ | $OCH_3$ | CH | |
| O | 0 | H | H | $CH_2N(CH_2CH_3)_2$ | H | $CH_3$ | $OCH_3$ | CH | |
| O | 0 | H | H | $CH_2N(CH_2CH_3)_2$ | H | $OCH_3$ | $OCH_3$ | CH | |
| O | 0 | H | H | $CH_2Si(CH_3)_3$ | H | $CH_3$ | $OCH_3$ | CH | 195-197 |
| O | 0 | H | H | $CH_2Si(CH_3)_3$ | H | $OCH_3$ | $OCH_3$ | CH | 196-198 |
| O | 0 | H | H | $C(=O)CH_3$ | H | $CH_3$ | $OCH_3$ | CH | 223-224 |
| O | 0 | H | H | $C(=O)CH_3$ | H | $OCH_3$ | $OCH_3$ | CH | 191-193 |
| O | 0 | H | H | $C(=O)CH_2CH_3$ | H | $CH_3$ | $OCH_3$ | CH | 205-206 |
| O | 0 | H | H | $C(=O)CH_2CH_3$ | H | $OCH_3$ | $OCH_3$ | CH | 160-161 |
| O | 0 | H | H | $C(=O)CH_2CH_2CH_3$ | H | $CH_3$ | $OCH_3$ | CH | 205-206 |
| O | 0 | H | H | $C(=O)CH_2CH_2CH_3$ | H | $OCH_3$ | $OCH_3$ | CH | 198-199 |
| O | 0 | H | H | $SO_2CH_3$ | H | $CH_3$ | $OCH_3$ | CH | |
| O | 0 | H | H | $SO_2CH_3$ | H | $OCH_3$ | $OCH_3$ | CH | |
| O | 0 | H | H | $SO_2CH_2CH_2CH_3$ | H | $CH_3$ | $OCH_3$ | CH | |
| O | 0 | H | H | $SO_2CH_2CH_2CH_3$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | cyclopropyl | H | $CH_3$ | $OCH_3$ | CH | |
| O | O | H | H | cyclopropyl | H | $OCH_3$ | $OCH_3$ | CH | |
| O | O | H | H | cyclopropyl | H | $CH_3$ | $OCH_3$ | N | |
| O | O | H | H | cyclopentyl | H | $CH_3$ | $OCH_3$ | CH | 168-170 |
| O | O | H | H | cyclopentyl | H | $OCH_3$ | $OCH_3$ | CH | 186-189 |
| O | O | H | H | cyclopentyl | H | $CH_3$ | $OCH_3$ | N | 182-183 |
| O | O | H | H | cyclopentyl | H | Cl | $OCH_3$ | CH | 233-235 |
| O | O | H | H | cyclohexyl | H | $CH_3$ | $OCH_3$ | CH | 222-224 |
| O | O | H | H | cyclohexyl | H | $OCH_3$ | $OCH_3$ | CH | 224-227 |
| O | O | H | H | $CH_2$-cyclopropyl | H | $CH_3$ | $CH_3$ | CH | 222°d |
| O | O | H | H | $CH_2$-cyclopropyl | H | $CH_3$ | $OCH_3$ | CH | 166-169 |
| O | O | H | H | $CH_2$-cyclopropyl | H | $OCH_3$ | $OCH_3$ | CH | 221-223 |
| O | O | H | H | $CH_2$-cyclopropyl | H | $CH_3$ | $OCH_3$ | N | 193-196 |
| O | O | H | H | $CH_2$-cyclopropyl | H | $OCH_3$ | $OCH_3$ | N | 204-206 |
| O | O | H | H | $CH_2$-cyclopropyl | H | Cl | $OCH_3$ | CH | 215-217 |
| O | O | H | H | $CH_2$-cyclopropyl | H | Br | $OCH_3$ | CH | |
| O | O | H | H | $CH_2$-cyclopentyl | H | $CH_3$ | $CH_3$ | CH | |
| O | O | H | H | $CH_2$-cyclopentyl | H | $CH_3$ | $OCH_3$ | CH | |
| O | O | H | H | $CH_2$-cyclopentyl | H | $OCH_3$ | $OCH_3$ | CH | |
| O | O | H | H | $CH_2$-cyclopentyl | H | $CH_3$ | $OCH_3$ | N | |
| O | O | H | H | $CH_2$-cyclopentyl | H | $OCH_3$ | $OCH_3$ | N | |
| O | O | H | H | $CH_2$-cyclopentyl | H | Cl | $OCH_3$ | CH | |
| O | O | H | H | $CH_2$-cyclopentyl | H | Br | $OCH_3$ | CH | |
| O | O | H | H | $CH_2$-cyclohexyl | H | $CH_3$ | $CH_3$ | CH | 195-198 |
| O | O | H | H | $CH_2$-cyclohexyl | H | $CH_3$ | $OCH_3$ | CH | 148-150 |
| O | O | H | H | $CH_2$-cyclohexyl | H | $OCH_3$ | $OCH_3$ | CH | 201-204 |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2$-cyclohexyl | H | Cl | $OCH_3$ | CH | 188-191 |
| O | O | H | H | $CH_2$-cyclohexyl | H | $CH_3$ | $CH_3$ | N | 168-170 |
| O | O | H | H | $CH_2$-cyclohexyl | H | $CH_3$ | $OCH_3$ | N | 175-178 |
| O | O | H | H | $CH_2$-cyclohexyl | H | $OCH_3$ | $OCH_3$ | N | 161-164 |
| O | O | H | H | $CH_2$ 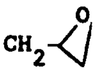 | H | $CH_3$ | $OCH_3$ | CH | |
| O | O | H | H | $CH_2$ 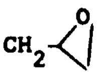 | H | $OCH_3$ | $OCH_3$ | CH | |
| O | O | H | H | $CH_2CHCl_2$ | H | $CH_3$ | $OCH_3$ | CH | |
| O | O | H | H | $CH_2CHCl_2$ | H | $OCH_3$ | $OCH_3$ | CH | |
| O | O | H | H | $CH_2CH_2CH_2Cl$ | H | $CH_3$ | $CH_3$ | CH | 194-197 |
| O | O | H | H | $CH_2CH_2CH_2Cl$ | H | $CH_3$ | $OCH_3$ | CH | 176-183 |
| O | O | H | H | $CH_2CH_2CH_2Cl$ | H | $OCH_3$ | $OCH_3$ | CH | 201-203 |
| O | O | H | H | $CH_2CH_2CH_2Cl$ | H | $CH_3$ | $OCH_3$ | N | 181-183 |
| O | O | H | H | $CH_2CH_2CH_2Cl$ | H | $OCH_3$ | $OCH_3$ | N | 176-178 |
| O | O | H | H | $CH_2CH_2CH_2Cl$ | H | Cl | $OCH_3$ | CH | 186-188 |
| O | O | H | H | $CH_2CH_2CH_2Cl$ | H | Br | $OCH_3$ | CH | |
| O | O | H | H | $CH_2CH_2CH_2Br$ | H | $CH_3$ | $CH_3$ | CH | |
| O | O | H | H | $CH_2CH_2CH_2Br$ | H | $CH_3$ | $OCH_3$ | CH | |
| O | O | H | H | $CH_2CH_2CH_2Br$ | H | $OCH_3$ | $OCH_3$ | CH | |
| O | O | H | H | $CH_2CH_2CH_2Br$ | H | $CH_3$ | $OCH_3$ | N | |
| O | O | H | H | $CH_2CH_2CH_2Br$ | H | $OCH_3$ | $OCH_3$ | N | |
| O | O | H | H | $CH_2CH_2CH_2Br$ | H | Cl | $OCH_3$ | CH | |
| O | O | H | H | $CH_2CH_2CH_2Br$ | H | Br | $OCH_3$ | CH | |
| O | O | H | H | $CH_2CH(Cl)CH_3$ | H | $CH_3$ | $OCH_3$ | CH | |
| O | O | H | H | $CH_2CH(Cl)CH_3$ | H | $OCH_3$ | $OCH_3$ | CH | |
| O | O | H | H | $CH_2CH_2I$ | H | $CH_3$ | $CH_3$ | CH | |
| O | O | H | H | $CH_2CH_2I$ | H | $CH_3$ | $OCH_3$ | CH | |
| O | O | H | H | $CH_2CH_2I$ | H | $OCH_3$ | $OCH_3$ | CH | |
| O | O | H | H | $CH_2CH_2I$ | H | $CH_3$ | $OCH_3$ | N | |
| O | O | H | H | $CH_2CH_2I$ | H | $OCH_3$ | $OCH_3$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2I$ | H | Cl | $OCH_3$ | CH | |
| O | O | H | H | $CH_2CH_2I$ | H | Br | $OCH_3$ | CH | |
| O | O | H | m-F | $CH_2CH_2OCH_3$ | H | $CH_3$ | $OCH_3$ | CH | |
| O | O | H | m-F | $CH_2CH_2OCH_3$ | H | $OCH_3$ | $OCH_3$ | CH | |
| O | O | H | m-F | $CH_2CH_2CH_2Cl$ | H | $CH_3$ | $OCH_3$ | CH | |
| O | O | H | m-F | $CH_2CH_2CH_2Cl$ | H | $OCH_3$ | $OCH_3$ | CH | |
| O | O | H | m-F | $CH_2CH(CH_3)CH_2CH_3$ | H | $CH_3$ | $OCH_3$ | CH | |
| O | O | H | m-F | $CH_2CH(CH_3)CH_2CH_3$ | H | $OCH_3$ | $OCH_3$ | CH | |
| O | O | H | m-Cl | $CH_2CH_2OCH_3$ | H | $CH_3$ | $OCH_3$ | CH | |
| O | O | H | m-Cl | $CH_2CH_2OCH_3$ | H | $OCH_3$ | $OCH_3$ | CH | |
| O | O | H | m-Cl | $CH_2CH_2CH_2Cl$ | H | $CH_3$ | $OCH_3$ | CH | |
| O | O | H | m-Cl | $CH_2CH_2CH_2Cl$ | H | $OCH_3$ | $OCH_3$ | CH | |
| O | O | H | m-Cl | $CH_2CH(CH_3)CH_2CH_3$ | H | $CH_3$ | $OCH_3$ | CH | |
| O | O | H | m-Cl | $CH_2CH(CH_3)CH_2CH_3$ | H | $OCH_3$ | $OCH_3$ | CH | |
| O | O | H | m-$CH_3$ | $CH_2CH_2OCH_3$ | H | CH | OCH | CH | 163-171 |
| O | O | H | m-$CH_3$ | $CH_2CH_2OCH_3$ | H | $OCH_3$ | $OCH_3$ | CH | 171-176 |
| O | O | H | m-$CH_3$ | $CH_2CH_2CH_2Cl$ | H | $CH_3$ | $OCH_3$ | CH | |
| O | O | H | m-$CH_3$ | $CH_2CH_2CH_2Cl$ | H | $OCH_3$ | $OCH_3$ | CH | |
| O | O | H | m-$CH_3$ | $CH_2CH(CH_3)CH_2CH_3$ | H | $CH_3$ | $OCH_3$ | CH | |
| O | O | H | m-$CH_3$ | $CH_2CH(CH_3)CH_2CH_3$ | H | $OCH_3$ | $OCH_3$ | CH | |
| O | O | H | m-$OCH_3$ | $CH_2CH_2OCH_3$ | H | $CH_3$ | $OCH_3$ | CH | |
| O | O | H | m-$OCH_3$ | $CH_2CH_2OCH_3$ | H | $OCH_3$ | $OCH_3$ | CH | |
| O | O | H | m-$OCH_3$ | $CH_2CH_2CH_2Cl$ | H | $CH_3$ | $OCH_3$ | CH | |
| O | O | H | m-$OCH_3$ | $CH_2CH_2CH_2Cl$ | H | $OCH_3$ | $OCH_3$ | CH | |
| O | O | H | m-$OCH_3$ | $CH_2CH(CH_3)CH_2CH_3$ | H | $CH_3$ | $OCH_3$ | CH | |
| O | O | H | m-$OCH_3$ | $CH_2CH(CH_3)CH_2CH_3$ | H | $OCH_3$ | $OCH_3$ | CH | |
| O | O | H | m-$CF_3$ | $CH_2CH_2OCH_3$ | H | $CH_3$ | $OCH_3$ | CH | |
| O | O | H | m-$CF_3$ | $CH_2CH_2OCH_3$ | H | $OCH_3$ | $OCH_3$ | CH | |
| O | O | H | m-$CF_3$ | $CH_2CH_2CH_2Cl$ | H | $CH_3$ | $OCH_3$ | CH | |
| O | O | H | m-$CF_3$ | $CH_2CH_2CH_2Cl$ | H | $OCH_3$ | $OCH_3$ | CH | |
| O | O | H | m-Br | $CH_2CN$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | m-$CH_2CH_3$ | $CH_2CN$ | H | $OCH_3$ | $OCH_3$ | CH | |
| O | O | H | m-$OCH_2CH_3$ | $CH_2CN$ | H | $OCH_3$ | $OCH_3$ | CH | |
| O | O | H | m-$OCF_2H$ | $CH_2CN$ | H | $OCH_3$ | $OCH_3$ | CH | |
| O | O | H | m-$SCH_3$ | $CH_2CN$ | H | $OCH_3$ | $OCH_3$ | CH | |
| O | O | H | o-F | $CH_2CN$ | H | $OCH_3$ | $OCH_3$ | CH | |
| O | O | H | o-Cl | $CH_2CN$ | H | $OCH_3$ | $OCH_3$ | CH | |
| O | O | H | o-Br | $CH_2CN$ | H | $OCH_3$ | $OCH_3$ | CH | |
| O | O | H | o-$CH_3$ | $CH_2CN$ | H | $OCH_3$ | $OCH_3$ | CH | |
| O | O | H | o-$CH_2CH_3$ | $CH_2CN$ | H | $OCH_3$ | $OCH_3$ | CH | |
| O | O | H | o-$OCH_3$ | $CH_2CN$ | H | $OCH_3$ | $OCH_3$ | CH | |
| O | O | H | o-$OCH_2CH_3$ | $CH_2CN$ | H | $OCH_3$ | $OCH_3$ | CH | |
| O | O | H | o-$CF_3$ | $CH_2CN$ | H | $OCH_3$ | $OCH_3$ | CH | |
| O | O | H | o-$OCF_2H$ | $CH_2CN$ | H | $OCH_3$ | $OCH_3$ | CH | |
| O | O | H | o-$SCH_3$ | $CH_2CN$ | H | $OCH_3$ | $OCH_3$ | CH | |
| O | O | H | m-$CH_2CH_3$ | $CH_3$ | H | $CH_3$ | $CH_3$ | CH | |
| O | O | H | m-$CH_2CH_3$ | $CH_3$ | H | $CH_3$ | $OCH_3$ | CH | |
| O | O | H | m-$CH_2CH_3$ | $CH_3$ | H | $OCH_3$ | $OCH_3$ | CH | |
| O | O | H | m-$CH_2CH_3$ | $CH_3$ | H | $CH_3$ | $CH_3$ | N | |
| O | O | H | m-$CH_2CH_3$ | $CH_3$ | H | $CH_3$ | $OCH_3$ | N | |
| O | O | H | m-$CH_2CH_3$ | $CH_3$ | H | $OCH_3$ | $OCH_3$ | N | |
| O | O | H | m-$CH_2CH_3$ | $CH_3$ | H | Cl | $OCH_3$ | CH | |
| O | O | H | m-$CH_2CH_3$ | $CH_3$ | H | Br | $OCH_3$ | CH | |
| O | O | H | m-$CH_2CH_3$ | $CH_2CH_3$ | H | $CH_3$ | $OCH_3$ | CH | |
| O | O | H | m-$CH_2CH_3$ | $CH_2CH_3$ | H | $OCH_3$ | $OCH_3$ | CH | |
| O | O | H | m-$CH_2CH_3$ | $CH_2CH_3$ | H | $CH_3$ | $OCH_3$ | N | |
| O | O | H | m-$CH_2CH_3$ | $CH_2CH_2CH_3$ | H | $CH_3$ | $OCH_3$ | CH | |
| O | O | H | m-$CH_2CH_3$ | $CH_2CH_2CH_3$ | H | $OCH_3$ | $OCH_3$ | CH | |
| O | O | H | m-$CH_2CH_3$ | $CH_2CH_2CH_2CH_3$ | H | $CH_3$ | $OCH_3$ | CH | |
| O | O | H | m-$CH_2CH_3$ | $CH_2CH_2CH_2CH_3$ | H | $OCH_3$ | $OCH_3$ | CH | |
| O | O | H | m-$CH_2CH_3$ | $CH(CH_3)_2$ | H | $CH_3$ | $OCH_3$ | CH | |
| O | O | H | m-$CH_2CH_3$ | $CH(CH_3)_2$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | 0 | H | m-$CH_2CH_3$ | $CH_2CH(CH_3)_2$ | H | $CH_3$ | $OCH_3$ | CH | |
| O | 0 | H | m-$CH_2CH_3$ | $CH_2CH(CH_3)_2$ | H | $OCH_3$ | $OCH_3$ | CH | |
| O | 0 | H | m-$OCH_2CH_3$ | $CH_3$ | H | $CH_3$ | $CH_3$ | CH | |
| O | 0 | H | m-$OCH_2CH_3$ | $CH_3$ | H | $CH_3$ | $OCH_3$ | CH | |
| O | 0 | H | m-$OCH_2CH_3$ | $CH_3$ | H | $OCH_3$ | $OCH_3$ | CH | |
| O | 0 | H | m-$OCH_2CH_3$ | $CH_3$ | H | $CH_3$ | $CH_3$ | N | |
| O | 0 | H | m-$OCH_2CH_3$ | $CH_3$ | H | $CH_3$ | $OCH_3$ | N | |
| O | 0 | H | m-$OCH_2CH_3$ | $CH_3$ | H | $OCH_3$ | $OCH_3$ | N | |
| O | 0 | H | m-$OCH_2CH_3$ | $CH_3$ | H | Cl | $OCH_3$ | CH | |
| O | 0 | H | m-$OCH_2CH_3$ | $CH_3$ | H | Br | $OCH_3$ | CH | |
| O | 0 | H | m-$OCH_2CH_3$ | $CH_2CH_3$ | H | $CH_3$ | $CH_3$ | CH | |
| O | 0 | H | m-$OCH_2CH_3$ | $CH_2CH_3$ | H | $CH_3$ | $OCH_3$ | CH | |
| O | 0 | H | m-$OCH_2CH_3$ | $CH_2CH_3$ | H | $OCH_3$ | $OCH_3$ | CH | |
| O | 0 | H | m-$OCH_2CH_3$ | $CH_2CH_3$ | H | $CH_3$ | $CH_3$ | N | |
| O | 0 | H | m-$OCH_2CH_3$ | $CH_2CH_3$ | H | $CH_3$ | $OCH_3$ | N | |
| O | 0 | H | m-$OCH_2CH_3$ | $CH_2CH_3$ | H | $OCH_3$ | $OCH_3$ | N | |
| O | 0 | H | m-$OCH_2CH_3$ | $CH_2CH_3$ | H | Cl | $OCH_3$ | CH | |
| O | 0 | H | m-$OCH_2CH_3$ | $CH_2CH_3$ | H | Br | $OCH_3$ | CH | |
| O | 0 | H | m-$OCH_2CH_3$ | $CH_2CH_2CH_3$ | H | $CH_3$ | $OCH_3$ | CH | |
| O | 0 | H | m-$OCH_2CH_3$ | $CH_2CH_2CH_3$ | H | $OCH_3$ | $OCH_3$ | CH | |
| O | 0 | H | m-$OCH_2CH_3$ | $CH_2CH_2CH_2CH_3$ | H | $CH_3$ | $OCH_3$ | CH | |
| O | 0 | H | m-$OCH_2CH_3$ | $CH_2CH_2CH_2CH_3$ | H | $OCH_3$ | $OCH_3$ | CH | |
| O | 0 | H | m-$OCH_2CH_3$ | $CH_2CH(CH_3)_2$ | H | $CH_3$ | $OCH_3$ | CH | |
| O | 0 | H | m-$OCH_2CH_3$ | $CH_2CH(CH_3)_2$ | H | $OCH_3$ | $OCH_3$ | CH | |
| O | 0 | H | H | $CH_2CN$ | $CH_3$ | $OCH_3$ | $OCH_3$ | CH | |
| O | 0 | H | H | $CH_2CN$ | $CH_3$ | $OCH_3$ | $OCH_3$ | CH | |
| O | 0 | H | H | $CH_2CN$ | H | $OCH_3$ | $OCH_3$ | CH | |
| O | 1 | H | H | $CH_2CH_2CH_2CH_2CH_3$ | H | $CH_3$ | $OCH_3$ | CH | |
| O | 1 | H | H | $CH_2CH_2CH_2CH_2CH_3$ | H | $OCH_3$ | $OCH_3$ | CH | |
| O | 1 | H | H | $CH_2CH_2CH(CH_3)_2$ | H | $CH_3$ | $OCH_3$ | CH | |
| O | 1 | H | H | $CH_2CH_2CH(CH_3)_2$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | 1 | H | H | $CH_2CH(CH_3)CH_2CH_3$ | H | $CH_3$ | $OCH_3$ | CH | |
| O | 1 | H | H | $CH_2CH(CH_3)CH_2CH_3$ | H | $OCH_3$ | $OCH_3$ | CH | |
| O | 1 | H | H | $CH(CH_3)CH_2CH_2CH_3$ | H | $CH_3$ | $OCH_3$ | CH | |
| O | 1 | H | H | $CH(CH_3)CH_2CH_2CH_3$ | H | $OCH_3$ | $OCH_3$ | CH | |
| O | 1 | H | H | $CH_2C(CH_3)_3$ | H | $CH_3$ | $OCH_3$ | CH | |
| O | 1 | H | H | $CH_2C(CH_3)_3$ | H | $OCH_3$ | $OCH_3$ | CH | |
| O | 1 | H | H | $CH_2CH_2OCH_3$ | H | $CH_3$ | $OCH_3$ | CH | |
| O | 1 | H | H | $CH_2CH_2OCH_3$ | H | $OCH_3$ | $OCH_3$ | CH | |
| O | 1 | H | H | $CH_2CH_2OCH_2CH_3$ | H | $CH_3$ | $OCH_3$ | CH | |
| O | 1 | H | H | $CH_2CH_2OCH_2CH_3$ | H | $OCH_3$ | $OCH_3$ | CH | |
| O | 1 | H | H | $CH_2CH_2CH_2Cl$ | H | $CH_3$ | $OCH_3$ | CH | |
| O | 1 | H | H | $CH_2CH_2CH_2Cl$ | H | $OCH_3$ | $OCH_3$ | CH | |
| O | 1 | H | H | $CH_2CH_2SCH_3$ | H | $CH_3$ | $OCH_3$ | CH | |
| O | 1 | H | H | $CH_2CH_2SCH_3$ | H | $OCH_3$ | $OCH_3$ | CH | |
| O | 1 | H | H | $CH_2C(CH_3)=CH_2$ | H | $OCH_3$ | $OCH_3$ | CH | |
| O | 0 | H | H | $CH_2CH_2OCH_3$ | H | $OCH_2CH_3$ | $OCH_3$ | CH | |
| O | 0 | H | H | $CH_2CH_2OCH_3$ | H | F | $OCH_3$ | CH | |
| O | 0 | H | H | $CH_2CH_2OCH_3$ | H | $OCF_2H$ | $OCH_3$ | CH | |
| O | 0 | H | H | $CH_2CH_2OCH_3$ | H | $CH_2F$ | $OCH_3$ | CH | |
| O | 0 | H | H | $CH_2CH_2OCH_3$ | H | $CF_3$ | $CF_3$ | CH | |
| O | 0 | H | H | $CH_2CH_2OCH_3$ | H | $OCH_3$ | H | CH | |
| O | 0 | H | H | $CH_2CH_2OCH_3$ | H | $CH_3$ | $OCH_2CH_3$ | CH | |
| O | 0 | H | H | $CH_2CH_2OCH_3$ | H | $OCH_3$ | $CH_2OCH_3$ | CH | |
| O | 0 | H | H | $CH_2CH_2OCH_3$ | H | $OCH_3$ | $NH_2$ | CH | |
| O | 0 | H | H | $CH_2CH_2OCH_3$ | H | $OCH_3$ | $NHCH_3$ | CH | |

**Table Ia (continued)**

| W | n | R | $R_1$ | $R_2$ | $R_3$ | X | Y | Z | m.p. (°C) |
|---|---|---|---|---|---|---|---|---|---|
| O | O | H | H | $CH_2CH_2OCH_3$ | H | $OCH_3$ | $N(CH_3)_2$ | CH | |
| O | O | H | H | $CH_2CH_2OCH_3$ | H | $OCH_3$ | $N(OCH_3)CH_3$ | CH | |
| O | O | H | H | $CH_2CH_2OCH_3$ | H | $OCH_3$ | $CH_2CH_3$ | CH | |
| O | O | H | H | $CH_2CH_2OCH_3$ | H | $OCH_3$ | $SCH_3$ | CH | |
| O | O | H | H | $CH_2CH_2OCH_3$ | H | $OCH_3$ | $OCH_2CH{=}CH_2$ | CH | |
| O | O | H | H | $CH_2CH_2OCH_3$ | H | $OCH_3$ | $OCH_2C{\equiv}CH$ | CH | |
| O | O | H | H | $CH_2CH_2OCH_3$ | H | $OCH_3$ | $OCH_2CF_3$ | CH | |
| O | O | H | H | $CH_2CH_2OCH_3$ | H | $OCH_3$ | CN | CH | |
| O | O | H | H | $CH_2CH_2OCH_3$ | H | $OCH_3$ | $N_3$ | CH | |
| O | O | H | H | $CH_2CH_2OCH_3$ | H | $OCH_3$ | $OCH_2CH_2OCH_3$ | CH | |
| O | O | H | H | $CH_2CH_2OCH_3$ | H | $OCH_3$ | $CH_2SCH_3$ | CH | |
| O | O | H | H | $CH_2CH_2OCH_3$ | H | $OCH_3$ | $OCF_2H$ | CH | |
| O | O | H | H | $CH_2CH_2OCH_3$ | H | $OCH_3$ | $SCF_2H$ | CH | |
| O | O | H | H | $CH_2CH_2OCH_3$ | H | $OCH_3$ | $OCF_2CHClF$ | CH | |
| O | O | H | H | $CH_2CH_2OCH_3$ | H | $OCH_3$ | $OCF_2CHBrF$ | CH | |
| O | O | H | H | $CH_2CH_2OCH_3$ | H | $OCH_3$ | $OCF_2CHFCF_3$ | CH | |
| O | O | H | H | $CH_2CH_2OCH_3$ | H | $OCH_3$ | CHO | CH | |
| O | O | H | H | $CH_2CH_2OCH_3$ | H | $OCH_3$ | $C(=O)CH_3$ | CH | |
| O | O | H | H | $CH_2CH_2OCH_3$ | H | $OCH_3$ | $C(=O)CH_2CH_3$ | | |
| O | O | H | H | $CH_2CH_2OCH_3$ | H | $OCH_3$ | $C(=O)CH_2CH_2CH_3$ | CH | |
| O | O | H | H | $CH_2CH_2OCH_3$ | H | $OCH_3$ | $-CH(OCH_3)_2$ | CH | |
| O | O | H | H | $CH_2CH_2OCH_3$ | H | $OCH_3$ | $CH(SCH_3)_2$ | CH | |
| O | O | H | H | $CH_2CH_2OCH_3$ | H | $OCH_3$ | $CH(OCH_2CH_3)_2$ | CH | |
| O | O | H | H | $CH_2CH_2OCH_3$ | H | $OCH_3$ | $CH(SCH_2CH_2CH_3)_2$ | CH | |
| O | O | H | H | $CH_2CH_2OCH_3$ | H | $OCH_3$ | /$OCH_3$<br>$-C-CH_3$<br>\\$SCH_3$ | CH | |
| O | O | H | H | $CH_2CH_2OCH_3$ | H | $OCH_3$ | 1,3-dioxolan-2-yl | CH | |
| O | O | H | H | $CH_2CH_2OCH_3$ | H | $OCH_3$ | 1,3-dioxolan-2-yl | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $CH_2CH_2OCH_3$ | H | $OCH_3$ | 1,3-dithiolan-2-yl | CH | |
| O | O | H | H | $CH_2CH_2OCH_3$ | H | $OCH_3$ | $C(OCH_3)_2$ $CH_2CH_2CH_3$ | CH | |
| O | O | H | H | $CH_2CH_2OCH_3$ | H | $OCH_3$ | $OCH_3$ | $CCH_3$ | |
| O | O | H | H | $CH_2CH_2OCH_3$ | H | $OCH_3$ | $OCH_3$ | $CCH_2CH_3$ | |
| O | O | H | H | $CH_2CH_2OCH_3$ | H | $OCH_3$ | $OCH_3$ | CCl | |
| O | O | H | H | $CH_2CH_2OCH_3$ | H | $OCH_3$ | $OCH_3$ | CBr | |
| O | O | H | H | $(CH_2)_5CH_3$ | H | $CH_3$ | $CH_3$ | CH | 195-196 |
| O | O | H | H | $(CH_2)_5CH_3$ | H | $CH_3$ | $OCH_3$ | CH | 196-197 |
| O | O | H | H | $(CH_2)_5CH_3$ | H | $OCH_3$ | $OCH_3$ | CH | 205-207 |
| O | O | H | H | $(CH_2)_5CH_3$ | H | $CH_3$ | $OCH_3$ | N | 149-151 |
| O | O | H | H | $(CH_2)_5CH_3$ | H | $OCH_3$ | $OCH_3$ | N | 164-166 |
| O | O | H | H | $(CH_2)_5CH_3$ | H | Cl | $OCH_3$ | CH | 215-218 |
| O | O | H | H | $(CH_2)_5CH_3$ | H | Br | $OCH_3$ | CH | |
| O | O | H | H | $(CH_2)_3CH(CH_3)_2$ | H | $CH_3$ | $OCH_3$ | CH | |
| O | O | H | H | $(CH_2)_3CH(CH_3)_2$ | H | $OCH_3$ | $OCH_3$ | CH | |
| O | O | H | H | $CH_2CH_2C(CH_3)_3$ | H | $CH_3$ | $OCH_3$ | CH | |
| O | O | H | H | $CH_2CH_2C(CH_3)_3$ | H | $OCH_3$ | $OCH_3$ | CH | |
| O | O | H | H | $(CH_2)_9CH_3$ | H | $OCH_3$ | $OCH_3$ | CH | 172-176 |
| O | O | H | H | $CH_2CH_2O(CH_2)_9CH_3$ | H | $OCH_3$ | $OCH_3$ | CH | |
| O | O | H | H | $CH_2CH_2OCH_2CH_2Cl$ | H | $OCH_3$ | $OCH_3$ | CH | |
| O | O | H | H | $CH_2CH_2OCF_2H$ | H | $OCH_3$ | $OCH_3$ | CH | |
| O | O | H | H | $CH_2CH_2O(CH_2)_9CF_3$ | H | $OCH_3$ | $OCH_3$ | CH | |
| O | O | H | H | $CH_2CH_2S(CH_2)_9CH_3$ | H | $OCH_3$ | $OCH_3$ | CH | |
| O | O | H | H | $CH_2CH_2SCF_2H$ | H | $OCH_3$ | $OCH_3$ | CH | |
| O | O | H | H | $CH_2CH_2S(CH_2)_9CF_3$ | H | $OCH_3$ | $OCH_3$ | CH | |
| O | O | H | H | $CH_2CH_2SOCH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | 0 | H | H | $CH_2CH_2SO(CH_2)_7CH(CH_3)_2$ | H | $OCH_3$ | $OCH_3$ | CH | |
| O | 0 | H | H | $CH_2CH_2SOCH_2CF_3$ | H | $OCH_3$ | $OCH_3$ | CH | |
| O | 0 | H | H | $CH_2CH_2SO_2(CH_2)_9CH_3$ | H | $OCH_3$ | $OCH_3$ | CH | |
| O | 0 | H | H | $CH_2CH_2SO_2CH_2CF_3$ | H | $OCH_3$ | $OCH_3$ | CH | |
| O | 0 | H | H | $CH_2CH_2OSO_2CH_3$ | H | $CH_3$ | $CH_3$ | CH | |
| O | 0 | H | H | $CH_2CH_2OSO_2CH_3$ | H | $CH_3$ | $OCH_3$ | CH | |
| O | 0 | H | H | $CH_2CH_2OSO_2CH_3$ | H | $OCH_3$ | $OCH_3$ | CH | |
| O | 0 | H | H | $CH_2CH_2OSO_2CH_3$ | H | $CH_3$ | $OCH_3$ | N | |
| O | 0 | H | H | $CH_2CH_2OSO_2CH_3$ | H | $OCH_3$ | $OCH_3$ | N | |
| O | 0 | H | H | $CH_2CH_2OSO_2CH_3$ | H | Cl | $OCH_3$ | CH | |
| O | 0 | H | H | $CH_2CH_2OSO_2CH_3$ | H | Br | $OCH_3$ | CH | |
| O | 0 | H | H | $CH_2CH_2OSO_2CH_2CH_3$ | H | $CH_3$ | $OCH_3$ | CH | |
| O | 0 | H | H | $CH_2CH_2OSO_2CH_2CH_3$ | H | $OCH_3$ | $OCH_3$ | CH | |
| O | 0 | H | H | $CH_2CH_2OSO_2CH_2CH_3$ | H | $OCH_3$ | $CH_3$ | N | |
| O | 0 | H | H | $CH_2CH_2OSO_2(CH_2)_9CH_3$ | H | $OCH_3$ | $OCH_3$ | CH | |
| O | 0 | H | H | $CH_2COCH_3$ | H | $CH_3$ | $CH_3$ | CH | |
| O | 0 | H | H | $CH_2COCH_3$ | H | $CH_3$ | $OCH_3$ | CH | |
| O | 0 | H | H | $CH_2COCH_3$ | H | $OCH_3$ | $OCH_3$ | CH | 178-182 |
| O | 0 | H | H | $CH_2COCH_3$ | H | $CH_3$ | $OCH_3$ | N | |
| O | 0 | H | H | $CH_2COCH_3$ | H | $OCH_3$ | $OCH_3$ | N | |
| O | 0 | H | H | $CH_2COCH_3$ | H | Cl | $OCH_3$ | CH | |
| O | 0 | H | H | $CH_2COCH_3$ | H | Br | $OCH_3$ | CH | |
| O | 0 | H | H | $CH_2CH_2COCH_3$ | H | $CH_3$ | $CH_3$ | CH | 200-201 |
| O | 0 | H | H | $CH_2CH_2COCH_3$ | H | $CH_3$ | $OCH_3$ | CH | 175-180 |
| O | 0 | H | H | $CH_2CH_2COCH_3$ | H | $OCH_3$ | $OCH_3$ | CH | 182-190 |
| O | 0 | H | H | $CH_2CH_2COCH_3$ | H | $CH_3$ | $OCH_3$ | N | |
| O | 0 | H | H | $CH_2CH_2COCH_3$ | H | $OCH_3$ | $OCH_3$ | N | |
| O | 0 | H | H | $CH_2CH_2COCH_3$ | H | Cl | $OCH_3$ | CH | 201-205 |
| O | 0 | H | H | $CH_2CH_2COCH_3$ | H | Br | $OCH_3$ | CH | |
| O | 0 | H | H | $CH_2COCH(CH_3)_2$ | H | $OCH_3$ | $OCH_3$ | CH | |
| O | 0 | H | H | $CH_2CO(CH_2)_9CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $CH_2COCH_2CH_2Cl$ | H | $OCH_3$ | $OCH_3$ | CH | |
| O | O | H | H | $CH_2CO(CH_2)_5CF_3$ | H | $OCH_3$ | $OCH_3$ | CH | |
| O | O | H | H | $CH_2CH_2OCO(CH_2)_9CH_3$ | H | $OCH_3$ | $OCH_3$ | CH | |
| O | O | H | H | $CH_2CH_2OCOCH_3$ | H | $CH_3$ | $CH_3$ | CH | |
| O | O | H | H | $CH_2CH_2OCOCH_3$ | H | $CH_3$ | $OCH_3$ | CH | |
| O | O | H | H | $CH_2CH_2OCOCH_3$ | H | $OCH_3$ | $OCH_3$ | CH | |
| O | O | H | H | $CH_2CH_2OCOCH_3$ | H | $CH_3$ | $OCH_3$ | N | |
| O | O | H | H | $CH_2CH_2OCOCH_3$ | H | $OCH_3$ | $OCH_3$ | N | |
| O | O | H | H | $CH_2CH_2OCOCH_3$ | H | Cl | $OCH_3$ | CH | |
| O | O | H | H | $CH_2CH_2OCOCH_3$ | H | Br | $OCH_3$ | CH | |
| O | O | H | H | $CH_2CH_2OCOCH_2CH_3$ | H | $OCH_3$ | $OCH_3$ | CH | |
| O | O | H | H | $CH_2CH(CH_3)OCOCH_3$ | H | $OCH_3$ | $OCH_3$ | CH | |
| O | O | H | H | $CH_2CH_2OCOCH_2Cl$ | H | $CH_3$ | $OCH_3$ | CH | |
| O | O | H | H | $CH_2CH_2OCOCH_2Cl$ | H | $OCH_3$ | $OCH_3$ | CH | |
| O | O | H | H | $CH_2CH_2OCO(CH_2)_9CH_2F$ | H | $OCH_3$ | $OCH_3$ | CH | |
| O | O | H | H | $CH_2CO_2(CH_2)_5CH_3$ | H | $OCH_3$ | $OCH_3$ | CH | |
| O | O | H | H | $CH_2CO_2CH_2CF_3$ | H | $OCH_3$ | $OCH_3$ | CH | |
| O | O | H | H | $CH_2CO_2(CH_2)_5CH_2Br$ | H | $OCH_3$ | $OCH_3$ | CH | |
| O | O | H | H | $CH_2CHO$ | H | $OCH_3$ | $OCH_3$ | CH | |
| O | O | H | H | $CH_2CH_2NH_2$ | H | $OCH_3$ | $OCH_3$ | CH | |
| O | O | H | H | $CH_2CH_2NHCH_3$ | H | $CH_3$ | $OCH_3$ | CH | |
| O | O | H | H | $CH_2CH_2NHCH_3$ | H | $OCH_3$ | $OCH_3$ | CH | |
| O | O | H | H | $CH_2CH_2NH(CH_2)_5CH_3$ | H | $OCH_3$ | $OCH_3$ | CH | |
| O | O | H | H | $CH_2CH_2N(CH_3)CH(CH_3)_2$ | H | $OCH_3$ | $OCH_3$ | CH | |
| O | O | H | H | $CH_2CH_2NHCOCH_3$ | H | $CH_3$ | $OCH_3$ | CH | |
| O | O | H | H | $CH_2CH_2NHCOCH_3$ | H | $OCH_3$ | $OCH_3$ | CH | |
| O | O | H | H | $CH_2CH_2N(CH_3)COCH_3$ | H | $CH_3$ | $OCH_3$ | CH | |
| O | O | H | H | $CH_2CH_2N(CH_3)COCH_3$ | H | $OCH_3$ | $OCH_3$ | CH | |
| O | O | H | H | $CH_2CH_2OH$ | H | $CH_3$ | $CH_3$ | CH | |
| O | O | H | H | $CH_2CH_2OH$ | H | $CH_3$ | $OCH_3$ | CH | |
| O | O | H | H | $CH_2CH_2OH$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $CH_2CH_2OH$ | H | $CH_3$ | $OCH_3$ | N | |
| O | O | H | H | $CH_2CH_2OH$ | H | $OCH_3$ | $OCH_3$ | N | |
| O | O | H | H | $CH_2CH_2OH$ | H | Cl | $OCH_3$ | CH | |
| O | O | H | H | $CH_2CH_2OH$ | H | Br | $OCH_3$ | CH | |
| O | O | H | H | $CH_2NO_2$ | H | $CH_3$ | $OCH_3$ | CH | |
| O | O | H | H | $CH_2NO_2$ | H | $OCH_3$ | $OCH_3$ | CH | |
| O | O | H | H | $CH_2NO_2$ | H | $OCH_3$ | $OCH_3$ | N | |
| O | O | H | H | $CH_2CH_2NO_2$ | H | $CH_3$ | $OCH_3$ | CH | |
| O | O | H | H | $CH_2CH_2NO_2$ | H | $OCH_3$ | $OCH_3$ | CH | |
| O | O | H | H | $CH_2CH_2NO_2$ | H | $OCH_3$ | $OCH_3$ | N | |
| O | O | H | H | $CH_2Si(CH_3)_2C(CH_3)_3$ | H | $OCH_3$ | $OCH_3$ | CH | |
| O | O | H | H | $CH_2Si(CH_3)_2$- 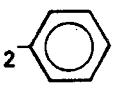 | H | $OCH_3$ | $OCH_3$ | CH | |
| O | O | H | H | $CH_2Si(CH_3)_2$- 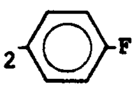 -F | H | $OCH_3$ | $OCH_3$ | CH | |
| O | O | H | H | $CH_2Si(CH_3)_2$- 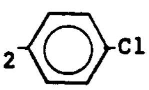 -Cl | H | $OCH_3$ | $OCH_3$ | CH | |
| O | O | H | H | $CH_2Si(CH_3)_2$- 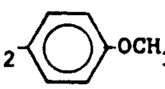 -$OCH_3$ | H | $OCH_3$ | $OCH_3$ | CH | |
| O | O | H | H | $CH_2Si(CH_3)_2$- 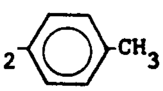 -$CH_3$ | H | $OCH_3$ | $OCH_3$ | CH | |
| O | O | H | H | $CH_2Si(CH_3)_2$- 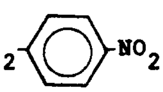 -$NO_2$ | H | $OCH_3$ | $OCH_3$ | CH | |
| O | O | H | H | $CH_2$- 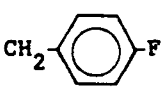 -F | H | $CH_3$ | $CH_3$ | CH | 214-216 |
| O | O | H | H | $CH_2$- 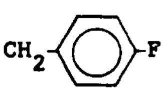 -F | H | $CH_3$ | $OCH_3$ | CH | 204-207 |
| O | O | H | H | $CH_2$- 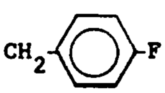 -F | H | $OCH_3$ | $OCH_3$ | CH | 208-210 |

**Table Ia (continued)**

| W | n | R | $R_1$ | $R_2$ | $R_3$ | X | Y | Z | m.p. (°C) |
|---|---|---|---|---|---|---|---|---|---|
| O | 0 | H | H | $CH_2$ 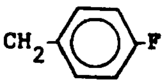 | H | Cl | $OCH_3$ | CH | 228-230 |
| O | 0 | H | H | $CH_2$ 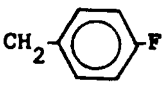 | H | Br | $OCH_3$ | CH | |
| O | 0 | H | H | $CH_2$ 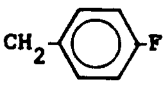 | H | $CH_3$ | $OCH_3$ | N | 201-203 |
| O | 0 | H | H | $CH_2$ 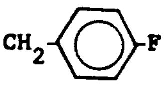 | H | $OCH_3$ | $OCH_3$ | N | 194-197 |
| O | 0 | H | H | $CH_2$ 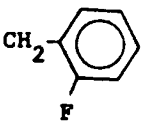 | H | $OCH_3$ | $OCH_3$ | CH | 201-204 |
| O | 0 | H | H | $CH_2CH_2C_6H_5$ | H | $CH_3$ | $CH_3$ | CH | 206-207 |
| O | 0 | H | H | $CH_2CH_2C_6H_5$ | H | $CH_3$ | $OCH_3$ | CH | 188-190 |
| O | 0 | H | H | $CH_2CH_2C_6H_5$ | H | $OCH_3$ | $OCH_3$ | CH | 208-210 |
| O | 0 | H | H | $CH_2CH_2C_6H_5$ | H | $CH_3$ | $OCH_3$ | N | 195-196 |
| O | 0 | H | H | $CH_2CH_2C_6H_5$ | H | $OCH_3$ | $OCH_3$ | N | 195-196 |
| O | 0 | H | H | $CH_2CH_2C_6H_5$ | H | Cl | $OCH_3$ | CH | 209-211 |
| O | 0 | H | H | $CH_2CH_2C_6H_5$ | H | Br | $OCH_3$ | CH | |
| O | 0 | H | H | $CH_2CH{=}CH(CH_2)_6CH_3$ | H | $OCH_3$ | $OCH_3$ | CH | |
| O | 0 | H | H | $CH_2C{\equiv}C(CH_2)_6CH_3$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $O(CH_2)_9CH_3$ | H | $OCH_3$ | $OCH_3$ | CH | |
| O | O | H | H | $CO(CH_2)_9CH_3$ | H | $OCH_3$ | $OCH_3$ | CH | |
| O | O | H | H | $CO_2(CH_2)_9CH_3$ | H | $OCH_3$ | $OCH_3$ | CH | |
| O | O | H | H | $SO_2(CH_2)_9CH_3$ | H | $OCH_3$ | $OCH_3$ | CH | |
| O | O | H | H | $SO_2CH(CH_3)_2$ | H | Cl | $OCH_3$ | CH | |
| O | O | H | H | $CH_2C{\equiv}CC_6H_5$ | H | $OCH_3$ | $OCH_3$ | CH | |
| O | O | H | H | $COCH_2Cl$ | H | $CH_3$ | $OCH_3$ | CH | |
| O | O | H | H | $COCH_2Cl$ | H | $OCH_3$ | $OCH_3$ | CH | |
| O | O | H | H | $CO_2C_6H_5$ | H | $OCH_3$ | $OCH_3$ | CH | |
| O | O | H | H | $SO_2C_6H_5$ | H | $OCH_3$ | $OCH_3$ | CH | |
| O | O | H | H | $COC_6H_5$ | H | $OCH_3$ | $OCH_3$ | CH | 214-219 |
| O | O | H | H | $CH{=}CHCH_2CH_3$ | H | $OCH_3$ | $OCH_3$ | CH | |
| O | O | H | H | $CH_2CH_2OCH_3$ | H | cyclo-propyl | $CH_3$ | CH | |
| O | O | H | H | $CH_2CH_2OCH_3$ | H | cyclo-propyl | $CH_2CH_3$ | CH | |
| O | O | H | H | $CH_2CH_2OCH_3$ | H | cyclo-propyl | $OCH_3$ | CH | |
| O | O | H | H | $CH_2CH_2OCH_3$ | H | cyclo-propyl | $OCH_2CH_3$ | CH | |
| O | O | H | H | $CH_2CH_2OCH_3$ | H | cyclo-propyl | $CH_2OCH_3$ | CH | |
| O | O | H | H | $CH_2CH_2OCH_3$ | H | cyclo-propyl | $OCF_2H$ | N | |
| O | O | H | H | $CH_2CH_2OCH_3$ | H | cyclo-propyl | $SCF_2H$ | N | |
| O | O | H | H | $CH_2CH_2OCH_3$ | H | cyclo-propyl | $OCH_2CF_3$ | CH | |
| O | O | H | H | $CH_2CH_2OCH_3$ | H | cyclo-propyl | $CF_3$ | N | |
| O | O | H | H | $CH_2CH_2OCH_3$ | H | cyclo-propyl | $OCH_2CH{=}CH_2$ | CH | |
| O | O | H | H | $CH_2CH_2OCH_3$ | H | cyclo-propyl | $OCH_2C{\equiv}CH$ | N | |
| O | O | H | H | $CH_2CH_2OCH_3$ | H | cyclo-propyl | $NHCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $CH_2CH_2OCH_3$ | H | cyclo-propyl | $N(CH_3)_2$ | N | |
| O | O | H | H | $CH_2CH_2OCH_3$ | H | cyclo-propyl | $CH(OCH_3)_2$ | CH | |
| O | O | H | H | $CH_2CH_2CH(CH_3)_2$ | H | cyclo-propyl | $OCH_3$ | CH | |
| O | O | H | H | $CH_2CH_2CH(CH_3)_2$ | H | cyclo-propyl | $OCH_3$ | N | |
| O | O | H | H | $CH_2CH=CH_2$ | H | cyclo-propyl | $OCH_3$ | CH | |
| O | O | H | H | $CH_2CH=CH_2$ | H | cyclo-propyl | $OCH_3$ | N | |
| O | O | H | H | $CH_2CH=CHCH_3$ | H | cyclo-propyl | $OCH_3$ | CH | |
| O | O | H | H | $CH_2CH=CHCH_3$ | H | cyclo-propyl | $OCH_3$ | N | |
| O | O | H | H | $CH_2CH_2CH_2Cl$ | H | cyclo-propyl | $OCH_3$ | CH | |
| O | O | H | H | $CH_2CH_2CH_2Cl$ | H | cyclo-propyl | $OCH_3$ | N | |
| O | O | H | H | $CH_2CH_2OCH_3$ | H | cyclo-propyl | $OCH_3$ | CH | |
| O | O | H | H | $CH_2CH_2OCH_3$ | H | cyclo-propyl | $OCH_3$ | N | |
| O | O | H | H | $CH_2CH=CH_2$ | H | cyclo-propyl | $OCH_3$ | CH | |
| O | O | H | H | $CH_2CH=CH_2$ | H | cyclo-propyl | $OCH_3$ | N | |
| O | O | H | H | $CH_2Si(CH_3)_3$ | H | $CH_3$ | $CH_3$ | CH | 190-193 |
| O | O | H | H | $CH_2Si(CH_3)_3$ | H | Cl | $OCH_3$ | CH | 201-202 |
| O | O | H | H | $CH_2Si(CH_3)_3$ | H | $CH_3$ | $OCH_3$ | N | 190-193 |
| O | O | H | H | $CH_2Si(CH_3)_3$ | H | $OCH_3$ | $OCH_3$ | N | 138-141 |
| O | O | H | H | $(CH_2)_9CH_3$ | H | $CH_3$ | $CH_3$ | CH | 167-169 |
| O | O | H | H | $(CH_2)_9CH_3$ | H | $CH_3$ | $OCH_3$ | CH | 159-163 |
| O | O | H | H | $(CH_2)_9CH_3$ | H | Cl | $OCH_3$ | N | 123-125 |
| O | O | H | H | $(CH_2)_9CH_3$ | H | $OCH_3$ | $OCH_3$ | N | 141-145 |

**Table Ia (continued)**

| W | n | R | $R_1$ | $R_2$ | $R_3$ | X | Y | Z | m.p. (°C) |
|---|---|---|---|---|---|---|---|---|---|
| O | O | H | H | $CO_2CH_3$ | H | $CH_3$ | $CH_3$ | CH | 218-220 |
| O | O | H | H | $CO_2CH_3$ | H | $CH_3$ | $OCH_3$ | CH | 241-244 |
| O | O | H | H | $CO_2Et$ | H | $OCH_3$ | $OCH_3$ | CH | 171-173 |
| O | O | H | H | $CO_2Et$ | H | $OCH_3$ | $CH_3$ | CH | 210-215 |
| O | O | H | H | $CO_2Et$ | H | $CH_3$ | $CH_3$ | CH | 185-187 |
| O | O | H | H | $CO_2Pr$ | H | $OCH_3$ | $OCH_3$ | CH | 143-150 |
| O | O | H | H | $CO_2Pr$ | H | $OCH_3$ | $CH_3$ | CH | 190-195 |
| O | O | H | H | $CO_2Pr$ | H | $CH_3$ | $CH_3$ | CH | 214-216 |
| O | O | H | H | $CO_2Bu$ | H | $OCH_3$ | $OCH_3$ | CH | 148-150 |
| O | O | H | H | $CO_2Bu$ | H | $OCH_3$ | $CH_3$ | CH | 162-165 |
| O | O | H | H | $CO_2Bu$ | H | $CH_3$ | $CH_3$ | CH | 198-202 |
| O | O | H | H | $CO_2iBu$ | H | $OCH_3$ | $OCH_3$ | CH | 182-186 |
| O | O | H | H | $CO_2iBu$ | H | $OCH_3$ | $CH_3$ | CH | 171-174 |
| O | O | H | H | $CO_2iBu$ | H | $CH_3$ | $CH_3$ | CH | 198-200 |
| O | O | H | H | $CO_2$Pentyl | H | $OCH_3$ | $OCH_3$ | CH | 147-149 |
| O | O | H | H | $CO_2$Pentyl | H | $OCH_3$ | $CH_3$ | CH | 140-148 |
| O | O | H | H | $CO_2$Pentyl | H | $CH_3$ | $CH_3$ | CH | 175-176 |
| O | O | H | H | $CO_2$Hexyl | H | $OCH_3$ | $OCH_3$ | CH | 130-135 |
| O | O | H | H | $CO_2$Hexyl | H | $OCH_3$ | $CH_3$ | CH | 155-156 |
| O | O | H | H | $CO_2$Hexyl | H | $CH_3$ | $CH_3$ | CH | 166-168 |
| O | O | H | H | $CO_2CH_2Ph$ | H | $OCH_3$ | $OCH_3$ | CH | 188-190 |
| O | O | H | H | $CO_2CH_2Ph$ | H | $OCH_3$ | $CH_3$ | CH | 194-197 |
| O | O | H | H | $CO_2CH_2Ph$ | H | $CH_3$ | $CH_3$ | CH | 173-179 |
| O | O | H | H | $CO_2$-*i*-Pr | H | $OCH_3$ | $OCH_3$ | CH | 210-215 |
| O | O | H | H | $CO_2$-*i*-Pr | H | $CH_3$ | $OCH_3$ | CH | 219-224 |
| O | O | H | H | $CO_2$-*i*-Pr | H | Cl | $OCH_3$ | CH | 194-199 |
| O | O | H | H | $CO_2C(CH_3)_3$ | H | $CH_3$ | $CH_3$ | CH | |
| O | O | H | H | $CO_2C(CH_3)_3$ | H | $CH_3$ | $OCH_3$ | N | |
| O | O | H | H | $CO_2C(CH_3)_3$ | H | $OCH_3$ | $OCH_3$ | N | |
| O | O | H | H | $CO_2C(CH_3)_3$ | H | Cl | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CO_2CH_3$ | H | $OCH_3$ | $OCH_3$ | CH | 239-241 |
| O | O | H | H | $CO_2CH_3$ | H | Cl | $OCH_3$ | CH | 238-241 |
| O | O | H | H | $CO_2CH_3$ | H | $CH_3$ | $OCH_3$ | N | 217-219 |
| O | O | H | H | $CO_2CH_3$ | H | $OCH_3$ | $OCH_3$ | N | 219-220 |
| O | O | H | H | $CH_2CH_2CO_2CH_3$ | H | $CH_3$ | $CH_3$ | CH | |
| O | O | H | H | $CH_2CH_2CO_2CH_3$ | H | Cl | $OCH_3$ | CH | 219-223 |
| O | O | H | H | $CH_2CH_2CO_2CH_3$ | H | $CH_3$ | $OCH_3$ | N | 197-199 |
| O | O | H | H | $CH_2CH_2CO_2CH_3$ | H | $OCH_3$ | $OCH_3$ | N | 183-189 |
| O | O | H | H | $CH_2OCH_2CH_2OCH_3$ | H | $CH_3$ | $CH_3$ | CH | |
| O | O | H | H | $CH_2OCH_2CH_2OCH_3$ | H | $CH_3$ | $OCH_3$ | CH | |
| O | O | H | H | $CH_2OCH_2CH_2OCH_3$ | H | $OCH_3$ | $OCH_3$ | CH | |
| O | O | H | H | $CH_2OCH_2CH_2OCH_3$ | H | Cl | $OCH_3$ | CH | |
| O | O | H | H | $CH_2OCH_2CH_2OCH_3$ | H | $CH_3$ | $OCH_3$ | N | |
| O | O | H | H | $CH_2OCH_2CH_2OCH_3$ | H | $OCH_3$ | $OCH_3$ | N | |
| O | O | H | H | $CH_2CH_2OCH_2CH_2OCH_3$ | H | $CH_3$ | $OCH_3$ | CH | |
| O | O | H | H | $CH_2CH_2OCH_2CH_2OCH_3$ | H | $OCH_3$ | $OCH_3$ | CH | |
| O | O | H | H | $CH_2CH_2OCH_2CH_2OCH_3$ | H | $OCH_3$ | $CH_3$ | CH | |
| O | O | H | H | $CH_2CH(CH_2CH_3)_2$ | H | $CH_3$ | $CH_3$ | CH | |
| O | O | H | H | $CH_2CH(CH_2CH_3)_2$ | H | $CH_3$ | $OCH_3$ | CH | |
| O | O | H | H | $CH_2CH(CH_2CH_3)_2$ | H | $OCH_3$ | $OCH_3$ | CH | |
| O | O | H | H | $CH_2CH(CH_2CH_3)_2$ | H | Cl | $OCH_3$ | CH | |
| O | O | H | H | $CH_2CH(CH_2CH_3)_2$ | H | $CH_3$ | $OCH_3$ | N | |
| O | O | H | H | $CH_2CH(CH_2CH_3)_2$ | H | $OCH_3$ | $OCH_3$ | N | |
| O | O | H | H | $CH(CH_2CH_3)_2$ | H | $CH_3$ | $CH_3$ | CH | 209-211 |
| O | O | H | H | $CH(CH_2CH_3)_2$ | H | $CH_3$ | $OCH_3$ | CH | 219-225 |
| O | O | H | H | $CH(CH_2CH_3)_2$ | H | $OCH_3$ | $OCH_3$ | CH | 223-225 |
| O | O | H | H | $CH(CH_2CH_3)_2$ | H | Cl | $OCH_3$ | CH | 220-223 |
| O | O | H | H | $CH(CH_2CH_3)_2$ | H | $CH_3$ | $OCH_3$ | N | 149-152 |
| O | O | H | H | $CH(CH_2CH_3)_2$ | H | $OCH_3$ | $OCH_3$ | N | 139-143 |
| O | O | H | H | $CH_2CH_2CH(CH_3)CH_2CH_3$ | H | $CH_3$ | $CH_3$ | N | |
| O | O | H | H | $CH_2CH_2CH(CH_3)CH_2CH_3$ | $CH_3$ | $OCH_3$ | $OCH_3$ | CH | |
| O | O | H | H | $CH_2CH_2CH(CH_3)CH_2CH_3$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2CH(CH_3)CH_2CH_3$ | H | $OCH_3$ | $OCH_3$ | CH | |
| O | O | H | H | $CH_2CH_2CH(CH_3)CH_2CH_3$ | H | $CH_3$ | $OCH_3$ | N | |
| O | O | H | H | $CH_2CH_2CH(CH_3)CH_2CH_3$ | H | $OCH_3$ | $OCH_3$ | N | |
| O | O | H | H | $CH_2CH(CH_3)CH_2CH_2CH_3$ | H | $CH_3$ | $CH_3$ | CH | |
| O | O | H | H | $CH_2CH(CH_3)CH_2CH_2CH_3$ | H | $CH_3$ | $OCH_3$ | CH | |
| O | O | H | H | $CH_2CH(CH_3)CH_2CH_2CH_3$ | H | $OCH_3$ | $OCH_3$ | CH | |
| O | O | H | H | $CH_2CH(CH_3)CH_2CH_2CH_3$ | H | Cl | $OCH_3$ | CH | |
| O | O | H | H | $CH_2CH(CH_3)CH_2CH_2CH_3$ | H | $CH_3$ | $OCH_3$ | N | |
| O | O | H | H | $CH_2CH(CH_3)CH_2CH_2CH_3$ | H | $OCH_3$ | $OCH_3$ | N | |
| O | O | H | H | $CH_2OCH_2CH_2OCH_2CH_3$ | H | $CH_3$ | $OCH_3$ | CH | |
| O | O | H | H | $CH_2OCH_2CH_2OCH_2CH_3$ | H | $OCH_3$ | $OCH_3$ | CH | |
| O | O | H | H | $CH_2OCH_2CH_2OCH_2CH_3$ | H | Cl | $OCH_3$ | CH | |
| O | O | H | H | $CH_2OCH_2CH_2OCH_2CH_3$ | H | $CH_3$ | $OCH_3$ | N | |
| O | O | H | H | $CH_2CH_2OCH_2OCH_3$ | H | $CH_3$ | $CH_3$ | CH | |
| O | O | H | H | $CH_2CH_2OCH_2OCH_3$ | H | $CH_3$ | $OCH_3$ | CH | |
| O | O | H | H | $CH_2CH_2OCH_2OCH_3$ | H | $OCH_3$ | $OCH_3$ | CH | |
| O | O | H | H | $CH_2CH_2OCH_2OCH_3$ | H | Cl | $OCH_3$ | CH | |
| O | O | H | H | $CH_2CH_2OCH_2OCH_3$ | H | $CH_3$ | $OCH_3$ | N | |
| O | O | H | H | $CH_2CH_2OCH_2OCH_3$ | H | $OCH_3$ | $OCH_3$ | N | |
| O | O | H | H | $CH_2CH_2OCH_2CHOCH_3$ | H | $CH_3$ | $OCH_3$ | CH | |
| O | O | H | H | $CH_2CH_2OCH_2CHOCH_3$ | H | $OCH_3$ | $OCH_3$ | CH | |
| O | O | H | H | $CH_2CH_2CH_2I$ | H | $CH_3$ | $CH_3$ | CH | |
| O | O | H | H | $CH_2CH_2CH_2I$ | H | $CH_3$ | $OCH_3$ | CH | |
| O | O | H | H | $CH_2CH_2CH_2I$ | H | $OCH_3$ | $OCH_3$ | CH | |
| O | O | H | H | $CH_2CH_2CH_2I$ | H | Cl | $OCH_3$ | CH | |
| O | O | H | H | $CH_2CH_2CH_2I$ | H | $CH_3$ | $OCH_3$ | N | |
| O | O | H | H | $CH_2CH_2CH_2I$ | H | $OCH_3$ | $OCH_3$ | N | |
| O | O | H | H | $CH_2CH_2CH_2CH_2Cl$ | H | $CH_3$ | $CH_3$ | CH | |
| O | O | H | H | $CH_2CH_2CH_2CH_2Cl$ | H | $CH_3$ | $OCH_3$ | CH | |
| O | O | H | H | $CH_2CH_2CH_2CH_2Cl$ | H | $OCH_3$ | $OCH_3$ | CH | |
| O | O | H | H | $CH_2CH_2CH_2CH_2Cl$ | H | Cl | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $CH_2CH_2CH_2CH_2Cl$ | H | $CH_3$ | $OCH_3$ | N | |
| O | O | H | H | $CH_2CH_2CH_2CH_2Cl$ | H | $OCH_3$ | $OCH_3$ | N | |
| O | O | H | H | $CH_2CH_2CH_2CH_2Br$ | H | $CH_3$ | $CH_3$ | CH | |
| O | O | H | H | $CH_2CH_2CH_2CH_2Br$ | H | $CH_3$ | $OCH_3$ | CH | |
| O | O | H | H | $CH_2CH_2CH_2CH_2Br$ | H | $OCH_3$ | $OCH_3$ | CH | |
| O | O | H | H | $CH_2CH_2CH_2CH_2Br$ | H | Cl | $OCH_3$ | CH | |
| O | O | H | H | $CH_2CH_2CH_2CH_2Br$ | H | $CH_3$ | $OCH_3$ | N | |
| O | O | H | H | $CH_2CH_2CH_2CH_2Br$ | H | $OCH_3$ | $OCH_3$ | N | |
| O | O | H | H | $CH_2CH_2CH_2F$ | H | $CH_3$ | $CH_3$ | CH | 210-211 |
| O | O | H | H | $CH_2CH_2CH_2F$ | H | $CH_3$ | $OCH_3$ | CH | 216-217 |
| O | O | H | H | $CH_2CH_2CH_2F$ | H | $OCH_3$ | $OCH_3$ | CH | 199-200 |
| O | O | H | H | $CH_2CH_2CH_2F$ | H | Cl | $OCH_3$ | CH | 222-224 |
| O | O | H | H | $CH_2CH_2CH_2F$ | H | $CH_3$ | $OCH_3$ | N | 148-149 |
| O | O | H | H | $CH_2CH_2CH_2F$ | H | $OCH_3$ | $OCH_3$ | N | 182-184 |
| O | O | H | H | $CH_2CH_2CH_2CH_2F$ | H | $CH_3$ | $CH_3$ | CH | |
| O | O | H | H | $CH_2CH_2CH_2CH_2F$ | H | $CH_3$ | $OCH_3$ | CH | |
| O | O | H | H | $CH_2CH_2CH_2CH$ F | H | $OCH_3$ | $OCH_3$ | CH | |
| O | O | H | H | $CH_2CH_2CH_2CH_2F$ | H | Cl | $OCH_3$ | CH | |
| O | O | H | H | $CH_2CH_2CH_2CH_2F$ | H | $CH_3$ | $OCH_3$ | N | |
| O | O | H | H | $CH_2CH_2CH_2CH_2F$ | H | $OCH_3$ | $OCH_3$ | N | |
| O | O | H | H | $CH_2CH_2CF_3$ | H | $CH_3$ | $CH_3$ | CH | |
| O | O | H | H | $CH_2CH_2CF_3$ | H | $CH_3$ | $OCH_3$ | CH | |
| O | O | H | H | $CH_2CH_2CF_3$ | H | $OCH_3$ | $OCH_3$ | CH | |
| O | O | H | H | $CH_2CH_2CF_3$ | H | Cl | $OCH_3$ | CH | |
| O | O | H | H | $CH_2CH_2CF_3$ | H | $CH_3$ | $OCH_3$ | N | |
| O | O | H | H | $CH_2CH_2CF_3$ | H | $OCH_3$ | $OCH_3$ | N | |
| O | O | H | H | $CH_2CH_2CH_2CF_3$ | H | $CH_3$ | $CH_3$ | CH | |
| O | O | H | H | $CH_2CH_2CH_2CF_3$ | H | $CH_3$ | $OCH_3$ | CH | |
| O | O | H | H | $CH_2CH_2CH_2CF_3$ | H | $OCH_3$ | $OCH_3$ | CH | |
| O | O | H | H | $CH_2CH_2CH_2CF_3$ | H | Cl | $OCH_3$ | CH | |

Table 1a (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2CH_2CF_3$ | H | $CH_3$ | $OCH_3$ | N | |
| O | O | H | H | $CH_2CH_2CH_2CF_3$ | H | $OCH_3$ | $OCH_3$ | N | |
| O | O | H | H | $CH_2CH(CF_3)_2$ | H | $CH_3$ | $OCH_3$ | CH | |
| O | O | H | H | $CH_2CH(CF_3)_2$ | H | $OCH_3$ | $OCH_3$ | CH | |
| O | O | H | H | $CH_2OCH_3$ | H | $CH_3$ | $CH_3$ | CH | 190-192 |
| O | O | H | H | $CH_2OCH_3$ | H | Cl | $OCH_3$ | CH | 172-174 |
| O | O | H | H | $CH_2OCH_3$ | H | $CH_3$ | $OCH_3$ | N | 171-173 |
| O | O | H | H | $CH_2OCH_3$ | H | $OCH_3$ | $OCH_3$ | N | 116-119 |
| O | O | H | H | $CH_2OCH_2CH_3$ | H | $CH_3$ | $CH_3$ | CH | |
| O | O | H | H | $CH_2OCH_2CH_3$ | H | $CH_3$ | $OCH_3$ | CH | |
| O | O | H | H | $CH_2OCH_2CH_3$ | H | $CH_3$ | $OCH_3$ | N | |
| O | O | H | H | $CH_2OCH_2CH_3$ | H | $OCH_3$ | $OCH_3$ | N | |
| O | O | H | H | $CH_2CH_2CH_2OCH_3$ | H | $CH_3$ | $CH_3$ | CH | |
| O | O | H | H | $CH_2CH_2CH_2OCH_3$ | H | Cl | $OCH_3$ | CH | |
| O | O | H | H | $CH_2CH_2CH_2OCH_3$ | H | $CH_3$ | $OCH_3$ | N | |
| O | O | H | H | $CH_2CH_2CH_2OCH_3$ | H | $OCH_3$ | $OCH_3$ | N | |
| O | O | H | H | $CH_2C(OCH_3)_2CH_3$ | H | $CH_3$ | $CH_3$ | CH | |
| O | O | H | H | $CH_2C(OCH_3)_2CH_3$ | H | $CH_3$ | $OCH_3$ | CH | |
| O | O | H | H | $CH_2C(OCH_3)\ CH_3$ | H | $OCH_3$ | $OCH_3$ | CH | |
| O | O | H | H | $CH_2C(OCH_3)_2CH_3$ | H | Cl | $OCH_3$ | CH | |
| O | O | H | H | $CH_2C(OCH_3)_2CH_3$ | H | $CH_3$ | $OCH_3$ | N | |
| O | O | H | H | $CH_2C(OCH_3)_2CH_3$ | H | $OCH_3$ | $OCH_3$ | N | |
| O | O | H | H | $CH_2CH_2C(OCH_3)_2CH_3$ | H | $CH_3$ | $CH_3$ | CH | |
| O | O | H | H | $CH_2CH_2C(OCH_3)_2CH_3$ | H | $CH_3$ | $OCH_3$ | CH | |
| O | O | H | H | $CH_2CH_2C(OCH_3)_2CH_3$ | H | $OCH_3$ | $OCH_3$ | CH | |
| O | O | H | H | $CH_2CH_2C(OCH_3)_2CH_3$ | H | Cl | $OCH_3$ | CH | |
| O | O | H | H | $CH_2CH_2C(OCH_3)_2CH_3$ | H | $CH_3$ | $OCH_3$ | N | |
| O | O | H | H | $CH_2CH_2C(OCH_3)_2CH_3$ | H | $OCH_3$ | $OCH_3$ | N | |
| O | O | H | H | $CH_2CH_2OCH_2CH{=}CH_2$ | H | $CH_3$ | $OCH_3$ | CH | |
| O | O | H | H | $CH_2CH_2OCH_2CH{=}CH_2$ | H | $OCH_3$ | $OCH_3$ | CH | |
| O | O | H | H | $CH_2CH_2OCH_2C{=}CH_2$ | H | $CH_3$ | $OCH_3$ | CH | |
| O | O | H | H | $CH_2CH_2OCH_2C{=}CH_2$ | H | $OCH_3$ | $OCH_3$ | CH | |

Table 1a (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2OCH{=}CH_2$ | H | $CH_3$ | $OCH_3$ | CH | |
| O | O | H | H | $CH_2CH_2OCH{=}CH_2$ | H | $OCH_3$ | $OCH_3$ | CH | |
| O | O | H | H | (2-cyclopentoxy)ethyl | H | $CH_3$ | $OCH_3$ | CH | |
| O | O | H | H | (2-cyclopentoxy)ethyl | H | $OCH_3$ | $OCH_3$ | CH | |
| O | O | H | H | phenoxymethyl | H | $CH_3$ | $OCH_3$ | CH | |
| O | O | H | H | phenoxymethyl | H | $OCH_3$ | $CH_3$ | CH | |
| O | O | H | H | 2-phenoxyethyl | H | $CH_3$ | $OCH_3$ | CH | |
| O | O | H | H | 2-phenoxyethyl | H | $OCH_3$ | $OCH_3$ | CH | |
| O | O | H | H | (4-chlorophenoxy)methyl | H | $OCH_3$ | $OCH_3$ | CH | |
| O | O | H | H | (4-bromophenoxy)methyl | H | $OCH_3$ | $OCH_3$ | CH | |
| O | O | H | H | (4-methylphenoxy)methyl | H | $OCH_3$ | $OCH_3$ | CH | |
| O | O | H | H | (4-nitrophenoxy)methyl | H | $OCH_3$ | $OCH_3$ | CH | |
| O | O | H | H | $CH_2CH_2OCH_2C{=}CH_2(CH_2)_6CH_3$ | H | $OCH_3$ | $OCH_3$ | CH | |
| O | O | H | H | $CH_2CH_2OCH_2C{=}CH(CH_2)_6CH_3$ | H | $OCH_3$ | $OCH_3$ | CH | |
| O | O | H | H | $CH_2CH_2OC(O)CH_2CH{=}CH_2$ | H | $OCH_3$ | $OCH_3$ | CH | |
| O | O | H | H | $CH_2CH_2OC(O)C{\equiv}CCH_3$ | H | $OCH_3$ | $OCH_3$ | CH | |
| O | O | H | H | $CH_2CH_2OC(O)$(cyclopropyl) | H | $OCH_3$ | $OCH_3$ | CH | |
| O | O | H | H | $CH_2CH_2OC(O)C_6H_5$ | H | $OCH_3$ | $OCH_3$ | CH | |
| O | O | H | H | $CH_2CH_2OC(O)C_6H_5$ | H | $OCH_3$ | $OCH_3$ | CH | |
| O | O | H | H | $CH_2CH_2OC(O)NHCH_3$ | H | $CH_3$ | $OCH_3$ | CH | |
| O | O | H | H | $CH_2CH_2OC(O)NHCH_3$ | H | $OCH_3$ | $OCH_3$ | CH | |
| O | O | H | H | $CH_2CH_2OC(O)NHCH_3$ | H | $CH_3$ | $OCH_3$ | CH | |
| O | O | H | H | $CH_2CH_2OC(O)N(CH_3)_2$ | H | $OCH_3$ | $OCH_3$ | CH | |
| O | O | H | H | $CH_2CH_2OC(O)NH(CH_2)_3CH_3$ | H | $CH_3$ | $OCH_3$ | CH | |
| O | O | H | H | $CH_2CH_2OC(O)NHC_6H_5$ | H | $OCH_3$ | $OCH_3$ | CH | |
| O | O | H | H | $CH_2CH_2OSO_2CF_3$ | H | $CH_3$ | $OCH_3$ | CH | |
| O | O | H | H | $CH_2CH_2OSO_2C_6H_5$ | H | $OCH_3$ | $OCH_3$ | CH | |
| O | O | H | H | $CH_2CH_2OP(O)(OCH_3)_2$ | H | $OCH_3$ | $OCH_3$ | CH | |
| O | O | H | H | $CH_2CH_2OP(O)(OCH_2CH_2CH_2CH_2)$ | H | $OCH_3$ | $OCH_3$ | CH | |
| O | O | H | H | $CH_2P(O)(OCH_3)_2$ | H | $CH_3$ | $CH_3$ | CH | |
| O | O | H | H | $CH_2P(O)(OCH_3)_2$ | H | $CH_3$ | $OCH_3$ | CH | |
| O | O | H | H | $CH_2P(O)(OCH_3)_2$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2P(O)(OCH_3)_2$ | H | Cl | $OCH_3$ | CH | |
| O | O | H | H | $CH_2P(O)(OCH_3)_2$ | H | $CH_3$ | $OCH_3$ | N | |
| O | O | H | H | $CH_2P(O)(OCH_3)_2$ | H | $OCH_3$ | $OCH_3$ | N | |
| O | O | H | H | $CH_2CH_2OCO_2CH_3$ | H | $CH_3$ | $CH_3$ | CH | |
| O | O | H | H | $CH_2CH_2OCO_2CH_3$ | H | $CH_3$ | $OCH_3$ | CH | |
| O | O | H | H | $CH_2CH_2OCO_2CH_3$ | H | $CH_3$ | $OCH_3$ | CH | |
| O | O | H | H | $CH_2CH_2OCO_2CH_3$ | H | Cl | $OCH_3$ | CH | |
| O | O | H | H | $CH_2CH_2OCO_2CH_3$ | H | $CH_3$ | $OCH_3$ | N | |
| O | O | H | H | $CH_2CH_2OCO_2CH_3$ | H | $OCH_3$ | $OCH_3$ | N | |
| O | O | H | H | $CH_2CH_2OCO_2CH_2CH_3$ | H | $CH_3$ | $OCH_3$ | CH | |
| O | O | H | H | $CH_2CH_2OCO_2CH_2CH_3$ | H | $OCH_3$ | $OCH_3$ | CH | |
| O | O | H | H | $CH_2CH_2OCO_2C_6H_5$ | H | $CH_3$ | $OCH_3$ | CH | |
| O | O | H | H | $CH_2CH_2OCO_2C_6H_5$ | H | $OCH_3$ | $OCH_3$ | CH | |
| O | O | H | H | $CH_2CH_2OP(S)(OCH_3)_2$ | H | $OCH_3$ | $OCH_3$ | CH | |
| O | O | H | H | $CH_2CH_2OP(S)(CH_3)_2$ | H | $OCH_3$ | $OCH_3$ | CH | |
| O | O | H | H | $CH_2CH_2OSi(CH_3)_3$ | H | $CH_3$ | $CH_3$ | CH | |
| O | O | H | H | $CH_2CH_2OSi(CH_3)_3$ | H | $CH_3$ | $OCH_3$ | CH | |
| O | O | H | H | $CH_2CH_2OSi(CH_3)_3$ | H | $OCH_3$ | $OCH_3$ | CH | |
| O | O | H | H | $CH_2CH_2OSi(CH_3)_3$ | H | Cl | $OCH_3$ | CH | |
| O | O | H | H | $CH_2CH_2OSi(CH_3)_3$ | H | $CH_3$ | $OCH_3$ | N | |
| O | O | H | H | $CH_2CH_2OSi(CH_3)_3$ | H | $OCH_3$ | $OCH_3$ | N | |
| O | O | H | H | $CH_2CH_2OSi(CH_3)_2C(CH_3)_3$ | H | $CH_3$ | $OCH_3$ | CH | |
| O | O | H | H | $CH_2CH_2OSi(CH_3)_2C(CH_3)_3$ | H | $OCH_3$ | $OCH_3$ | CH | |
| O | O | H | H | $CH_2CH_2OSi(CH_3)_2(CH_2)_9CH_3$ | H | $OCH_3$ | $OCH_3$ | CH | |
| O | O | H | H | $CH_2CH_2OSi(CH_3)_2C_6H_5$ | H | $OCH_3$ | $OCH_3$ | CH | |
| O | O | H | H | $CH_2CH_2OSi(CH_3)_2CH_2C_6H_5$ | H | $OCH_3$ | $OCH_3$ | CH | |
| O | O | H | H | $CH_2CH_2CH_2OH$ | H | $CH_3$ | $CH_3$ | CH | |
| O | O | H | H | $CH_2CH_2CH_2OH$ | H | $CH_3$ | $OCH_3$ | CH | |
| O | O | H | H | $CH_2CH_2CH_2OH$ | H | $OCH_3$ | $OCH_3$ | CH | |
| O | O | H | H | $CH_2CH_2CH_2OH$ | H | $OCH_3$ | $OCH_3$ | N | |
| O | O | H | H | $CH_2CH_2CH_2OH$ | H | Cl | $OCH_3$ | CH | |
| O | O | H | H | $CH_2CH_2CH_2OH$ | H | $OCH_3$ | $CH_3$ | N | |

Table 1a (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2CH_2OC(O)CH_3$ | H | $OCH_3$ | $OCH_3$ | CH | |
| O | O | H | H | $CH_2CH_2CH_2OSi(CH_3)_3$ | H | $OCH_3$ | $OCH_3$ | CH | |
| O | O | H | H | $CH_2CH_2CH_2OSO_2CH_3$ | H | $OCH_3$ | $OCH_3$ | CH | |
| O | O | H | H | $CH_2SO_2CH_3$ | H | $CH_3$ | $OCH_3$ | CH | |
| O | O | H | H | $CH_2SO_2CH_3$ | H | $OCH_3$ | $OCH_3$ | CH | |
| O | O | H | H | $CH_2SO_2CH_3$ | H | Cl | $OCH_3$ | CH | |
| O | O | H | H | $CH_2SO_2CH_3$ | H | $CH_3$ | $OCH_3$ | N | |
| O | O | H | H | $CH_2CH_2SOCH_2CH_3$ | H | $CH_3$ | $OCH_3$ | CH | |
| O | O | H | H | $CH_2CH_2SOCH_2CH_3$ | H | $OCH_3$ | $OCH_3$ | CH | |
| O | O | H | H | $CH_2CH_2SO_2CH_2CH_3$ | H | $CH_3$ | $OCH_3$ | CH | |
| O | O | H | H | $CH_2CH_2SO_2CH_2CH_3$ | H | $OCH_3$ | $OCH_3$ | CH | |
| O | O | H | H | $CH_2SC_6H_5$ | H | $CH_3$ | $CH_3$ | CH | |
| O | O | H | H | $CH_2SC_6H_5$ | H | $CH_3$ | $OCH_3$ | CH | |
| O | O | H | H | $CH_2SC_6H_5$ | H | $OCH_3$ | $OCH_3$ | CH | |
| O | O | H | H | $CH_2SC_6H_5$ | H | Cl | $OCH_3$ | CH | |
| O | O | H | H | $CH_2SC_6H_5$ | H | $CH_3$ | $OCH_3$ | N | |
| O | O | H | H | $CH_2SC_6H_5$ | H | $OCH_3$ | $OCH_3$ | N | |
| O | O | H | H | $CH_2SO_2C_6H_5$ | H | $CH_3$ | $OCH_3$ | CH | |
| O | O | H | H | $CH_2SO_2C_6H_5$ | H | $OCH_3$ | $OCH_3$ | CH | |
| O | O | H | H | $CH_2SO_2C_6H_5$ | H | $CH_3$ | $OCH_3$ | N | |
| O | O | H | H | $CH_2SO_2C_6H_5$ | H | $OCH_3$ | $OCH_3$ | N | |
| O | O | H | H | $CH_2SCN$ | H | $CH_3$ | $OCH_3$ | CH | |
| O | O | H | H | $CH_2SCN$ | H | $OCH_3$ | $OCH_3$ | CH | |
| O | O | H | H | $CH_2CH_2SCN$ | H | $CH_3$ | $OCH_3$ | CH | |
| O | O | H | H | $CH_2CH_2SCN$ | H | $OCH_3$ | $OCH_3$ | CH | 193-195 |
| O | O | H | H | $CH_2CN$ | H | $CH_3$ | $CH_3$ | CH | 230-234 |
| O | O | H | H | $CH_2CN$ | H | Cl | $OCH_3$ | CH | 238-241 |
| O | O | H | H | $CH_2CN$ | H | $CH_3$ | $OCH_3$ | N | |
| O | O | H | H | $CH_2CN$ | H | $OCH_3$ | $OCH_3$ | N | |
| O | O | H | H | $CH_2CH_2SP(O)(OCH_3)_2$ | H | $OCH_3$ | $OCH_3$ | CH | |
| O | O | H | H | $CH_2CH_2SP(S)(OCH_3)_2$ | H | $OCH_3$ | $OCH_3$ | CH | |
| O | O | H | H | $CH_2P(S)_2(OCH_3)_2$ | H | $OCH_3$ | $OCH_3$ | CH | |

**Table Ia (continued)**

| W | n | R | $R_1$ | $R_2$ | $R_3$ | X | Y | Z | m.p.(°C) |
|---|---|---|---|---|---|---|---|---|---|
| O | O | H | H | $CH_2NH_2$ | H | $CH_3$ | $CH_3$ | CH | |
| O | O | H | H | $CH_2NH_2$ | H | $OCH_3$ | $OCH_3$ | CH | |
| O | O | H | H | $CH_2NH\ CH_2CH_2CH_2CH_3$ | H | $OCH_3$ | $OCH_3$ | CH | |
| O | O | H | H | $CH_2N^{\oplus}(CH_3)_3Cl^{\ominus}$ | H | $OCH_3$ | $OCH_3$ | CH | |
| O | O | H | H | $CH_2CH_2N^{\oplus}(CH_3)_3I^{\ominus}$ | H | $OCH_3$ | $OCH_3$ | CH | |
| O | O | H | H | $CH_2CH_2NHCO_2CH_3$ | H | $OCH_3$ | $OCH_3$ | CH | |
| O | O | H | H | $CH_2CH_2NHC(O)NH\ CH_3$ | H | $CH_3$ | $OCH_3$ | CH | |
| O | O | H | H | $CH_2CH_2NHC(O)NH\ CH_3$ | H | $OCH_3$ | $OCH_3$ | CH | |
| O | O | H | H | $CH_2CH_2NHSO_2CH_3$ | H | $CH_3$ | $CH_3$ | CH | |
| O | O | H | H | $CH_2CH_2NHSO_2CH_3$ | H | $CH_3$ | $OCH_3$ | CH | |
| O | O | H | H | $CH_2CH_2NHSO_2CH_3$ | H | $OCH_3$ | $OCH_3$ | CH | |
| O | O | H | H | $CH_2CH_2NHSO_2CH_3$ | H | Cl | $OCH_3$ | CH | |
| O | O | H | H | $CH_2CH_2NHSO_2CH_3$ | H | $CH_3$ | $OCH_3$ | N | |
| O | O | H | H | $CH_2CH_2NHP(O)(OCH_3)_2$ | H | $OCH_3$ | $OCH_3$ | CH | |
| O | O | H | H | $CH_2CH_2NHP(S)(OCH_3)_2$ | H | $OCH_3$ | $OCH_3$ | CH | |
| O | O | H | H | $CH_2NO_2$ | H | Cl | $CH_3$ | CH | |
| O | O | H | H | $CH_2NO_2$ | H | $OCH_3$ | $OCH_3$ | N | |
| O | O | H | H | $CH_2CH_2NO_2$ | H | $CH_3$ | $CH_3$ | CH | |
| O | O | H | H | $CH_2CH_2NO_2$ | H | Cl | $OCH_3$ | CH | |
| O | O | H | H | $CH_2CH_2NO_2$ | H | $OCH_3$ | $OCH_3$ | N | |
| O | O | H | H | $CH_2C(O)C_6H_5$ | H | $CH_3$ | $CH_3$ | CH | |
| O | O | H | H | $CH_2C(O)C_6H_5$ | H | $CH_3$ | $OCH_3$ | CH | |
| O | O | H | H | $CH_2C(O)\ C_6H_5$ | H | $OCH_3$ | $OCH_3$ | CH | 193-199 |
| O | O | H | H | $CH_2C(O)C_6H_5$ | H | Cl | $OCH_3$ | CH | |
| O | O | H | H | $CH_2C(O)C_6H_5$ | H | $CH_3$ | $OCH_3$ | N | |
| O | O | H | H | $CH_2C(O)C_6H_5$ | H | $OCH_3$ | $OCH_3$ | N | |
| O | O | H | H | $CH_2CH_2C(O)C_6H_5$ | H | $CH_3$ | $OCH_3$ | CH | |
| O | O | H | H | $CH_2CH_2C(O)C_6H_5$ | H | $OCH_3$ | $OCH_3$ | CH | |
| O | O | H | H | $CH_2CO_2CH_2CH_3$ | H | $CH_3$ | $CH_3$ | CH | |
| O | O | H | H | $CH_2CO_2CH_2CH_3$ | H | Cl | $OCH_3$ | CH | 164-166 |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CO_2CH_2CH_3$ | H | $CH_3$ | $OCH_3$ | N | |
| O | O | H | H | $CH_2CO_2CH_2CH_3$ | H | $OCH_3$ | $OCH_3$ | N | |
| O | O | H | H | $CH_2CO_2C_6H_5$ | H | $CH_3$ | $CH_3$ | CH | |
| O | O | H | H | $CH_2CO_2C_6H_5$ | H | $CH_3$ | $OCH_3$ | CH | |
| O | O | H | H | $CH_2CO_2C_6H_5$ | H | $OCH_3$ | $OCH_3$ | CH | |
| O | O | H | H | $CH_2CO_2C_6H_5$ | H | Cl | $OCH_3$ | CH | |
| O | O | H | H | $CH_2CO_2C_6H_5$ | H | $CH_3$ | $OCH_3$ | N | |
| O | O | H | H | $CH_2CO_2C_6H_5$ | H | $OCH_3$ | $OCH_3$ | N | |
| O | O | H | H | $CH_2CH_2CO_2CH_2CH_3$ | H | $CH_3$ | $OCH_3$ | CH | |
| O | O | H | H | $CH_2CH_2CO_2CH_2CH_3$ | H | $OCH_3$ | $OCH_3$ | CH | |
| O | O | H | H | $CH_2CH_2CO_2C_6H_5$ | H | $CH_3$ | $OCH_3$ | CH | |
| O | O | H | H | $CH_2CH_2CO_2C_6H_5$ | H | $OCH_3$ | $OCH_3$ | CH | |
| O | O | H | H | $CH_2CO_2C(CH_3)_3$ | H | $CH_3$ | $CH_3$ | CH | |
| O | O | H | H | $CH_2CO_2C(CH_3)_3$ | H | $CH_3$ | $OCH_3$ | CH | 168-172 |
| O | O | H | H | $CH_2CO_2C(CH_3)_3$ | H | $OCH_3$ | $OCH_3$ | CH | 178-180 |
| O | O | H | H | $CH_2CO_2C(CH_3)_3$ | H | Cl | $OCH_3$ | CH | 156-159 |
| O | O | H | H | $CH_2CO_2C(CH_3)_3$ | H | $CH_3$ | $OCH_3$ | N | |
| O | O | H | H | $CH_2CO_2C(CH_3)_3$ | H | $OCH_3$ | $OCH_3$ | N | |
| O | O | H | H | $CH_2CO_2H$ | H | $CH_3$ | $CH_3$ | CH | |
| O | O | H | H | $CH_2CO_2H$ | H | $CH_3$ | $OCH_3$ | CH | |
| O | O | H | H | $CH_2CO_2H$ | H | $OCH_3$ | $OCH_3$ | CH | |
| O | O | H | H | $CH_2CO_2H$ | H | Cl | $OCH_3$ | CH | |
| O | O | H | H | $CH_2CO_2H$ | H | $CH_3$ | $OCH_3$ | N | |
| O | O | H | H | $CH_2CO_2H$ | H | $OCH_3$ | $OCH_3$ | N | |
| O | O | H | H | $CH_2CH(CH_3)CN$ | H | $CH_3$ | $CH_3$ | CH | |
| O | O | H | H | $CH_2CH(CH_3)CN$ | H | Cl | $OCH_3$ | CH | |
| O | O | H | H | $CH_2CH(CH_3)CN$ | H | $CH_3$ | $OCH_3$ | N | |
| O | O | H | H | $CH_2CH(CH_3)CN$ | H | $OCH_3$ | $OCH_3$ | N | |
| O | O | H | H | $CH_2C(O)NHCH_3$ | H | $CH_3$ | $OCH_3$ | CH | |
| O | O | H | H | $CH_2C(O)NHCH_3$ | H | $OCH_3$ | $OCH_3$ | CH | |
| O | O | H | H | $CH_2C(O)N(CH_3)_2$ | H | $CH_3$ | $OCH_3$ | CH | |
| O | O | H | H | $CH_2C(O)N(CH_3)_2$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | (1-naphthyl)methyl | H | $CH_3$ | $OCH_3$ | CH | 163-169 |
| O | O | H | H | (1-naphthyl)methyl | H | $OCH_3$ | $OCH_3$ | CH | 220-228 |
| O | O | H | H | (2-naphthyl)methyl | H | $CH_3$ | $OCH_3$ | CH | |
| O | O | H | H | (2-naphthyl)methyl | H | $OCH_3$ | $OCH_3$ | CH | |
| O | O | H | H | (2-methylpyridinium)-methyl iodide | H | $CH_3$ | $OCH_3$ | CH | |
| O | O | H | H | (2-methylpyridinium)-methyl iodide | H | $OCH_3$ | $OCH_3$ | CH | |
| O | O | H | H | pyridin-2-ylmethyl | H | $CH_3$ | $CH_3$ | CH | |
| O | O | H | H | pyridin-2-ylmethyl | H | $CH_3$ | $OCH_3$ | CH | |
| O | O | H | H | pyridin-2-ylmethyl | H | $OCH_3$ | $OCH_3$ | CH | 172-174 |
| O | O | H | H | pyridin-2-ylmethyl | H | Cl | $OCH_3$ | CH | |
| O | O | H | H | pyridin-2-ylmethyl | H | $CH_3$ | $OCH_3$ | N | |
| O | O | H | H | pyridin-2-ylmethyl | H | $OCH_3$ | $OCH_3$ | N | |
| O | O | H | H | pyridin-3-ylmethyl | H | $CH_3$ | $CH_3$ | CH | |
| O | O | H | H | pyridin-3-ylmethyl | H | $CH_3$ | $OCH_3$ | CH | |
| O | O | H | H | pyridin-3-ylmethyl | H | $OCH_3$ | $OCH_3$ | CH | |
| O | O | H | H | pyridin-3-ylmethyl | H | Cl | $OCH_3$ | CH | |
| O | O | H | H | pyridin-3-ylmethyl | H | $CH_3$ | $OCH_3$ | N | |
| O | O | H | H | pyridin-3-ylmethyl | H | $OCH_3$ | $OCH_3$ | N | |
| O | O | H | H | pyridin-4-ylmethyl | H | $CH_3$ | $CH_3$ | CH | |
| O | O | H | H | pyridin-4-ylmethyl | H | $CH_3$ | $OCH_3$ | CH | |
| O | O | H | H | pyridin-4-ylmethyl | H | $OCH_3$ | $OCH_3$ | CH | |
| O | O | H | H | pyridin-4-ylmethyl | H | Cl | $OCH_3$ | CH | |
| O | O | H | H | pyridin-4-ylmethyl | H | $CH_3$ | $OCH_3$ | N | |
| O | O | H | H | pyridin-4-ylmethyl | H | $OCH_3$ | $OCH_3$ | N | |
| O | O | H | H | thien-2-ylmethyl | H | $CH_3$ | $CH_3$ | CH | |
| O | O | H | H | thien-2-ylmethyl | H | $CH_3$ | $OCH_3$ | CH | |
| O | O | H | H | thien-2-ylmethyl | H | $OCH_3$ | $OCH_3$ | CH | |
| O | O | H | H | thien-2-ylmethyl | H | Cl | $OCH_3$ | CH | |
| O | O | H | H | thien-2-ylmethyl | H | $CH_3$ | $OCH_3$ | N | |
| O | O | H | H | thien-2-ylmethyl | H | $OCH_3$ | $OCH_3$ | N | |
| O | O | H | H | thien-3-ylmethyl | H | $CH_3$ | $CH_3$ | CH | |

Table 1a (continued)

| W | n | R | $R_1$ | $R_2$ | $R_3$ | X | Y | Z | m.p. (°C) |
|---|---|---|---|---|---|---|---|---|---|
| O | O | H | H | thien-3-ylmethyl | H | $CH_3$ | $OCH_3$ | CH | |
| O | O | H | H | thien-3-ylmethyl | H | $OCH_3$ | $OCH_3$ | CH | |
| O | O | H | H | thien-3-ylmethyl | H | Cl | $OCH_3$ | CH | |
| O | O | H | H | thien-3-ylmethyl | H | $CH_3$ | $OCH_3$ | N | |
| O | O | H | H | thien-3-ylmethyl | H | $OCH_3$ | $OCH_3$ | N | |
| O | O | H | H | (5-methylthien-2-yl)methyl | H | $CH_3$ | $OCH_3$ | CH | |
| O | O | H | H | (5-methylthien-2-yl)methyl | H | $OCH_3$ | $OCH_3$ | CH | |
| O | O | H | H | (5-chlorothien-2-yl)methyl | H | $CH_3$ | $OCH_3$ | CH | |
| O | O | H | H | (5-chlorothien-2-yl)methyl | H | $OCH_3$ | $OCH_3$ | CH | |
| O | O | H | H | (5-cyanothien-2-yl)methyl | H | $CH_3$ | $OCH_3$ | CH | |
| O | O | H | H | (5-cyanothien-2-yl)methyl | H | $OCH_3$ | $OCH_3$ | CH | |
| O | O | H | H | furan-2-ylmethyl | H | $CH_3$ | $OCH_3$ | CH | |
| O | O | H | H | furan-2-ylmethyl | H | $OCH_3$ | $OCH_3$ | CH | |
| O | O | H | H | (5-methylfuran-2-yl)methyl | H | $CH_3$ | $OCH_3$ | CH | |
| O | O | H | H | (5-methylfuran-2-yl)methyl | H | $OCH_3$ | $OCH_3$ | CH | |
| O | O | H | H | furan-3-ylmethyl | H | $CH_3$ | $OCH_3$ | CH | |
| O | O | H | H | furan-3-ylmethyl | H | $OCH_3$ | $OCH_3$ | CH | |
| O | O | H | H | (4.5-dihydrofuran-2-yl)methyl | H | $CH_3$ | $OCH_3$ | CH | |
| O | O | H | H | (4.5-dihydrofuran-2-yl)methyl | H | $OCH_3$ | $OCH_3$ | CH | |
| O | O | H | H | (1H-pyrrol-1-yl)methyl | H | $CH_3$ | $OCH_3$ | CH | |
| O | O | H | H | (1H-pyrrol-1-yl)methyl | H | $OCH_3$ | $OCH_3$ | CH | |
| O | O | H | H | (1H-pyrrol-2-yl)methyl | H | $CH_3$ | $OCH_3$ | CH | |
| O | O | H | H | (1H-pyrrol-2-yl)methyl | H | $OCH_3$ | $OCH_3$ | CH | |
| O | O | H | H | (1-methyl-1H-pyrrol-3-yl)methyl | H | $CH_3$ | $OCH_3$ | CH | |
| O | O | H | H | (1-methyl-1H-pyrrol-3-yl)methyl | H | $OCH_3$ | $OCH_3$ | CH | |
| O | O | H | H | oxazol-2-ylmethyl | H | $CH_3$ | $OCH_3$ | CH | |
| O | O | H | H | oxazol-2-ylmethyl | H | $OCH_3$ | $OCH_3$ | CH | |
| O | O | H | H | thiazol-2-ylmethyl | H | $CH_3$ | $OCH_3$ | CH | |
| O | O | H | H | thiazol-2-ylmethyl | H | $OCH_3$ | $OCH_3$ | CH | |
| O | O | H | H | (4.5-dihydro-4.4-dimethyl-oxazol-2-yl)methyl | H | $CH_3$ | $OCH_3$ | CH | |
| O | O | H | H | (4.5-dihydro-4.4-dimethyl-oxazol-2-yl)methyl | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | isoxazol-3-ylmethyl | H | $CH_3$ | $OCH_3$ | CH | |
| O | O | H | H | iosxazol-3-ylmethyl | H | $OCH_3$ | $OCH_3$ | CH | |
| O | O | H | H | (1,2,4-oxadiazol-3-yl)methyl | H | $CH_3$ | $OCH_3$ | CH | |
| O | O | H | H | (1,2,4-oxadiazol-3-yl)methyl | H | $OCH_3$ | $OCH_3$ | CH | |
| O | O | H | H | (5-methyl-1,2,4-oxadiazol-3-yl)methyl | H | $CH_3$ | $OCH_3$ | CH | |
| O | O | H | H | (5-methyl-1,2,4-oxadiazol-3-yl)methyl | H | $OCH_3$ | $OCH_3$ | CH | |
| O | O | H | H | (5-methyl-1,3,4-oxadiazol-2-yl)methyl | H | $CH_3$ | $OCH_3$ | CH | |
| O | O | H | H | (5-methyl-1,3,4-oxadiazol-2-yl)methyl | H | $OCH_3$ | $OCH_3$ | CH | |
| O | O | H | H | 1H-pyrazol-1-ylmethyl | H | $CH_3$ | $OCH_3$ | CH | |
| O | O | H | H | 1H-pyrazol-1-ylmethyl | H | $OCH_3$ | $OCH_3$ | CH | |
| O | O | H | H | 4H-pyrazol-1-ylmethyl | H | $CH_3$ | $OCH_3$ | CH | |
| O | O | H | H | 1H-1,2,4,triazol-1-ylmethyl | H | $CH_3$ | $OCH_3$ | CH | |
| O | O | H | H | 1H-1,2,4,triazol-1-ylmethyl | H | $OCH_3$ | $OCH_3$ | CH | |
| O | O | H | H | (1-methyl-1H-imidazol-2-yl)methyl | H | $CH_3$ | $OCH_3$ | CH | |
| O | O | H | H | (1-methyl-1H-imidazol-2-yl)methyl | H | $OCH_3$ | $OCH_3$ | CH | |
| O | O | H | H | (1-methyl-1H-1,2,4-triazol-5-yl)methyl | H | $CH_3$ | $OCH_3$ | CH | |
| O | O | H | H | (1-methyl-1H-1,2,4-triazol-5-yl)methyl | H | $OCH_3$ | $OCH_3$ | CH | |
| O | O | H | H | (4,5-dihydro-1,4,4-trimethyl-1H-imidazol-2-yl)methyl | H | $CH_3$ | $OCH_3$ | CH | |
| O | O | H | H | (4,5-dihydro-1,4,4-trimethyl-1H-imidazol-2-yl)methyl | H | $OCH_3$ | $OCH_3$ | CH | |
| O | O | H | H | (1,2,5-thiadiazol-3-yl)methyl | H | $CH_3$ | $OCH_3$ | CH | |
| O | O | H | H | (1,2,5-thiadiazol-3-yl)methyl | H | $OCH_3$ | $OCH_3$ | CH | |
| O | O | H | H | 4-(2,6-dimethylmorpholino)methyl | H | $CH_3$ | $OCH_3$ | CH | |
| O | O | H | H | 4-(2,6-dimethylmorpholino)methyl | H | $OCH_3$ | $OCH_3$ | CH | |
| O | O | H | H | (4,6-dimethoxy-1,3,5-triazin-2-yl)methyl | H | $CH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | (4,6-dimethoxy-1,3,5-triazin-2-yl)methyl | H | $OCH_3$ | $OCH_3$ | CH | |
| O | O | H | H | (4-dimethyamino-6-methylthio 1,3,5-triazin-2-yl)methyl | H | $CH_3$ | $OCH_3$ | CH | |
| O | O | H | H | (4-dimethyamino-6-methylthio 1,3,5-triazin-2-yl)methyl | H | $OCH_3$ | $OCH_3$ | CH | |
| O | O | H | H | (cyclopentanon-2-yl)methyl | H | $CH_3$ | $CH_3$ | CH | |
| O | O | H | H | (cyclopentanon-2-yl)methyl | H | $CH_3$ | $OCH_3$ | CH | |
| O | O | H | H | (cyclopentanon-2-yl)methyl | H | $OCH_3$ | $OCH_3$ | CH | |
| O | O | H | H | (cyclopentanon-2-yl)methyl | H | Cl | $OCH_3$ | CH | |
| O | O | H | H | (cyclopentanon-2-yl)methyl | H | $CH_3$ | $OCH_3$ | N | |
| O | O | H | H | (cyclopentanon-2-yl)methyl | H | $OCH_3$ | $OCH_3$ | N | |
| O | O | H | H | γ-butyryllactone-2-yl)-methyl | H | $CH_3$ | $CH_3$ | CH | |
| O | O | H | H | γ-butyryllactone-2-yl)-methyl | H | $CH_3$ | $OCH_3$ | CH | |
| O | O | H | H | γ-butyryllactone-2-yl)-methyl | H | $OCH_3$ | $OCH_3$ | CH | |
| O | O | H | H | γ-butyryllactone-2-yl)-methyl | H | Cl | $OCH_3$ | CH | |
| O | O | H | H | γ-butyryllactone-2-yl)-methyl | H | $CH_3$ | $OCH_3$ | N | |
| O | O | H | H | γ-butyryllactone-2-yl)-methyl | H | $OCH_3$ | $OCH_3$ | N | |
| O | O | H | H | γ-butyryllactone-3-yl)-methyl | H | $CH_3$ | $CH_3$ | CH | |
| O | O | H | H | γ-butyryllactone-3-yl)-methyl | H | $CH_3$ | $OCH_3$ | CH | |
| O | O | H | H | γ-butyryllactone-3-yl)-methyl | H | $OCH_3$ | $OCH_3$ | CH | |
| O | O | H | H | γ-butyryllactone-3-yl)-methyl | H | Cl | $OCH_3$ | CH | |
| O | O | H | H | γ-butyryllactone-3-yl)-methyl | H | $CH_3$ | $OCH_3$ | N | |
| O | O | H | H | γ-butyryllactone-3-yl)-methyl | H | $OCH_3$ | $OCH_3$ | N | |
| O | O | H | H | cyclohexanon-2-yl)methyl | H | $CH_3$ | $CH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | (cyclohexanon-2-yl)methyl | H | $CH_3$ | $OCH_3$ | CH | |
| O | O | H | H | (cyclohexanon-2-yl)methyl | H | $OCH_3$ | $OCH_3$ | CH | |
| O | O | H | H | (cyclohexanon-2-yl)methyl | H | Cl | $OCH_3$ | CH | |
| O | O | H | H | (cyclohexanon-2-yl)methyl | H | $CH_3$ | $OCH_3$ | N | |
| O | O | H | H | (cyclohexanon-2-yl)methyl | H | $OCH_3$ | $OCH_3$ | N | |
| O | O | H | H | 2-chloro-6-fluorobenzyl | H | $OCH_3$ | $OCH_3$ | CH | |
| O | O | H | H | 4-methylthiobenzyl | H | $OCH_3$ | $OCH_3$ | CH | |
| O | O | H | H | 4-methylsulfonylbenzyl | H | $OCH_3$ | $OCH_3$ | CH | |
| O | O | H | H | 1,1'-biphenylmethyl | H | $OCH_3$ | $OCH_3$ | CH | |
| O | O | H | H | 3-phenoxybenzyl | H | $OCH_3$ | $OCH_3$ | CH | |
| O | O | H | H | 2-[4-(2,2-dimethyl-1,3-dioxolan-2-yl)methoxy]ethyl | H | $OCH_3$ | $OCH_3$ | CH | |
| O | O | H | H | 4-(2,2-dimethyl-1,3-dioxolan-2-yl)methoxy | H | $OCH_3$ | $OCH_3$ | CH | |
| O | O | H | H | (2-methyl-1,3-dioxolan-2-yl)methyl | H | $CH_3$ | $OCH_3$ | CH | |
| O | O | H | H | (2-methyl-1,3-dioxolan-2-yl)methyl | H | $OCH_3$ | $OCH_3$ | CH | |
| O | O | H | H | 2-(2-methyl-1,3-dioxolan-2-yl)ethyl | H | $CH_3$ | $OCH_3$ | CH | |
| O | O | H | H | 2-(2-methyl-1,3-dioxolan-2-yl)ethyl | H | $OCH_3$ | $OCH_3$ | CH | |
| O | O | H | H | (1,3-dioxolan-2-yl)methyl | H | $CH_3$ | $OCH_3$ | CH | |
| O | O | H | H | (1,3-dioxolan-2-yl)methyl | H | $OCH_3$ | $OCH_3$ | CH | |
| O | O | H | H | 2-(1,3-dioxolan-2-yl)methyl | H | $CH_3$ | $OCH_3$ | CH | |
| O | O | H | H | 2-(1,3-dioxolan-2-yl)methyl | H | $OCH_3$ | $OCH_3$ | CH | |
| O | O | H | H | (1,3-dioxoan-2-yl)methyl | H | $CH_3$ | $OCH_3$ | CH | |
| O | O | H | H | (1,3-dioxoan-2-yl)methyl | H | $OCH_3$ | $OCH_3$ | CH | |
| O | O | H | H | 2-(2-tetrahydropyranyloxy)ethyl | H | $CH_3$ | $OCH_3$ | CH | |
| O | O | H | H | 2-(2-tetrahydropyranyloxy)ethyl | H | $OCH_3$ | $OCH_3$ | CH | |
| O | O | H | H | $CH_2C(CH_3)=NOH$ | H | $CH_3$ | $OCH_3$ | CH | |
| O | O | H | H | $CH_2C(CH_3)=NOH$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CHC(CH_3)=NOH$ | H | $CH_3$ | $OCH_3$ | CH | |
| O | O | H | H | $CH_2CHC(CH_3)=NOH$ | H | $OCH_3$ | $OCH_3$ | CH | |
| O | O | H | H | $CH_2C(CH_3)(CN)OSi(CH_3)_3$ | H | $OCH_3$ | $OCH_3$ | CH | |
| O | O | H | H | $CH(C_6H_5)_2$ | H | $CH_3$ | $OCH_3$ | CH | |
| O | O | H | H | $CH(C_6H_5)_2$ | H | $OCH_3$ | $OCH_3$ | CH | |
| O | O | H | H | $CH(CN)_2$ | H | $CH_3$ | $OCH_3$ | CH | |
| O | O | H | H | $CH(CN)_2$ | H | $OCH_3$ | $OCH_3$ | CH | |
| O | O | H | H | C(Cl)=CHCl | H | $CH_3$ | $OCH_3$ | CH | |
| O | O | H | H | C(Cl)=CHCl | H | $OCH_3$ | $OCH_3$ | CH | |
| O | O | H | H | $CH=CHC(O)CH_3$ | H | $OCH_3$ | $OCH_3$ | CH | |
| O | O | H | H | CH=CHCN | H | $OCH_3$ | $OCH_3$ | CH | |
| O | O | H | H | $CH=CHCO_2CH_3$ | H | $OCH_3$ | $OCH_3$ | CH | |
| O | O | H | H | $CH=CHCH_3$ | H | $CH_3$ | $OCH_3$ | CH | |
| O | O | H | H | $CH=CHCH_3$ | H | $OCH_3$ | $OCH_3$ | CH | |
| O | O | H | H | $CH=CHCH_3$ | H | $CH_3$ | $OCH_3$ | CH | |
| O | O | H | H | $CH=CHCH_3$ | H | $OCH_3$ | $OCH_3$ | CH | |
| O | O | H | H | 2-epoxypropyl | H | $CH_3$ | $CH_3$ | CH | |
| O | O | H | H | 2-epoxypropyl | H | Cl | $OCH_3$ | CH | |
| O | O | H | H | 2-epoxypropyl | H | $CH_3$ | $OCH_3$ | N | |
| O | O | H | H | 2-epoxypropyl | H | $OCH_3$ | $OCH_3$ | N | |
| O | O | H | H | 2-epoxybutyl | H | $CH_3$ | $CH_3$ | CH | |
| O | O | H | H | 2-epoxybutyl | H | $CH_3$ | $OCH_3$ | CH | |
| O | O | H | H | 2-epoxybutyl | H | $OCH_3$ | $OCH_3$ | CH | |
| O | O | H | H | 2-epoxybutyl | H | Cl | $OCH_3$ | CH | |
| O | O | H | H | 2-epoxybutyl | H | $CH_3$ | $OCH_3$ | N | |
| O | O | H | H | 2-epoxybutyl | H | $OCH_3$ | $OCH_3$ | N | |
| O | O | H | H | (4-t-butylcyclohexyl)methyl | H | $OCH_3$ | $OCH_3$ | CH | |
| O | O | H | H | (4-chlorocyclopentyl)methyl | H | $OCH_3$ | $OCH_3$ | CH | |
| O | O | H | H | $C_6H_5$ | H | $CH_3$ | $CH_3$ | CH | |
| O | O | H | H | $C_6H_5$ | H | $CH_3$ | $OCH_3$ | CH | |
| O | O | H | H | $C_6H_5$ | H | $OCH_3$ | $OCH_3$ | CH | |
| O | O | H | H | $C_6H_5$ | H | Cl | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | (cyclohexanon-2-yl)methyl | H | $CH_3$ | $OCH_3$ | CH | |
| O | O | H | H | (cyclohexanon-2-yl)methyl | H | $OCH_3$ | $OCH_3$ | CH | |
| O | O | H | H | (cyclohexanon-2-yl)methyl | H | Cl | $OCH_3$ | CH | |
| O | O | H | H | (cyclohexanon-2-yl)methyl | H | $CH_3$ | $OCH_3$ | N | |
| O | O | H | H | (cyclohexanon-2-yl)methyl | H | $OCH_3$ | $OCH_3$ | N | |
| O | O | H | H | 2-chloro-6-fluorobenzyl | H | $OCH_3$ | $OCH_3$ | CH | |
| O | O | H | H | 4-methylthiobenzyl | H | $OCH_3$ | $OCH_3$ | CH | |
| O | O | H | H | 4-methylsulfonylbenzyl | H | $OCH_3$ | $OCH_3$ | CH | |
| O | O | H | H | 1,1'-biphenylmethyl | H | $OCH_3$ | $OCH_3$ | CH | |
| O | O | H | H | 3-phenoxybenzyl | H | $OCH_3$ | $OCH_3$ | CH | |
| O | O | H | H | 2-[4-(2,2-dimethyl-1,3-dioxolan-2-yl)methoxy]ethyl | H | $OCH_3$ | $OCH_3$ | CH | |
| O | O | H | H | 4-(2,2-dimethyl-1,3-dioxolan-2-yl)methoxy | H | $OCH_3$ | $OCH_3$ | CH | |
| O | O | H | H | (2-methyl-1,3-dioxolan-2-yl)methyl | H | $CH_3$ | $OCH_3$ | CH | |
| O | O | H | H | (2-methyl-1,3-dioxolan-2-yl)methyl | H | $OCH_3$ | $OCH_3$ | CH | |
| O | O | H | H | 2-(2-methyl-1,3-dioxolan-2-yl)ethyl | H | $CH_3$ | $OCH_3$ | CH | |
| O | O | H | H | 2-(2-methyl-1,3-dioxolan-2-yl)ethyl | H | $OCH_3$ | $OCH_3$ | CH | |
| O | O | H | H | (1,3-dioxolan-2-yl)methyl | H | $CH_3$ | $OCH_3$ | CH | |
| O | O | H | H | (1,3-dioxolan-2-yl)methyl | H | $OCH_3$ | $OCH_3$ | CH | |
| O | O | H | H | 2-(1,3-dioxolan-2-yl)methyl | H | $CH_3$ | $OCH_3$ | CH | |
| O | O | H | H | 2-(1,3-dioxolan-2-yl)methyl | H | $OCH_3$ | $OCH_3$ | CH | |
| O | O | H | H | (1,3-dioxoan-2-yl)methyl | H | $CH_3$ | $OCH_3$ | CH | |
| O | O | H | H | (1,3-dioxoan-2-yl)methyl | H | $OCH_3$ | $OCH_3$ | CH | |
| O | O | H | H | 2-(2-tetrahydropyranyloxy)ethyl | H | $CH_3$ | $OCH_3$ | CH | |
| O | O | H | H | 2-(2-tetrahydropyranyloxy)ethyl | H | $OCH_3$ | $OCH_3$ | CH | |
| O | O | H | H | $CH_2C(CH_3)=NOH$ | H | $CH_3$ | $OCH_3$ | CH | |
| O | O | H | H | $CH_2C(CH_3)=NOH$ | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $C_6H_5$ | H | $CH_3$ | $OCH_3$ | N | |
| O | O | H | H | $C_6H_5$ | H | $OCH_3$ | $OCH_3$ | N | |
| O | O | H | H | p-$NO_2C_6H_4$ | H | $CH_3$ | $OCH_3$ | CH | |
| O | O | H | H | p-$NO_2C_6H_4$ | H | $OCH_3$ | $OCH_3$ | CH | 193-194 |
| O | O | H | H | p-$CNC_6H_4$ | H | $CH_3$ | $OCH_3$ | CH | |
| O | O | H | H | p-$CNC_6H_4$ | H | $OCH_3$ | $OCH_3$ | CH | |
| O | O | H | H | p-$CH_3SO_2C_6H_4$ | H | $CH_3$ | $OCH_3$ | CH | |
| O | O | H | H | p-$CH_3SO_2C_6H_4$ | H | $OCH_3$ | $OCH_3$ | CH | |
| O | O | H | H | p-$ClC_6H_4$ | H | $CH_3$ | $OCH_3$ | CH | |
| O | O | H | H | p-$ClC_6H_4$ | H | $OCH_3$ | $OCH_3$ | CH | |
| O | O | H | H | 4-pyrimidinyl | H | $CH_3$ | $OCH_3$ | CH | |
| O | O | H | H | 4-pyrimidinyl | H | $OCH_3$ | $OCH_3$ | CH | |
| O | O | H | H | 2-pyrimidinyl | H | $CH_3$ | $OCH_3$ | CH | |
| O | O | H | H | 2-pyrimidinyl | H | $OCH_3$ | $OCH_3$ | CH | |
| O | O | H | H | 2-thienyl | H | $OCH_3$ | $OCH_3$ | CH | |
| O | O | H | H | 3-thienyl | H | $OCH_3$ | $OCH_3$ | CH | |
| O | O | H | H | 2-thiazolyl | H | $OCH_3$ | $OCH_3$ | CH | |
| O | O | H | H | 1-methyl-1H-imidazol-2-yl | H | $OCH_3$ | $CH_3$ | CH | |
| O | O | H | H | 2-oxazolyl | H | $OCH_3$ | $OCH_3$ | CH | |
| O | O | H | H | 2-(5-nitrothienyl) | H | $CH_3$ | $OCH_3$ | CH | |
| O | O | H | H | pyrimidin-2-yl | H | $CH_3$ | $OCH_3$ | CH | |
| O | O | H | H | 4.6-dimethoxypyrimidin-2-yl | H | $CH_3$ | $OCH_3$ | CH | |
| O | O | H | H | 2-cyclopentanonyl | H | $CH_3$ | $OCH_3$ | CH | |
| O | O | H | H | 2-cyclopentanonyl | H | $OCH_3$ | $OCH_3$ | CH | |
| O | O | H | H | 3-cyclopentanonyl | H | $CH_3$ | $OCH_3$ | CH | |
| O | O | H | H | 3-cyclopentanonyl | H | $OCH_3$ | $OCH_3$ | CH | |
| O | O | H | H | 2-cyclohexanonyl | H | $CH_3$ | $OCH_3$ | CH | |
| O | O | H | H | 2-cyclohexanonyl | H | $OCH_3$ | $OCH_3$ | CH | |
| O | O | H | H | 3-cyclohexanonyl | H | $CH_3$ | $OCH_3$ | CH | |
| O | O | H | H | 3-cyclohexanonyl | H | $OCH_3$ | $OCH_3$ | CH | |
| O | O | H | H | γ-butyrolactone-2-yl | H | $CH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | γ-butyrolactone-2-yl | H | $OCH_3$ | $OCH_3$ | CH | 206-211 |
| O | O | H | H | γ-butyrolactone-3-yl | H | $CH_3$ | $OCH_3$ | CH | |
| O | O | H | H | γ-butyrolactone-3-yl | H | $OCH_3$ | $OCH_3$ | CH | |
| O | O | H | H | $SCH_3$ | H | $CH_3$ | $OCH_3$ | CH | |
| O | O | H | H | $SCH_3$ | H | $OCH_3$ | $OCH_3$ | CH | |
| O | O | H | H | $SCH_2CH_2CH_3$ | H | $CH_3$ | $OCH_3$ | CH | |
| O | O | H | H | $SCH_2CH_2CH_3CH$ | H | $OCH_3$ | $OCH_3$ | CH | |
| O | O | H | H | $S(CH_2)_9CH$ | H | $OCH_3$ | $OCH_3$ | CH | |
| O | O | H | H | $SCCl_3$ | H | $CH_3$ | $CH_3$ | CH | |
| O | O | H | H | $SCCl_3$ | H | $CH_3$ | $OCH_3$ | CH | |
| O | O | H | H | $SCCl_3$ | H | $OCH_3$ | $OCH_3$ | CH | |
| O | O | H | H | $SCCl_3$ | H | Cl | $OCH_3$ | CH | |
| O | O | H | H | $SCCl_3$ | H | $CH_3$ | $OCH_3$ | N | |
| O | O | H | H | $SCCl_3$ | H | $OCH_3$ | $OCH_3$ | N | |
| O | O | H | H | $SC_6H_5$ | H | $CH_3$ | $OCH_3$ | CH | |
| O | O | H | H | $SC_6H_5$ | H | $OCH_3$ | $OCH_3$ | CH | |
| O | O | H | H | 4-nitrophenylthio | H | $CH_3$ | $OCH_3$ | CH | |
| O | O | H | H | 4-nitrophenylthio | H | $OCH_3$ | $OCH_3$ | CH | |
| O | O | H | H | $SO_2CH_3$ | H | $CH_3$ | $CH_3$ | CH | |
| O | O | H | H | $SO_2CH_3$ | H | Cl | $OCH_3$ | CH | |
| O | O | H | H | $SO_2CH_3$ | H | $CH_3$ | $OCH_3$ | N | |
| O | O | H | H | $SO_2CH_3$ | H | $OCH_3$ | $OCH_3$ | N | |
| O | O | H | H | $SO_2CH_2CH_3$ | H | $CH_3$ | $OCH_3$ | CH | |
| O | O | H | H | $SO_2CH_2CH_3$ | H | $OCH_3$ | $OCH_3$ | CH | |
| O | O | H | H | $SO_2CH(CH_3)_2$ | H | $CH_3$ | $OCH_3$ | CH | |
| O | O | H | H | $SO_2CH(CH_3)_2$ | H | $OCH_3$ | $OCH_3$ | CH | |
| O | O | H | H | phenylsulfonyl | H | $CH_3$ | $OCH_3$ | CH | |
| O | O | H | H | phenylsulfonyl | H | $OCH_3$ | $OCH_3$ | CH | |
| O | O | H | H | 4-toluenesulfonyl | H | $OCH_3$ | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $OCH_3$ | H | $CH_3$ | $CH_3$ | CH | |
| O | O | H | H | $OCH_3$ | H | Cl | $OCH_3$ | CH | |
| O | O | H | H | $OCH_3$ | H | $CH_3$ | $OCH_3$ | N | |
| O | O | H | H | $OCH_3$ | H | $OCH_3$ | $OCH_3$ | N | |
| O | O | H | H | OH | H | $CH_3$ | $OCH_3$ | N | |
| O | O | H | H | OH | H | $OCH_3$ | $OCH_3$ | N | |
| O | O | H | H | $OCH_2CH_2CH_2CH_3$ | H | $CH_3$ | $OCH_3$ | N | |
| O | O | H | H | $OCH_2CH_2CH_2CH_3$ | H | $OCH_3$ | $OCH_3$ | N | |
| O | O | H | H | $C(O)CH_3$ | H | $CH_3$ | $CH_3$ | CH | 227-228 |
| O | O | H | H | $C(O)CH_3$ | H | Cl | $OCH_3$ | CH | 218-220 |
| O | O | H | H | $C(O)CH_3$ | H | Br | $OCH_3$ | CH | |
| O | O | H | H | $C(O)CH_3$ | H | $CH_3$ | $OCH_3$ | N | 195-197 |
| O | O | H | H | $C(O)CH_3$ | H | $OCH_3$ | $OCH_3$ | N | 199-201 |
| O | O | H | H | $C(O)CH_2CH_3$ | H | $CH_3$ | $CH_3$ | CH | 190-191 |
| O | O | H | H | $C(O)CH_2CH_3$ | H | Cl | $OCH_3$ | CH | 203-205 |
| O | O | H | H | $C(O)CH_2CH_3$ | H | $CH_3$ | $OCH_3$ | N | 185-186 |
| O | O | H | H | $C(O)CH_2CH_3$ | H | $OCH_3$ | $OCH_3$ | N | 189-190 |
| O | O | H | H | $C(O)CH_2CH_2CH_3$ | H | $CH_3$ | $CH_3$ | CH | 196-198 |
| O | O | H | H | $C(O)CH_2CH_2CH_3$ | H | Cl | $OCH_3$ | CH | 203-205 |
| O | O | H | H | $C(O)CH_2CH_2CH_3$ | H | $CH_3$ | $OCH_3$ | N | 185-186 |
| O | O | H | H | $C(O)CH_2CH_2CH_3$ | H | $OCH_3$ | $OCH_3$ | N | 170-171 |
| O | O | H | H | $C(O)C_6H_5$ | H | $CH_3$ | $CH_3$ | CH | |
| O | O | H | H | $C(O)C_6H_5$ | H | $CH_3$ | $OCH_3$ | CH | |
| O | O | H | H | $C(O)C_6H_5$ | H | $OCH_3$ | $OCH_3$ | CH | |
| O | O | H | H | $C(O)C_6H_5$ | H | Cl | $OCH_3$ | CH | |
| O | O | H | H | $C(O)C_6H_5$ | H | $CH_3$ | $OCH_3$ | CH | |
| O | O | H | H | $C(O)C_6H_5$ | H | $OCH_3$ | $OCH_3$ | CH | 88, dec. |
| O | O | H | H | $C(O)CH_2C_6H_5$ | H | $CH_3$ | $OCH_3$ | N | |
| O | O | H | H | $C(O)CH_2C_6H_5$ | H | $OCH_3$ | $OCH_3$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | C(O)thienyl | H | $CH_3$ | $OCH_3$ | CH | |
| O | O | H | H | C(O)thienyl | H | $OCH_3$ | $OCH_3$ | CH | |
| O | O | H | H | $CO_2CH(CH_3)_2$ | H | $CH_3$ | $OCH_3$ | CH | |
| O | O | H | H | $CO_2CH(CH_3)_2$ | H | $OCH_3$ | $OCH_3$ | CH | |
| O | O | H | H | $CO_2C(CH_3)_3$ | H | $CH_3$ | $OCH_3$ | CH | |
| O | O | H | H | $CO_2C(CH_3)_3$ | H | $OCH_3$ | $OCH_3$ | CH | |
| O | O | H | H | $CO_2CH_2CH_2OH$ | H | $CH_3$ | $OCH_3$ | CH | |
| O | O | H | H | $CO_2CH_2CH_2OH$ | H | $OCH_3$ | $OCH_3$ | CH | |
| O | O | H | H | $C(O)NHCH_3$ | H | $CH_3$ | $OCH_3$ | CH | |
| O | O | H | H | $C(O)NHCH_3$ | H | $OCH_3$ | $OCH_3$ | CH | |
| O | O | H | H | $C(O)NHCH_2CH_3$ | H | $CH_3$ | $OCH_3$ | CH | |
| O | O | H | H | $C(O)NHCH_2CH_3$ | H | $OCH_3$ | $OCH_3$ | CH | |
| O | O | H | H | $C(O)NHCH_2CH_2CH_2CH_3$ | H | $CH_3$ | $OCH_3$ | CH | |
| O | O | H | H | $C(O)NH_2CH_2CH_2CH_2CH_3$ | H | $OCH_3$ | $OCH_3$ | CH | |
| O | O | H | H | $C(O)N(CH_3)_2$ | H | $CH_3$ | $OCH_3$ | CH | |
| O | O | H | H | $C(O)N(CH_3)_2$ | H | $OCH_3$ | $OCH_3$ | CH | 202-212 |
| O | O | H | H | C(O)NH(cyclohexyl) | H | $CH_3$ | $OCH_3$ | CH | |
| O | O | H | H | C(O)NH(cyclohexyl) | H | $OCH_3$ | $OCH_3$ | CH | |
| O | O | H | H | $C(O)NHC_6H_5$ | H | $CH_3$ | $OCH_3$ | CH | |
| O | O | H | H | $C(O)NHC_6H_5$ | H | $OCH_3$ | $OCH_3$ | CH | |
| O | O | H | H | (4.6-dimethylpyrimidin-2-yl)aminocarbonyl | H | $CH_3$ | $CH_3$ | CH | 181-183 |
| O | O | H | H | (4-methoxy-6-methylpyrimidin-2-yl)aminocarbonyl | H | $CH_3$ | $OCH_3$ | CH | |
| O | O | H | H | (4.6-dimethoxypyrimidin-2-yl) aminocarbonyl | H | $OCH_3$ | $OCH_3$ | CH | 192-194 |

Table Ia (continued)

| W | n | R | $R_1$ | $R_2$ | $R_3$ | X | Y | Z | m.p. (°C) |
|---|---|---|---|---|---|---|---|---|---|
| O | O | H | H | (4-chloro-6-methoxypyrimidin-2-yl)aminocarbonyl | H | Cl | $OCH_3$ | CH | 206-208 |
| O | O | H | H | (4-methoxy-6-methyl-1,3,5-triazin-2-yl)aminocarbonyl | H | $CH_3$ | $OCH_3$ | CH | |
| O | O | H | H | (4,6-dimethoxy-1,3,5,-triazin-2-yl)aminocarbonyl | H | $OCH_3$ | $OCH_3$ | N | |
| O | O | H | H | $CS_2CH_3$ | H | $CH_3$ | $OCH_3$ | CH | |
| O | O | H | H | $CS_2CH_3$ | H | $OCH_3$ | $OCH_3$ | CH | |
| O | O | H | H | $CS_2CH_2CH_3$ | H | $CH_3$ | $OCH_3$ | CH | |
| O | O | H | H | $CS_2CH_2CH_3$ | H | $OCH_3$ | $OCH_3$ | CH | |
| O | O | H | H | $CS_2CH_2CH_2CH_3$ | H | $CH_3$ | $OCH_3$ | CH | |
| O | O | H | H | $CS_2CH_2CH_2CH_3$ | H | $OCH_3$ | $OCH_3$ | CH | |
| O | O | H | H | $NH_2$ | H | $CH_3$ | $OCH_3$ | CH | |
| O | O | H | H | $NH_2$ | H | $OCH_3$ | $OCH_3$ | CH | |
| O | O | H | H | $NHCH_3$ | H | $CH_3$ | $OCH_3$ | CH | |
| O | O | H | H | $NHCH_3$ | H | $OCH_3$ | $OCH_3$ | CH | |
| O | O | H | H | $NHCH_2CH_3$ | H | $CH_3$ | $OCH_3$ | CH | |
| O | O | H | H | $NHCH_2CH_3$ | H | $OCH_3$ | $OCH_3$ | CH | |
| O | O | H | H | $NHC_6H_5$ | H | $CH_3$ | $OCH_3$ | CH | |
| O | O | H | H | $NHC_6H_5$ | H | $OCH_3$ | $OCH_3$ | CH | |
| O | O | H | H | 4-chlorobenzyl | H | $CH_3$ | $CH_3$ | CH | |
| O | O | H | H | 4-chlorobenzyl | H | $CH_3$ | $OCH_3$ | CH | |
| O | O | H | H | 4-chlorobenzyl | H | $OCH_3$ | $OCH_3$ | CH | |
| O | O | H | H | 4-chlorobenzyl | H | Cl | $OCH_3$ | CH | |
| O | O | H | H | 4-chlorobenzyl | H | $CH_3$ | $OCH_3$ | N | |
| O | O | H | H | 4-chlorobenzyl | H | $OCH_3$ | $OCH_3$ | N | |
| O | O | H | H | 4-methylbenzyl | H | $CH_3$ | $CH_3$ | CH | 194-197 |
| O | O | H | H | 4-methylbenzyl | H | $CH_3$ | $OCH_3$ | CH | 193-196 |
| O | O | H | H | 4-methylbenzyl | H | $OCH_3$ | $OCH_3$ | CH | 170-173 |
| O | O | H | H | 4-methylbenzyl | H | Cl | $OCH_3$ | CH | 147-150 |
| O | O | H | H | 4-methylbenzyl | H | $CH_3$ | $OCH_3$ | N | 154-157 |

Table Ia (continued)

| W | n | R | $R_1$ | $R_2$ | $R_3$ | X | Y | Z | m.p. (°C) |
|---|---|---|---|---|---|---|---|---|---|
| O | O | H | H | 4-methylbenzyl | H | $OCH_3$ | $OCH_3$ | N | 139-143 |
| O | O | H | H | 4-nitrobenzyl | H | $CH_3$ | $CH_3$ | CH | 223-225 |
| O | O | H | H | 4-nitrobenzyl | H | $CH_3$ | $OCH_3$ | CH | 218-219 |
| O | O | H | H | 4-nitrobenzyl | H | $OCH_3$ | $OCH_3$ | CH | 180-183 |
| O | O | H | H | 4-nitrobenzyl | H | Cl | $OCH_3$ | CH | 200-203 |
| O | O | H | H | 4-nitrobenzyl | H | $CH_3$ | $OCH_3$ | N | 189-192 |
| O | O | H | H | 4-nitrobenzyl | H | $OCH_3$ | $OCH_3$ | N | 198-200 |
| O | O | H | H | 3-trifluoromethylbenzyl | H | $CH_3$ | $CH_3$ | CH | |
| O | O | H | H | 3-trifluoromethylbenzyl | H | $CH_3$ | $OCH_3$ | CH | |
| O | O | H | H | 3-trifluoromethylbenzyl | H | $OCH_3$ | $OCH_3$ | CH | |
| O | O | H | H | 3-trifluoromethylbenzyl | H | Cl | $OCH_3$ | CH | |
| O | O | H | H | 3-trifluoromethylbenzyl | H | $CH_3$ | $OCH_3$ | N | |
| O | O | H | H | 3-trifluoromethylbenzyl | H | $OCH_3$ | $OCH_3$ | N | |
| O | O | H | H | 3-methylbenzyl | H | $CH_3$ | $CH_3$ | CH | |
| O | O | H | H | 3-methylbenzyl | H | $CH_3$ | $OCH_3$ | CH | |
| O | O | H | H | 3-methylbenzyl | H | $OCH_3$ | $OCH_3$ | CH | |
| O | O | H | H | 3-methylbenzyl | H | Cl | $OCH_3$ | CH | |
| O | O | H | H | 3-methylbenzyl | H | $CH_3$ | $OCH_3$ | N | |
| O | O | H | H | 3-methylbenzyl | H | $OCH_3$ | $OCH_3$ | N | |
| O | O | H | H | 2-methylbenzyl | H | $CH_3$ | $CH_3$ | CH | |
| O | O | H | H | 2-methylbenzyl | H | $CH_3$ | $OCH_3$ | CH | |
| O | O | H | H | 2-methylbenzyl | H | $OCH_3$ | $OCH_3$ | CH | |
| O | O | H | H | 2-methylbenzyl | H | Cl | $OCH_3$ | CH | |
| O | O | H | H | 2-methylbenzyl | H | $CH_3$ | $OCH_3$ | N | |
| O | O | H | H | 2-methylbenzyl | H | $OCH_3$ | $OCH_3$ | N | |
| O | O | H | H | OH | H | $CH_3$ | $OCH_3$ | CH | |
| O | O | H | H | OH | H | $OCH_3$ | $OCH_3$ | CH | |
| O | O | H | H | CN | H | $CH_3$ | $CH_3$ | CH | |
| O | O | H | H | CN | H | $CH_3$ | $OCH_3$ | CH | |
| O | O | H | H | CN | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | CN | H | Cl | $OCH_3$ | CH | |
| O | O | H | H | CN | H | $CH_3$ | $OCH_3$ | N | |
| O | O | H | H | CN | H | $OCH_3$ | $OCH_3$ | N | |
| O | O | H | H | $P(O)(OCH_3)_2$ | H | $CH_3$ | $CH_3$ | CH | |
| O | O | H | H | $P(O)(OCH_3)_2$ | H | $CH_3$ | $OCH_3$ | CH | |
| O | O | H | H | $P(O)(OCH_3)_2$ | H | $OCH_3$ | $OCH_3$ | CH | |
| O | O | H | H | $P(O)(OCH_3)_2$ | H | Cl | $OCH_3$ | CH | |
| O | O | H | H | $P(O)(OCH_3)_2$ | H | $CH_3$ | $OCH_3$ | N | |
| O | O | H | H | $P(O)(OCH_3)_2$ | H | $OCH_3$ | $OCH_3$ | N | |
| O | O | H | H | $P(O)(OCH_2CH_3)_2$ | H | $OCH_3$ | $OCH_3$ | CH | |
| O | O | H | H | $P(S)(OCH_3)_2$ | H | $OCH_3$ | $OCH_3$ | CH | |
| O | O | H | H | $P(O)(CH_3)_2$ | H | $OCH_3$ | $OCH_3$ | CH | |
| O | O | H | H | $P(S)(CH_3)_2$ | H | $OCH_3$ | $OCH_3$ | CH | |
| O | O | H | H | $P(O)(S\ CH_2CH_2CH_2CH_3)_2$ | H | $OCH_3$ | $OCH_3$ | CH | |
| O | O | H | H | $Si(CH_3)_3$ | H | $CH_3$ | $CH_3$ | CH | |
| O | O | H | H | $Si(CH_3)_3$ | H | $CH_3$ | $OCH_3$ | CH | |
| O | O | H | H | $Si(CH_3)_3$ | H | $OCH_3$ | $OCH_3$ | CH | |
| O | O | H | H | $Si(CH_3)_3$ | H | Cl | $OCH_3$ | CH | |
| O | O | H | H | $Si(CH_3)_3$ | H | $CH_3$ | $OCH_3$ | N | |
| O | O | H | H | $Si(CH_3)_3$ | H | $OCH_3$ | $OCH_3$ | N | |
| O | O | H | H | $Si(CH_3)_2C(CH_3)_3$ | H | $CH_3$ | $CH_3$ | CH | |
| O | O | H | H | $Si(CH_3)_2C(CH_3)_3$ | H | $CH_3$ | $OCH_3$ | CH | |
| O | O | H | H | $Si(CH_3)_2C(CH_3)_3$ | H | $OCH_3$ | $OCH_3$ | CH | |
| O | O | H | H | $Si(CH_3)_2C(CH_3)_3$ | H | Cl | $OCH_3$ | CH | |
| O | O | H | H | $Si(CH_3)_2C(CH_3)_3$ | H | $CH_3$ | $OCH_3$ | N | |
| O | O | H | H | $Si(CH_3)_2C(CH_3)_3$ | H | $OCH_3$ | $OCH_3$ | N | |
| O | O | H | H | $Si(CH_3)_2C_6H_5$ | H | $OCH_3$ | $OCH_3$ | CH | |
| O | O | H | H | $Si(CH_3)_2(CH_2)_9CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| S | 0 | H | H | $C(O)CH_3$ | H | $OCH_3$ | $OCH_3$ | CH | |
| S | 0 | H | H | $CO_2CH_3$ | H | $OCH_3$ | $OCH_3$ | CH | |
| S | 0 | H | H | $CH_2CH=CH_2$ | H | $OCH_3$ | $OCH_3$ | CH | |
| S | 0 | H | H | $CH_2CH_2F$ | H | $OCH_3$ | $OCH_3$ | CH | |
| S | 0 | H | H | $CH_2C(O)CH_3$ | H | $OCH_3$ | $OCH_3$ | CH | |
| S | 0 | H | H | $CF_2H$ | H | $OCH_3$ | $OCH_3$ | CH | |
| S | 0 | H | H | $SO_2CH_3$ | H | $OCH_3$ | $OCH_3$ | CH | |
| O | 1 | H | H | $OCH_3$ | H | $OCH_3$ | $OCH_3$ | CH | |
| O | 1 | H | H | $C(O)NHCH_3$ | H | $OCH_3$ | $OCH_3$ | CH | |
| O | 1 | H | H | $CS_2CH_3$ | H | $OCH_3$ | $OCH_3$ | CH | |
| O | 1 | H | H | $NH_2$ | H | $OCH_3$ | $OCH_3$ | CH | |
| O | 1 | H | H | OH | H | $OCH_3$ | $OCH_3$ | CH | |
| O | 1 | H | H | $P(O)(OCH_3)_2$ | H | $OCH_3$ | $OCH_3$ | CH | |
| O | 1 | H | H | $Si(CH_3)_2C(CH_3)_3$ | H | $OCH_3$ | $OCH_3$ | CH | |
| O | 1 | H | H | $CH_2CH_2Cl$ | H | $OCH_3$ | $OCH_3$ | CH | |
| O | 1 | H | H | $CH_2CN$ | H | $OCH_3$ | $OCH_3$ | CH | |
| O | 1 | H | H | $CH_2F$ | H | $OCH_3$ | $OCH_3$ | CH | |
| O | 1 | H | H | $CH_2OCH_3$ | H | $OCH_3$ | $OCH_3$ | CH | |
| O | 1 | H | H | $CH_2SCN$ | H | $OCH_3$ | $OCH_3$ | CH | |
| O | 1 | H | H | $CH_2NO_2$ | H | $OCH_3$ | $OCH_3$ | CH | |
| O | 1 | H | H | $CH_2CO_2CH_3$ | H | $OCH_3$ | $OCH_3$ | CH | |
| O | 1 | H | H | $CH_2CH_2Cl$ | H | $OCH_3$ | $OCH_3$ | CH | |
| O | 1 | H | H | $CH_2CH_2Br$ | H | $OCH_3$ | $OCH_3$ | CH | |
| O | 1 | H | H | $CH_2CH_2OH$ | H | $OCH_3$ | $OCH_3$ | CH | |
| O | 1 | H | H | $CH_2SO_2CH_3$ | H | $OCH_3$ | $OCH_3$ | CH | |
| O | 1 | H | H | $CH_2SCH_3$ | H | $OCH_3$ | $OCH_3$ | CH | |
| O | 1 | H | H | $CH_2CF_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $C(O)CH_3$ | H | H | $OCH_3$ | CH | |
| O | O | H | H | $C(O)CH_3$ | H | $CH_2CH_3$ | $OCH_3$ | CH | |
| O | O | H | H | $C(O)CH_3$ | H | $CH_2CH_2CH_3$ | $OCH_3$ | CH | |
| O | O | H | H | $C(O)CH_3$ | H | $CH_2CH_2CH_2CH_3$ | $OCH_3$ | CH | |
| O | O | H | H | $C(O)CH_3$ | H | $OCH_2CH_3$ | $OCH_3$ | CH | |
| O | O | H | H | $C(O)CH_3$ | H | $OCH_2CH_2CH_3$ | $OCH_3$ | CH | |
| O | O | H | H | $C(O)CH_3$ | H | $OCH_2CH_2CH_2CH_3$ | $OCH_3$ | CH | |
| O | O | H | H | $C(O)CH_3$ | H | $OCH(CH_3)_2$ | $OCH_3$ | CH | |
| O | O | H | H | $C(O)CH_3$ | H | $OCH_2CF_3$ | $OCH_3$ | CH | |
| O | O | H | H | $C(O)CH_3$ | H | $CH_2Cl$ | $OCH_3$ | CH | |
| O | O | H | H | $C(O)CH_3$ | H | $CH_2Br$ | $OCH_3$ | CH | |
| O | O | H | H | $C(O)CH_3$ | H | $CH_2CH_2Cl$ | $OCH_3$ | CH | |
| O | O | H | H | $C(O)CH_3$ | H | $SCH_3$ | $OCH_3$ | CH | |
| O | O | H | H | $C(O)CH_3$ | H | $SCH_2CH_3$ | $OCH_3$ | CH | |
| O | O | H | H | $C(O)CH_3$ | H | $SCH_2CH_3Cl$ | $OCH_3$ | CH | |
| O | O | H | H | $C(O)CH_3$ | H | F | $OCH_3$ | CH | |
| O | O | H | H | $C(O)CH_3$ | H | Cl | $OCH_3$ | CH | |
| O | O | H | H | $C(O)CH_3$ | H | Br | $OCH_3$ | CH | |
| O | O | H | H | $C(O)CH_3$ | H | $OCH_2CH_2F$ | $OCH_3$ | CH | |
| O | O | H | H | $C(O)CH_3$ | H | $CH_2OCH_3$ | $OCH_3$ | CH | |
| O | O | H | H | $C(O)CH_3$ | H | $CH_2OCH_2CH_3$ | $OCH_3$ | CH | |
| O | O | H | H | $C(O)CH_3$ | H | $CH_2OCH_2CH_2CH_2CH_3$ | $OCH_3$ | CH | |
| O | O | H | H | $C(O)CH_3$ | H | $OCH_2OCH_3$ | $OCH_3$ | CH | |
| O | O | H | H | $C(O)CH_3$ | H | $OCH_2CH_2OCH_3$ | $OCH_3$ | CH | |
| O | O | H | H | $C(O)CH_3$ | H | $NH_2$ | $OCH_3$ | CH | |
| O | O | H | H | $C(O)CH_3$ | H | $NHCH_3$ | $OCH_3$ | CH | |
| O | O | H | H | $C(O)CH_3$ | H | $NHCH_2CH_3$ | $OCH_3$ | CH | |
| O | O | H | H | $C(O)CH_3$ | H | $NHCH(CH_3)_2$ | $OCH_3$ | CH | |
| O | O | H | H | $C(O)CH_3$ | H | $NHCH_2CH_2CH_3$ | $OCH_3$ | CH | |
| O | O | H | H | $C(O)CH_3$ | H | $N(CH_3)_2$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $C(O)CH_3$ | H | $N(CH_3)CH_2CH_3$ | $OCH_3$ | CH | |
| O | O | H | H | $C(O)CH_3$ | H | $N(CH_3)C(CH_3)_2$ | $OCH_3$ | CH | |
| O | O | H | H | $C(O)CH_3$ | H | cyclopropyl | $OCH_3$ | CH | |
| O | O | H | H | $C(O)CH_3$ | H | cyclobutyl | $OCH_3$ | CH | |
| O | O | H | H | $C(O)CH_3$ | H | cyclopentyl | $OCH_3$ | CH | |
| O | O | H | H | $C(O)CH_3$ | H | $OCH_3$ | H | CH | |
| O | O | H | H | $C(O)CH_3$ | H | $OCH_3$ | $OCH_2CH=CH_2$ | CH | |
| O | O | H | H | $C(O)CH_3$ | H | $OCH_3$ | $OCH_2C(CH_3)=CH_2$ | CH | |
| O | O | H | H | $C(O)CH_3$ | H | $OCH_3$ | $OCH_2C\equiv CH$ | CH | |
| O | O | H | H | $C(O)CH_3$ | H | $OCH_3$ | $OCH_2C\equiv CCH_3$ | CH | |
| O | O | H | H | $C(O)CH_3$ | H | $OCH_3$ | $CH_2SCH_3$ | CH | |
| O | O | H | H | $C(O)CH_3$ | H | $OCH_3$ | $CH_2SCH_2CH_3$ | CH | |
| O | O | H | H | $C(O)CH_3$ | H | $OCH_3$ | $CH_2SC(CH_3)_3$ | CH | |
| O | O | H | H | $C(O)CH_3$ | H | $OCH_3$ | $CH_2CH_2CH_2CH_2Cl$ | CH | |
| O | O | H | H | $C(O)CH_3$ | H | $OCH_3$ | $N_3$ | CH | |
| O | O | H | H | $C(O)CH_3$ | H | $OCH_3$ | CN | CH | |
| O | O | H | H | $C(O)CH_3$ | H | $OCH_3$ | $C(O)CH_3$ | CH | |
| O | O | H | H | $C(O)CH_3$ | H | $OCH_3$ | CHO | CH | |
| O | O | H | H | $C(O)CH_3$ | H | $OCH_3$ | $C(O)CH(CH_3)_2$ | CH | |
| O | O | H | H | $C(O)CH_3$ | H | $OCH_3$ | $CH(OCH_3)_2$ | CH | |
| O | O | H | H | $C(O)CH_3$ | H | $OCH_3$ | $CH(OCH_2CH_3)_2$ | CH | |

Table 1a (continued)

| W | n | R | $R_1$ | $R_2$ | $R_3$ | X | Y | Z | m.p. (°C) |
|---|---|---|---|---|---|---|---|---|---|
| O | O | H | H | $CH_2CH_2CH_2CH_3$ | H | $SCH_3$ | $SCH_3$ | CH | |
| O | O | H | H | $CH_2CH_2CH_2CH_3$ | H | $NHCH_3$ | $NHCH_3$ | CH | |
| O | O | H | m-$CH_3$ | $C(O)CH_3$ | H | $OCH_3$ | $OCH_3$ | CH | |
| O | O | H | o-$CH_3$ | $C(O)CH_3$ | H | $OCH_3$ | $OCH_3$ | CH | |
| O | O | H | o-$CH_2CH_3$ | $C(O)CH_3$ | H | $OCH_3$ | $OCH_3$ | CH | |
| O | O | H | o-$CH_2CH_2CH_3$ | $C(O)CH_3$ | H | $OCH_3$ | $OCH_3$ | CH | |
| O | O | H | m-$OCH_3$ | $C(O)CH_3$ | H | $OCH_3$ | $OCH_3$ | CH | |
| O | O | H | m-$OCH_2CH_3$ | $C(O)CH_3$ | H | $OCH_3$ | $OCH_3$ | CH | |
| O | O | H | m-$OCH_2CH_2CH_3$ | $C(O)CH_3$ | H | $OCH_3$ | $OCH_3$ | CH | |
| O | O | H | m-$OCH(CH_3)_2$ | $C(O)CH_3$ | H | $OCH_3$ | $OCH_3$ | CH | |
| O | O | H | o-$OCH_3$ | $C(O)CH_3$ | H | $OCH_3$ | $OCH_3$ | CH | |
| O | O | H | o-$OCH_2CH_3$ | $C(O)CH_3$ | H | $OCH_3$ | $OCH_3$ | CH | |
| O | O | H | m-$OCF_2H$ | $C(O)CH_3$ | H | $OCH_3$ | $OCH_3$ | CH | |
| O | O | H | m-$OCH_2CH_2F$ | $C(O)CH_3$ | H | $OCH_3$ | $OCH_3$ | CH | |
| O | O | H | m-$OCH_2CF_3$ | $C(O)CH_3$ | H | $OCH_3$ | $OCH_3$ | CH | |
| O | O | H | O-$OCH_2CH_2CH_2F$ | $C(O)CH_3$ | H | $OCH_3$ | $OCH_3$ | CH | |
| O | O | H | m-$CF_3$ | $C(O)CH_3$ | H | $OCH_3$ | $OCH_3$ | CH | |
| O | O | H | o-$CF_3$ | $C(O)CH_3$ | H | $OCH_3$ | $OCH_3$ | CH | |
| O | O | H | o-$CH_2CH_2Cl$ | $C(O)CH_3$ | H | $OCH_3$ | $OCH_3$ | CH | |
| O | O | H | o-$CH_2CH_2CH_2F$ | $C(O)CH_3$ | H | $OCH_3$ | $OCH_3$ | CH | |
| O | O | H | m-$SCH_3$ | $C(O)CH_3$ | H | $OCH_3$ | $OCH_3$ | CH | |
| O | O | H | o-$SCH_3$ | $C(O)CH_3$ | H | $OCH_3$ | $OCH_3$ | CH | |
| O | O | H | o-$SCH_2CH_2CH_3$ | $C(O)CH_3$ | H | $OCH_3$ | $OCH_3$ | CH | |
| O | O | H | o-$SCF_3$ | $C(O)CH_3$ | H | $OCH_3$ | $OCH_3$ | CH | |
| O | O | H | o-$SCH_2CH_2CH_2Cl$ | $C(O)CH_3$ | H | $OCH_3$ | $OCH_3$ | CH | |
| O | O | H | o-$NH_2$ | $C(O)CH_3$ | H | $OCH_3$ | $OCH_3$ | CH | |
| O | O | H | m-$NHCH_3$ | $C(O)CH_3$ | H | $OCH_3$ | $OCH_3$ | CH | |
| O | O | H | o-$NHCH_3$ | $C(O)CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | m-NH$(CH_3)_2$ | C(O)$CH_3$ | H | $OCH_3$ | $OCH_3$ | CH | |
| O | O | H | o-NH $CH_2CH_2CH_3$ | C(O)$CH_3$ | H | $OCH_3$ | $OCH_3$ | CH | |
| O | O | H | o-NH $CH_2CH_2CH_3$ | C(O)$CH_3$ | H | $OCH_3$ | $OCH_3$ | CH | |
| O | O | H | o-N$(CH_3)CH_2CH_2CH_3$ | C(O)$CH_3$ | H | $OCH_3$ | $OCH_3$ | CH | |
| O | O | H | m-F | C(O)$CH_3$ | H | $OCH_3$ | $OCH_3$ | CH | |
| O | O | H | o-F | C(O)$CH_3$ | H | $OCH_3$ | $OCH_3$ | CH | |
| O | O | H | p-F | C(O)$CH_3$ | H | $OCH_3$ | $OCH_3$ | CH | |
| O | O | H | m-Cl | C(O)$CH_3$ | H | $OCH_3$ | $OCH_3$ | CH | |
| O | O | H | o-Cl | C(O)$CH_3$ | H | $OCH_3$ | $OCH_3$ | CH | |
| O | O | H | p-Cl | C(O)$CH_3$ | H | $OCH_3$ | $OCH_3$ | CH | |
| O | O | H | m-Br | C(O)$CH_3$ | H | $OCH_3$ | $OCH_3$ | CH | |
| O | O | H | o-Br | C(O)$CH_3$ | H | $OCH_3$ | $OCH_3$ | CH | |
| O | O | H | m-I | C(O)$CH_3$ | H | $OCH_3$ | $OCH_3$ | CH | |
| O | O | H | o-$NO_2$ | C(O)$CH_3$ | H | $OCH_3$ | $OCH_3$ | CH | |
| O | O | H | m-$CH_3$ | C(O)$CH_3$ | H | $CH_3$ | $OCH_3$ | CH | |
| O | O | H | o-$CH_3$ | C(O)$CH_3$ | H | $CH_3$ | $OCH_3$ | CH | |
| O | O | H | o-$CH_2CH_3$ | C(O)$CH_3$ | H | $CH_3$ | $OCH_3$ | CH | |
| O | O | H | m-$OCH_3$ | C(O)$CH_3$ | H | $CH_3$ | $OCH_3$ | CH | |
| O | O | H | o-$OCH_3$ | C(O)$CH_3$ | H | $CH_3$ | $OCH_3$ | CH | |
| O | O | H | m-$OCH_2CH_3$ | C(O)$CH_3$ | H | $CH_3$ | $OCH_3$ | CH | |
| O | O | H | m-$CF_3$ | C(O)$CH_3$ | H | $CH_3$ | $OCH_3$ | CH | |
| O | O | H | m-$SCH_3$ | C(O)$CH_3$ | H | $CH_3$ | $OCH_3$ | CH | |
| O | O | H | o-$SCH_3$ | C(O)$CH_3$ | H | $CH_3$ | $OCH_3$ | CH | |
| O | O | H | m-N$(CH_3)_2$ | C(O)$CH_3$ | H | $CH_3$ | $OCH_3$ | CH | |
| O | O | H | m-F | C(O)$CH_3$ | H | $CH_3$ | $OCH_3$ | CH | |
| O | O | H | o-F | C(O)$CH_3$ | H | $CH_3$ | $OCH_3$ | CH | |
| O | O | H | m-Cl | C(O)$CH_3$ | H | $CH_3$ | $OCH_3$ | CH | |
| O | O | H | o-Cl | C(O)$CH_3$ | H | $CH_3$ | $OCH_3$ | CH | |
| O | O | H | m-Br | C(O)$CH_3$ | H | $CH_3$ | $OCH_3$ | CH | |
| O | O | H | m-$CH_3$ | C(O)$CH_2CH_3$ | H | $CH_3$ | $OCH_3$ | CH | |
| O | O | H | m-$CH_3$ | C(O)$CH_2CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | m-$OCH_3$ | $C(O)CH_2CH_3$ | H | $CH_3$ | $OCH_3$ | CH | |
| O | O | H | m-$OCH_3$ | $C(O)CH_2CH_3$ | H | $OCH_3$ | $OCH_3$ | CH | |
| O | O | H | m-$N(CH_3)_2$ | $C(O)CH_2CH_3$ | H | $CH_3$ | $OCH_3$ | CH | |
| O | O | H | m-$N(CH_3)_2$ | $C(O)CH_2CH_3$ | H | $OCH_3$ | $OCH_3$ | CH | |
| O | O | H | m-F | $C(O)CH_2CH_3$ | H | $CH_3$ | $OCH_3$ | CH | |
| O | O | H | m-F | $C(O)CH_2CH_3$ | H | $OCH_3$ | $OCH_3$ | CH | |
| O | O | H | m-Cl | $C(O)CH_2CH_3$ | H | $CH_3$ | $OCH_3$ | CH | |
| O | O | H | m-Cl | $C(O)CH_2CH_3$ | H | $OCH_3$ | $OCH_3$ | CH | |
| O | O | H | m-Br | $C(O)CH_2CH_3$ | H | $CH_3$ | $OCH_3$ | CH | |
| O | O | H | m-Br | $C(O)CH_2CH_3$ | H | $OCH_3$ | $OCH_3$ | CH | |
| O | O | H | m-$SCH_3$ | $C(O)CH_2CH_3$ | H | $CH_3$ | $OCH_3$ | CH | |
| O | O | H | m-$SCH_3$ | $C(O)CH_2CH_3$ | H | $OCH_3$ | $OCH_3$ | CH | |
| O | O | H | m-$CH_3$ | $C(O)CH_2CH_3$ | H | $CH_3$ | $OCH_3$ | CH | |
| O | O | H | m-$CH_3$ | $C(O)CH_2CH_3$ | H | $OCH_3$ | $OCH_3$ | CH | |
| O | O | H | o-$OCH_3$ | $C(O)CH_2CH_3$ | H | $CH_3$ | $OCH_3$ | CH | |
| O | O | H | o-$OCH_3$ | $C(O)CH_2CH_3$ | H | $OCH_3$ | $OCH_3$ | CH | |
| O | O | H | o-F | $C(O)CH_2CH_3$ | H | $CH_3$ | $OCH_3$ | CH | |
| O | O | H | m-$CH_3$ | $C(O)CH_2CH_3$ | H | $CH_3$ | $OCH_3$ | CH | |
| O | O | H | m-$CH_3$ | $C(O)CH_2CH_3$ | H | $OCH_3$ | $OCH_3$ | CH | |
| O | O | H | m-$OCH_3$ | $C(O)CH_2CH_3$ | H | $CH_3$ | $OCH_3$ | CH | |
| O | O | H | m-$OCH_3$ | $C(O)CH_2CH_3$ | H | $OCH_3$ | $OCH_3$ | CH | |
| O | O | H | m-$N(CH_3)_2$ | $C(O)CH_2CH_3$ | H | $CH_3$ | $OCH_3$ | CH | |
| O | O | H | m-$N(CH_3)_2$ | $C(O)CH_2CH_3$ | H | $OCH_3$ | $OCH_3$ | CH | |
| O | O | H | m-F | $C(O)CH_2CH_3$ | H | $CH_3$ | $OCH_3$ | CH | |
| O | O | H | m-F | $C(O)CH_2CH_3$ | H | $OCH_3$ | $OCH_3$ | CH | |
| O | O | H | m-Cl | $C(O)CH_2CH_3$ | H | $CH_3$ | $OCH_3$ | CH | |
| O | O | H | m-Cl | $C(O)CH_2CH_3$ | H | $OCH_3$ | $OCH_3$ | CH | |
| O | O | H | m-Br | $C(O)CH_2CH_3$ | H | $CH_3$ | $OCH_3$ | CH | |
| O | O | H | m-Br | $C(O)CH_2CH_3$ | H | $OCH_3$ | $OCH_3$ | CH | |
| O | O | H | m-$SCH_3$ | $C(O)CH_2CH_3$ | H | $CH_3$ | $OCH_3$ | CH | |
| O | O | H | m-$SCH_3$ | $C(O)CH_2CH_3$ | H | $OCH_3$ | $OCH_3$ | CH | |
| O | O | H | m-$CH_3$ | $C(O)CH_2CH_3$ | H | $CH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | m-$CH_3$ | $C(O)CH_2CH_3$ | H | $OCH_3$ | $OCH_3$ | CH | |
| O | O | H | o-$OCH_3$ | $C(O)CH_2CH_3$ | H | $CH_3$ | $OCH_3$ | CH | |
| O | O | H | o-$OCH_3$ | $C(O)CH_2CH_3$ | H | $OCH_3$ | $OCH_3$ | CH | |
| O | O | H | o-F | $C(O)CH_2CH_3$ | H | $CH_3$ | $OCH_3$ | CH | |
| O | O | H | o-F | $C(O)CH_2CH_3$ | H | $OCH_3$ | $OCH_3$ | CH | |
| O | O | H | o-Cl | $C(O)CH_2CH_3$ | H | $CH_3$ | $OCH_3$ | CH | |
| O | O | H | o-Cl | $C(O)CH_2CH_3$ | H | $OCH_3$ | $OCH_3$ | CH | |
| O | O | H | o-Br | $C(O)CH_2CH_3$ | H | $CH_3$ | $OCH_3$ | CH | |
| O | O | H | o-Br | $C(O)CH_2CH_3$ | H | $OCH_3$ | $OCH_3$ | CH | |
| O | O | H | o-$SCH_3$ | $C(O)CH_2CH_3$ | H | $CH_3$ | $OCH_3$ | CH | |
| O | O | H | o-$SCH_3$ | $C(O)CH_2CH_3$ | H | $OCH_3$ | $OCH_3$ | CH | |
| O | O | H | m-$OCH_2CH_3$ | $C(O)CH_2CH_3$ | H | $CH_3$ | $OCH_3$ | CH | |
| O | O | H | m-$OCH_2CH_3$ | $C(O)CH_2CH_3$ | H | $OCH_3$ | $OCH_3$ | CH | |
| O | O | H | m-$CH_3$ | $CH_2C(O)CH_3$ | H | $CH_3$ | $OCH_3$ | CH | |
| O | O | H | m-$CH_3$ | $CH_2C(O)CH_3$ | H | $OCH_3$ | $OCH_3$ | CH | |
| O | O | H | m-$OCH_3$ | $CH_2C(O)CH_3$ | H | $CH_3$ | $OCH_3$ | CH | |
| O | O | H | m-$OCH_3$ | $CH_2C(O)CH_3$ | H | $OCH_3$ | $OCH_3$ | CH | |
| O | O | H | m-$N(CH_3)_2$ | $CH_2C(O)CH_3$ | H | $CH_3$ | $OCH_3$ | CH | |
| O | O | H | m-$N(CH_3)_2$ | $CH_2C(O)CH_3$ | H | $OCH_3$ | $OCH_3$ | CH | |
| O | O | H | m-F | $CH_2C(O)CH_3$ | H | $CH_3$ | $OCH_3$ | CH | |
| O | O | H | m-F | $CH_2C(O)CH_3$ | H | $OCH_3$ | $OCH_3$ | CH | |
| O | O | H | m-Cl | $CH_2C(O)CH_3$ | H | $CH_3$ | $OCH_3$ | CH | |
| O | O | H | m-Cl | $CH_2C(O)CH_3$ | H | $OCH_3$ | $OCH_3$ | CH | |
| O | O | H | m-Br | $CH_2C(O)CH_3$ | H | $CH_3$ | $OCH_3$ | CH | |
| O | O | H | m-Br | $CH_2C(O)CH_3$ | H | $OCH_3$ | $OCH_3$ | CH | |
| O | O | H | m-$SCH_3$ | $CH_2C(O)CH_3$ | H | $CH_3$ | $OCH_3$ | CH | |
| O | O | H | m-$SCH_3$ | $CH_2C(O)CH_3$ | H | $OCH_3$ | $OCH_3$ | CH | |
| O | O | H | m-$CH_3$ | $CH_2C(O)CH_3$ | H | $CH_3$ | $OCH_3$ | CH | |
| O | O | H | m-$CH_3$ | $CH_2C(O)CH_3$ | H | $OCH_3$ | $OCH_3$ | CH | |
| O | O | H | o-$OCH_3$ | $CH_2C(O)CH_3$ | H | $CH_3$ | $OCH_3$ | CH | |
| O | O | H | o-$OCH_3$ | $CH_2C(O)CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | o-F | $CH_2C(O)CH_3$ | H | $CH_3$ | $OCH_3$ | CH | |
| O | O | H | o-F | $C(O)CH_2CH_3$ | H | $OCH_3$ | $OCH_3$ | CH | |
| O | O | H | o-Cl | $C(O)CH_2CH_3$ | H | $CH_3$ | $OCH_3$ | CH | |
| O | O | H | o-Cl | $C(O)CH_2CH_3$ | H | $OCH_3$ | $OCH_3$ | CH | |
| O | O | H | o-Br | $C(O)CH_2CH_3$ | H | $CH_3$ | $OCH_3$ | CH | |
| O | O | H | o-Br | $C(O)CH_2CH_3$ | H | $OCH_3$ | $OCH_3$ | CH | |
| O | O | H | o-$SCH_3$ | $C(O)CH_2CH_3$ | H | $CH_3$ | $OCH_3$ | CH | |
| O | O | H | o-$SCH_3$ | $C(O)CH_2CH_3$ | H | $OCH_3$ | $OCH_3$ | CH | |
| O | O | H | m-$OCH_2CH_3$ | $C(O)CH_2CH_3$ | H | $CH_3$ | $OCH_3$ | CH | |
| O | O | H | m-$OCH_2CH_3$ | $C(O)CH_2CH_3$ | H | $OCH_3$ | $OCH_3$ | CH | |
| O | O | H | m-$CH_3$ | $CH_2OCH_3$ | H | $CH_3$ | $OCH_3$ | CH | |
| O | O | H | m-$CH_3$ | $CH_2OCH_3$ | H | $OCH_3$ | $OCH_3$ | CH | |
| O | O | H | m-$OCH_3$ | $CH_2OCH_3$ | H | $CH_3$ | $OCH_3$ | CH | |
| O | O | H | m-$OCH_3$ | $CH_2OCH_3$ | H | $OCH_3$ | $OCH_3$ | CH | |
| O | O | H | m-$N(CH_3)_2$ | $CH_2OCH_3$ | H | $CH_3$ | $OCH_3$ | CH | |
| O | O | H | m-$N(CH_3)_2$ | $CH_2OCH_3$ | H | $OCH_3$ | $OCH_3$ | CH | |
| O | O | H | m-F | $CH_2OCH_3$ | H | $CH_3$ | $OCH_3$ | CH | |
| O | O | H | m-F | $CH_2OCH_3$ | H | $OCH_3$ | $OCH_3$ | CH | |
| O | O | H | m-Cl | $CH_2OCH_3$ | H | $CH_3$ | $OCH_3$ | CH | |
| O | O | H | m-Cl | $CH_2OCH_3$ | H | $OCH_3$ | $OCH_3$ | CH | |
| O | O | H | m-Br | $CH_2OCH_3$ | H | $CH_3$ | $OCH_3$ | CH | |
| O | O | H | m-Br | $CH_2OCH_3$ | H | $OCH_3$ | $OCH_3$ | CH | |
| O | O | H | m-$SCH_3$ | $CH_2OCH_3$ | H | $CH_3$ | $OCH_3$ | CH | |
| O | O | H | m-$SCH_3$ | $CH_2OCH_3$ | H | $OCH_3$ | $OCH_3$ | CH | |
| O | O | H | m-$CH_3$ | $CH_2OCH_3$ | H | $CH_3$ | $OCH_3$ | CH | |
| O | O | H | m-$CH_3$ | $CH_2OCH_3$ | H | $OCH_3$ | $OCH_3$ | CH | |
| O | O | H | o-$OCH_3$ | $CH_2OCH_3$ | H | $CH_3$ | $OCH_3$ | CH | |
| O | O | H | o-$OCH_3$ | $CH_2OCH_3$ | H | $OCH_3$ | $OCH_3$ | CH | |
| O | O | H | o-F | $CH_2OCH_3$ | H | $CH_3$ | $OCH_3$ | CH | |
| O | O | H | o-F | $CH_2OCH_3$ | H | $OCH_3$ | $OCH_3$ | CH | |
| O | O | H | o-Cl | $CH_2OCH_3$ | H | $CH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | o-Cl | $CH_2OCH_3$ | H | $OCH_3$ | $OCH_3$ | CH | |
| O | O | H | o-Br | $CH_2OCH_3$ | H | $CH_3$ | $OCH_3$ | CH | |
| O | O | H | o-Br | $CH_2OCH_3$ | H | $OCH_3$ | $OCH_3$ | CH | |
| O | O | H | o-$SCH_3$ | $CH_2OCH_3$ | H | $CH_3$ | $OCH_3$ | CH | |
| O | O | H | o-$SCH_3$ | $CH_2OCH_3$ | H | $OCH_3$ | $OCH_3$ | CH | |
| O | O | H | m-$OCH_2CH_3$ | $CH_2OCH_3$ | H | $CH_3$ | $OCH_3$ | CH | |
| O | O | H | m-$OCH_2CH_3$ | $CH_2OCH_3$ | H | $OCH_3$ | $OCH_3$ | CH | |
| O | O | H | m-$CH_3$ | $CO_2CH_3$ | H | $CH_3$ | $OCH_3$ | CH | |
| O | O | H | m-$CH_3$ | $CO_2CH_3$ | H | $OCH_3$ | $OCH_3$ | CH | |
| O | O | H | m-$OCH_3$ | $CO_2CH_3$ | H | $CH_3$ | $OCH_3$ | CH | |
| O | O | H | m-$OCH_3$ | $CO_2CH_3$ | H | $OCH_3$ | $OCH_3$ | CH | |
| O | O | H | m-$N(CH_3)_2$ | $CO_2CH_3$ | H | $CH_3$ | $OCH_3$ | CH | |
| O | O | H | m-$N(CH_3)_2$ | $CO_2CH_3$ | H | $OCH_3$ | $OCH_3$ | CH | |
| O | O | H | m-F | $CO_2CH_3$ | H | $CH_3$ | $OCH_3$ | CH | |
| O | O | H | m-F | $CO_2CH_3$ | H | $OCH_3$ | $OCH_3$ | CH | |
| O | O | H | m-Cl | $CO_2CH_3$ | H | $CH_3$ | $OCH_3$ | CH | |
| O | O | H | m-Cl | $CO_2CH_3$ | H | $OCH_3$ | $OCH_3$ | CH | |
| O | O | H | m-Br | $CO_2CH_3$ | H | $CH_3$ | $OCH_3$ | CH | |
| O | O | H | m-Br | $CO_2CH_3$ | H | $OCH_3$ | $OCH_3$ | CH | |
| O | O | H | m-$SCH_3$ | $CO_2CH_3$ | H | $CH_3$ | $OCH_3$ | CH | |
| O | O | H | m-$SCH_3$ | $CO_2CH_3$ | H | $OCH_3$ | $OCH_3$ | CH | |
| O | O | H | m-$CH_3$ | $CO_2CH_3$ | H | $CH_3$ | $OCH_3$ | CH | |
| O | O | H | m-$CH_3$ | $CO_2CH_3$ | H | $OCH_3$ | $OCH_3$ | CH | |
| O | O | H | o-$OCH_3$ | $CO_2CH_3$ | H | $CH_3$ | $OCH_3$ | CH | |
| O | O | H | o-$OCH_3$ | $CO_2CH_3$ | H | $OCH_3$ | $OCH_3$ | CH | |
| O | O | H | o-F | $CO_2CH_3$ | H | $CH_3$ | $OCH_3$ | CH | |
| O | O | H | o-F | $CO_2CH_3$ | H | $OCH_3$ | $OCH_3$ | CH | |
| O | O | H | o-Cl | $CO_2CH_3$ | H | $CH_3$ | $OCH_3$ | CH | |
| O | O | H | o-Cl | $CO_2CH_3$ | H | $OCH_3$ | $OCH_3$ | CH | |
| O | O | H | o-Br | $CO_2CH_3$ | H | $CH_3$ | $OCH_3$ | CH | |
| O | O | H | o-Br | $CO_2CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | o-$SCH_3$ | $CO_2CH_3$ | H | $CH_3$ | $OCH_3$ | CH | |
| O | O | H | o-$SCH_3$ | $CO_2CH_3$ | H | $OCH_3$ | $OCH_3$ | CH | |
| O | O | H | m-$OCH_2CH_3$ | $CO_2CH_3$ | H | $CH_3$ | $OCH_3$ | CH | |
| O | O | H | m-$OCH_2CH_3$ | $CO_2CH_3$ | H | $OCH_3$ | $OCH_3$ | CH | |
| O | O | H | m-$CH_3$ | CN | H | $CH_3$ | $OCH_3$ | CH | |
| O | O | H | m-$CH_3$ | CN | H | $OCH_3$ | $OCH_3$ | CH | |
| O | O | H | m-$OCH_3$ | CN | H | $CH_3$ | $OCH_3$ | CH | |
| O | O | H | m-$OCH_3$ | CN | H | $OCH_3$ | $OCH_3$ | CH | |
| O | O | H | m-$N(CH_3)_2$ | CN | H | $CH_3$ | $OCH_3$ | CH | |
| O | O | H | m-$N(CH_3)_2$ | CN | H | $OCH_3$ | $OCH_3$ | CH | |
| O | O | H | m-F | CN | H | $CH_3$ | $OCH_3$ | CH | |
| O | O | H | m-F | CN | H | $OCH_3$ | $OCH_3$ | CH | |
| O | O | H | m-Cl | CN | H | $CH_3$ | $OCH_3$ | CH | |
| O | O | H | m-Cl | CN | H | $OCH_3$ | $OCH_3$ | CH | |
| O | O | H | m-Br | CN | H | $CH_3$ | $OCH_3$ | CH | |
| O | O | H | m-Br | CN | H | $OCH_3$ | $OCH_3$ | CH | |
| O | O | H | m-$SCH_3$ | CN | H | $CH_3$ | $OCH_3$ | CH | |
| O | O | H | m-$SCH_3$ | CN | H | $OCH_3$ | $OCH_3$ | CH | |
| O | O | H | m-$CH_3$ | CN | H | $CH_3$ | $OCH_3$ | CH | |
| O | O | H | m-$CH_3$ | CN | H | $OCH_3$ | $OCH_3$ | CH | |
| O | O | H | o-$OCH_3$ | CN | H | $CH_3$ | $OCH_3$ | CH | |
| O | O | H | o-$OCH_3$ | CN | H | $OCH_3$ | $OCH_3$ | CH | |
| O | O | H | o-F | CN | H | $CH_3$ | $OCH_3$ | CH | |
| O | O | H | o-F | CN | H | $OCH_3$ | $OCH_3$ | CH | |
| O | O | H | o-Cl | CN | H | $CH_3$ | $OCH_3$ | CH | |
| O | O | H | o-Cl | CN | H | $OCH_3$ | $OCH_3$ | CH | |
| O | O | H | o-Br | CN | H | $CH_3$ | $OCH_3$ | CH | |
| O | O | H | o-Br | CN | H | $OCH_3$ | $OCH_3$ | CH | |
| O | O | H | o-$SCH_3$ | CN | H | $CH_3$ | $OCH_3$ | CH | |
| O | O | H | o-$SCH_3$ | CN | H | $OCH_3$ | $OCH_3$ | CH | |
| O | O | H | m-$OCH_2CH_3$ | CN | H | $CH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | m-$OCH_2CH_3$ | CN | H | $OCH_3$ | $OCH_3$ | CH | |
| O | O | H | m-$CH_3$ | $CH_2CN$ | H | $CH_3$ | $OCH_3$ | CH | |
| O | O | H | m-$CH_3$ | $CH_2CN$ | H | $OCH_3$ | $OCH_3$ | CH | |
| O | O | H | m-$OCH_3$ | $CH_2CN$ | H | $CH_3$ | $OCH_3$ | CH | |
| O | O | H | m-$OCH_3$ | $CH_2CN$ | H | $OCH_3$ | $OCH_3$ | CH | |
| O | O | H | m-$N(CH_3)_2$ | $CH_2CN$ | H | $CH_3$ | $OCH_3$ | CH | |
| O | O | H | m-$N(CH_3)_2$ | $CH_2CN$ | H | $OCH_3$ | $OCH_3$ | CH | |
| O | O | H | m-F | $CH_2CN$ | H | $CH_3$ | $OCH_3$ | CH | |
| O | O | H | m-F | $CH_2CN$ | H | $OCH_3$ | $OCH_3$ | CH | |
| O | O | H | m-Cl | $CH_2CN$ | H | $CH_3$ | $OCH_3$ | CH | |
| O | O | H | m-Cl | $CH_2CN$ | H | $OCH_3$ | $OCH_3$ | CH | |
| O | O | H | m-Br | $CH_2CN$ | H | $CH_3$ | $OCH_3$ | CH | |
| O | O | H | m-Br | $CH_2CN$ | H | $OCH_3$ | $OCH_3$ | CH | |
| O | O | H | m-$SCH_3$ | $CH_2CN$ | H | $CH_3$ | $OCH_3$ | CH | |
| O | O | H | m-$SCH_3$ | $CH_2CN$ | H | $OCH_3$ | $OCH_3$ | CH | |
| O | O | H | m-$CH_3$ | $CH_2CN$ | H | $CH_3$ | $OCH_3$ | CH | |
| O | O | H | m-$CH_3$ | $CH_2CN$ | H | $OCH_3$ | $OCH_3$ | CH | |
| O | O | H | o-$OCH_3$ | $CH_2CN$ | H | $CH_3$ | $OCH_3$ | CH | |
| O | O | H | o-$OCH_3$ | $CH_2CN$ | H | $OCH_3$ | $OCH_3$ | CH | |
| O | O | H | o-F | $CH_2CN$ | H | $CH_3$ | $OCH_3$ | CH | |
| O | O | H | o-F | $CH_2CN$ | H | $OCH_3$ | $OCH_3$ | CH | |
| O | O | H | o-Cl | $CH_2CN$ | H | $CH_3$ | $OCH_3$ | CH | |
| O | O | H | o-Cl | $CH_2CN$ | H | $OCH_3$ | $OCH_3$ | CH | |
| O | O | H | o-Br | $CH_2CN$ | H | $CH_3$ | $OCH_3$ | CH | |
| O | O | H | o-Br | $CH_2CN$ | H | $OCH_3$ | $OCH_3$ | CH | |
| O | O | H | o-$SCH_3$ | $CH_2CN$ | H | $CH_3$ | $OCH_3$ | CH | |
| O | O | H | o-$SCH_3$ | $CH_2CN$ | H | $OCH_3$ | $OCH_3$ | CH | |
| O | O | H | m-$OCH_2CH_3$ | $CH_2CN$ | H | $CH_3$ | $OCH_3$ | CH | |
| O | O | H | m-$OCH_2CH_3$ | $CH_2CN$ | H | $OCH_3$ | $OCH_3$ | CH | |
| O | O | H | m-$CH_3$ | $CH_2CH=CH_2$ | H | $CH_3$ | $OCH_3$ | CH | |
| O | O | H | m-$CH_3$ | $CH_2CH=CH_2$ | H | $OCH_3$ | $OCH_3$ | CH | |

Table Ia (continued)

| W | n | R | R1 | R2 | R3 | X | Y | Z | m.p. (°C) |
|---|---|---|---|---|---|---|---|---|---|
| O | O | H | m-$OCH_3$ | $CH_2CH{=}CH_2$ | H | $CH_3$ | $OCH_3$ | CH | |
| O | O | H | m-$OCH_3$ | $CH_2CH{=}CH_2$ | H | $OCH_3$ | $OCH_3$ | CH | |
| O | O | H | m-$N(CH_3)_2$ | $CH_2CH{=}CH_2$ | H | $CH_3$ | $OCH_3$ | CH | |
| O | O | H | m-$N(CH_3)_2$ | $CH_2CH{=}CH_2$ | H | $OCH_3$ | $OCH_3$ | CH | |
| O | O | H | m-F | $CH_2CH{=}CH_2$ | H | $CH_3$ | $OCH_3$ | CH | |
| O | O | H | m-F | $CH_2CH{=}CH_2$ | H | $OCH_3$ | $OCH_3$ | CH | |
| O | O | H | m-Cl | $CH_2CH{=}CH_2$ | H | $CH_3$ | $OCH_3$ | CH | |
| O | O | H | m-Cl | $CH_2CH{=}CH_2$ | H | $OCH_3$ | $OCH_3$ | CH | |
| O | O | H | m-Br | $CH_2CH{=}CH_2$ | H | $CH_3$ | $OCH_3$ | CH | |
| O | O | H | m-Br | $CH_2CH{=}CH_2$ | H | $OCH_3$ | $OCH_3$ | CH | |
| O | O | H | m-$SCH_3$ | $CH_2CH{=}CH_2$ | H | $CH_3$ | $OCH_3$ | CH | |
| O | O | H | m-$SCH_3$ | $CH_2CH{=}CH_2$ | H | $OCH_3$ | $OCH_3$ | CH | |
| O | O | H | m-$CH_3$ | $CH_2CH{=}CH_2$ | H | $CH_3$ | $OCH_3$ | CH | |
| O | O | H | m-$CH_3$ | $CH_2CH{=}CH_2$ | H | $OCH_3$ | $OCH_3$ | CH | |
| O | O | H | o-$OCH_3$ | $CH_2CH{=}CH_2$ | H | $CH_3$ | $OCH_3$ | CH | |
| O | O | H | o-$OCH_3$ | $CH_2CH{=}CH_2$ | H | $OCH_3$ | $OCH_3$ | CH | |
| O | O | H | o-F | $CH_2CH{=}CH_2$ | H | $CH_3$ | $OCH_3$ | CH | |
| O | O | H | o-F | $CH_2CH{=}CH_2$ | H | $OCH_3$ | $OCH_3$ | CH | |
| O | O | H | o-Cl | $CH_2CH{=}CH_2$ | H | $CH_3$ | $OCH_3$ | CH | |
| O | O | H | o-Cl | $CH_2CH{=}CH_2$ | H | $OCH_3$ | $OCH_3$ | CH | |
| O | O | H | o-Br | $CH_2CH{=}CH_2$ | H | $CH_3$ | $OCH_3$ | CH | |
| O | O | H | o-Br | $CH_2CH{=}CH_2$ | H | $OCH_3$ | $OCH_3$ | CH | |
| O | O | H | o-$SCH_3$ | $CH_2CH{=}CH_2$ | H | $CH_3$ | $OCH_3$ | CH | |
| O | O | H | o-$SCH_3$ | $CH_2CH{=}CH_2$ | H | $OCH_3$ | $OCH_3$ | CH | |
| O | O | H | m-$OCH_2CH_3$ | $CH_2CH{=}CH_2$ | H | $CH_3$ | $OCH_3$ | CH | |
| O | O | H | m-$OCH_2CH_3$ | $CH_2CH{=}CH_2$ | H | $OCH_3$ | $OCH_3$ | CH | |
| O | O | H | m-$CH_3$ | $CH_2C{\equiv}CH$ | H | $CH_3$ | $OCH_3$ | CH | |
| O | O | H | m-$CH_3$ | $CH_2C{\equiv}CH$ | H | $OCH_3$ | $OCH_3$ | CH | |
| O | O | H | m-$OCH_3$ | $CH_2C{\equiv}CH$ | H | $CH_3$ | $OCH_3$ | CH | |
| O | O | H | m-$OCH_3$ | $CH_2C{\equiv}CH$ | H | $OCH_3$ | $OCH_3$ | CH | |

**Table Ia (continued)**

| W | n | R | $R_1$ | $R_2$ | $R_3$ | X | Y | Z | m.p. (°C) |
|---|---|---|---|---|---|---|---|---|---|
| O | O | H | m-$N(CH_3)_2$ | $CH_2C\equiv CH$ | H | $CH_3$ | $OCH_3$ | CH | |
| O | O | H | m-$N(CH_3)_2$ | $CH_2C\equiv CH$ | H | $OCH_3$ | $OCH_3$ | CH | |
| O | O | H | m-F | $CH_2C\equiv CH$ | H | $CH_3$ | $OCH_3$ | CH | |
| O | O | H | m-F | $CH_2C\equiv CH$ | H | $OCH_3$ | $OCH_3$ | CH | |
| O | O | H | m-Cl | $CH_2C\equiv CH$ | H | $CH_3$ | $OCH_3$ | CH | |
| O | O | H | m-Cl | $CH_2C\equiv CH$ | H | $OCH_3$ | $OCH_3$ | CH | |
| O | O | H | m-Br | $CH_2C\equiv CH$ | H | $CH_3$ | $OCH_3$ | CH | |
| O | O | H | m-Br | $CH_2C\equiv CH$ | H | $OCH_3$ | $OCH_3$ | CH | |
| O | O | H | m-$SCH_3$ | $CH_2C\equiv CH$ | H | $CH_3$ | $OCH_3$ | CH | |
| O | O | H | m-$SCH_3$ | $CH_2C\equiv CH$ | H | $OCH_3$ | $OCH_3$ | CH | |
| O | O | H | m-$CH_3$ | $CH_2C\equiv CH$ | H | $CH_3$ | $OCH_3$ | CH | |
| O | O | H | m-$CH_3$ | $CH_2C\equiv CH$ | H | $OCH_3$ | $OCH_3$ | CH | |
| O | O | H | o-$OCH_3$ | $CH_2C\equiv CH$ | H | $CH_3$ | $OCH_3$ | CH | |
| O | O | H | o-$OCH_3$ | $CH_2C\equiv CH$ | H | $OCH_3$ | $OCH_3$ | CH | |
| O | O | H | o-F | $CH_2C\equiv CH$ | H | $CH_3$ | $OCH_3$ | CH | |
| O | O | H | o-F | $CH_2C\equiv CH$ | H | $OCH_3$ | $OCH_3$ | CH | |
| O | O | H | o-Cl | $CH_2C\equiv CH$ | H | $CH_3$ | $OCH_3$ | CH | |
| O | O | H | o-Cl | $CH_2C\equiv CH$ | H | $OCH_3$ | $OCH_3$ | CH | |
| O | O | H | o-Br | $CH_2C\equiv CH$ | H | $CH_3$ | $OCH_3$ | CH | |
| O | O | H | o-Br | $CH_2C\equiv CH$ | H | $OCH_3$ | $OCH_3$ | CH | |
| O | O | H | o-$SCH_3$ | $CH_2C\equiv CH$ | H | $CH_3$ | $OCH_3$ | CH | |
| O | O | H | o-$SCH_3$ | $CH_2C\equiv CH$ | H | $OCH_3$ | $OCH_3$ | CH | |
| O | O | H | m-$OCH_2CH_3$ | $CH_2C\equiv CH$ | H | $CH_3$ | $OCH_3$ | CH | |
| O | O | H | m-$OCH_2CH_3$ | $CH_2C\equiv CH$ | H | $OCH_3$ | $OCH_3$ | CH | |
| O | O | H | m-$CH_3$ | $CH_2CH_2F$ | H | Cl | $OCH_3$ | CH | |
| O | O | H | m-$CH_3$ | $CH_2CH_2F$ | H | $OCH_3$ | $OCH_3$ | CH | |
| O | O | H | m-$OCH_3$ | $CH_2CH_2F$ | H | Cl | $OCH_3$ | CH | |
| O | O | H | m-$OCH_3$ | $CH_2CH_2F$ | H | $OCH_3$ | $OCH_3$ | CH | |
| O | O | H | m-$N(CH_3)_2$ | $CH_2CH_2F$ | H | Cl | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | m-$N(CH_3)_2$ | $CH_2CH_2F$ | H | $OCH_3$ | $OCH_3$ | CH | |
| O | O | H | m-F | $CH_2CH_2F$ | H | Cl | $OCH_3$ | CH | |
| O | O | H | m-F | $CH_2CH_2F$ | H | $OCH_3$ | $OCH_3$ | CH | |
| O | O | H | m-Cl | $CH_2CH_2F$ | H | Cl | $OCH_3$ | CH | |
| O | O | H | m-Cl | $CH_2CH_2F$ | H | $OCH_3$ | $OCH_3$ | CH | |
| O | O | H | m-Br | $CH_2CH_2F$ | H | Cl | $OCH_3$ | CH | |
| O | O | H | m-Br | $CH_2CH_2F$ | H | $OCH_3$ | $OCH_3$ | CH | |
| O | O | H | m-$SCH_3$ | $CH_2CH_2F$ | H | Cl | $OCH_3$ | CH | |
| O | O | H | m-$SCH_3$ | $CH_2CH_2F$ | H | $OCH_3$ | $OCH_3$ | CH | |
| O | O | H | m-$CH_3$ | $CH_2CH_2F$ | H | Cl | $OCH_3$ | CH | |
| O | O | H | m-$CH_3$ | $CH_2CH_2F$ | H | $OCH_3$ | $OCH_3$ | CH | |
| O | O | H | o-$OCH_3$ | $CH_2CH_2F$ | H | Cl | $OCH_3$ | CH | |
| O | O | H | o-$OCH_3$ | $CH_2CH_2F$ | H | $OCH_3$ | $OCH_3$ | CH | |
| O | O | H | o-F | $CH_2CH_2F$ | H | Cl | $OCH_3$ | CH | |
| O | O | H | o-F | $CH_2CH_2F$ | H | $OCH_3$ | $OCH_3$ | CH | |
| O | O | H | o-Cl | $CH_2CH_2F$ | H | Cl | $OCH_3$ | CH | |
| O | O | H | o-Cl | $CH_2CH_2F$ | H | $OCH_3$ | $OCH_3$ | CH | |
| O | O | H | o-Br | $CH_2CH_2F$ | H | Cl | $OCH_3$ | CH | |
| O | O | H | o-Br | $CH_2CH_2F$ | H | $OCH_3$ | $OCH_3$ | CH | |
| O | O | H | o-$SCH_3$ | $CH_2CH_2F$ | H | Cl | $OCH_3$ | CH | |
| O | O | H | o-$SCH_3$ | $CH_2CH_2F$ | H | $OCH_3$ | $OCH_3$ | CH | |
| O | O | H | m-$OCH_2CH_3$ | $CH_2CH_2F$ | H | Cl | $OCH_3$ | CH | |
| O | O | H | m-$OCH_2CH_3$ | $CH_2CH_2F$ | H | $OCH_3$ | $OCH_3$ | CH | |
| O | O | H | m-$CH_3$ | $CH_2NO_2$ | H | $CH_3$ | $OCH_3$ | CH | |
| O | O | H | m-$CH_3$ | $CH_2NO_2$ | H | $OCH_3$ | $OCH_3$ | CH | |
| O | O | H | m-$OCH_3$ | $CH_2NO_2$ | H | $CH_3$ | $OCH_3$ | CH | |
| O | O | H | m-$OCH_3$ | $CH_2NO_2$ | H | $OCH_3$ | $OCH_3$ | CH | |
| O | O | H | m-$N(CH_3)_2$ | $CH_2NO_2$ | H | $CH_3$ | $OCH_3$ | CH | |
| O | O | H | m-$N(CH_3)_2$ | $CH_2NO_2$ | H | $OCH_3$ | $OCH_3$ | CH | |
| O | O | H | m-F | $CH_2NO_2$ | H | $CH_3$ | $OCH_3$ | CH | |
| O | O | H | m-F | $CH_2NO_2$ | H | $OCH_3$ | $OCH_3$ | CH | |

**Table Ia (continued)**

| W | n | R | $R_1$ | $R_2$ | $R_3$ | X | Y | Z | m.p. (°C) |
|---|---|---|---|---|---|---|---|---|---|
| O | O | H | m-Cl | $CH_2NO_2$ | H | $CH_3$ | $OCH_3$ | CH | |
| O | O | H | m-Cl | $CH_2NO_2$ | H | $OCH_3$ | $OCH_3$ | CH | |
| O | O | H | m-Br | $CH_2NO_2$ | H | $CH_3$ | $OCH_3$ | CH | |
| O | O | H | m-Br | $CH_2NO_2$ | H | $OCH_3$ | $OCH_3$ | CH | |
| O | O | H | m-$SCH_3$ | $CH_2NO_2$ | H | $CH_3$ | $OCH_3$ | CH | |
| O | O | H | m-$SCH_3$ | $CH_2NO_2$ | H | $OCH_3$ | $OCH_3$ | CH | |
| O | O | H | m-$CH_3$ | $CH_2NO_2$ | H | $CH_3$ | $OCH_3$ | CH | |
| O | O | H | m-$CH_3$ | $CH_2NO_2$ | H | $OCH_3$ | $OCH_3$ | CH | |
| O | O | H | o-$OCH_3$ | $CH_2NO_2$ | H | $CH_3$ | $OCH_3$ | CH | |
| O | O | H | o-$OCH_3$ | $CH_2NO_2$ | H | $OCH_3$ | $OCH_3$ | CH | |
| O | O | H | o-F | $CH_2NO_2$ | H | $CH_3$ | $OCH_3$ | CH | |
| O | O | H | o-F | $CH_2NO_2$ | H | $OCH_3$ | $OCH_3$ | CH | |
| O | O | H | o-Cl | $CH_2NO_2$ | H | $CH_3$ | $OCH_3$ | CH | |
| O | O | H | o-Cl | $CH_2NO_2$ | H | $OCH_3$ | $OCH_3$ | CH | |
| O | O | H | o-Br | $CH_2NO_2$ | H | $CH_3$ | $OCH_3$ | CH | |
| O | O | H | o-Br | $CH_2NO_2$ | H | $OCH_3$ | $OCH_3$ | CH | |
| O | O | H | o-$SCH_3$ | $CH_2NO_2$ | H | $CH_3$ | $OCH_3$ | CH | |
| O | O | H | o-$SCH_3$ | $CH_2NO_2$ | H | $OCH_3$ | $OCH_3$ | CH | |
| O | O | H | m-$OCH_2CH_3$ | $CH_2NO_2$ | H | $CH_3$ | $OCH_3$ | CH | |
| O | O | H | m-$OCH_2CH_3$ | $CH_2NO_2$ | H | $OCH_3$ | $OCH_3$ | CH | |
| O | O | H | m-$OCH_2CH_2CH_3$ | H | H | $OCH_3$ | $OCH_3$ | CH | |
| O | O | H | m-$OCH(CH_3)_2$ | H | H | $OCH_3$ | $OCH_3$ | CH | |
| O | O | H | m-$N(CH_3)_2$ | H | H | $OCH_3$ | $OCH_3$ | CH | |
| O | O | H | m-$OCH_2CH_2CH_3$ | $CH_3$ | H | $OCH_3$ | $OCH_3$ | CH | |
| O | O | H | m-$OCH(CH_3)_2$ | $CH_3$ | H | $OCH_3$ | $OCH_3$ | CH | |
| O | O | H | m-$N(CH_3)_2$ | $CH_3$ | H | $OCH_3$ | $OCH_3$ | CH | |
| O | O | H | m-$OCH_2CH_2CH_3$ | $CH_2CH_3$ | H | $OCH_3$ | $OCH_3$ | CH | |
| O | O | H | m-$OCH(CH_3)_2$ | $CH_2CH_3$ | H | $OCH_3$ | $OCH_3$ | CH | |
| O | O | H | m-$N(CH_3)_2$ | $CH_2CH_3$ | H | $OCH_3$ | $OCH_3$ | CH | |
| O | O | H | m-$OCH_2CH_2CH_3$ | $CH_2CH_2CH_3$ | H | $OCH_3$ | $OCH_3$ | CH | |
| O | O | H | m-$OCH(CH_3)_2$ | $CH_2CH_2CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | m-$N(CH_3)_2$ | $CH_2CH_2CH_3$ | H | $OCH_3$ | $OCH_3$ | CH | |
| O | O | H | m-$OCH_2CH_2CH_3$ | CH $CH(CH_3)_2$ | H | $OCH_3$ | $OCH_3$ | CH | |
| O | O | H | m-$OCH(CH_3)_2$ | CH $CH(CH_3)_2$ | H | $OCH_3$ | $OCH_3$ | CH | |
| O | O | H | m-$N(CH_3)_2$ | CH $CH(CH_3)_2$ | H | $OCH_3$ | $OCH_3$ | CH | |
| O | O | H | m-$OCH_2CH_2CH_3$ | $CH_2CH_2CH_2CH_3$ | H | $OCH_3$ | $OCH_3$ | CH | |
| O | O | H | m-$OCH(CH_3)_2$ | $CH_2CH_2CH_2CH_3$ | H | $OCH_3$ | $OCH_3$ | CH | |
| O | O | H | m-$N(CH_3)_2$ | $CH_2CH_2CH_2CH_3$ | H | $OCH_3$ | $OCH_3$ | CH | |
| O | O | H | m-$NHCH_3$ | $CH_2CH_2CH_2CH_3$ | H | $OCH_3$ | $OCH_3$ | CH | |
| O | O | H | m-$OCH_2CH_2F$ | $CH_2CH_2CH_2CH_3$ | H | $OCH_3$ | $OCH_3$ | CH | |
| O | O | H | m-$SCH_2CH_3$ | $CH_2CH_2CH_2CH_3$ | H | $OCH_3$ | $OCH_3$ | CH | |
| O | O | H | H | $CH_2C{\equiv}CH$ | H | $CH_3$ | $CH_3$ | CH | 206-209 |
| O | O | H | H | $CH_2C{\equiv}CH$ | H | Cl | $OCH_3$ | CH | 198-200 |
| O | O | H | H | $CH_2C{\equiv}CH$ | H | $CH_3$ | $OCH_3$ | N | 188-190 |
| O | O | H | H | $CH_2C{\equiv}CH$ | H | $OCH_3$ | $OCH_3$ | N | 190-192 |
| O | O | H | H | $CH_2CH_2CH_2OSO_2CH_3$ | H | $CH_3$ | $CH_3$ | CH | |
| O | O | H | H | $CH_2CH_2CH_2OSO_2CH_3$ | H | $CH_3$ | $OCH_3$ | CH | |
| O | O | H | H | $CH_2CH_2CH_2OSO_2CH_3$ | H | $OCH_3$ | $OCH_3$ | CH | |
| O | O | H | H | $CH_2CH_2CH_2OSO_2CH_3$ | H | Cl | $OCH_3$ | CH | |
| O | O | H | H | $CH_2CH_2CH_2OSO_2CH_3$ | H | $CH_3$ | $OCH_3$ | N | |
| O | O | H | H | $CH_2CH_2CH_2OSO_2CH_3$ | H | $OCH_3$ | $OCH_3$ | N | |
| O | O | H | H | $CH_2CH{=}C\ (CH_3)_2$ | H | $CH_3$ | $CH_3$ | CH | 204-204.5(d) |
| O | O | H | H | $CH_2CH{=}C\ (CH_3)_2$ | H | $CH_3$ | $OCH_3$ | CH | 205-208(d) |
| O | O | H | H | $CH_2CH{=}C\ (CH_3)_2$ | H | $OCH_3$ | $OCH_3$ | CH | 224-226(d) |
| O | O | H | H | $CH_2CH{=}C\ (CH_3)_2$ | H | Cl | $OCH_3$ | CH | 214-215(d) |
| O | O | H | H | $CH_2CH{=}C\ (CH_3)_2$ | H | $CH_3$ | $OCH_3$ | N | 183-185(d) |
| O | O | H | H | $CH_2CH{=}C\ (CH_3)_2$ | H | $OCH_3$ | $OCH_3$ | N | 170-173(d) |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $C(O)CH_3$ | H | $OCH_2CH_3$ | $NHCH_3$ | N | |
| O | O | H | H | $C(O)CH_2CH_3$ | H | $OCH_2CH_3$ | $NHCH_3$ | N | |
| O | O | H | H | $C(O)CH_2CH_2CH_3$ | H | $OCH_2CH_3$ | $NHCH_3$ | N | |
| O | O | H | H | $C(O)C(CH_3)_3$ | H | $OCH_2CH_3$ | $NHCH_3$ | N | |
| O | O | H | H | $CO_2CH_3$ | H | $OCH_2CH_3$ | $NHCH_3$ | N | |
| O | O | H | H | $CO_2CH_2CH_3$ | H | $OCH_2CH_3$ | $NHCH_3$ | N | |
| O | O | H | H | $CO_2CH_2CH_2CH_3$ | H | $OCH_2CH_3$ | $NHCH_3$ | N | |
| O | O | H | H | $CO_2CH(CH_3)_2$ | H | $OCH_2CH_3$ | $NHCH_3$ | N | |
| O | O | H | H | $CO_2C(CH_3)_3$ | H | $OCH_2CH_3$ | $NHCH_3$ | N | |
| O | O | H | H | $CH_2C(O)CH_3$ | H | $OCH_2CH_3$ | $NHCH_3$ | N | |
| O | O | H | H | $CH_2CH{=}CH_2$ | H | $OCH_2CH_3$ | $NHCH_3$ | N | |
| O | O | H | H | $CH_2C{\equiv}CH$ | H | $OCH_2CH_3$ | $NHCH_3$ | N | |
| O | O | H | H | $CH_2CH_2F$ | H | $OCH_2CH_3$ | $NHCH_3$ | N | |
| O | O | H | H | $CH_2CH_2Cl$ | H | $OCH_2CH_3$ | $NHCH_3$ | N | |
| O | O | H | H | $CH_2CH_2Br$ | H | $OCH_2CH_3$ | $NHCH_3$ | N | |
| O | O | H | H | $CH_2CH_2CH_2F$ | H | $OCH_2CH_3$ | $NHCH_3$ | N | |
| O | O | H | H | $CH_2CF_3$ | H | $OCH_2CH_3$ | $NHCH_3$ | N | |
| O | O | H | H | $CH_2CH_2CF_3$ | H | $OCH_2CH_3$ | $NHCH_3$ | N | |
| O | O | H | H | $CH_2CHF_2$ | H | $OCH_2CH_3$ | $NHCH_3$ | N | |
| O | O | H | H | $CH_2CN$ | H | $OCH_2CH_3$ | $NHCH_3$ | N | |
| O | O | H | H | $CH_2OCH_3$ | H | $OCH_2CH_3$ | $NHCH_3$ | N | |
| O | O | H | H | $CH_2OCH_2CH_3$ | H | $OCH_2CH_3$ | $NHCH_3$ | N | |
| O | O | H | H | $C(O)CH_3$ | H | $OCH_3$ | H | CH | |
| O | O | H | H | $C(O)CH_2CH_3$ | H | $OCH_3$ | H | CH | |
| O | O | H | H | $C(O)CH_2CH_2CH_3$ | H | $OCH_3$ | H | CH | |
| O | O | H | H | $C(O)C(CH_3)_3$ | H | $OCH_3$ | H | CH | |
| O | O | H | H | $CO_2CH_3$ | H | $OCH_3$ | H | CH | |
| O | O | H | H | $CO_2CH_2CH_3$ | H | $OCH_3$ | H | CH | |
| O | O | H | H | $CO_2CH_2CH_2CH_3$ | H | $OCH_3$ | H | CH | |
| O | O | H | H | $CO_2CH(CH_3)_2$ | H | $OCH_3$ | H | CH | |
| O | O | H | H | $CO_2C(CH_3)_3$ | H | $OCH_3$ | H | CH | |
| O | O | H | H | $CH_2C(O)CH_3$ | H | $OCH_3$ | H | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2C(O)CH_2CH_3$ | H | $OCH_3$ | H | CH | |
| O | O | H | H | $CH_2CH=CH_2$ | H | $OCH_3$ | H | CH | |
| O | O | H | H | $CH_2C\equiv CH$ | H | $OCH_3$ | H | CH | |
| O | O | H | H | $CH_2CH_2F$ | H | $OCH_3$ | H | CH | |
| O | O | H | H | $CH_2CH_2Cl$ | H | $OCH_3$ | H | CH | |
| O | O | H | H | $CH_2CH_2Br$ | H | $OCH_3$ | H | CH | |
| O | O | H | H | $CH_2CH_2CH_2F$ | H | $OCH_3$ | H | CH | |
| O | O | H | H | $CH_2CF_3$ | H | $OCH_3$ | H | CH | |
| O | O | H | H | $CH_2CH_2CF_3$ | H | $OCH_3$ | H | CH | |
| O | O | H | H | $CH_2CHF_2$ | H | $OCH_3$ | H | CH | |
| O | O | H | H | $CH_2CN$ | H | $OCH_3$ | H | CH | |
| O | O | H | H | $CH_2OCH_3$ | H | $OCH_3$ | H | CH | |
| O | O | H | H | $OCH_2CH_3$ | H | $OCH_3$ | H | CH | |
| O | O | H | H | $C(O)CH_3$ | H | $OCH_3$ | $OCH_2CH_2F$ | N | |
| O | O | H | H | $C(O)CH_2CH_3$ | H | $OCH_3$ | $OCH_2CH_2F$ | N | |
| O | O | H | H | $C(O)CH_2CH_2CH_3$ | H | $OCH_3$ | $OCH_2CH_2F$ | N | |
| O | O | H | H | $C(O)C(CH_3)_3$ | H | $OCH_3$ | $OCH_2CH_2F$ | N | |
| O | O | H | H | $CO_2CH_3$ | H | $OCH_3$ | $OCH_2CH_2F$ | N | |
| O | O | H | H | $CO_2CH_2CH_3$ | H | $OCH_3$ | $OCH_2CH_2F$ | N | |
| O | O | H | H | $CO_2CH_2CH_2CH_3$ | H | $OCH_3$ | $OCH_2CH_2F$ | N | |
| O | O | H | H | $CO_2CH(CH_3)_2$ | H | $OCH_3$ | $OCH_2CH_2F$ | N | |
| O | O | H | H | $CO_2C(CH_3)_3$ | H | $OCH_3$ | $OCH_2CH_2F$ | N | |
| O | O | H | H | $CH_2C(O)CH_3$ | H | $OCH_3$ | $OCH_2CH_2F$ | N | |
| O | O | H | H | $CH_2C(O)CH_2CH_3$ | H | $OCH_3$ | $OCH_2CH_2F$ | N | |
| O | O | H | H | $CH_2CH=CH_2$ | H | $OCH_3$ | $OCH_2CH_2F$ | N | |
| O | O | H | H | $CH_2C\equiv CH$ | H | $OCH_3$ | $OCH_2CH_2F$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CN$ | H | $OCH_3$ | $OCH_2CH_2F$ | N | |
| O | O | H | H | $CH_2OCH_3$ | H | $OCH_3$ | $OCH_2CH_2F$ | N | |
| O | O | H | H | $C(O)CH_3$ | H | $OCH_3$ | $OCH_2CF_3$ | N | |
| O | O | H | H | $C(O)CH_2CH_3$ | H | $OCH_3$ | $OCH_2CF_3$ | N | |
| O | O | H | H | $C(O)CH_2CH_2CH_3$ | H | $OCH_3$ | $OCH_2CF_3$ | N | |
| O | O | H | H | $C(O)C(CH_3)_3$ | H | $OCH_3$ | $OCH_2CF_3$ | N | |
| O | O | H | H | $CO_2CH_3$ | H | $OCH_3$ | $OCH_2CF_3$ | N | |
| O | O | H | H | $CO_2CH_2CH_3$ | H | $OCH_3$ | $OCH_2CF_3$ | N | |
| O | O | H | H | $CO_2CH_2CH_2CH_3$ | H | $OCH_3$ | $OCH_2CF_3$ | N | |
| O | O | H | H | $CO_2CH(CH_3)_2$ | H | $OCH_3$ | $OCH_2CF_3$ | N | |
| O | O | H | H | $CO_2C(CH_3)_3$ | H | $OCH_3$ | $OCH_2CF_3$ | N | |
| O | O | H | H | $CH_2C(O)CH_3$ | H | $OCH_3$ | $OCH_2CF_3$ | N | |
| O | O | H | H | $CH_2C(O)CH_2CH_3$ | H | $OCH_3$ | $OCH_2CF_3$ | N | |
| O | O | H | H | $CH_2CH=CH_2$ | H | $OCH_3$ | $OCH_2CF_3$ | N | |
| O | O | H | H | $CH_2C\equiv CH$ | H | $OCH_3$ | $OCH_2CF_3$ | N | |
| O | O | H | H | $CH_2CH_2F$ | H | $OCH_3$ | $OCH_2CF_3$ | N | |
| O | O | H | H | $CH_2CH_2Cl$ | H | $OCH_3$ | $OCH_2CF_3$ | N | |
| O | O | H | H | $CH_2CH_2Br$ | H | $OCH_3$ | $OCH_2CF_3$ | N | |
| O | O | H | H | $CH_2CH_2CH_2F$ | H | $OCH_3$ | $OCH_2CF_3$ | N | |
| O | O | H | H | $CH_2CF_3$ | H | $OCH_3$ | $OCH_2CF_3$ | N | |
| O | O | H | H | $CH_2CH_2CF_3$ | H | $OCH_3$ | $OCH_2CF_3$ | N | |
| O | O | H | H | $CH_2CHF_2$ | H | $OCH_3$ | $OCH_2CF_3$ | N | |
| O | O | H | H | $CH_2CN$ | H | $OCH_3$ | $OCH_2CF_3$ | N | |
| O | O | H | H | $CH_2OCH_3$ | H | $OCH_3$ | $OCH_2CF_3$ | N | |
| O | O | H | H | $CH_2OCH_2CH_3$ | H | $OCH_3$ | $OCH_2CF_3$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | m-$(CH_2)_5CH_3$ | $C(O)CH_3$ | H | $OCH_3$ | $OCH_3$ | CH | |
| O | O | H | m-$(CH_2)_3CH(CH_3)_2$ | $C(O)CH_3$ | H | $OCH_3$ | $OCH_3$ | CH | |
| O | O | H | m-$(CH_2)_5CF_3$ | $C(O)CH_3$ | H | $OCH_3$ | $OCH_3$ | CH | |
| O | O | H | m-$O(CH_2)_5CH_3$ | $C(O)CH_3$ | H | $OCH_3$ | $OCH_3$ | CH | |
| O | O | H | m-$S(CH_2)_5CH_3$ | $C(O)CH_3$ | H | $OCH_3$ | $OCH_3$ | CH | |
| O | O | H | o-$S(O)(CH_2)_5CH_3$ | $C(O)CH_3$ | H | $OCH_3$ | $OCH_3$ | CH | |
| O | O | H | o-$SO_2(CH_2)_5CH_3$ | $C(O)CH_3$ | H | $OCH_3$ | $OCH_3$ | CH | |
| O | O | H | m-CN | $C(O)CH_3$ | H | $OCH_3$ | $OCH_3$ | CH | |
| O | O | H | m-$O(CH_2)_5CF_3$ | $C(O)CH_3$ | H | $OCH_3$ | $OCH_3$ | CH | |
| O | O | H | m-$S(CH_2)_5CF_3$ | $C(O)CH_3$ | H | $OCH_3$ | $OCH_3$ | CH | |
| O | O | H | m-$NH(CH_2)_5CH_3$ | $C(O)CH_3$ | H | $OCH_3$ | $OCH_3$ | CH | |
| O | O | H | m-$N(CH_3)(CH_2)_5CH_3$ | $C(O)CH_3$ | H | $OCH_3$ | $OCH_3$ | CH | |
| O | O | H | o-$SO_2NH_2$ | $C(O)CH_3$ | H | $OCH_3$ | $OCH_3$ | CH | |
| O | O | H | o-$SO_2NH(CH_2)_3CH_3$ | $C(O)CH_3$ | H | $OCH_3$ | $OCH_3$ | CH | |
| O | O | H | o-$SO_2N(CH_3)_2$ | $C(O)CH_3$ | H | $OCH_3$ | $OCH_3$ | CH | |
| O | O | H | o-$SO_2N(CH_3)OCH_3$ | $C(O)CH_3$ | H | $OCH_3$ | $OCH_3$ | CH | |
| O | O | H | o-$SO_2N(CH_3)OCH_2CH_3$ | $C(O)CH_3$ | H | $OCH_3$ | $OCH_3$ | CH | |
| O | O | H | o-$SO_2N(CH_3)CH_2CN$ | $C(O)CH_3$ | H | $OCH_3$ | $OCH_3$ | CH | |
| O | O | H | o-$SO_2NH(CH_2)_3CN$ | $C(O)CH_3$ | H | $OCH_3$ | $OCH_3$ | CH | |
| O | O | H | o-$SO_2NHCH_2CH{=}CH_2$ | $C(O)CH_3$ | H | $OCH_3$ | $OCH_3$ | CH | |
| O | O | H | o-azetidinyl-sulfonyl | $C(O)CH_3$ | H | $OCH_3$ | $OCH_3$ | CH | |
| O | O | H | o-pyrrolidinyl-sulfonyl | $C(O)CH_3$ | H | $OCH_3$ | $OCH_3$ | CH | |
| O | O | H | o-piperidinyl-sulfonyl | $C(O)CH_3$ | H | $OCH_3$ | $OCH_3$ | CH | |
| O | O | H | o-morpholino-sulfonyl | $C(O)CH_3$ | H | $OCH_3$ | $OCH_3$ | CH | |
| O | O | H | m-$CH_2OCH_3$ | $C(O)CH_3$ | H | $OCH_3$ | $OCH_3$ | CH | |
| O | O | H | o-$CH_2OCH_3$ | $C(O)CH_3$ | H | $OCH_3$ | $OCH_3$ | CH | |
| O | O | H | m-$(CH_2)_3OCH_3$ | $C(O)CH_3$ | H | $OCH_3$ | $OCH_3$ | CH | |
| O | O | H | o-$CH_2O(CH_2)_2CH_3$ | $C(O)CH_3$ | H | $OCH_3$ | $OCH_3$ | CH | |
| O | O | H | o-$CH_2SCH_3$ | $C(O)CH_3$ | H | $OCH_3$ | $OCH_3$ | CH | |

**Table Ia (continued)**

| W | n | R | $R_1$ | $R_2$ | $R_3$ | X | Y | Z | m.p.(°C) |
|---|---|---|---|---|---|---|---|---|---|
| O | O | H | o-$CH_2S(CH_2)_2CH_3$ | $C(O)CH_3$ | H | $OCH_3$ | $OCH_3$ | CH | |
| O | O | H | o-$CH_2S(O)Pr$ | $C(O)CH_3$ | H | $OCH_3$ | $OCH_3$ | CH | |
| O | O | H | o-$CH_2SO_2Pr$ | $C(O)CH_3$ | H | $OCH_3$ | $OCH_3$ | CH | |
| O | O | H | m-$CH_2SO_2N(CH_3)_2$ | $C(O)CH_3$ | H | $OCH_3$ | $OCH_3$ | CH | |
| O | O | H | m-$CH_2SO_2NHPr$ | $C(O)CH_3$ | H | $OCH_3$ | $OCH_3$ | CH | |
| O | O | H | o-$CH_2N(CH_3)_2$ | $C(O)CH_3$ | H | $OCH_3$ | $OCH_3$ | CH | |
| O | O | H | o-$CH_2NHPr$ | $C(O)CH_3$ | H | $OCH_3$ | $OCH_3$ | CH | |
| O | O | H | m-$CH_2CH_2N(CH_3)_2$ | $C(O)CH_3$ | H | $OCH_3$ | $OCH_3$ | CH | |
| O | O | H | m-$CH_2NO_2$ | $C(O)CH_3$ | H | $OCH_3$ | $OCH_3$ | CH | |
| O | O | H | o-$(CH_2)_3NO_2$ | $C(O)CH_3$ | H | $OCH_3$ | $OCH_3$ | CH | |
| O | O | H | m-$CH_2CN$ | $C(O)CH_3$ | H | $OCH_3$ | $OCH_3$ | CH | |
| O | O | H | o-$(CH_2)_3CN$ | $C(O)CH_3$ | H | $OCH_3$ | $OCH_3$ | CH | |
| O | O | H | o-$CH_2CO_2CH_3$ | $C(O)CH_3$ | H | $OCH_3$ | $OCH_3$ | CH | |
| O | O | H | o-$(CH_2)_2CO_2H$ | $C(O)CH_3$ | H | $OCH_3$ | $OCH_3$ | CH | |
| O | O | H | m-$CH_2CO_2Pr$ | $C(O)CH_3$ | H | $OCH_3$ | $OCH_3$ | CH | |
| O | O | H | o-$CH_2O(CH_2)_2F$ | $C(O)CH_3$ | H | $OCH_3$ | $OCH_3$ | CH | |
| O | O | H | o-$CH_2O(CH_2)_2CF_3$ | $C(O)CH_3$ | H | $OCH_3$ | $OCH_3$ | CH | |
| O | O | H | m-$CH_2OCH_2CF_3$ | $C(O)CH_3$ | H | $OCH_3$ | $OCH_3$ | CH | |
| O | O | H | o-$CH_2O(CH_2)_3Cl$ | $C(O)CH_3$ | H | $OCH_3$ | $OCH_3$ | CH | |
| O | O | H | o-$(CH_2)_2S(CH_2)_2Cl$ | $C(O)CH_3$ | H | $OCH_3$ | $OCH_3$ | CH | |
| O | O | H | o-$Si(CH_3)_3$ | $C(O)CH_3$ | H | $OCH_3$ | $OCH_3$ | CH | |
| O | O | H | o-$Si(CH_3)_2C(CH_3)_3$ | $C(O)CH_3$ | H | $OCH_3$ | $OCH_3$ | CH | |
| O | O | H | o-$Si(CH_3)_2Ph$ | $C(O)CH_3$ | H | $OCH_3$ | $OCH_3$ | CH | |
| O | O | H | o-$CO_2CH_3$ | $C(O)CH_3$ | H | $OCH_3$ | $OCH_3$ | CH | |
| O | O | H | o-$CO_2(CH_2)_2CH_3$ | $C(O)CH_3$ | H | $OCH_3$ | $OCH_3$ | CH | |
| O | O | H | o-$CO_2CH_2CH{=}CH_2$ | $C(O)CH_3$ | H | $OCH_3$ | $OCH_3$ | CH | |
| O | O | H | o-$CO_2(CH_2)_2CH{=}CH_2$ | $C(O)CH_3$ | H | $OCH_3$ | $OCH_3$ | CH | |
| O | O | H | o-$CO_2CH_2C{\equiv}CCH_3$ | $C(O)CH_3$ | H | $OCH_3$ | $OCH_3$ | CH | |
| O | O | H | o-$CO_2CH_2CF_3$ | $C(O)CH_3$ | H | $OCH_3$ | $OCH_3$ | CH | |
| O | O | H | o-$CO_2(CH_2)_4Cl$ | $C(O)CH_3$ | H | $OCH_3$ | $OCH_3$ | CH | |
| O | O | H | o-$CO_2CH_2CN$ | $C(O)CH_3$ | H | $OCH_3$ | $OCH_3$ | CH | |
| O | O | H | o-$CO_2(CH_2)_3CN$ | $C(O)CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | o-$CO_2$-cyclopentyl | $C(O)CH_3$ | H | $OCH_3$ | $OCH_3$ | CH | |
| O | O | H | o-$CO_2$-cyclohexyl | $C(O)CH_3$ | H | $OCH_3$ | $OCH_3$ | CH | |
| O | O | H | o-$CO_2$-cyclopropylmethyl | $C(O)CH_3$ | H | $OCH_3$ | $OCH_3$ | CH | |
| O | O | H | o-$CO_2$-cyclohexylmethyl | $C(O)CH_3$ | H | $OCH_3$ | $OCH_3$ | CH | |
| O | O | H | o-$CO_2(CH_2)_2OCH_3$ | $C(O)CH_3$ | H | $OCH_3$ | $OCH_3$ | CH | |
| O | O | H | o-$CO_2(CH_2)_2OCH_2CH_3$ | $C(O)CH_3$ | H | $OCH_3$ | $OCH_3$ | CH | |
| O | O | H | m-CN | $CH_3$ | H | $CH_3$ | $OCH_3$ | CH | |
| O | O | H | m-CN | $CH_3$ | H | $OCH_3$ | $OCH_3$ | CH | |
| O | O | H | m-CN | $CH_3$ | H | Cl | $OCH_3$ | CH | |
| O | O | H | o-CN | $CH_3$ | H | $CH_3$ | $OCH_3$ | CH | |
| O | O | H | o-CN | $CH_3$ | H | $OCH_3$ | $OCH_3$ | CH | |
| O | O | H | o-CN | $CH_3$ | H | Cl | $OCH_3$ | CH | |
| O | O | H | o-$CH_2OCH_3$ | $CH_3$ | H | $CH_3$ | $OCH_3$ | CH | |
| O | O | H | o-$CH_2OCH_3$ | $CH_3$ | H | $OCH_3$ | $OCH_3$ | CH | |
| O | O | H | o-$CH_2OCH_3$ | $CH_3$ | H | Cl | $OCH_3$ | CH | |
| O | O | H | o-$Si(CH_3)_3$ | $CH_3$ | H | $CH_3$ | $OCH_3$ | CH | |
| O | O | H | o-$Si(CH_3)_3$ | $CH_3$ | H | $OCH_3$ | $OCH_3$ | CH | |
| O | O | H | o-$Si(CH_3)_3$ | $CH_3$ | H | Cl | $OCH_3$ | CH | |
| O | O | H | o-$CH_2SCH_3$ | $CH_3$ | H | $CH_3$ | $OCH_3$ | CH | |
| O | O | H | o-$CH_2SCH_3$ | $CH_3$ | H | $OCH_3$ | $OCH_3$ | CH | |
| O | O | H | o-$CH_2SCH_3$ | $CH_3$ | H | Cl | $OCH_3$ | CH | |
| O | O | H | o-$CH_2CN$ | $CH_3$ | H | $CH_3$ | $OCH_3$ | CH | |
| O | O | H | o-$CH_2CN$ | $CH_3$ | H | $OCH_3$ | $OCH_3$ | CH | |
| O | O | H | o-$CH_2CN$ | $CH_3$ | H | Cl | $OCH_3$ | CH | |
| O | O | H | m-CN | $CH_2CH_2CH_2CH_3$ | H | $CH_3$ | $OCH_3$ | CH | |
| O | O | H | m-CN | $CH_2CH_2CH_2CH_3$ | H | $OCH_3$ | $OCH_3$ | CH | |
| O | O | H | m-CN | $CH_2CH_2CH_2CH_3$ | H | Cl | $OCH_3$ | CH | |
| O | O | H | o-CN | $CH_2CH_2CH_2CH_3$ | H | $CH_3$ | $OCH_3$ | CH | |
| O | O | H | o-CN | $CH_2CH_2CH_2CH_3$ | H | $OCH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | o-CN | $CH_2CH_2CH_2CH_3$ | H | Cl | $OCH_3$ | CH | |
| O | O | H | o-$CH_2OCH_3$ | $CH_2CH_2CH_2CH_3$ | H | $CH_3$ | $OCH_3$ | CH | |
| O | O | H | o-$CH_2OCH_3$ | $CH_2CH_2CH_2CH_3$ | H | $OCH_3$ | $OCH_3$ | CH | |
| O | O | H | o-$CH_2OCH_3$ | $CH_2CH_2CH_2CH_3$ | H | Cl | $OCH_3$ | CH | |
| O | O | H | o-$Si(CH_3)_3$ | $CH_2CH_2CH_2CH_3$ | H | $CH_3$ | $OCH_3$ | CH | |
| O | O | H | o-$Si(CH_3)_3$ | $CH_2CH_2CH_2CH_3$ | H | $OCH_3$ | $OCH_3$ | CH | |
| O | O | H | o-$Si(CH_3)_3$ | $CH_2CH_2CH_2CH_3$ | H | Cl | $OCH_3$ | CH | |
| O | O | H | o-$CH_2SCH_3$ | $CH_2CH_2CH_2CH_3$ | H | $CH_3$ | $OCH_3$ | CH | |
| O | O | H | o-$CH_2SCH_3$ | $CH_2CH_2CH_2CH_3$ | H | $OCH_3$ | $OCH_3$ | CH | |
| O | O | H | o-$CH_2SCH_3$ | $CH_2CH_2CH_2CH_3$ | H | Cl | $OCH_3$ | CH | |
| O | O | H | o-$CH_2CN$ | $CH_2CH_2CH_2CH_3$ | H | $CH_3$ | $OCH_3$ | CH | |
| O | O | H | o-$CH_2CN$ | $CH_2CH_2CH_2CH_3$ | H | $OCH_3$ | $OCH_3$ | CH | |
| O | O | H | o-$CH_2CN$ | $CH_2CH_2CH_2CH_3$ | H | Cl | $OCH_3$ | CH | |
| O | O | H | H | $(CO)_2OCH_3$ | H | $CH_3$ | $CH_3$ | CH | |
| O | O | H | H | $(CO)_2OCH_3$ | H | $CH_3$ | $OCH_3$ | CH | |
| O | O | H | H | $(CO)_2OCH_3$ | H | $OCH_3$ | $OCH_3$ | CH | |
| O | O | H | H | $(CO)_2OCH_3$ | H | Cl | $OCH_3$ | CH | |
| O | O | H | H | $(CO)_2OCH_3$ | H | Br | $OCH_3$ | CH | |
| O | O | H | H | $(CO)_2OCH_3$ | H | $CH_3$ | $OCH_3$ | N | |
| O | O | H | H | $(CO)_2OCH_3$ | H | $OCH_3$ | $OCH_3$ | N | |
| O | O | H | H | $(CO)_2OCH_2CH_3$ | H | $CH_3$ | $CH_3$ | CH | |
| O | O | H | H | $(CO)_2OCH_2CH_3$ | H | $CH_3$ | $OCH_3$ | CH | |
| O | O | H | H | $(CO)_2OCH_2CH_3$ | H | $OCH_3$ | $OCH_3$ | CH | |
| O | O | H | H | $(CO)_2OCH_2CH_3$ | H | Cl | $OCH_3$ | CH | |
| O | O | H | H | $(CO)_2OCH_2CH_3$ | H | Br | $OCH_3$ | CH | |
| O | O | H | H | $(CO)_2OCH_2CH_3$ | H | $CH_3$ | $OCH_3$ | N | |
| O | O | H | H | $(CO)_2OCH_2CH_3$ | H | $OCH_3$ | $OCH_3$ | N | |
| O | O | H | H | $(CO)_2OPh$ | H | $CH_3$ | $OCH_3$ | CH | |
| O | O | H | H | $(CO)_2OPh$ | H | $OCH_3$ | $OCH_3$ | CH | |
| O | O | H | H | $CH_2CH_2SeCH_3$ | H | $CH_3$ | $CH_3$ | CH | |
| O | O | H | H | $CH_2CH_2SeCH_3$ | H | $CH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | $CH_2CH_2SeCH_3$ | H | $OCH_3$ | $OCH_3$ | CH | |
| O | O | H | H | $CH_2CH_2SeCH_3$ | H | Cl | $OCH_3$ | CH | |
| O | O | H | H | $CH_2CH_2SeCH_3$ | H | Br | $OCH_3$ | CH | |
| O | O | H | H | $CH_2CH_2SeCH_3$ | H | $CH_3$ | $OCH_3$ | N | |
| O | O | H | H | $CH_2CH_2SeCH_3$ | H | $OCH_3$ | $OCH_3$ | N | |
| O | O | H | H | $CH_2CH_2SeCH_2CH_3$ | H | $CH_3$ | $OCH_3$ | CH | |
| O | O | H | H | $CH_2CH_2SeCH_2CH_3$ | H | $OCH_3$ | $OCH_3$ | CH | |
| O | O | H | H | $CH_2SeCH_3$ | H | $CH_3$ | $OCH_3$ | CH | |
| O | O | H | H | $CH_2SeCH_3$ | H | $OCH_3$ | $OCH_3$ | CH | |
| O | O | H | H | $CH_2CH_2SePh$ | H | $CH_3$ | $CH_3$ | CH | |
| O | O | H | H | $CH_2CH_2SePh$ | H | $CH_3$ | $OCH_3$ | CH | |
| O | O | H | H | $CH_2CH_2SePh$ | H | $OCH_3$ | $OCH_3$ | CH | |
| O | O | H | H | $CH_2CH_2SePh$ | H | Cl | $OCH_3$ | CH | |
| O | O | H | H | $CH_2CH_2SePh$ | H | Br | $OCH_3$ | CH | |
| O | O | H | H | $CH_2CH_2SePh$ | H | $CH_3$ | $OCH_3$ | N | |
| O | O | H | H | $CH_2CH_2SePh$ | H | $OCH_3$ | $OCH_3$ | N | |
| O | O | H | H | $(CO)_2CH_3$ | H | $CH_3$ | $OCH_3$ | CH | |
| O | O | H | H | $(CO)_2CH_3$ | H | $OCH_3$ | $OCH_3$ | CH | |
| O | O | H | H | $(CO)_2CH_2CH_3$ | H | $CH_3$ | $OCH_3$ | CH | |
| O | O | H | H | $(CO)_2CH_2CH_3$ | H | $OCH_3$ | $OCH_3$ | CH | |
| O | O | H | H | $(CO)_2Ph$ | H | $CH_3$ | $OCH_3$ | CH | |
| O | O | H | H | $(CO)_2Ph$ | H | $OCH_3$ | $OCH_3$ | CH | |
| O | O | H | H | 2-thienoyl | H | $CH_3$ | $CH_3$ | CH | |
| O | O | H | H | 2-thienoyl | H | $CH_3$ | $OCH_3$ | CH | |
| O | O | H | H | 2-thienoyl | H | $OCH_3$ | $OCH_3$ | CH | |
| O | O | H | H | 2-thienoyl | H | Cl | $OCH_3$ | CH | |
| O | O | H | H | 2-thienoyl | H | Br | $OCH_3$ | CH | |
| O | O | H | H | 2-thienoyl | H | $CH_3$ | $OCH_3$ | N | |
| O | O | H | H | 2-thienoyl | H | $OCH_3$ | $OCH_3$ | N | |
| O | O | H | H | 3-thienoyl | H | $CH_3$ | $CH_3$ | CH | |
| O | O | H | H | 3-thienoyl | H | $CH_3$ | $OCH_3$ | CH | |

Table Ia (continued)

| W | n | R | $R_1$ | $R_2$ | $R_3$ | X | Y | Z | m.p. (°C) |
|---|---|---|---|---|---|---|---|---|---|
| O | O | H | H | 3-thienoyl | H | $OCH_3$ | $OCH_3$ | CH | |
| O | O | H | H | 3-thienoyl | H | Cl | $OCH_3$ | CH | |
| O | O | H | H | 3-thienoyl | H | Br | $OCH_3$ | CH | |
| O | O | H | H | 3-thienoyl | H | $CH_3$ | $OCH_3$ | N | |
| O | O | H | H | 3-thienoyl | H | $OCH_3$ | $OCH_3$ | N | |
| O | O | H | H | 2-furoyl | H | $CH_3$ | $CH_3$ | CH | |
| O | O | H | H | 2-furoyl | H | $CH_3$ | $OCH_3$ | CH | |
| O | O | H | H | 2-furoyl | H | $OCH_3$ | $OCH_3$ | CH | |
| O | O | H | H | 2-furoyl | H | Cl | $OCH_3$ | CH | |
| O | O | H | H | 2-furoyl | H | Br | $OCH_3$ | CH | |
| O | O | H | H | 2-furoyl | H | $CH_3$ | $OCH_3$ | N | |
| O | O | H | H | 2-furoyl | H | $OCH_3$ | $OCH_3$ | N | |
| O | O | H | H | 3-furoyl | H | $CH_3$ | $CH_3$ | CH | |
| O | O | H | H | 3-furoyl | H | $CH_3$ | $OCH_3$ | CH | |
| O | O | H | H | 3-furoyl | H | $OCH_3$ | $OCH_3$ | CH | |
| O | O | H | H | 3-furoyl | H | Cl | $OCH_3$ | CH | |
| O | O | H | H | 3-furoyl | H | Br | $OCH_3$ | CH | |
| O | O | H | H | 3-furoyl | H | $CH_3$ | $OCH_3$ | N | |
| O | O | H | H | 3-furoyl | H | $OCH_3$ | $OCH_3$ | N | |
| O | O | H | H | $CH_2CH_2$-pyridinium bromide | H | $OCH_3$ | $OCH_3$ | CH | |

**Table Ia (continued)**

| W | n | R | $R_1$ | $R_2$ | $R_3$ | X | Y | Z | m.p.(°C) |
|---|---|---|---|---|---|---|---|---|---|
| O | O | H | H | $C(O)C(CH_3)_3$ | H | $CH_3$ | $CH_3$ | CH | 176-178 |
| O | O | H | H | $C(O)C(CH_3)_3$ | H | $CH_3$ | $OCH_3$ | CH | 201-202 |
| O | O | H | H | $C(O)C(CH_3)_3$ | H | $OCH_3$ | $OCH_3$ | CH | 203-204 |
| O | O | H | H | $C(O)C(CH_3)_3$ | H | Cl | $OCH_3$ | CH | 199-200 |
| O | O | H | H | $C(O)C(CH_3)_3$ | H | Br | $OCH_3$ | CH | |
| O | O | H | H | $C(O)C(CH_3)_3$ | H | $CH_3$ | $OCH_3$ | N | |
| O | O | H | H | $C(O)C(CH_3)_3$ | H | $OCH_3$ | $OCH_3$ | N | |
| O | O | H | H | $C(O)CH_2CH(CH_3)_2$ | H | $CH_3$ | $CH_3$ | CH | |
| O | O | H | H | $C(O)CH_2CH(CH_3)_2$ | H | $CH_3$ | $OCH_3$ | CH | |
| O | O | H | H | $C(O)CH_2CH(CH_3)_2$ | H | $OCH_3$ | $OCH_3$ | CH | |
| O | O | H | H | $C(O)CH_2CH(CH_3)_2$ | H | Cl | $OCH_3$ | CH | |
| O | O | H | H | $C(O)CH_2CH(CH_3)_2$ | H | Br | $OCH_3$ | CH | |
| O | O | H | H | $C(O)CH_2CH(CH_3)_2$ | H | $CH_3$ | $OCH_3$ | N | |
| O | O | H | H | $C(O)CH_2CH(CH_3)_2$ | H | $OCH_3$ | $OCH_3$ | N | |
| O | O | H | H | $C(O)CH(CH_3)_2$ | H | $CH_3$ | $OCH_3$ | CH | |
| O | O | H | H | $C(O)CH(CH_3)_2$ | H | $OCH_3$ | $OCH_3$ | CH | |
| O | O | H | H | $CH_2C(Br)=CH_2$ | H | $CH_3$ | $CH_3$ | CH | 220-222 |
| O | O | H | H | $CH_2C(Br)=CH_2$ | H | $CH_3$ | $OCH_3$ | CH | 213-214 |
| O | O | H | H | $CH_2C(Br)=CH_2$ | H | $OCH_3$ | $OCH_3$ | CH | 211-213 |
| O | O | H | H | $CH_2C(Br)=CH_2$ | H | Cl | $OCH_3$ | CH | 188-190 |
| O | O | H | H | $CH_2C(Br)=CH_2$ | H | Br | $OCH_3$ | CH | |
| O | O | H | H | $CH_2C(Br)=CH_2$ | H | $CH_3$ | $OCH_3$ | N | 190-192 |
| O | O | H | H | $CH_2C(Br)=CH_2$ | H | $OCH_3$ | $OCH_3$ | N | 184-185 |
| O | O | H | H | $CH=CH_2$ | H | $CH_3$ | $CH_3$ | CH | |
| O | O | H | H | $CH=CH_2$ | H | $CH_3$ | $OCH_3$ | CH | |
| O | O | H | H | $CH=CH_2$ | H | $OCH_3$ | $OCH_3$ | CH | |
| O | O | H | H | $CH=CH_2$ | H | Cl | $OCH_3$ | CH | |
| O | O | H | H | $CH=CH_2$ | H | Br | $OCH_3$ | CH | |
| O | O | H | H | $CH=CH_2$ | H | $CH_3$ | $OCH_3$ | N | |
| O | O | H | H | $CH=CH_2$ | H | $OCH_3$ | $OCH_3$ | N | |
| O | O | H | H | $CH_2CH_2SO_2CH_3$ | H | $CH_3$ | $CH_3$ | CH | 207-208 |
| O | O | H | H | $CH_2CH_2SO_2CH_3$ | H | $CH_3$ | $OCH_3$ | CH | 149-150 |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2SO_2CH_3$ | H | $OCH_3$ | $OCH_3$ | CH | 204-205 |
| O | O | H | H | $CH_2CH_2SO_2CH_3$ | H | Cl | $OCH_3$ | CH | 212-214 |
| O | O | H | H | $CH_2CH_2SO_2CH_3$ | H | Br | $OCH_3$ | CH | |
| O | O | H | H | $CH_2CH_2SO_2CH_3$ | H | $CH_3$ | $OCH_3$ | N | 197-198 |
| O | O | H | H | $CH_2CH_2SO_2CH_3$ | H | $OCH_3$ | $OCH_3$ | N | 195-196 |
| O | O | H | H | $CH_2CHF_2$ | H | $CH_3$ | $CH_3$ | CH | |
| O | O | H | H | $CH_2CHF_2$ | H | $CH_3$ | $OCH_3$ | CH | |
| O | O | H | H | $CH_2CHF_2$ | H | $OCH_3$ | $OCH_3$ | CH | |
| O | O | H | H | $CH_2CHF_2$ | H | Cl | $CH_3$ | CH | |
| O | O | H | H | $CH_2CHF_2$ | H | $CH_3$ | $OCH_3$ | N | |
| O | O | H | H | $CH_2CHF_2$ | H | $OCH_3$ | $OCH_3$ | N | |
| O | O | H | H | $CH_2CHCl_2$ | H | $CH_3$ | $CH_3$ | CH | |
| O | O | H | H | $CH_2CHCl_2$ | H | Cl | $OCH_3$ | CH | |
| O | O | H | H | $CH_2CHCl_2$ | H | $CH_3$ | $OCH_3$ | N | |
| O | O | H | H | $CH_2CHCl_2$ | H | $OCH_3$ | $OCH_3$ | N | |
| O | O | H | H | $CH_2OCH_2CH_3$ | H | $CH_3$ | $CH_3$ | CH | |
| O | O | H | H | $CH_2OCH_2CH_3$ | H | $CH_3$ | $OCH_3$ | CH | |
| O | O | H | H | $CH_2OCH_2CH_3$ | H | $OCH_3$ | $OCH_3$ | CH | |
| O | O | H | H | $CH_2OCH_2CH_3$ | H | Cl | $OCH_3$ | CH | |
| O | O | H | H | $CH_2OCH_2CH_3$ | H | $CH_3$ | $OCH_3$ | N | |
| O | O | H | H | $CH_2OCH_2CH_3$ | H | $OCH_3$ | $OCH_3$ | N | |
| O | O | H | H | $CH_2OCH_2CH_2CH_3$ | H | $CH_3$ | $CH_3$ | CH | |
| O | O | H | H | $CH_2OCH_2CH_2CH_3$ | H | $CH_3$ | $OCH_3$ | CH | |
| O | O | H | H | $CH_2OCH_2CH_2CH_3$ | H | $OCH_3$ | $OCH_3$ | CH | |
| O | O | H | H | $CH_2OCH_2CH_2CH_3$ | H | Cl | $OCH_3$ | CH | |
| O | O | H | H | $CH_2OCH_2CH_2CH_3$ | H | $CH_3$ | $OCH_3$ | N | |
| O | O | H | H | $CH_2OCH_2CH_2CH_3$ | H | $OCH_3$ | $OCH_3$ | N | |
| O | O | H | H | $CH_2OCH(CH_3)_2$ | H | $CH_3$ | $CH_3$ | CH | |
| O | O | H | H | $CH_2OCH(CH_3)_2$ | H | $CH_3$ | $OCH_3$ | CH | |
| O | O | H | H | $CH_2OCH(CH_3)_2$ | H | $OCH_3$ | $OCH_3$ | CH | |
| O | O | H | H | $CH_2OCH(CH_3)_2$ | H | Cl | $OCH_3$ | CH | |
| O | O | H | H | $CH_2OCH(CH_3)_2$ | H | $CH_3$ | $OCH_3$ | N | |
| O | O | H | H | $CH_2OCH(CH_3)_2$ | H | $OCH_3$ | $OCH_3$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2OC(CH_3)_3$ | H | $CH_3$ | $CH_3$ | CH | |
| O | O | H | H | $CH_2OC(CH_3)_3$ | H | $CH_3$ | $OCH_3$ | CH | |
| O | O | H | H | $CH_2OC(CH_3)_3$ | H | $OCH_3$ | $OCH_3$ | CH | |
| O | O | H | H | $CH_2OC(CH_3)_3$ | H | Cl | $OCH_3$ | CH | |
| O | O | H | H | $CH_2OC(CH_3)_3$ | H | $CH_3$ | $OCH_3$ | N | |
| O | O | H | H | $CH_2OC(CH_3)_3$ | H | $OCH_3$ | $OCH_3$ | N | |
| O | O | H | H | $CH_2CH_2SPh$ | H | $CH_3$ | $CH_3$ | CH | |
| O | O | H | H | $CH_2CH_2SPh$ | H | $CH_3$ | $OCH_3$ | CH | |
| O | O | H | H | $CH_2CH_2SPh$ | H | $OCH_3$ | $OCH_3$ | CH | |
| O | O | H | H | $CH_2CH_2SPh$ | H | Cl | $OCH_3$ | CH | |
| O | O | H | H | $CH_2CH_2SPh$ | H | $CH_3$ | $OCH_3$ | N | |
| O | O | H | H | $CH_2CH_2SPh$ | H | $OCH_3$ | $OCH_3$ | N | |
| O | O | H | H | $N(CH_3)_2$ | H | $CH_3$ | $CH_3$ | CH | |
| O | O | H | H | $N(CH_3)_2$ | H | $CH_3$ | $OCH_3$ | CH | |
| O | O | H | H | $N(CH_3)_2$ | H | $OCH_3$ | $OCH_3$ | CH | |
| O | O | H | H | $N(CH_3)_2$ | H | Cl | $OCH_3$ | CH | |
| O | O | H | H | $N(CH_3)_2$ | H | $CH_3$ | $OCH_3$ | N | |
| O | O | H | H | $N(CH_3)_2$ | H | $OCH_3$ | $OCH_3$ | N | |
| O | O | H | H | cyclopropyl | H | $CH_3$ | $CH_3$ | CH | |
| O | O | H | H | cyclopropyl | H | $CH_3$ | $OCH_3$ | CH | |
| O | O | H | H | cyclopropyl | H | $OCH_3$ | $OCH_3$ | CH | |
| O | O | H | H | cyclopropyl | H | Cl | $OCH_3$ | CH | |
| O | O | H | H | cyclopropyl | H | $CH_3$ | $OCH_3$ | N | |
| O | O | H | H | cyclopropyl | H | $OCH_3$ | $OCH_3$ | N | |
| O | O | H | H | $CH_2CH(CH_3)OH$ | H | $CH_3$ | $CH_3$ | CH | |
| O | O | H | H | $CH_2CH(CH_3)OH$ | H | Cl | $OCH_3$ | CH | |
| O | O | H | H | $CH_2CH(CH_3)OH$ | H | $CH_3$ | $OCH_3$ | N | |
| O | O | H | H | $CH_2CH(CH_3)OH$ | H | $OCH_3$ | $OCH_3$ | N | |

**Table Ia (continued)**

| W | n | R | $R_1$ | $R_2$ | $R_3$ | X | Y | Z | m.p.(°C) |
|---|---|---|---|---|---|---|---|---|---|
| O | O | H | H | $CH_2CH(CH_3)OC(O)CH_3$ | H | $CH_3$ | $CH_3$ | CH | |
| O | O | H | H | $CH_2CH(CH_3)OC(O)CH_3$ | H | $CH_3$ | $OCH_3$ | CH | |
| O | O | H | H | $CH_2CH(CH_3)OC(O)CH_3$ | H | $OCH_3$ | $OCH_3$ | CH | |
| O | O | H | H | $CH_2CH(CH_3)OC(O)CH_3$ | H | Cl | $OCH_3$ | CH | |
| O | O | H | H | $CH_2CH(CH_3)OC(O)CH_3$ | H | $CH_3$ | $OCH_3$ | N | |
| O | O | H | H | $CH_2CH(CH_3)OC(O)CH_3$ | H | $OCH_3$ | $OCH_3$ | N | |
| O | O | H | H | $CH_2CH(CH_3)OSO_2CH_3$ | H | $CH_3$ | $CH_3$ | CH | |
| O | O | H | H | $CH_2CH(CH_3)OSO_2CH_3$ | H | $CH_3$ | $OCH_3$ | CH | |
| O | O | H | H | $CH_2CH(CH_3)OSO_2CH_3$ | H | $OCH_3$ | $OCH_3$ | CH | |
| O | O | H | H | $CH_2CH(CH_3)OSO_2CH_3$ | H | Cl | $OCH_3$ | CH | |
| O | O | H | H | $CH_2CH(CH_3)OSO_2CH_3$ | H | $CH_3$ | $OCH_3$ | N | |
| O | O | H | H | $CH_2CH(CH_3)OSO_2CH_3$ | H | $OCH_3$ | $OCH_3$ | N | |
| O | O | H | H | $CH_2CH_2CH_2OH$ | H | $CH_3$ | $CH_3$ | CH | |
| O | O | H | H | $CH_2CH_2CH_2OH$ | H | Cl | $OCH_3$ | CH | |
| O | O | H | H | $CH_2CH_2CH_2OH$ | H | $CH_3$ | $OCH_3$ | N | |
| O | O | H | H | $CH_2CH_2CH_2OH$ | H | $OCH_3$ | $OCH_3$ | N | |
| O | O | H | H | $CH_2CH_2CH_2OC(O)CH_3$ | H | $CH_3$ | $CH_3$ | CH | |
| O | O | H | H | $CH_2CH_2CH_2OC(O)CH_3$ | H | $CH_3$ | $OCH_3$ | CH | |
| O | O | H | H | $CH_2CH_2CH_2OC(O)CH_3$ | H | $OCH_3$ | $OCH_3$ | CH | |
| O | O | H | H | $CH_2CH_2CH_2OC(O)CH_3$ | H | Cl | $OCH_3$ | CH | |
| O | O | H | H | $CH_2CH_2CH_2OC(O)CH_3$ | H | $CH_3$ | $OCH_3$ | N | |
| O | O | H | H | $CH_2CH_2CH_2OC(O)CH_3$ | H | $OCH_3$ | $OCH_3$ | N | |
| O | O | H | H | $CH_2CH_2CH_2CH_2OH$ | H | $CH_3$ | $CH_3$ | CH | |
| O | O | H | H | $CH_2CH_2CH_2CH_2OH$ | H | $CH_3$ | $OCH_3$ | CH | |
| O | O | H | H | $CH_2CH_2CH_2CH_2OH$ | H | $OCH_3$ | $OCH_3$ | CH | |
| O | O | H | H | $CH_2CH_2CH_2CH_2OH$ | H | Cl | $OCH_3$ | CH | |
| O | O | H | H | $CH_2CH_2CH_2CH_2OH$ | H | $CH_3$ | $OCH_3$ | N | |
| O | O | H | H | $CH_2CH_2CH_2CH_2OH$ | H | $OCH_3$ | $OCH_3$ | N | |
| O | O | H | H | $CH_2CH_2CH_2CH_2OC(O)CH_3$ | H | $CH_3$ | $CH_3$ | CH | |
| O | O | H | H | $CH_2CH_2CH_2CH_2OC(O)CH_3$ | H | $CH_3$ | $OCH_3$ | CH | 170-173 |
| O | O | H | H | $CH_2CH_2CH_2CH_2OC(O)CH_3$ | H | $OCH_3$ | $OCH_3$ | CH | 199-201 |
| O | O | H | H | $CH_2CH_2CH_2CH_2OC(O)CH_3$ | H | Cl | $OCH_3$ | CH | 196-198 |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2CH_2CH_2OC(O)CH_3$ | H | $CH_3$ | $OCH_3$ | N | |
| O | O | H | H | $CH_2CH_2CH_2CH_2OC(O)CH_3$ | H | $OCH_3$ | $OCH_3$ | N | |
| O | O | H | H | $CH_2CH_2CH_2CH_2OSO_2CH_3$ | H | $CH_3$ | $CH_3$ | CH | |
| O | O | H | H | $CH_2CH_2CH_2CH_2OSO_2CH_3$ | H | $CH_3$ | $OCH_3$ | CH | |
| O | O | H | H | $CH_2CH_2CH_2CH_2OSO_2CH_3$ | H | $OCH_3$ | $OCH_3$ | CH | |
| O | O | H | H | $CH_2CH_2CH_2CH_2OSO_2CH_3$ | H | Cl | $OCH_3$ | CH | |
| O | O | H | H | $CH_2CH_2CH_2CH_2OSO_2CH_3$ | H | $CH_3$ | $OCH_3$ | N | |
| O | O | H | H | $CH_2CH_2CH_2CH_2OSO_2CH_3$ | H | $OCH_3$ | $OCH_3$ | N | |
| O | O | H | H | $CH_2CH(CH_2CH_3)OH$ | H | $CH_3$ | $CH_3$ | CH | |
| O | O | H | H | $CH_2CH(CH_2CH_3)OH$ | H | $CH_3$ | $OCH_3$ | CH | |
| O | O | H | H | $CH_2CH(CH_2CH_3)OH$ | H | $OCH_3$ | $OCH_3$ | CH | |
| O | O | H | H | $CH_2CH(CH_2CH_3)OH$ | H | Cl | $OCH_3$ | CH | |
| O | O | H | H | $CH_2CH(CH_2CH_3)OH$ | H | $CH_3$ | $OCH_3$ | N | |
| O | O | H | H | $CH_2CH(CH_2CH_3)OH$ | H | $OCH_3$ | $OCH_3$ | N | |
| O | O | H | H | $CH_2CH(CH_2CH_3)OC(O)CH_3$ | H | $CH_3$ | $CH_3$ | CH | |
| O | O | H | H | $CH_2CH(CH_2CH_3)OC(O)CH_3$ | H | $CH_3$ | $OCH_3$ | CH | |
| O | O | H | H | $CH_2CH(CH_2CH_3)OC(O)CH_3$ | H | $OCH_3$ | $OCH_3$ | CH | |
| O | O | H | H | $CH_2CH(CH_2CH_3)OC(O)CH_3$ | H | Cl | $OCH_3$ | CH | |
| O | O | H | H | $CH_2CH(CH_2CH_3)OC(O)CH_3$ | H | $CH_3$ | $OCH_3$ | N | |
| O | O | H | H | $CH_2CH(CH_2CH_3)OC(O)CH_3$ | H | $OCH_3$ | $OCH_3$ | N | |
| O | O | H | H | $CH_2CH(CH_2CH_3)OSO_2CH_3$ | H | $CH_3$ | $CH_3$ | CH | |
| O | O | H | H | $CH_2CH(CH_2CH_3)OSO_2CH_3$ | H | $CH_3$ | $OCH_3$ | CH | |
| O | O | H | H | $CH_2CH(CH_2CH_3)OSO_2CH_3$ | H | $OCH_3$ | $OCH_3$ | CH | |
| O | O | H | H | $CH_2CH(CH_2CH_3)OSO_2CH_3$ | H | Cl | $OCH_3$ | CH | |
| O | O | H | H | $CH_2CH(CH_2CH_3)OSO_2CH_3$ | H | $CH_3$ | $OCH_3$ | N | |
| O | O | H | H | $CH_2CH(CH_2CH_3)OSO_2CH_3$ | H | $OCH_3$ | $OCH_3$ | N | |
| O | O | H | H | $CH_2CH_2C{\equiv}CH$ | H | $CH_3$ | $CH_3$ | CH | |
| O | O | H | H | $CH_2CH_2C{\equiv}CH$ | H | Cl | $OCH_3$ | CH | |
| O | O | H | H | $CH_2CH_2C{\equiv}CH$ | H | $CH_3$ | $OCH_3$ | N | |
| O | O | H | H | $CH_2CH_2C{\equiv}CH$ | H | $OCH_3$ | $OCH_3$ | N | |
| O | O | H | H | $CH_2C{\equiv}C-CH_3$ | H | $CH_3$ | $CH_3$ | CH | |
| O | O | H | H | $CH_2C{\equiv}C-CH_3$ | H | Cl | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2C{\equiv}C{-}CH_3$ | H | $CH_3$ | $OCH_3$ | N | |
| O | O | H | H | $CH_2C{\equiv}C{-}CH_3$ | H | $OCH_3$ | $OCH_3$ | N | |
| O | O | H | H | $CH(CH_3)C{\equiv}CH$ | H | $CH_3$ | $CH_3$ | CH | |
| O | O | H | H | $CH(CH_3)C{\equiv}CH$ | H | Cl | $OCH_3$ | CH | |
| O | O | H | H | $CH(CH_3)C{\equiv}CH$ | H | $CH_3$ | $OCH_3$ | N | |
| O | O | H | H | $CH(CH_3)C{\equiv}CH$ | H | $OCH_3$ | $OCH_3$ | N | |
| O | O | H | H | 3-epoxybutyl | H | $CH_3$ | $CH_3$ | CH | |
| O | O | H | H | 3-epoxybutyl | H | $CH_3$ | $OCH_3$ | CH | |
| O | O | H | H | 3-epoxybutyl | H | $OCH_3$ | $OCH_3$ | CH | |
| O | O | H | H | 3-epoxybutyl | H | Cl | $OCH_3$ | CH | |
| O | O | H | H | 3-epoxybutyl | H | $CH_3$ | $OCH_3$ | N | |
| O | O | H | H | 3-epoxybutyl | H | $OCH_3$ | $OCH_3$ | N | |
| O | O | H | H | $CH_2CH_2NH_2$ | H | $CH_3$ | $CH_3$ | CH | |
| O | O | H | H | $CH_2CH_2NH_2$ | H | $CH_3$ | $OCH_3$ | CH | |
| O | O | H | H | $CH_2CH_2NH_2$ | H | $OCH_3$ | $OCH_3$ | CH | |
| O | O | H | H | $CH_2CH_2NH_2$ | H | Cl | $OCH_3$ | CH | |
| O | O | H | H | $CH_2CH_2NH_2$ | H | $CH_3$ | $OCH_3$ | N | |
| O | O | H | H | $CH_2CH_2NH_2$ | H | $OCH_3$ | $OCH_3$ | N | |
| O | O | H | H | $CH_2CH_2N(CH_3)_2$ | H | $CH_3$ | $CH_3$ | CH | |
| O | O | H | H | $CH_2CH_2N(CH_3)_2$ | H | $CH_3$ | $OCH_3$ | CH | |
| O | O | H | H | $CH_2CH_2N(CH_3)_2$ | H | $OCH_3$ | $OCH_3$ | CH | |
| O | O | H | H | $CH_2CH_2N(CH_3)_2$ | H | Cl | $OCH_3$ | CH | |
| O | O | H | H | $CH_2CH_2N(CH_3)_2$ | H | $CH_3$ | $OCH_3$ | N | |
| O | O | H | H | $CH_2CH_2N(CH_3)_2$ | H | $OCH_3$ | $OCH_3$ | N | |
| O | O | H | H | $CH_2CH_2CH_2N(CH_3)_2$ | H | $CH_3$ | $CH_3$ | CH | |
| O | O | H | H | $CH_2CH_2CH_2N(CH_3)_2$ | H | $CH_3$ | $OCH_3$ | CH | |
| O | O | H | H | $CH_2CH_2CH_2N(CH_3)_2$ | H | $OCH_3$ | $OCH_3$ | CH | |
| O | O | H | H | $CH_2CH_2CH_2N(CH_3)_2$ | H | Cl | $OCH_3$ | CH | |
| O | O | H | H | $CH_2CH_2CH_2N(CH_3)_2$ | H | $CH_3$ | $OCH_3$ | N | |
| O | O | H | H | $CH_2CH_2CH_2N(CH_3)_2$ | H | $OCH_3$ | $OCH_3$ | N | |
| O | O | H | H | $CH_2CH_2NHC(O)CH_3$ | H | $CH_3$ | $CH_3$ | CH | |
| O | O | H | H | $CH_2CH_2NHC(O)CH_3$ | H | Cl | $OCH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | H | $CH_2CH_2NHC(O)CH_3$ | H | $CH_3$ | $OCH_3$ | N | |
| O | O | H | H | $CH_2CH_2NHC(O)CH_3$ | H | $OCH_3$ | $OCH_3$ | N | |
| O | O | H | H | $CH_2CH_2NHCH_3$ | H | $CH_3$ | $CH_3$ | CH | |
| O | O | H | H | $CH_2CH_2NHCH_3$ | H | Cl | $OCH_3$ | CH | |
| O | O | H | H | $CH_2CH_2NHCH_3$ | H | $CH_3$ | $OCH_3$ | N | |
| O | O | H | H | $CH_2CH_2NHCH_3$ | H | $OCH_3$ | $OCH_3$ | N | |
| O | O | H | H | $CH_2C(CH_3)=NOH$ | H | $CH_3$ | $CH_3$ | CH | |
| O | O | H | H | $CH_2C(CH_3)=NOH$ | H | Cl | $OCH_3$ | CH | |
| O | O | H | H | $CH_2C(CH_3)=NOH$ | H | $CH_3$ | $OCH_3$ | N | |
| O | O | H | H | $CH_2C(CH_3)=NOH$ | H | $OCH_3$ | $OCH_3$ | N | |
| O | O | H | H | $CH_2C(CH_3)=NNH_2$ | H | $CH_3$ | $CH_3$ | CH | |
| O | O | H | H | $CH_2C(CH_3)=NNH_2$ | H | $CH_3$ | $OCH_3$ | CH | |
| O | O | H | H | $CH_2C(CH_3)=NNH_2$ | H | $OCH_3$ | $OCH_3$ | CH | |
| O | O | H | H | $CH_2C(CH_3)=NNH_2$ | H | Cl | $OCH_3$ | CH | |
| O | O | H | H | $CH_2C(CH_3)=NNH_2$ | H | $CH_3$ | $OCH_3$ | N | |
| O | O | H | H | $CH_2C(CH_3)=NNH_2$ | H | $OCH_3$ | $OCH_3$ | N | |
| O | O | H | H | $CH_2C(CH_3)=NNHPh$ | H | $CH_3$ | $CH_3$ | CH | |
| O | O | H | H | $CH_2C(CH_3)=NNHPh$ | H | $CH_3$ | $OCH_3$ | CH | |
| O | O | H | H | $CH_2C(CH_3)=NNHPh$ | H | $OCH_3$ | $OCH_3$ | CH | |
| O | O | H | H | $CH_2C(CH_3)=NNHPh$ | H | Cl | $OCH_3$ | CH | |
| O | O | H | H | $CH_2C(CH_3)=NNHPh$ | H | $CH_3$ | $OCH_3$ | N | |
| O | O | H | H | $CH_2C(CH_3)=NNHPh$ | H | $OCH_3$ | $OCH_3$ | N | |
| O | O | H | H | $CH_2C(CH_3)=NNHCH_3$ | H | $CH_3$ | $CH_3$ | CH | |
| O | O | H | H | $CH_2C(CH_3)=NNHCH_3$ | H | $CH_3$ | $OCH_3$ | CH | |
| O | O | H | H | $CH_2C(CH_3)=NNHCH_3$ | H | $OCH_3$ | $OCH_3$ | CH | |
| O | O | H | H | $CH_2C(CH_3)=NNHCH_3$ | H | Cl | $OCH_3$ | CH | |
| O | O | H | H | $CH_2C(CH_3)=NNHCH_3$ | H | $CH_3$ | $OCH_3$ | N | |
| O | O | H | H | $CH_2C(CH_3)=NNHCH_3$ | H | $OCH_3$ | $OCH_3$ | N | |
| O | O | H | H | $CH_2CH_2P(O)(OCH_3)_2$ | H | $CH_3$ | $CH_3$ | CH | |
| O | O | H | H | $CH_2CH_2P(O)(OCH_3)_2$ | H | $CH_3$ | $OCH_3$ | CH | |
| O | O | H | H | $CH_2CH_2P(O)(OCH_3)_2$ | H | $OCH_3$ | $OCH_3$ | CH | |
| O | O | H | H | $CH_2CH_2P(O)(OCH_3)_2$ | H | Cl | $OCH_3$ | CH | |

**Table Ia (continued)**

| W | n | R | $R_1$ | $R_2$ | $R_3$ | X | Y | Z | m.p.(°C) |
|---|---|---|---|---|---|---|---|---|---|
| O | O | H | H | $CH_2CH_2P(O)(OCH_3)_2$ | H | $CH_3$ | $OCH_3$ | N | |
| O | O | H | H | $CH_2CH_2P(O)(OCH_3)_2$ | H | $OCH_3$ | $OCH_3$ | N | |
| O | O | H | H | $CH_2SCN$ | H | $CH_3$ | $CH_3$ | CH | |
| O | O | H | H | $CH_2SCN$ | H | Cl | $OCH_3$ | CH | |
| O | O | H | H | $CH_2SCN$ | H | $CH_3$ | $OCH_3$ | N | |
| O | O | H | H | $CH_2SCN$ | H | $OCH_3$ | $OCH_3$ | N | |
| O | O | H | H | $CH_2CO_2CH(CH_3)_2$ | H | $CH_3$ | $CH_3$ | CH | |
| O | O | H | H | $CH_2CO_2CH(CH_3)_2$ | H | $CH_3$ | $OCH_3$ | CH | |
| O | O | H | H | $CH_2CO_2CH(CH_3)_2$ | H | $OCH_3$ | $OCH_3$ | CH | |
| O | O | H | H | $CH_2CO_2CH(CH_3)_2$ | H | Cl | $OCH_3$ | CH | |
| O | O | H | H | $CH_2CO_2CH(CH_3)_2$ | H | $CH_3$ | $OCH_3$ | N | |
| O | O | H | H | $CH_2CO_2CH(CH_3)_2$ | H | $OCH_3$ | $OCH_3$ | N | |
| O | O | H | H | cyclopentanone-3-yl | H | $CH_3$ | $CH_3$ | CH | |
| O | O | H | H | cyclopentanone-3-yl | H | $CH_3$ | $OCH_3$ | CH | |
| O | O | H | H | cyclopentanone-3-yl | H | $OCH_3$ | $OCH_3$ | CH | |
| O | O | H | H | cyclopentanone-3-yl | H | Cl | $OCH_3$ | CH | |
| O | O | H | H | cyclopentanone-3-yl | H | $CH_3$ | $OCH_3$ | N | |
| O | O | H | H | cyclopentanone-3-yl | H | $OCH_3$ | $OCH_3$ | N | |
| O | O | H | H | cyclohexanone-3-yl | H | $CH_3$ | $CH_3$ | CH | |
| O | O | H | H | cyclohexanone-3-yl | H | $CH_3$ | $OCH_3$ | CH | |
| O | O | H | H | cyclohexanone-3-yl | H | $OCH_3$ | $OCH_3$ | CH | |
| O | O | H | H | cyclohexanone-3-yl | H | Cl | $OCH_3$ | CH | |
| O | O | H | H | cyclohexanone-3-yl | H | $CH_3$ | $OCH_3$ | N | |
| O | O | H | H | cyclohexanone-3-yl | H | $OCH_3$ | $OCH_3$ | N | |
| O | O | H | H | $CH_2CH{=}CH{-}C{\equiv}CC(CH_3)_3$ | H | $OCH_3$ | $OCH_3$ | CH | |
| O | O | H | H | $CH_2CH{=}CH{-}CH{=}CH_2$ | H | $CH_3$ | $CH_3$ | CH | |
| O | O | H | H | $CH_2CH{=}CH{-}CH{=}CH_2$ | H | $CH_3$ | $OCH_3$ | CH | |
| O | O | H | H | $CH_2CH{=}CH{-}CH{=}CH_2$ | H | $OCH_3$ | $OCH_3$ | CH | |
| O | O | H | H | $CH_2CH{=}CH{-}CH{=}CH_2$ | H | Cl | $OCH_3$ | CH | |
| O | O | H | H | $CH_2CH{=}CH{-}CH{=}CH_2$ | H | $CH_3$ | $OCH_3$ | N | |
| O | O | H | H | $CH_2CH{=}CH{-}CH{=}CH_2$ | H | $OCH_3$ | $OCH_3$ | N | |
| O | O | H | m-Cl | $CH_2CH_2F$ | H | $CH_3$ | $CH_3$ | CH | |

Table Ia (continued)

| W | n | R | $R_1$ | $R_2$ | $R_3$ | X | Y | Z | m.p.(°C) |
|---|---|---|---|---|---|---|---|---|---|
| O | O | H | m-Cl | $CH_2CH_2F$ | H | Cl | $OCH_3$ | CH | |
| O | O | H | m-Cl | $CH_2CH_2F$ | H | $CH_3$ | $OCH_3$ | N | |
| O | O | H | m-Cl | $CH_2CH_2F$ | H | $OCH_3$ | $OCH_3$ | N | |
| O | O | H | m-Cl | $C(O)CH_3$ | H | $CH_3$ | $CH_3$ | CH | |
| O | O | H | m-Cl | $C(O)CH_3$ | H | $CH_3$ | $OCH_3$ | CH | |
| O | O | H | m-Cl | $C(O)CH_3$ | H | Cl | $OCH_3$ | CH | |
| O | O | H | m-Cl | $C(O)CH_3$ | H | $CH_3$ | $OCH_3$ | N | |
| O | O | H | m-Cl | $C(O)CH_3$ | H | $OCH_3$ | $OCH_3$ | N | |
| O | O | H | m-Cl | $CH_2CH_2CH_2F$ | H | $CH_3$ | $CH_3$ | CH | |
| O | O | H | m-Cl | $CH_2CH_2CH_2F$ | H | $CH_3$ | $OCH_3$ | CH | |
| O | O | H | m-Cl | $CH_2CH_2CH_2F$ | H | $OCH_3$ | $OCH_3$ | CH | |
| O | O | H | m-Cl | $CH_2CH_2CH_2F$ | H | Cl | $OCH_3$ | CH | |
| O | O | H | m-Cl | $CH_2CH_2CH_2F$ | H | $CH_3$ | $OCH_3$ | N | |
| O | O | H | m-Cl | $CH_2CH_2CH_2F$ | H | $OCH_3$ | $OCH_3$ | N | |
| O | O | H | m-Cl | $CH_2OCH_3$ | H | $CH_3$ | $CH_3$ | CH | |
| O | O | H | m-Cl | $CH_2OCH_3$ | H | Cl | $OCH_3$ | CH | |
| O | O | H | m-Cl | $CH_2OCH_3$ | H | $CH_3$ | $OCH_3$ | N | |
| O | O | H | m-Cl | $CH_2OCH_3$ | H | $OCH_3$ | $OCH_3$ | N | |
| O | O | H | m-$CH_3$ | $CH_2CH_2OCH_3$ | H | $CH_3$ | $CH_3$ | CH | 188-193 |
| O | O | H | m-$CH_3$ | $CH_2CH_2OCH_3$ | H | Cl | $OCH_3$ | CH | 119-124 |
| O | O | H | m-$CH_3$ | $CH_2CH_2OCH_3$ | H | $CH_3$ | $OCH_3$ | N | 164-172 |
| O | O | H | m-$CH_3$ | $CH_2CH_2OCH_3$ | H | $OCH_3$ | $OCH_3$ | N | 153-158 |
| O | O | H | p-$CH_3$ | $CH_2CH_2OCH_3$ | H | $CH_3$ | $CH_3$ | CH | |
| O | O | H | p-$CH_3$ | $CH_2CH_2OCH_3$ | H | $CH_3$ | $OCH_3$ | CH | |
| O | O | H | p-$CH_3$ | $CH_2CH_2OCH_3$ | H | $OCH_3$ | $OCH_3$ | CH | |
| O | O | H | p-$CH_3$ | $CH_2CH_2OCH_3$ | H | Cl | $OCH_3$ | CH | |
| O | O | H | p-$CH_3$ | $CH_2CH_2OCH_3$ | H | $CH_3$ | $OCH_3$ | N | |
| O | O | H | p-$CH_3$ | $CH_2CH_2OCH_3$ | H | $OCH_3$ | $OCH_3$ | N | |
| O | O | H | m-$SCH_3$ | $CH_2CH_2F$ | H | $CH_3$ | $CH_3$ | CH | |
| O | O | H | m-$SCH_3$ | $CH_2CH_2F$ | H | Cl | $OCH_3$ | CH | |
| O | O | H | m-$SCH_3$ | $CH_2CH_2F$ | H | $CH_3$ | $OCH_3$ | N | |
| O | O | H | m-$SCH_3$ | $CH_2CH_2F$ | H | $OCH_3$ | $OCH_3$ | N | |

Table Ia (continued)

| W | n | R | $R_1$ | $R_2$ | $R_3$ | X | Y | Z | m.p.(°C) |
|---|---|---|---|---|---|---|---|---|---|
| O | O | H | m-$SO_2CH_3$ | $CH_2CH_2F$ | H | $CH_3$ | $CH_3$ | CH | |
| O | O | H | m-$SO_2CH_3$ | $CH_2CH_2F$ | H | $CH_3$ | $OCH_3$ | CH | |
| O | O | H | m-$SO_2CH_3$ | $CH_2CH_2F$ | H | $OCH_3$ | $OCH_3$ | CH | |
| O | O | H | m-$SO_2CH_3$ | $CH_2CH_2F$ | H | Cl | $OCH_3$ | CH | |
| O | O | H | m-$SO_2CH_3$ | $CH_2CH_2F$ | H | $CH_3$ | $OCH_3$ | N | |
| O | O | H | m-$SO_2CH_3$ | $CH_2CH_2F$ | H | $OCH_3$ | $OCH_3$ | N | |
| O | O | H | m-$CF_3$ | $C(O)CH_3$ | H | $CH_3$ | $CH_3$ | CH | |
| O | O | H | m-$CF_3$ | $C(O)CH_3$ | H | $CH_3$ | $OCH_3$ | CH | |
| O | O | H | m-$CF_3$ | $C(O)CH_3$ | H | Cl | $OCH_3$ | CH | |
| O | O | H | m-$CF_3$ | $C(O)CH_3$ | H | $CH_3$ | $OCH_3$ | N | |
| O | O | H | m-$CF_3$ | $C(O)CH_3$ | H | $OCH_3$ | $OCH_3$ | N | |
| O | O | H | H | $CF_2CF_2H$ | H | $CH_3$ | $CH_3$ | CH | |
| O | O | H | H | $CF_2CF_2H$ | H | Cl | $OCH_3$ | CH | |
| O | O | H | H | $CF_2CF_2H$ | H | $CH_3$ | $OCH_3$ | N | |
| O | O | H | H | $CF_2CF_2H$ | H | $OCH_3$ | $OCH_3$ | N | |
| O | O | H | H | $CH(CH_3)CH(CH_3)_2$ | H | $CH_3$ | $CH_3$ | CH | 214-216 |
| O | O | H | H | $CH(CH_3)CH(CH_3)_2$ | H | $CH_3$ | $OCH_3$ | CH | 230-233 |
| O | O | H | H | $CH(CH_3)CH(CH_3)_2$ | H | $OCH_3$ | $OCH_3$ | CH | 228-231 |
| O | O | H | H | $CH(CH_3)CH(CH_3)_2$ | H | Cl | $OCH_3$ | CH | 224-232 |
| O | O | H | H | $CH(CH_3)CH(CH_3)_2$ | H | $CH_3$ | $OCH_3$ | N | 167-170 |
| O | O | H | H | $CH(CH_3)CH(CH_3)_2$ | H | $OCH_3$ | $OCH_3$ | N | 130-133 |
| O | O | H | H | cyclohexyl | H | $CH_3$ | $CH_3$ | CH | 168-171 |
| O | O | H | H | cyclohexyl | H | Cl | $OCH_3$ | CH | 224-227 |
| O | O | H | H | cyclohexyl | H | $CH_3$ | $OCH_3$ | N | 139-142 |
| O | O | H | H | cyclohexyl | H | $OCH_3$ | $OCH_3$ | N | 184-187 |
| O | O | H | H | cyclopropyl | H | $CH_3$ | $CH_3$ | CH | |
| O | O | H | H | cyclopropyl | H | Cl | $OCH_3$ | CH | |
| O | O | H | H | cyclopropyl | H | $OCH_3$ | $OCH_3$ | N | |
| O | O | H | H | 2-fluorobenzyl | H | $CH_3$ | $CH_3$ | CH | 180-183 |
| O | O | H | H | 2-fluorobenzyl | H | $CH_3$ | $OCH_3$ | CH | 182-185 |
| O | O | H | H | 2-fluorobenzyl | H | Cl | $OCH_3$ | CH | 180-183 |
| O | O | H | H | 2-fluorobenzyl | H | $CH_3$ | $OCH_3$ | N | 172-174 |

Table Ia (continued)

| W | n | R | $R_1$ | $R_2$ | $R_3$ | X | Y | Z | m.p.(°C) |
|---|---|---|---|---|---|---|---|---|---|
| O | O | H | H | 2-fluorobenzyl | H | $OCH_3$ | $OCH_3$ | N | 173-175 |
| O | O | H | H | 2-chlorobenzyl | H | $CH_3$ | $CH_3$ | CH | 194-197 |
| O | O | H | H | 2-chlorobenzyl | H | $CH_3$ | $OCH_3$ | CH | 178-180 |
| O | O | H | H | 2-chlorobenzyl | H | $OCH_3$ | $OCH_3$ | CH | 200-203 |
| O | O | H | H | 2-chlorobenzyl | H | Cl | $OCH_3$ | CH | 194-197 |
| O | O | H | H | 2-chlorobenzyl | H | $CH_3$ | $OCH_3$ | N | 180-182 |
| O | O | H | H | 2-chlorobenzyl | H | $OCH_3$ | $OCH_3$ | N | 163-166 |
| O | O | H | H | 2-bromobenzyl | H | $CH_3$ | $CH_3$ | CH | |
| O | O | H | H | 2-bromobenzyl | H | $CH_3$ | $OCH_3$ | CH | |
| O | O | H | H | 2-bromobenzyl | H | $OCH_3$ | $OCH_3$ | CH | |
| O | O | H | H | 2-bromobenzyl | H | Cl | $OCH_3$ | CH | |
| O | O | H | H | 2-bromobenzyl | H | $CH_3$ | $OCH_3$ | N | |
| O | O | H | H | 2-bromobenzyl | H | $OCH_3$ | $OCH_3$ | N | |
| O | O | H | m-Cl | $CH_2CH_2CH_2F$ | H | Cl | $OCH_3$ | CH | |
| O | O | H | m-$OCH_3$ | $CH_2CH_2CH_2F$ | H | Cl | $OCH_3$ | CH | |
| O | O | H | m-$SCH_3$ | $CH_2CH_2CH_2F$ | H | Cl | $OCH_3$ | CH | |
| O | O | H | m-CN | $CH_2CH_2CH_2F$ | H | Cl | $OCH_3$ | CH | |
| O | O | H | m-F | $CH_2CH_2CH_2F$ | H | Cl | $OCH_3$ | CH | |
| O | O | H | m-$CH_3$ | $CH_2CH_2CH_2F$ | H | Cl | $OCH_3$ | CH | |
| O | O | H | o-Cl | $CH_2CH_2CH_2F$ | H | Cl | $OCH_3$ | CH | |
| O | O | H | o-$OCH_3$ | $CH_2CH_2CH_2F$ | H | Cl | $OCH_3$ | CH | |
| O | O | H | o-$SCH_3$ | $CH_2CH_2CH_2F$ | H | Cl | $OCH_3$ | CH | |
| O | O | H | o-F | $CH_2CH_2CH_2F$ | H | Cl | $OCH_3$ | CH | |
| O | O | H | o-CN | $CH_2CH_2CH_2F$ | H | Cl | $OCH_3$ | CH | |
| O | 1 | H | H | $CH_2CH_2CH_2F$ | H | Cl | $OCH_3$ | CH | |
| O | 1 | H | H | $CH_2CH_2F$ | H | Cl | $OCH_3$ | CH | |
| O | 1 | H | H | $CH_2CH_2F$ | H | $OCH_3$ | $OCH_3$ | CH | |
| O | 1 | H | H | $CH_2CH_2F$ | H | $CH_3$ | $OCH_3$ | CH | |

Table Ib

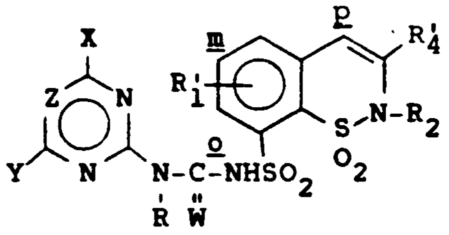

| W | R | $R'_1$ | $R_2$ | $R'_4$ | X | Y | Z | m.p.(°C) |
|---|---|---|---|---|---|---|---|---|
| O | H | H | $CH_2CH_2CH_2CH_2CH_3$ | H | $CH_3$ | $OCH_3$ | CH | |
| O | H | H | $CH_2CH_2CH_2CH_2CH_3$ | H | $OCH_3$ | $OCH_3$ | CH | |
| O | H | H | $CH_2CH_2CH(CH_3)_2$ | H | $CH_3$ | $OCH_3$ | CH | |
| O | H | H | $CH_2CH_2CH(CH_3)_2$ | H | $OCH_3$ | $OCH_3$ | CH | |
| O | H | H | $CH_2CH(CH_3)CH_2CH_3$ | H | $CH_3$ | $OCH_3$ | CH | |
| O | H | H | $CH_2CH(CH_3)CH_2CH_3$ | H | $OCH_3$ | $OCH_3$ | CH | |
| O | H | H | $CH_2CH_2CH_2Cl$ | H | $CH_3$ | $OCH_3$ | CH | |
| O | H | H | $CH_2CH_2CH_2Cl$ | H | $OCH_3$ | $OCH_3$ | CH | |
| O | H | H | $CH_2CH_2OCH_3$ | H | $CH_3$ | $OCH_3$ | CH | |
| O | H | H | $CH_2CH_2OCH_3$ | H | $OCH_3$ | $OCH_3$ | CH | |
| O | H | H | $CH_2OCH_3$ | H | $CH_3$ | $OCH_3$ | CH | |
| O | H | H | $CH_2CH_2F$ | H | $CH_3$ | $CH_3$ | CH | |
| O | H | H | $CH_2CH_2F$ | H | $CH_3$ | $OCH_3$ | CH | |
| O | H | H | $CH_2CH_2F$ | H | $OCH_3$ | $OCH_3$ | CH | |
| O | H | H | $CH_2CH_2F$ | H | $CH_3$ | $OCH_3$ | N | |
| O | H | H | $CH_3$ | $CH_3$ | $CH_3$ | $CH_3$ | CH | |
| O | H | H | $CH_3$ | $CH_3$ | $CH_3$ | $OCH_3$ | CH | |
| O | H | H | $CH_3$ | $CH_3$ | $OCH_3$ | $OCH_3$ | CH | |
| O | H | H | $CH_3$ | $CH_3$ | $CH_3$ | $OCH_3$ | N | |
| O | H | H | $CH_3$ | $CH_3$ | $OCH_3$ | $OCH_3$ | N | |
| O | H | H | $CH_3$ | $CH_3$ | Cl | $OCH_3$ | CH | |
| O | H | H | $CH_3$ | $CH_3$ | Br | $OCH_3$ | CH | |
| O | H | H | $CH_2CH_3$ | $CH_3$ | $CH_3$ | $OCH_3$ | CH | |
| O | H | H | $CH_2CH_3$ | $CH_3$ | $OCH_3$ | $OCH_3$ | CH | |
| O | H | H | $CH_2CH_3$ | $CH_3$ | $CH_3$ | $OCH_3$ | N | |

Table Ib (continued)

| W | R | $R'_1$ | $R_2$ | $R'_4$ | X | Y | Z | m.p. (°C) |
|---|---|---|---|---|---|---|---|---|
| O | H | H | $CH_2CH_2CH_3$ | $CH_3$ | $OCH_3$ | $OCH_3$ | CH | |
| O | H | H | $CH_3$ | $CH_2CH_3$ | $OCH_3$ | $OCH_3$ | CH | |
| O | H | H | $CH_3$ | $CH_2CH_2CH_2CH_3$ | $OCH_3$ | $OCH_3$ | CH | |
| O | H | o-$CH_2CH_3$ | $CH_3$ | H | $CH_3$ | $OCH_3$ | CH | |
| O | H | m-$CH_2CH_3$ | $CH_3$ | H | $OCH_3$ | $OCH_3$ | CH | |
| O | H | m-$CH_2CH_3$ | $CH_3$ | H | $CH_3$ | $OCH_3$ | N | |
| O | H | o-$OCH_2CH_3$ | $CH_3$ | H | $CH_3$ | $OCH_3$ | CH | |
| O | H | m-$OCH_2CH_3$ | $CH_3$ | H | $OCH_3$ | $OCH_3$ | CH | |
| O | H | m-$OCH_2CH_3$ | $CH_3$ | H | $CH_3$ | $OCH_3$ | N | |
| O | H | H | $CH_2CH_2CH_2CH_3$ | $CH_3$ | $CH_3$ | $CH_3$ | CH | |
| O | H | H | $CH_2CH_2CH_2CH_3$ | $CH_3$ | $CH_3$ | $OCH_3$ | CH | |
| O | H | H | $CH_2CH_2CH_2CH_3$ | $CH_3$ | $OCH_3$ | $OCH_3$ | CH | |
| O | H | H | $CH_2CH_2CH_2CH_3$ | $CH_3$ | $CH_3$ | $OCH_3$ | N | |
| O | H | H | $CH_2CH_2CH_2CH_3$ | $CH_3$ | $OCH_3$ | $OCH_3$ | N | |
| O | H | H | $CH_2CH_2CH_2CH_3$ | $CH_3$ | Cl | $OCH_3$ | CH | |
| O | H | H | $CH_2CH_2CH_2CH_3$ | $CH_3$ | Br | $OCH_3$ | CH | |
| O | H | H | $CH_2CH(CH_3)_2$ | $CH_3$ | $CH_3$ | $CH_3$ | CH | |
| O | H | H | $CH_2CH(CH_3)_2$ | $CH_3$ | $CH_3$ | $OCH_3$ | CH | |
| O | H | H | $CH_2CH(CH_3)_2$ | $CH_3$ | $OCH_3$ | $OCH_3$ | CH | |
| O | H | H | $CH_2CH(CH_3)_2$ | $CH_3$ | $CH_3$ | $OCH_3$ | N | |
| O | H | H | $CH_2CH(CH_3)_2$ | $CH_3$ | $OCH_3$ | $OCH_3$ | N | |
| O | H | H | $CH_2CH(CH_3)_2$ | $CH_3$ | Cl | $OCH_3$ | CH | |
| O | H | H | $CH_2CH(CH_3)_2$ | $CH_3$ | Br | $OCH_3$ | CH | |
| O | H | H | $CH_2CH_2OCH_3$ | H | - | $OCH_3$ | N | |
| O | H | H | $CH_2CH_2OCH_3$ | H | - | $OCH_3$ | CH | |
| O | H | o-$CH_3$ | $C(O)CH_3$ | H | $OCH_3$ | $OCH_3$ | CH | |
| O | H | m-$CH_3$ | $C(O)CH_3$ | H | $OCH_3$ | $OCH_3$ | CH | |
| O | H | o-$CH_2CH_2CH_3$ | $C(O)CH_3$ | H | $OCH_3$ | $OCH_3$ | CH | |
| O | H | o-$OCH_3$ | $C(O)CH_3$ | H | $OCH_3$ | $OCH_3$ | CH | |
| O | H | m-$OCH_3$ | $C(O)CH_3$ | H | $OCH_3$ | $OCH_3$ | CH | |
| O | H | o-$OCH_2CH_2CH_3$ | $C(O)CH_3$ | H | $OCH_3$ | $OCH_3$ | CH | |

Table Ib (continued)

| W | R | $R'_1$ | $R_2$ | $R'_4$ | X | Y | Z | m.p. (°C) |
|---|---|---|---|---|---|---|---|---|
| O | H | o-$OCH_2CH_2CH_2Cl$ | $C(O)CH_3$ | H | $OCH_3$ | $OCH_3$ | CH | |
| O | H | o-$CH_2CH_2Br$ | $C(O)CH_3$ | H | $OCH_3$ | $OCH_3$ | CH | |
| O | H | m-$CH_2CF_3$ | $C(O)CH_3$ | H | $OCH_3$ | $OCH_3$ | CH | |
| O | H | o-$CF_3$ | $C(O)CH_3$ | H | $OCH_3$ | $OCH_3$ | CH | |
| O | H | m-$CF_3$ | $C(O)CH_3$ | H | $OCH_3$ | $OCH_3$ | CH | |
| O | H | o-$CH_2CH_2CH_2F$ | $C(O)CH_3$ | H | $OCH_3$ | $OCH_3$ | CH | |
| O | H | o-$SCH_3$ | $C(O)CH_3$ | H | $OCH_3$ | $OCH_3$ | CH | |
| O | H | m-$SCH_3$ | $C(O)CH_3$ | H | $OCH_3$ | $OCH_3$ | CH | |
| O | H | o-$SCH_2CH_2CH_3$ | $C(O)CH_3$ | H | $OCH_3$ | $OCH_3$ | CH | |
| O | H | o-$SCH_2CH_2CH_2F$ | $C(O)CH_3$ | H | $OCH_3$ | $OCH_3$ | CH | |
| O | H | o-$NH_2$ | $C(O)CH_3$ | H | $OCH_3$ | $OCH_3$ | CH | |
| O | H | o-$NHCH_3$ | $C(O)CH_3$ | H | $OCH_3$ | $OCH_3$ | CH | |
| O | H | m-$N(CH_3)_2$ | $C(O)CH_3$ | H | $OCH_3$ | $OCH_3$ | CH | |
| O | H | m-$NH(CH_2)_2CH_3$ | $C(O)CH_3$ | H | $OCH_3$ | $OCH_3$ | CH | |
| O | H | m-$N(CH_2CH_2CH_3)_2$ | $C(O)CH_3$ | H | $OCH_3$ | $OCH_3$ | CH | |
| O | H | o-Cl | $C(O)CH_3$ | H | $OCH_3$ | $OCH_3$ | CH | |
| O | H | m-Cl | $C(O)CH_3$ | H | $OCH_3$ | $OCH_3$ | CH | |
| O | H | p-Cl | $C(O)CH_3$ | H | $OCH_3$ | $OCH_3$ | CH | |
| O | H | o-F | $C(O)CH_3$ | H | $OCH_3$ | $OCH_3$ | CH | |
| O | H | m-F | $C(O)CH_3$ | H | $OCH_3$ | $OCH_3$ | CH | |
| O | H | p-F | $C(O)CH_3$ | H | $OCH_3$ | $OCH_3$ | CH | |
| O | H | o-Br | $C(O)CH_3$ | H | $OCH_3$ | $OCH_3$ | CH | |
| O | H | m-Br | $C(O)CH_3$ | H | $OCH_3$ | $OCH_3$ | CH | |
| O | H | o-$NO_2$ | $C(O)CH_3$ | H | $OCH_3$ | $OCH_3$ | CH | |
| O | H | m-$NO_2$ | $C(O)CH_3$ | H | $OCH_3$ | $OCH_3$ | CH | |

Table Ic

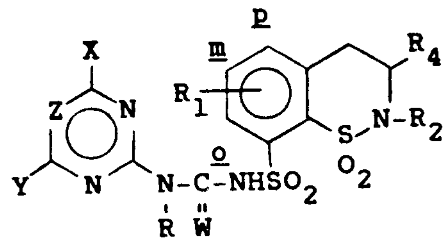

| W | R | $R_1$ | $R_2$ | $R_4$ | X | Y | Z | m.p. (°C) |
|---|---|---|---|---|---|---|---|---|
| O | H | H | $CH_3$ | $CH_3$ | $CH_3$ | $CH_3$ | CH | |
| O | H | H | $CH_3$ | $CH_3$ | $CH_3$ | $OCH_3$ | CH | |
| O | H | H | $CH_3$ | $CH_3$ | $OCH_3$ | $OCH_3$ | CH | |
| O | H | H | $CH_3$ | $CH_3$ | $CH_3$ | $CH_3$ | N | |
| O | H | H | $CH_3$ | $CH_3$ | $CH_3$ | $OCH_3$ | N | |
| O | H | H | $CH_3$ | $CH_3$ | $OCH_3$ | $OCH_3$ | N | |
| O | H | H | $CH_3$ | $CH_3$ | Cl | $OCH_3$ | CH | |
| O | H | H | $CH_3$ | $CH_3$ | Br | $OCH_3$ | CH | |
| O | H | H | $CH_3$ | $CH_3$ | $OCH_3$ | $CH(OCH_3)_2$ | CH | |
| O | H | H | $CH_3$ | $CH_2CH_3$ | $CH_3$ | $OCH_3$ | CH | |
| O | H | H | $CH_3$ | $CH_2CH_3$ | $OCH_3$ | $OCH_3$ | CH | |
| O | H | H | $CH_3$ | $CH_2CH_3$ | $CH_3$ | $OCH_3$ | N | |
| O | H | H | $CH_3$ | $CH_2CH_2CH_3$ | $CH_3$ | $OCH_3$ | CH | |
| O | H | H | $CH_3$ | $CH_2CH_2CH_3$ | $OCH_3$ | $OCH_3$ | CH | |
| O | H | H | $CH_3$ | $CH_2CH_2CH_3$ | $CH_3$ | $OCH_3$ | N | |
| O | H | H | $CH_3$ | $CH(CH_3)_2$ | $OCH_3$ | $OCH_3$ | CH | |
| O | H | H | $CH_3$ | $CH_2CH_2CH_2CH_3$ | $OCH_3$ | $OCH_3$ | CH | |
| O | H | H | $CH_3$ | $CH_2CH(CH_3)_2$ | $OCH_3$ | $OCH_3$ | CH | |
| O | H | H | $CH_3$ | $CH(CH_3)CH_2CH_3$ | $OCH_3$ | $OCH_3$ | CH | |
| O | H | H | $CH_2CH_3$ | $CH_3$ | $CH_3$ | $CH_3$ | CH | |
| O | H | H | $CH_2CH_3$ | $CH_3$ | $CH_3$ | $OCH_3$ | CH | |
| O | H | H | $CH_2CH_3$ | $CH_3$ | $OCH_3$ | $OCH_3$ | CH | |
| O | H | H | $CH_2CH_3$ | $CH_3$ | $CH_3$ | $OCH_3$ | N | |
| O | H | H | $CH_2CH_3$ | $CH_3$ | $OCH_3$ | $OCH_3$ | N | |
| O | H | H | $CH_2CH_3$ | $CH_3$ | Cl | $OCH_3$ | CH | |

## Table Ic (continued)

| W | R | $R_1$ | $R_2$ | $R_4$ | X | Y | Z | m.p.(°C) |
|---|---|---|---|---|---|---|---|---|
| O | H | H | $CH_2CH_3$ | $CH_3$ | Br | $OCH_3$ | CH | |
| O | H | H | $CH_2CH_2CH_3$ | $CH_3$ | $CH_3$ | $OCH_3$ | CH | |
| O | H | H | $CH_2CH_2CH_3$ | $CH_3$ | $OCH_3$ | $OCH_3$ | CH | |
| O | H | H | $CH_2CH_2CH_2CH_3$ | $CH_3$ | $CH_3$ | $CH_3$ | CH | |
| O | H | H | $CH_2CH_2CH_2CH_3$ | $CH_3$ | $CH_3$ | $OCH_3$ | CH | |
| O | H | H | $CH_2CH_2CH_2CH_3$ | $CH_3$ | $OCH_3$ | $OCH_3$ | CH | |
| O | H | H | $CH_2CH_2CH_2CH_3$ | $CH_3$ | $CH_3$ | $OCH_3$ | N | |
| O | H | H | $CH_2CH_2CH_2CH_3$ | $CH_3$ | $OCH_3$ | $OCH_3$ | N | |
| O | H | H | $CH_2CH_2CH_2CH_3$ | $CH_3$ | Cl | $OCH_3$ | CH | |
| O | H | H | $CH_2CH_2CH_2CH_3$ | $CH_3$ | Br | $OCH_3$ | CH | |
| O | H | H | $CH_2CH(CH_3)_2$ | $CH_3$ | $CH_3$ | $CH_3$ | CH | |
| O | H | H | $CH_2CH(CH_3)_2$ | $CH_3$ | $CH_3$ | $OCH_3$ | CH | |
| O | H | H | $CH_2CH(CH_3)_2$ | $CH_3$ | $OCH_3$ | $OCH_3$ | CH | |
| O | H | H | $CH_2CH(CH_3)_2$ | $CH_3$ | $CH_3$ | $OCH_3$ | N | |
| O | H | H | $CH_2CH(CH_3)_2$ | $CH_3$ | $OCH_3$ | $OCH_3$ | N | |
| O | H | H | $CH_2CH(CH_3)_2$ | $CH_3$ | Cl | $OCH_3$ | CH | |
| O | H | H | $CH_2CH(CH_3)_2$ | $CH_3$ | Br | $OCH_3$ | CH | |
| O | H | H | $CH_2CH(CH_3)CH_2CH_3$ | H | $CH_3$ | $OCH_3$ | CH | |
| O | H | H | $CH_2CH(CH_3)CH_2CH_3$ | H | $OCH_3$ | $OCH_3$ | CH | |
| O | H | H | $CH_2CH_2CH_2CH_2CH_3$ | H | $CH_3$ | $OCH_3$ | CH | |
| O | H | H | $CH_2CH_2CH_2CH_2CH_3$ | H | $OCH_3$ | $OCH_3$ | CH | |
| O | H | H | $CH_2CH_2OCH_3$ | H | $CH_3$ | $OCH_3$ | CH | |
| O | H | H | $CH_2CH_2OCH_3$ | H | $OCH_3$ | $OCH_3$ | CH | |
| O | H | H | $CH_2CH_2OCH_2CH_3$ | H | $CH_3$ | $OCH_3$ | CH | |
| O | H | H | $CH_2CH_2OCH_2CH_3$ | H | $OCH_3$ | $OCH_3$ | CH | |
| O | H | H | $CH_2CH_2CH_2Cl$ | H | $CH_3$ | $OCH_3$ | CH | |
| O | H | H | $CH_2CH_2CH_2Cl$ | H | $OCH_3$ | $OCH_3$ | CH | |
| O | H | H | $CH_2CH_2SCH_3$ | H | $CH_3$ | $OCH_3$ | CH | |
| O | H | H | $CH_2CH_2SCH_3$ | H | $OCH_3$ | $OCH_3$ | CH | |
| O | H | H | $CH_2CH_2CN$ | H | $CH_3$ | $OCH_3$ | CH | |
| O | H | H | $CH_2CH_2CN$ | H | $OCH_3$ | $OCH_3$ | CH | |

Table Ic (continued)

| W | R | $R_1$ | $R_2$ | $R_4$ | X | Y | Z | m.p. (°C) |
|---|---|---|---|---|---|---|---|---|
| O | H | m-Cl | $CH_3$ | $CH_3$ | $OCH_3$ | $OCH_3$ | CH | |
| O | H | m-Cl | $CH_2CH_3$ | $CH_3$ | $OCH_3$ | $OCH_3$ | CH | |
| O | H | m-Cl | $CH_2CH_2CH_3$ | $CH_3$ | $OCH_3$ | $OCH_3$ | CH | |
| O | H | m-Cl | $CH_2CH_2CH_2CH_3$ | $CH_3$ | $OCH_3$ | $OCH_3$ | CH | |
| O | H | m-Cl | $CH_2CH(CH_3)_2$ | $CH_3$ | $OCH_3$ | $OCH_3$ | CH | |
| O | H | m-Cl | $CH_2CH_2CH_2CH_2CH_3$ | $CH_3$ | $OCH_3$ | $OCH_3$ | CH | |
| O | H | m-Cl | $CH_2CH(CH_3)CH_2CH_3$ | $CH_3$ | $OCH_3$ | $OCH_3$ | CH | |
| O | H | m-Cl | $CH_2CH_2OCH_3$ | $CH_3$ | $OCH_3$ | $OCH_3$ | CH | |
| O | H | m-Cl | $CH_2CH_2OCH_2CH_3$ | $CH_3$ | $OCH_3$ | $OCH_3$ | CH | |
| O | H | m-Cl | $CH_2CH_2CH_2Cl$ | $CH_3$ | $OCH_3$ | $OCH_3$ | CH | |
| O | H | m-Br | $CH_3$ | $CH_3$ | $OCH_3$ | $OCH_3$ | CH | |
| O | H | m-Br | $CH_2CH_3$ | $CH_3$ | $OCH_3$ | $OCH_3$ | CH | |
| O | H | m-Br | $CH_2CH_2CH_3$ | $CH_3$ | $OCH_3$ | $OCH_3$ | CH | |
| O | H | m-Br | $CH_2CH_2CH_2CH_3$ | $CH_3$ | $OCH_3$ | $OCH_3$ | CH | |
| O | H | m-Br | $CH_2CH(CH_3)_2$ | $CH_3$ | $OCH_3$ | $OCH_3$ | CH | |
| O | H | m-Br | $CH_2CH_2CH_2CH_2CH_3$ | $CH_3$ | $OCH_3$ | $OCH_3$ | CH | |
| O | H | m-Br | $CH_2CH(CH_3)CH_2CH_3$ | $CH_3$ | $OCH_3$ | $OCH_3$ | CH | |
| O | H | m-Br | $CH_2CH_2OCH_3$ | $CH_3$ | $OCH_3$ | $OCH_3$ | CH | |
| O | H | m-Br | $CH_2CH_2OCH_2CH_3$ | $CH_3$ | $OCH_3$ | $OCH_3$ | CH | |
| O | H | m-Br | $CH_2CH_2CH_2Cl$ | $CH_3$ | $OCH_3$ | $OCH_3$ | CH | |
| O | H | m-$CH_3$ | $CH_3$ | $CH_3$ | $OCH_3$ | $OCH_3$ | CH | |
| O | H | m-$CH_3$ | $CH_2CH_3$ | $CH_3$ | $OCH_3$ | $OCH_3$ | CH | |
| O | H | m-$CH_3$ | $CH_2CH_2CH_3$ | $CH_3$ | $OCH_3$ | $OCH_3$ | CH | |
| O | H | m-$CH_3$ | $CH_2CH_2CH_2CH_3$ | $CH_3$ | $OCH_3$ | $OCH_3$ | CH | |
| O | H | m-$CH_3$ | $CH_2CH(CH_3)_2$ | $CH_3$ | $OCH_3$ | $OCH_3$ | CH | |
| O | H | m-$CH_3$ | $CH_2CH_2CH_2CH_2CH_3$ | $CH_3$ | $OCH_3$ | $OCH_3$ | CH | |
| O | H | m-$CH_3$ | $CH_2CH(CH_3)CH_2CH_3$ | $CH_3$ | $OCH_3$ | $OCH_3$ | CH | |
| O | H | m-$CH_3$ | $CH_2CH_2OCH_3$ | $CH_3$ | $OCH_3$ | $OCH_3$ | CH | |
| O | H | m-$CH_3$ | $CH_2CH_2OCH_2CH_3$ | $CH_3$ | $OCH_3$ | $OCH_3$ | CH | |
| O | H | m-$CH_3$ | $CH_2CH_2CH_2Cl$ | $CH_3$ | $OCH_3$ | $OCH_3$ | CH | |
| O | H | o-Cl | $CH_3$ | $CH_3$ | $OCH_3$ | $OCH_3$ | CH | |
| O | H | o-Cl | $CH_2CH_3$ | $CH_3$ | $OCH_3$ | $OCH_3$ | CH | |

Table Ic (continued)

| W | R | $R_1$ | $R_2$ | $R_4$ | X | Y | Z | m.p. (°C) |
|---|---|---|---|---|---|---|---|---|
| O | H | o-Cl | $CH_2CH_2CH_3$ | $CH_3$ | $OCH_3$ | $OCH_3$ | CH | |
| O | H | o-Cl | $CH_2CH_2CH_2CH_3$ | $CH_3$ | $OCH_3$ | $OCH_3$ | CH | |
| O | H | o-Cl | $CH_2CH(CH_3)_2$ | $CH_3$ | $OCH_3$ | $OCH_3$ | CH | |
| O | H | o-Cl | $CH_2CH_2CH_2CH_2CH_3$ | $CH_3$ | $OCH_3$ | $OCH_3$ | CH | |
| O | H | o-Cl | $CH_2CH(CH_3)CH_2CH_3$ | $CH_3$ | $OCH_3$ | $OCH_3$ | CH | |
| O | H | o-Cl | $CH_2CH_2OCH_3$ | $CH_3$ | $OCH_3$ | $OCH_3$ | CH | |
| O | H | o-Cl | $CH_2CH_2OCH_2CH_3$ | $CH_3$ | $OCH_3$ | $OCH_3$ | CH | |
| O | H | o-Cl | $CH_2CH_2CH_2Cl$ | $CH_3$ | $OCH_3$ | $OCH_3$ | CH | |
| O | H | o-$CH_3$ | $CH_3$ | $CH_3$ | $OCH_3$ | $OCH_3$ | CH | |
| O | H | o-$CH_3$ | $CH_2CH_3$ | $CH_3$ | $OCH_3$ | $OCH_3$ | CH | |
| O | H | o-$CH_3$ | $CH_2CH_2CH_3$ | $CH_3$ | $OCH_3$ | $OCH_3$ | CH | |
| O | H | o-$CH_3$ | $CH_2CH_2CH_2CH_3$ | $CH_3$ | $OCH_3$ | $OCH_3$ | CH | |
| O | H | o-$CH_3$ | $CH_2CH(CH_3)_2$ | $CH_3$ | $OCH_3$ | $OCH_3$ | CH | |
| O | H | o-$CH_3$ | $CH_2CH_2CH_2CH_2CH_3$ | $CH_3$ | $OCH_3$ | $OCH_3$ | CH | |
| O | H | o-$CH_3$ | $CH_2CH(CH_3)CH_2CH_3$ | $CH_3$ | $OCH_3$ | $OCH_3$ | CH | |
| O | H | o-$CH_3$ | $CH_2CH_2OCH_3$ | $CH_3$ | $OCH_3$ | $OCH_3$ | CH | |
| O | H | o-$CH_3$ | $CH_2CH_2OCH_2CH_3$ | $CH_3$ | $OCH_3$ | $OCH_3$ | CH | |
| O | H | o-$CH_3$ | $CH_2CH_2CH_2Cl$ | $CH_3$ | $OCH_3$ | $OCH_3$ | CH | |
| O | H | H | $CH_2CH(CH_3)=CH_2$ | $CH_3$ | $OCH_3$ | $OCH_3$ | CH | |
| O | H | H | $CH_3$ | $CH_3$ | cyclo-propyl | $OCH_3$ | CH | |
| O | H | H | $CH_3$ | $CH_3$ | cyclo-propyl | $OCH_3$ | N | |
| O | H | H | $C(O)CH_3$ | $CH_3$ | $OCH_3$ | $OCH_3$ | CH | |
| O | H | H | $C(O)CH_2CH_3$ | $CH_3$ | $OCH_3$ | $OCH_3$ | CH | |
| O | H | H | $C(O)CH_2CH_2CH_3$ | $CH_3$ | $OCH_3$ | $OCH_3$ | CH | |
| O | H | H | $CO_2CH_3$ | $CH_3$ | $OCH_3$ | $OCH_3$ | CH | |
| O | H | H | $CO_2CH_2CH_3$ | $CH_3$ | $OCH_3$ | $OCH_3$ | CH | |
| O | H | H | $CH_2CH_2F$ | $CH_3$ | $OCH_3$ | $OCH_3$ | CH | |
| O | H | H | $CH_2CH_2CH_2F$ | $CH_3$ | $OCH_3$ | $OCH_3$ | CH | |
| O | H | H | $CH_2CH_2Cl$ | $CH_3$ | $OCH_3$ | $OCH_3$ | CH | |
| O | H | H | $CH_2OCH_3$ | $CH_3$ | $OCH_3$ | $OCH_3$ | CH | |

Table Id

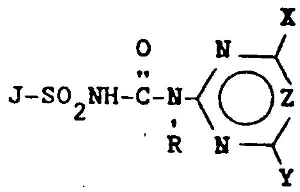

| J | $W_2$ | m' | n | R | $R'_1$ | $R_4$ | $R_5$ | $R_6$ | X | Y | Z | m.p. (°C) |
|---|---|---|---|---|---|---|---|---|---|---|---|---|
| J-11 | O | – | 0 | H | H | $CH_3$ | H | H | $OCH_3$ | $OCH_3$ | CH | |
| J-11 | O | – | 0 | H | H | $CH_3$ | $CH_3$ | H | $OCH_3$ | $OCH_3$ | CH | |
| J-11 | O | – | 1 | H | H | $CH_3$ | H | H | $OCH_3$ | $OCH_3$ | CH | |
| J-11 | O | – | 1 | H | H | $CH_3$ | H | $CH_3$ | $OCH_3$ | $OCH_3$ | CH | |
| J-11 | S | – | 0 | H | H | $CH_3$ | H | H | $OCH_3$ | $OCH_3$ | CH | |
| J-12 | O | 1 | 0 | H | H | – | H | H | $OCH_3$ | $OCH_3$ | CH | |
| J-12 | O | 2 | 0 | H | H | – | H | H | $OCH_3$ | $OCH_3$ | CH | |
| J-12 | S | 1 | 0 | H | H | – | H | H | $OCH_3$ | $OCH_3$ | CH | |
| J-11 | O | – | 0 | H | H | Butyl | H | H | $OCH_3$ | $OCH_3$ | CH | |
| J-11 | O | – | 0 | H | H | $CH_3$ | Butyl | H | $OCH_3$ | $OCH_3$ | CH | |

Table IIa

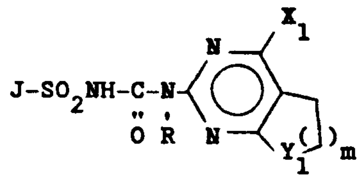

| J | n | R | R'1 or R1 | R2 | R3 | R4 | X1 | Y1 | m | m.p. (°C) |
|---|---|---|---|---|---|---|---|---|---|---|
| J-1 | 0 | H | H | $CH_2CH_2OCH_3$ | H | - | $CH_3$ | $CH_2$ | 1 | |
| J-1 | 0 | H | H | $CH_2CH_2OCH_3$ | H | - | $OCH_3$ | $CH_2$ | 1 | |
| J-1 | 0 | H | H | $CH_2CH_2OCH_3$ | H | - | $OCH_2CH_3$ | $CH_2$ | 1 | |
| J-1 | 0 | H | H | $CH_2CH_2OCH_3$ | H | - | $OCF_2H$ | $CH_2$ | 1 | |
| J-1 | 0 | H | H | $CH_2CH_2OCH_3$ | H | - | $CH_3$ | O | 1 | |
| J-1 | 0 | H | H | $CH_2CH_2OCH_3$ | H | - | $OCH_3$ | O | 1 | |
| J-1 | 0 | H | H | $CH_2CH_2OCH_3$ | H | - | $OCH_2CH_3$ | O | 1 | |
| J-1 | 0 | H | H | $CH_2CH_2OCH_3$ | H | - | $OCF_2H$ | O | 1 | |
| J-1 | 0 | H | H | $CH_2CH_2OCH_3$ | H | - | $CH_3$ | O | 2 | |
| J-1 | 0 | H | H | $CH_2CH_2OCH_3$ | H | - | $OCH_3$ | O | 2 | |
| J-1 | 0 | H | H | $CH_2CH_2OCH_3$ | H | - | $OCH_2CH_3$ | O | 2 | |
| J-1 | 0 | H | H | $CH_2CH_2OCH_2CH_3$ | H | - | $OCF_2H$ | O | 2 | |
| J-1 | 0 | H | H | $CH_2CH_2OCH_2CH_3$ | H | - | $OCH_3$ | $CH_2$ | 1 | |
| J-1 | 0 | H | H | $CH_2CH_2OCH_2CH_3$ | H | - | $OCH_3$ | O | 1 | |
| J-1 | 0 | H | H | $CH_2CH_2OCH_2CH_3$ | H | - | $OCH_3$ | O | 2 | |
| J-1 | 0 | H | H | $CH_2CH_2CH_2CH_2CH_3$ | H | - | $OCH_3$ | $CH_2$ | 1 | |
| J-1 | 0 | H | H | $CH_2CH_2CH_2CH_2CH_3$ | H | - | $OCH_3$ | O | 1 | |
| J-1 | 0 | H | H | $CH_2CH_2CH_2CH_2CH_3$ | H | - | $OCH_3$ | O | 2 | |
| J-1 | 0 | H | H | $CH_2CH(CH_3)CH_2CH_3$ | H | - | $OCH_3$ | $CH_2$ | 1 | |
| J-1 | 0 | H | H | $CH_2CH(CH_3)CH_2CH_3$ | H | - | $OCH_3$ | O | 1 | |
| J-1 | 0 | H | H | $CH_2CH(CH_3)CH_2CH_3$ | H | - | $OCH_3$ | O | 2 | |
| J-1 | 0 | H | H | $CH_2CH_2CH_2Cl$ | H | - | $OCH_3$ | $CH_2$ | 1 | |
| J-1 | 0 | H | H | $CH_2CH_2CH_2Cl$ | H | - | $OCH_3$ | O | 1 | |
| J-1 | 0 | H | H | $CH_2CH_2CH_2Cl$ | H | - | $OCH_3$ | O | 2 | |
| J-1 | 0 | H | H | $CH_2C(CH_3)=CH_2$ | H | - | $OCH_3$ | $CH_2$ | 1 | |
| J-1 | 0 | H | H | $CH_2C(CH_3)=CH_2$ | H | - | $OCH_3$ | O | 1 | |

Table IIa (continued)

| J | n | R | $R'_1$* or $R_1$* | $R_2$ | $R_3$ | $R_4$ | $X_1$ | $Y_1$ | m | m.p. (°C) |
|---|---|---|---|---|---|---|---|---|---|---|
| J-1 | 0 | H | H | $CH_2C(CH_3)=CH_2$ | H | – | $OCH_3$ | O | 2 | |
| J-1 | 0 | H | o-$CH_2CH_3$ | $CH_3$ | H | – | $OCH_3$ | $CH_2$ | 1 | |
| J-1 | 0 | H | o-$CH_2CH_3$ | $CH_3$ | H | – | $OCH_3$ | O | 1 | |
| J-1 | 0 | H | o-$CH_2CH_3$ | $CH_3$ | H | – | $OCH_3$ | O | 2 | |
| J-1 | 0 | H | o-$OCH_2CH_3$ | $CH_3$ | H | – | $OCH_3$ | $CH_2$ | 1 | |
| J-1 | 0 | H | o-$OCH_2CH_3$ | $CH_3$ | H | – | $OCH_3$ | O | 1 | |
| J-1 | 0 | H | o-$OCH_2CH_3$ | $CH_3$ | H | – | $OCH_3$ | O | 2 | |
| J-1 | 0 | H | H | $CH_2CH_2OCH_3$ | H | – | $OCH_3$ | O | 1 | |
| J-1 | 0 | H | H | $CH_2CH_2OCH_3$ | H | – | $OCH_3$ | O | 2 | |
| J-1 | 0 | H | H | $CH_2CH_2OCH_2CH_3$ | H | – | $OCH_3$ | O | 1 | |
| J-1 | 0 | H | H | $CH_2CH_2OCH_2CH_3$ | H | – | $OCH_3$ | O | 2 | |
| J-1 | 0 | H | H | $CH_2CH_2CH_2CH_2CH_3$ | H | – | $OCH_3$ | O | 1 | |
| J-1 | 0 | H | H | $CH_2CH_2CH_2CH_2CH_3$ | H | – | $OCH_3$ | O | 2 | |
| J-1 | 0 | H | H | $CH_2CH(CH_3)CH_2CH_3$ | H | – | $OCH_3$ | O | 1 | |
| J-1 | 0 | H | H | $CH_2CH(CH_3)CH_2CH_3$ | H | – | $OCH_3$ | O | 2 | |
| J-1 | 0 | H | H | $CH_2CH_2CH_2Cl$ | H | – | $OCH_3$ | O | 1 | |
| J-1 | 0 | H | H | $CH_2CH_2CH_2Cl$ | H | – | $OCH_3$ | O | 2 | |
| J-1 | 0 | H | H | $CH_2C(CH_3)=CH_2$ | H | – | $OCH_3$ | O | 1 | |
| J-1 | 0 | H | H | $CH_2C(CH_3)=CH_2$ | H | – | $OCH_3$ | O | 2 | |
| J-4 | – | H | H | H | – | $CH_3$ | $OCH_3$ | O | 1 | |
| J-4 | – | H | H | $CH_3$ | – | $CH_3$ | $OCH_3$ | O | 1 | |
| J-4 | – | H | H | $CH_2CH_3$ | – | $CH_3$ | $OCH_3$ | O | 1 | |
| J-4 | – | H | H | $CH_2CH_2CH_3$ | – | $CH_3$ | $OCH_3$ | O | 1 | |
| J-4 | – | H | H | $CH_2CH_2CH_2CH_3$ | – | $CH_3$ | $OCH_3$ | O | 1 | |
| J-4 | – | H | H | $CH_2CH(CH_3)_2$ | – | $CH_3$ | $OCH_3$ | O | 1 | |
| J-2 | – | H | H | $CH_2CH_2OCH_3$ | – | $CH_3$ | $OCH_3$ | O | 1 | |
| J-2 | – | H | H | $CH_2CH(CH_3)CH_2CH_3$ | – | $CH_3$ | $OCH_3$ | O | 1 | |
| J-2 | – | H | H | $CH_2CH_2CH_2Cl$ | – | $CH_3$ | $OCH_3$ | O | 1 | |
| J-2 | – | H | H | $CH_2C(CH_3)=CH_2$ | – | $CH_3$ | $OCH_3$ | O | 1 | |
| J-3 | – | H | H | $CH_3$ | – | $CH_3$ | $OCH_3$ | O | 1 | |

Table IIa (continued)

| J | n | R | $R_1'$* or $R_1$* | $R_2$ | $R_3$ | $R_4$ | $X_1$ | $Y_1$ | m | m.p. (°C) |
|---|---|---|---|---|---|---|---|---|---|---|
| J-3 | - | H | H | $CH_2CH_3$ | - | $CH_3$ | $OCH_3$ | O | 1 | |
| J-3 | - | H | H | $CH_2CH_2CH_3$ | - | $CH_3$ | $OCH_3$ | O | 1 | |
| J-3 | - | H | H | $CH_2CH_2CH_2CH_3$ | - | $CH_3$ | $OCH_3$ | O | 1 | |
| J-3 | - | H | H | $CH_2CH(CH_3)_2$ | - | $CH_3$ | $OCH_3$ | O | 1 | |
| J-1 | 0 | H | o-$CH_2CH_3$ | $CH_2CH_3$ | H | - | $OCH_3$ | O | 1 | |
| J-1 | 0 | H | o-$CH_2CH_3$ | $CH_2CH_2CH_3$ | H | - | $OCH_3$ | O | 1 | |
| J-1 | 0 | H | m-$CH_2CH_3$ | $CH_2CH_2CH_2CH_3$ | H | - | $OCH_3$ | O | 1 | |
| J-1 | 0 | H | m-$CH_2CH_3$ | $CH_2CH(CH_3)_2$ | H | - | $OCH_3$ | O | 1 | |
| J-1 | 0 | H | m-$CH_2CH_3$ | H | H | - | $OCH_3$ | O | 1 | |
| J-1 | 0 | H | m-$OCH_2CH_3$ | H | H | - | $OCH_3$ | O | 1 | |
| J-1 | 0 | H | m-$OCH_2CH_3$ | $CH_3$ | H | - | $OCH_3$ | O | 1 | |
| J-1 | 0 | H | m-$OCH_2CH_3$ | $CH_2CH_3$ | H | - | $OCH_3$ | O | 1 | |
| J-1 | 0 | H | m-$OCH_2CH_3$ | $CH_2CH_2CH_3$ | H | - | $OCH_3$ | O | 1 | |
| J-1 | 0 | H | o-$OCH_2CH_3$ | $CH_2CH_2CH_2CH_3$ | H | - | $OCH_3$ | O | 1 | |
| J-1 | 0 | H | o-$OCH_2CH_3$ | $CH_2CH(CH_3)_2$ | H | - | $OCH_3$ | O | 1 | |

* - symbols o and m refer to ortho and meta to sulfonylurea bridge respectively.

Table IIb

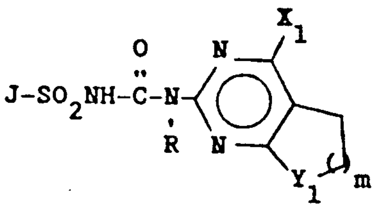

| J | $W_2$ | m' | n | R | $R_1'$ | $R_4$ | $R_5$ | $R_6$ | $X_1$ | $Y_1$ | m | m.p. (°C) |
|---|---|---|---|---|---|---|---|---|---|---|---|---|
| J-11 | O | - | 0 | H | H | $CH_3$ | H | H | $OCH_3$ | O | 1 | |
| J-11 | O | - | 1 | H | H | $CH_3$ | H | H | $OCH_3$ | O | 1 | |
| J-11 | S | - | 0 | H | H | $CH_3$ | H | H | $OCH_3$ | O | 1 | |
| J-11 | S | - | 1 | H | H | $CH_3$ | H | H | $OCH_3$ | O | 1 | |
| J-12 | O | 1 | 0 | H | H | - | H | H | $OCH_3$ | O | 1 | |
| J-12 | O | 2 | 0 | H | H | - | H | H | $OCH_3$ | O | 1 | |
| J-12 | S | 1 | 0 | H | H | - | H | H | $OCH_3$ | O | 1 | |
| J-12 | S | 2 | 0 | H | H | - | H | H | $OCH_3$ | O | 1 | |
| J-12 | O | 1 | 1 | H | H | - | H | H | $OCH_3$ | O | 1 | |
| J-12 | O | 2 | 1 | H | H | - | H | H | $OCH_3$ | O | 1 | |
| J-12 | S | 1 | 1 | H | H | - | H | H | $OCH_3$ | O | 1 | |
| J-12 | S | 2 | 1 | H | H | - | H | H | $OCH_3$ | O | 1 | |

**Table IIIa**

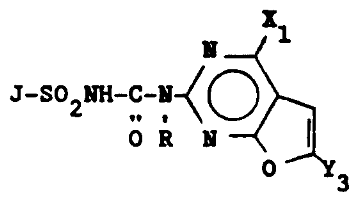

| J | n | R | $R_1$* or $R'_1$* | $R_2$ | $R_3$ | $R_4$ | $X_1$ | $Y_3$ | m.p. (°C) |
|---|---|---|---|---|---|---|---|---|---|
| J-1 | 0 | H | H | $CH_2CH_2OCH_3$ | H | – | $CH_3$ | $CH_3$ | |
| J-1 | 0 | H | H | $CH_2CH_2OCH_3$ | H | – | $OCH_3$ | H | |
| J-1 | 0 | H | H | $CH_2CH_2OCH_3$ | H | – | $OCH_3$ | $CH_3$ | |
| J-1 | 0 | H | H | $CH_2CH_2OCH_3$ | H | – | $OCH_2CH_3$ | $CH_3$ | |
| J-1 | 0 | H | H | $CH_2CH_2OCH_3$ | H | – | $OCF_2H$ | $CH_3$ | |
| J-1 | 0 | H | H | $CH_2CH_2OCH_2CH_3$ | H | – | $OCH_3$ | $CH_3$ | |
| J-1 | 0 | H | H | $CH_2CH_2CH_2CH_2CH_3$ | H | – | $OCH_3$ | $CH_3$ | |
| J-1 | 0 | H | H | $CH_2CH(CH_3)CH_2CH_3$ | H | – | $OCH_3$ | $CH_3$ | |
| J-1 | 0 | H | H | $CH_2CH_2CH_2Cl$ | H | – | $OCH_3$ | $CH_3$ | |
| J-1 | 0 | H | H | $CH_2C(CH_3)=CH_2$ | H | – | $OCH_3$ | $CH_3$ | |
| J-1 | 0 | H | o-$CH_2CH_3$ | $CH_3$ | H | – | $OCH_3$ | $CH_3$ | |
| J-1 | 0 | H | o-$CH_2CH_3$ | $CH_2CH_3$ | H | – | $OCH_3$ | $CH_3$ | |
| J-1 | 0 | H | o-$CH_2CH_3$ | $CH_2CH_2CH_3$ | H | – | $OCH_3$ | $CH_3$ | |
| J-1 | 0 | H | m-$CH_2CH_3$ | $CH_2CH_2CH_2CH_3$ | H | – | $OCH_3$ | $CH_3$ | |
| J-1 | 0 | H | m-$CH_2CH_3$ | $CH_2CH(CH_3)_2$ | H | – | $OCH_3$ | $CH_3$ | |
| J-1 | 0 | H | m-$CH_2CH_3$ | H | H | – | $OCH_3$ | $CH_3$ | |
| J-1 | 0 | H | o-$OCH_2CH_3$ | H | H | – | $OCH_3$ | $CH_3$ | |
| J-1 | 0 | H | o-$OCH_2CH_3$ | $CH_3$ | H | – | $OCH_3$ | $CH_3$ | |
| J-1 | 0 | H | o-$OCH_2CH_3$ | $CH_2CH_3$ | H | – | $OCH_3$ | $CH_3$ | |
| J-1 | 0 | H | m-$OCH_2CH_3$ | $CH_2CH_2CH_3$ | H | – | $OCH_3$ | $CH_3$ | |
| J-1 | 0 | H | m-$OCH_2CH_3$ | $CH_2CH_2CH_2CH_3$ | H | – | $OCH_3$ | $CH_3$ | |
| J-1 | 0 | H | m-$OCH_2CH_3$ | $CH_2CH(CH_3)_2$ | H | – | $OCH_3$ | $CH_3$ | |
| J-1 | 1 | H | H | $CH_2CH_2OCH_3$ | H | – | $OCH_3$ | $CH_3$ | |
| J-1 | 1 | H | H | $CH_2CH_2OCH_2CH_3$ | H | – | $OCH_3$ | $CH_3$ | |
| J-1 | 1 | H | H | $CH_2CH_2CH_2CH_2CH_3$ | H | – | $OCH_3$ | $CH_3$ | |
| J-1 | 1 | H | H | $CH_2CH(CH_3)CH_2CH_3$ | H | – | $OCH_3$ | $CH_3$ | |

Table IIIa (continued)

| J | n | R | $R_1$ or $R_1'$ | $R_2$ | $R_3$ | $R_4$ | $X_1$ | $Y_3$ | m.p. (°C) |
|---|---|---|---|---|---|---|---|---|---|
| J-1 | 1 | H | H | $CH_2CH_2CH_2Cl$ | H | - | $OCH_3$ | $CH_3$ | |
| J-1 | 1 | H | H | $CH_2C(CH_3)=CH_2$ | H | - | $OCH_3$ | $CH_3$ | |
| J-2 | - | H | H | $CH_2CH_2OCH_3$ | - | - | $OCH_3$ | $CH_3$ | |
| J-2 | - | H | H | $CH_2CH_2OCH_2CH_3$ | - | - | $OCH_3$ | $CH_3$ | |
| J-2 | - | H | H | $CH_2CH_2CH_2CH_2CH_3$ | - | - | $OCH_3$ | $CH_3$ | |
| J-2 | - | H | H | $CH_2CH(CH_3)CH_2CH_3$ | - | - | $OCH_3$ | $CH_3$ | |
| J-2 | - | H | H | $CH_2CH_2CH_2Cl$ | - | - | $OCH_3$ | $CH_3$ | |
| J-2 | - | H | H | $CH_2C(CH_3)=CH_2$ | - | - | $OCH_3$ | $CH_3$ | |
| J-3 | - | H | H | H | - | $CH_3$ | $OCH_3$ | $CH_3$ | |
| J-3 | - | H | H | $CH_3$ | - | $CH_3$ | $OCH_3$ | $CH_3$ | |
| J-3 | - | H | H | $CH_2CH_3$ | - | $CH_3$ | $OCH_3$ | $CH_3$ | |
| J-3 | - | H | H | $CH_2CH_2CH_3$ | - | $CH_3$ | $OCH_3$ | $CH_3$ | |
| J-3 | - | H | H | $CH_2CH_2CH_2CH_3$ | - | $CH_3$ | $OCH_3$ | $CH_3$ | |
| J-3 | - | H | H | $CH_2CH(CH_3)_2$ | - | $CH_3$ | $OCH_3$ | $CH_3$ | |
| J-3 | - | H | H | $CH_2CH_2CH_2CH_2CH_3$ | - | $CH_3$ | $OCH_3$ | $CH_3$ | |
| J-3 | - | H | H | $CH_2CH(CH_3)CH_2CH_3$ | - | $CH_3$ | $OCH_3$ | $CH_3$ | |
| J-3 | - | H | H | $CH_2CH_2OCH_3$ | - | $CH_3$ | $OCH_3$ | $CH_3$ | |
| J-3 | - | H | H | $CH_2CH_2CH_2Cl$ | - | $CH_3$ | $OCH_3$ | $CH_3$ | |
| J-4 | - | H | H | H | - | $CH_3$ | $OCH_3$ | $CH_3$ | |
| J-4 | - | H | H | $CH_3$ | - | $CH_3$ | $OCH_3$ | $CH_3$ | |
| J-4 | - | H | H | $CH_2CH_3$ | - | $CH_3$ | $OCH_3$ | $CH_3$ | |
| J-4 | - | H | H | $CH_2CH_2CH_3$ | - | $CH_3$ | $OCH_3$ | $CH_3$ | |
| J-4 | - | H | H | $CH_2CH_2CH_2CH_3$ | - | $CH_3$ | $OCH_3$ | $CH_3$ | |
| J-4 | - | H | H | $CH_2CH(CH_3)_2$ | - | $CH_3$ | $OCH_3$ | $CH_3$ | |
| J-4 | - | H | H | $CH_2CH_2CH_2CH_2CH_3$ | - | $CH_3$ | $OCH_3$ | $CH_3$ | |
| J-4 | - | H | H | $CH_2CH(CH_3)CH_2CH_3$ | - | $CH_3$ | $OCH_3$ | $CH_3$ | |
| J-4 | - | H | H | $CH_2CH_2CH_2Cl$ | - | $CH_3$ | $OCH_3$ | $CH_3$ | |
| J-4 | - | H | H | $CH_2CH_2OCH_3$ | - | $CH_3$ | $OCH_3$ | $CH_3$ | |

* - symbols o and m refer to ortho and meta to the sulfonylurea bridge respectively.

Table IIIb

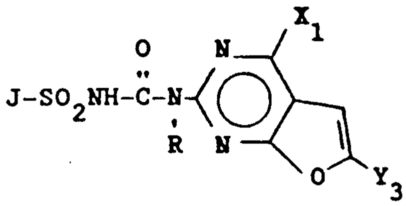

| J | $W_2$ | m' | n | R | $R_1'$ | $R_4$ | $R_5$ | $R_6$ | $X_1$ | $Y_3$ | m.p. (°C) |
|---|---|---|---|---|---|---|---|---|---|---|---|
| J-11 | O | – | 0 | H | H | $CH_3$ | H | H | $OCH_3$ | $CH_3$ | |
| J-11 | O | – | 1 | H | H | $CH_3$ | H | H | $OCH_3$ | $CH_3$ | |
| J-11 | S | – | 0 | H | H | $CH_3$ | H | H | $OCH_3$ | $CH_3$ | |
| J-11 | S | – | 1 | H | H | $CH_3$ | H | H | $OCH_3$ | $CH_3$ | |
| J-12 | O | 1 | 0 | H | H | – | H | H | $OCH_3$ | $CH_3$ | |
| J-12 | O | 2 | 0 | H | H | – | H | H | $OCH_3$ | $CH_3$ | |
| J-12 | O | 1 | 1 | H | H | – | H | H | $OCH_3$ | $CH_3$ | |
| J-12 | O | 2 | 1 | H | H | – | H | H | $OCH_3$ | $CH_3$ | |

## Table IVa

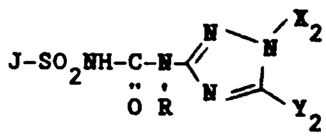

| J | n | R | $R_1$* or $R_1'$* | $R_2$ | $R_3$ | $R_4$ | $X_2$ | $Y_2$ | m.p. (°C) |
|---|---|---|---|---|---|---|---|---|---|
| J-1 | 0 | H | H | $CH_2CH_2OCH_3$ | H | – | $CH_3$ | $OCH_3$ | |
| J-1 | 0 | H | H | $CH_2CH_2OCH_3$ | H | – | $CH_2CH_3$ | $OCH_3$ | |
| J-1 | 0 | H | H | $CH_2CH_2OCH_3$ | H | – | $CH_2CF_3$ | $OCH_3$ | |
| J-1 | 0 | H | H | $CH_2CH_2OCH_3$ | H | – | $CH_3$ | $OCH_2CH_3$ | |
| J-1 | 0 | H | H | $CH_2CH_2OCH_3$ | H | – | $CH_3$ | $SCH_3$ | |
| J-1 | 0 | H | H | $CH_2CH_2OCH_3$ | H | – | $CH_3$ | $SCH_2CH_3$ | |
| J-1 | 0 | H | H | $CH_2CH_2OCH_3$ | H | – | $CH_3$ | $CH_3$ | |
| J-1 | 0 | H | H | $CH_2CH_2OCH_3$ | H | – | $CH_3$ | $CH_2CH_3$ | |
| J-1 | 0 | H | H | $CH_2CH_2CH_2CH_2CH_3$ | H | – | $CH_3$ | $OCH_3$ | |
| J-1 | 0 | H | H | $CH_2CH(CH_3)CH_2CH_3$ | H | – | $CH_3$ | $OCH_3$ | |
| J-1 | 0 | H | H | $CH_2CH_2CH_2Cl$ | H | – | $CH_3$ | $OCH_3$ | |
| J-1 | 0 | H | o-$CH_2CH_3$ | H | H | – | $CH_3$ | $OCH_3$ | |
| J-1 | 0 | H | o-$CH_2CH_3$ | $CH_3$ | H | – | $CH_3$ | $OCH_3$ | |
| J-1 | 0 | H | o-$CH_2CH_3$ | $CH_2CH_3$ | H | – | $CH_3$ | $OCH_3$ | |
| J-1 | 0 | H | o-$CH_2CH_3$ | $CH_2CH_2CH_3$ | H | – | $CH_3$ | $OCH_3$ | |
| J-1 | 0 | H | o-$CH_2CH_3$ | $CH_2CH_2CH_2CH_3$ | H | – | $CH_3$ | $OCH_3$ | |
| J-1 | 0 | H | o-$CH_2CH_3$ | $CH_2CH(CH_3)_2$ | H | – | $CH_3$ | $OCH_3$ | |
| J-1 | 1 | H | H | $CH_2CH_2OCH_3$ | H | – | $CH_3$ | $OCH_3$ | |
| J-1 | 1 | H | H | $CH_2CH_2CH_2CH_2CH_3$ | H | – | $CH_3$ | $OCH_3$ | |
| J-1 | 1 | H | H | $CH_2CH(CH_3)CH_2CH_3$ | H | – | $CH_3$ | $OCH_3$ | |
| J-1 | 1 | H | H | $CH_2CH_2CH_2Cl$ | H | – | $CH_3$ | $OCH_3$ | |
| J-2 | – | H | H | $CH_2CH_2OCH_3$ | – | – | $CH_3$ | $OCH_3$ | |
| J-2 | – | H | H | $CH_2CH_2CH_2CH_2CH_3$ | – | – | $CH_3$ | $OCH_3$ | |
| J-2 | – | H | H | $CH_2CH(CH_3)CH_2CH_3$ | – | – | $CH_3$ | $OCH_3$ | |
| J-2 | – | H | H | $CH_2CH_2CH_2Cl$ | – | – | $CH_3$ | $OCH_3$ | |
| J-3 | – | H | H | $CH_3$ | – | $CH_3$ | $CH_3$ | $OCH_3$ | |
| J-3 | – | H | H | $CH_2CH_3$ | – | $CH_3$ | $CH_3$ | $OCH_3$ | |

Table IVa (continued)

| J | n | R | $R_1$ or $R_1'$ | $R_2$ | $R_3$ | $R_4$ | $X_2$ | $Y_2$ | m.p. (°C) |
|---|---|---|---|---|---|---|---|---|---|
| J-3 | - | H | H | $CH_2CH_2CH_3$ | - | $CH_3$ | $CH_3$ | $OCH_3$ | |
| J-3 | - | H | H | $CH_2CH_2CH_2CH_3$ | - | $CH_3$ | $CH_3$ | $OCH_3$ | |
| J-3 | - | H | H | $CH_2CH(CH_3)_2$ | - | $CH_3$ | $CH_3$ | $OCH_3$ | |
| J-3 | - | H | H | $CH_2CH_2OCH_3$ | - | $CH_3$ | $CH_3$ | $OCH_3$ | |
| J-3 | - | H | H | $CH_2CH_2CH_2CH_2CH_3$ | - | $CH_3$ | $CH_3$ | $OCH_3$ | |
| J-3 | - | H | H | $CH_2CH(CH_3)CH_2CH_3$ | - | $CH_3$ | $CH_3$ | $OCH_3$ | |
| J-3 | - | H | H | $CH_2CH_2CH_2Cl$ | - | $CH_3$ | $CH_3$ | $OCH_3$ | |
| J-4 | - | H | H | $CH_3$ | - | $CH_3$ | $CH_3$ | $OCH_3$ | |
| J-4 | - | H | H | $CH_2CH_3$ | - | $CH_3$ | $CH_3$ | $OCH_3$ | |
| J-4 | - | H | H | $CH_2CH_2CH_3$ | - | $CH_3$ | $CH_3$ | $OCH_3$ | |
| J-4 | - | H | H | $CH_2CH_2CH_2CH_3$ | - | $CH_3$ | $CH_3$ | $OCH_3$ | |
| J-4 | - | H | H | $CH_2CH(CH_3)_2$ | - | $CH_3$ | $CH_3$ | $OCH_3$ | |
| J-4 | - | H | H | $CH_2CH_2OCH_3$ | - | $CH_3$ | $CH_3$ | $OCH_3$ | |
| J-4 | - | H | H | $CH_2CH_2CH_2CH_2CH_3$ | - | $CH_3$ | $CH_3$ | $OCH_3$ | |
| J-4 | - | H | H | $CH_2CH(CH_3)CH_2CH_3$ | - | $CH_3$ | $CH_3$ | $OCH_3$ | |
| J-4 | - | H | H | $CH_2CH_2CH_2Cl$ | - | $CH_3$ | $CH_3$ | $OCH_3$ | |

* - symbols o and m refer to ortho and meta to sulfonylurea bridge respectively.

Table IVb

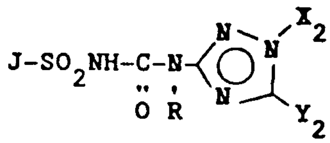

| J | $W_2$ | m' | n | R | $R'_1$ | $R_4$ | $R_5$ | $R_6$ | $X_2$ | $Y_2$ | m.p. (°C) |
|---|---|---|---|---|---|---|---|---|---|---|---|
| J-11 | O | – | 0 | H | H | $CH_3$ | H | H | $CH_3$ | $OCH_3$ | |
| J-11 | O | – | 1 | H | H | $CH_3$ | H | H | $CH_3$ | $OCH_3$ | |
| J-11 | S | – | 0 | H | H | $CH_3$ | H | H | $CH_3$ | $OCH_3$ | |
| J-11 | S | – | 1 | H | H | $CH_3$ | H | H | $CH_3$ | $OCH_3$ | |
| J-12 | O | 1 | 0 | H | H | – | H | H | $CH_3$ | $OCH_3$ | |
| J-12 | O | 1 | 1 | H | H | – | H | H | $CH_3$ | $OCH_3$ | |
| J-12 | O | 2 | 0 | H | H | – | H | H | $CH_3$ | $OCH_3$ | |
| J-12 | O | 2 | 1 | H | H | – | H | H | $CH_3$ | $OCH_3$ | |

Table Va

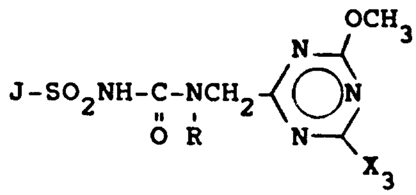

| J | n | R | $R_1$ or $R'_1$ | $R_2$ | $R_3$ | $R_4$ | $X_3$ | m.p. (°C) |
|---|---|---|---|---|---|---|---|---|
| J-1 | 0 | H | H | $CH_2CH_2OCH_3$ | H | - | $CH_3$ | |
| J-1 | 0 | H | H | $CH_2CH_2OCH_3$ | H | - | $OCH_3$ | |
| J-1 | 0 | H | H | $CH_2CH_2CH_2CH_2CH_3$ | H | - | $OCH_3$ | |
| J-1 | 0 | H | H | $CH_2CH_2CH_2Cl$ | H | - | $OCH_3$ | |
| J-1 | 1 | H | H | $CH_2CH_2OCH_3$ | H | - | $OCH_3$ | |
| J-1 | 1 | H | H | $CH_2CH_2CH_2CH_2CH_3$ | H | - | $OCH_3$ | |
| J-1 | 1 | H | H | $CH_2CH_2CH_2Cl$ | H | - | $OCH_3$ | |
| J-2 | - | H | H | $CH_2CH_2OCH_3$ | - | - | $OCH_3$ | |
| J-2 | - | H | H | $CH_2CH_2CH_2CH_2CH_3$ | - | - | $OCH_3$ | |
| J-2 | - | H | H | $CH_2CH_2CH_2Cl$ | - | - | $OCH_3$ | |
| J-3 | - | H | H | $CH_2CH_2OCH_3$ | - | $CH_3$ | $OCH_3$ | |
| J-3 | - | H | H | $CH_2CH_2CH_2CH_2CH_3$ | - | $CH_3$ | $OCH_3$ | |
| J-3 | - | H | H | $CH_2CH_2CH_2Cl$ | - | $CH_3$ | $OCH_3$ | |
| J-3 | - | H | H | $CH_3$ | - | $CH_3$ | $OCH_3$ | |
| J-3 | - | H | H | $CH_2CH_3$ | - | $CH_3$ | $OCH_3$ | |
| J-3 | - | H | H | $CH_2CH_2CH_3$ | - | $CH_3$ | $OCH_3$ | |
| J-3 | - | H | H | $CH_2CH_2CH_2CH_3$ | - | $CH_3$ | $OCH_3$ | |
| J-3 | - | H | H | $CH_2CH(CH_3)_2$ | - | $CH_3$ | $OCH_3$ | |
| J-4 | - | H | H | $CH_2CH_2OCH_3$ | - | $CH_3$ | $OCH_3$ | |
| J-4 | - | H | H | $CH_2CH_2CH_2CH_2CH_3$ | - | $CH_3$ | $OCH_3$ | |
| J-4 | - | H | H | $CH_2CH_2CH_2Cl$ | - | $CH_3$ | $OCH_3$ | |
| J-4 | - | H | H | H | - | $CH_3$ | $OCH_3$ | |
| J-4 | - | H | H | $CH_3$ | - | $CH_3$ | $OCH_3$ | |
| J-4 | - | H | H | $CH_2CH_3$ | - | $CH_3$ | $OCH_3$ | |
| J-4 | - | H | H | $CH_2CH_2CH_3$ | - | $CH_3$ | $OCH_3$ | |
| J-4 | - | H | H | $CH_2CH_2CH_2CH_3$ | - | $CH_3$ | $OCH_3$ | |
| J-4 | - | H | H | $CH_2CH(CH_3)_2$ | - | $CH_3$ | $OCH_3$ | |

Table Vb

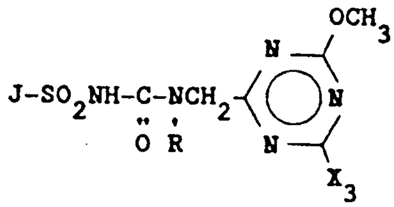

| J | $W_2$ | m' | n | R | $R'_1$ | $R_4$ | $R_5$ | $R_6$ | $X_3$ | m.p.(°C) |
|---|---|---|---|---|---|---|---|---|---|---|
| J-11 | O | - | 0 | H | H | $CH_3$ | H | H | $OCH_3$ | |
| J-11 | O | - | 1 | H | H | $CH_3$ | H | H | $OCH_3$ | |
| J-11 | S | - | 0 | H | H | $CH_3$ | H | H | $OCH_3$ | |
| J-11 | S | - | 1 | H | H | $CH_3$ | H | H | $OCH_3$ | |
| J-12 | O | 1 | 0 | H | H | - | H | H | $OCH_3$ | |
| J-12 | O | 1 | 1 | H | H | - | H | H | $OCH_3$ | |
| J-12 | O | 2 | 0 | H | H | - | H | H | $OCH_3$ | |
| J-12 | O | 2 | 1 | H | H | - | H | H | $OCH_3$ | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 5% to 99% solid or liquid inert diluent(s). More specifically, they will contain these ingredients in the following approximate proportions:

Table VI

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspension | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 °C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 4th Ed., McGraw-Hill, New York, 1963, pp. 8-59ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 8

Wettable Powder

| | |
|---|---|
| 2,3-dihydro-N-[(4,6-dimethoxypyrimidin-2-yl)aminocarbonyl]-2-(2-methoxyethyl)-1,2-benzisothiazole-7-sulfonamide, 1,1-dioxide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 9

Granule

| | |
|---|---|
| Wettable Powder of Example 8 | 5% |
| attapulgite granules (U.S.S. 20-40 mesh; 0.84-0.42 mm) | 95% |

A slurry of wettable powder containing ≈25% solids is sprayed on the surface of attapulgite granules in a double-cone blender. The granules are dried and packaged.

Example 10

Extruded Pellet

| | |
|---|---|
| 2,3-dihydro-N-[(4,6-dimethoxypyrimidin-2-yl)aminocarbonyl]-2-(2-methoxyethyl)-1,2-benzisothiazole-7-sulfonamide, 1,1-dioxide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 11

Low Strength Granule

| | |
|---|---|
| 2,3-dihydro-N-[(4,6-dimethoxypyrimidin-2-yl)aminocarbonyl]-2-(3-methylbutyl)-1,2-benzisothiazole-7-sulfonamide, 1,1-dioxide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 12

Aqueous Suspension

| | |
|---|---|
| 2,3-dihydro-N-[(4,6-dimethoxypyrimidin-2-yl)aminocarbonyl]-2-(2-methoxyethyl)-1,2-benzisothiazole-7-sulfonamide, 1,1-dioxide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 13

Oil Suspension

| | |
|---|---|
| 2,3-dihydro-N-[(4,6-dimethoxypyrimidin-2-yl)aminocarbonyl]-2-(3-methylbutyl)-1,2-benzisothiazole-7-sulfonamide, 1,1-dioxide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 14

Granule

| | |
|---|---|
| 2,3-dihydro-N-[(4,6-dimethoxypyrimidin-2-yl)aminocarbonyl]-2-(2-methoxyethyl)-1,2-benzisothiazole-7-sulfonamide, 1,1-dioxide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 15

High Strength Concentrate

| | |
|---|---|
| 2,3-dihydro-N-[(4,6-dimethoxypyrimidin-2-yl)aminocarbonyl]-2-(2-methoxyethyl)-1,2-benzisothiazole-7-sulfonamide, 1,1-dioxide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 16

Wettable Powder

| | |
|---|---|
| 2,3-dihydro-N-[(4,6-dimethoxypyrimidin-2-yl)aminocarbonyl]-2-(3-methylbutyl)-1,2-benzisothiazole-7-sulfonamide, 1,1-dioxide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 17

Wettable Powder

| | |
|---|---|
| 2,3-dihydro-N-[(4,6-dimethoxypyrimidin-2-yl)aminocarbonyl]-2-(3-methylbutyl)-1,2-benzisothiazole-7-sulfonamide, 1,1-dioxide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 18

Dust

| | |
|---|---|
| 2,3-dihydro-N-[(4,6-dimethoxypyrimidin-2-yl)aminocarbonyl]-2-(3-methylbutyl)-1,2-benzisothiazole-7-sulfonamide, 1,1-dioxide | 10% |
| attapulgite | 10% |
| talc | 80% |

The active ingredient is blended with attapulgite and then passed through a hammer mill to produce particles substantially all below 200 microns. The ground concentrate is then blended with powdered talc until homogeneous.

Example 19

Solution

| | |
|---|---|
| 2,3-dihydro-N-[(4,6-dimethoxypyrimidin-2-yl)aminocarbonyl]-2-(3-methylbutyl)-1,2-benzisothiazole-7-sulfonamide, 1,1-dioxide | 30% |
| dimethylformamide | 70% |

The ingredients are combined and stirred to produce a solution, which can be used for low volume applications.

Solutions of compounds of Formula I with J = J-5 to J-10 should be avoided due to the instability of the compounds when dissolved.

## Utility

The compounds of the present invention are active herbicides. They have utility for broad-spectrum pre- and/or post-emergence weed control in areas where complete control of all vegetation is desired, such as around fuel storage tanks, ammunition depots, industrial storage areas, oil-well sites, drive-in theaters, around billboards, highway and railroad structures. Alternatively, the subject compounds are useful to modify plant growth. Certain of the compounds can be used to control weeds in crops such as wheat. The related compounds where J is $J_1$, n is O, $R_1$ = $R_3$ = H and $R_2$ is isobutyl show excellent control of wild oats both pre- and postemergence and are also useful for preemergence and postemergence control of grassy weeds in broadlead crops such as cotton and soybeans.

The rates of application for the compounds of the invention are determined by a number of factors. including their use as selective or general herbicides, the crop species involved, the types of weeds to be controlled, weather and climate, formulations selected, mode of application, amount of foliage present, etc. In general terms, the subject compounds should be applied at levels of around 0.01 to 5 kg/ha, the lower rates being suggested for use on lighter soils and/or those having a low organic matter content, for selective weed control or for situations where only short-term persistence is required.

The compounds of the invention may be used in combination with any other commercial herbicide; examples of which are those of the triazine, triazole, uracil, urea, amide, diphenylether, carbamate and bipyridylium types.

The herbicidal properties of the subject compounds were discovered in a number of greenhouse tests. The test procedures and results follow.

## Test A

Seeds of crabgrass (Digitaria sp.), barnyard-grass (Echinochloa crusgalli), wild oats (Avena fatua), sicklepod (Cassia obtusifolia), morningglory (Ipomoea sp.), cocklebur (Xanthium sp.), sorghum, corn, soybean, sugarbeet, rice, wheat and purple nutsedge (Cyperus rotundus) tubers, and in certain cases, cotton were planted and treated pre-emergence with the test chemicals dissolved in a non-phytotoxic solvent. At the same time, these crop and weed species, along with cotton and bush bean, were treated post-emergence with a soil/foliage application. At the time of treatment, the plants ranged in height from 2 to 19 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

- C = chlorosis or necrosis;
- E = emergence inhibition;
- G = growth retardation;
- H = formative effects;
- L = lodging;
- U = unusual pigmentation;
- X = axillary stimulation; and
- 6Y = abscised buds or flowers.

The data show that most of the compounds tested are highly active herbicides with growth modifying properties and that some of the compounds tested provide selective weed control in crops such as wheat, cotton and soybeans. It will be noted that all compounds were tested at the low rate of 50 g/ha.

## Compounds

Compound 1

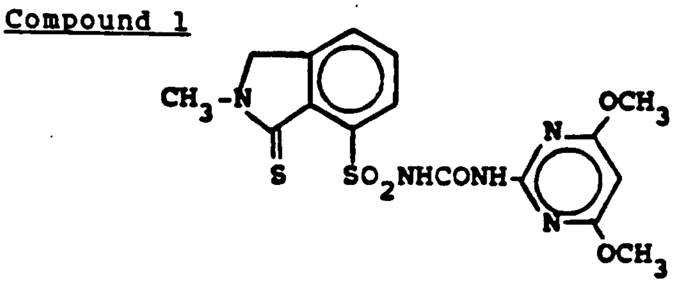

Compound 2

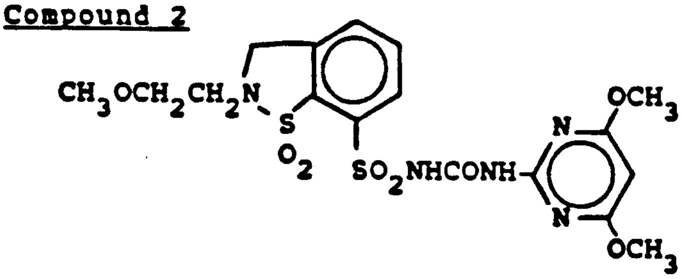

Compound 3

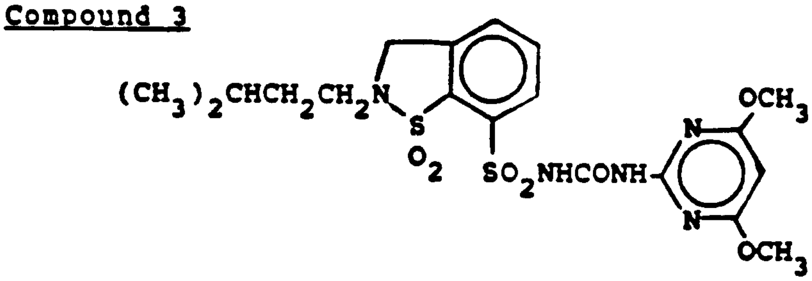

Compound 4

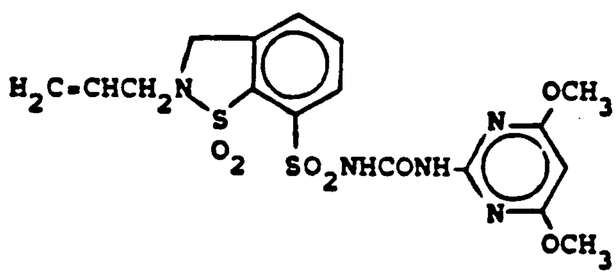

Compound 5

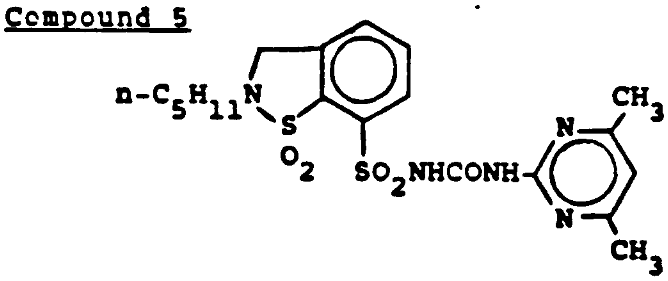

Compounds

Compound 6

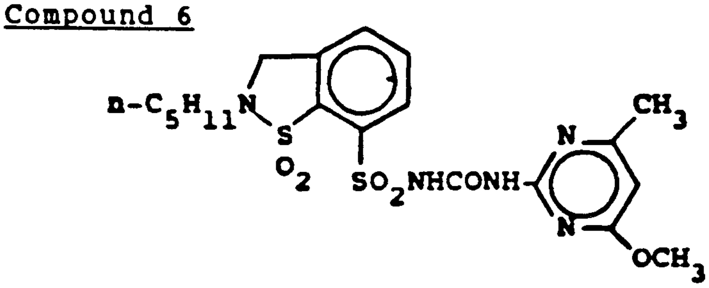

Compound 7

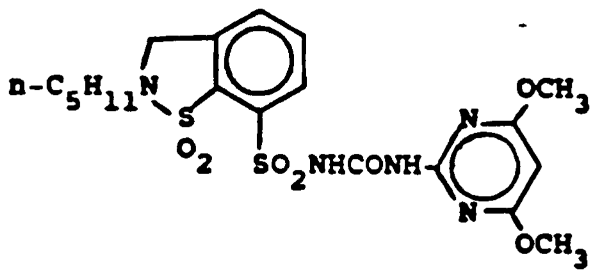

Compound 8

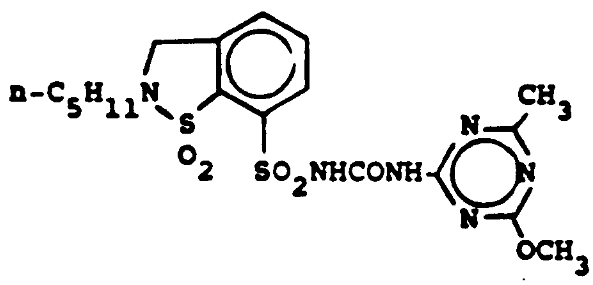

Compound 9

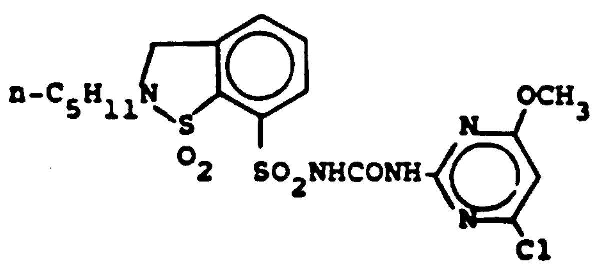

Compounds

Compound 10

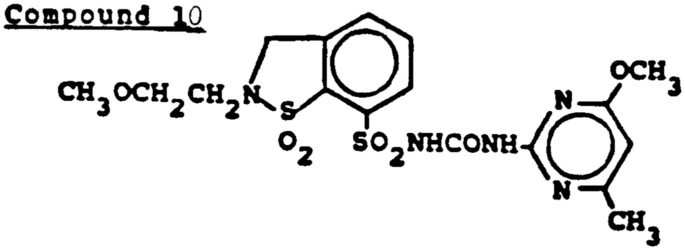

Compound 11

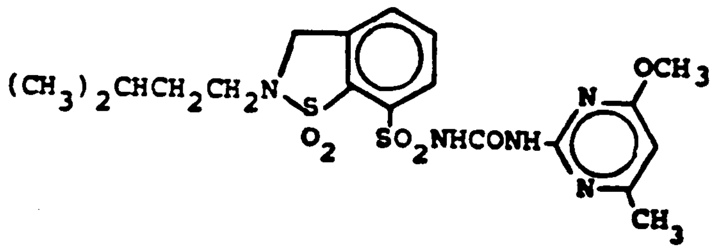

Compound 12

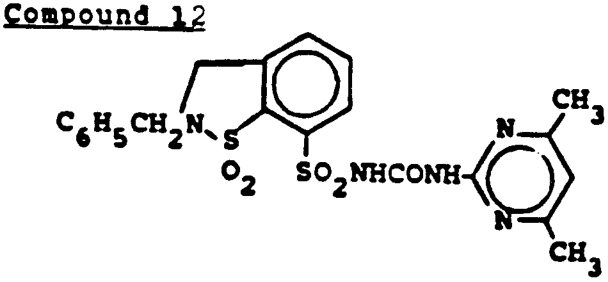

Compound 13

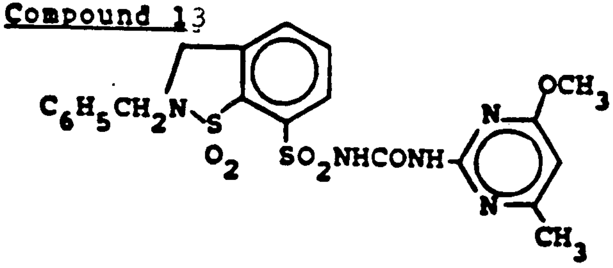

Compound 14

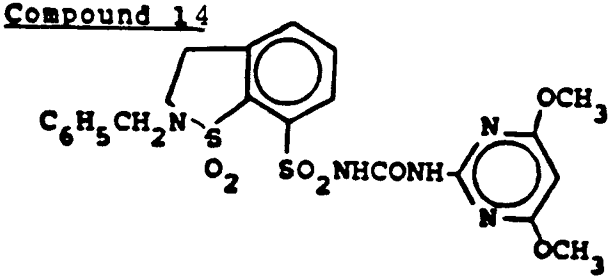

**Compounds**

**Compound 15**

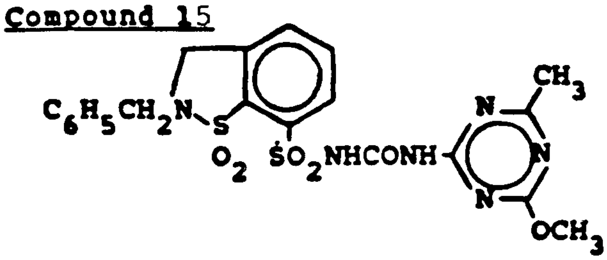

**Compound 16**

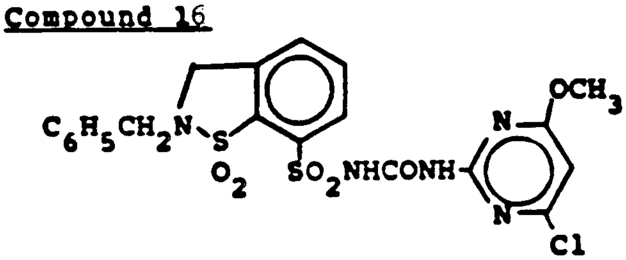

**Compound 17**

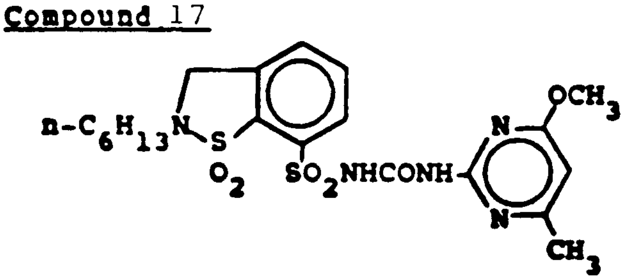

Compounds

Compound 18

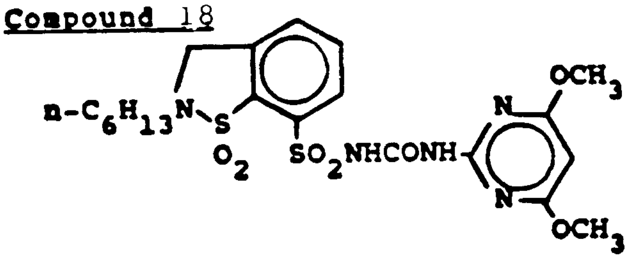

Compound 19

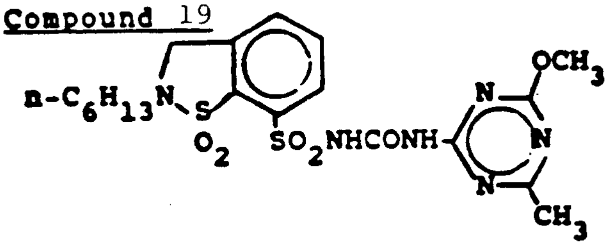

Compound 20

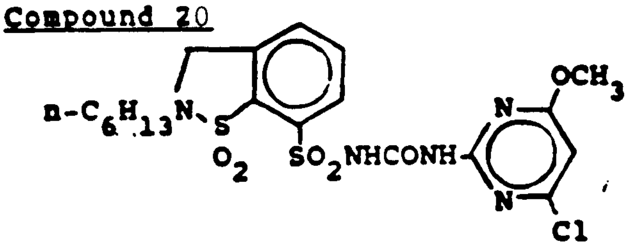

Compounds

Compound 21

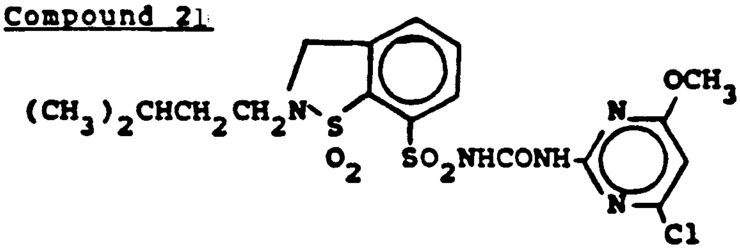

Compound 22

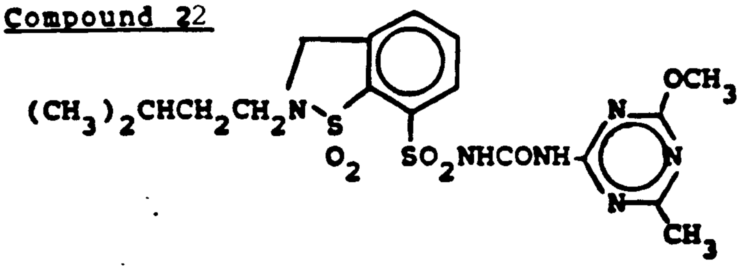

Compound 23

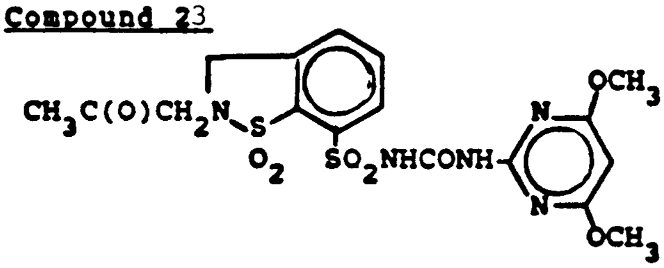

Compound 24

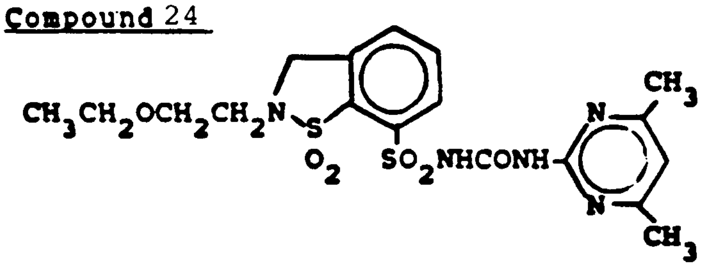

Compounds

Compound 25

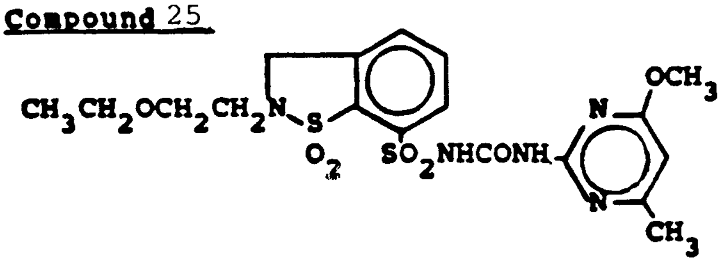

Compound 26

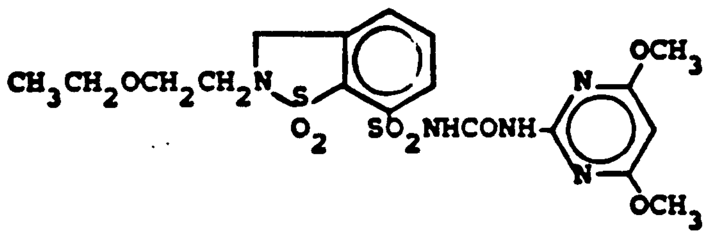

Compound 27

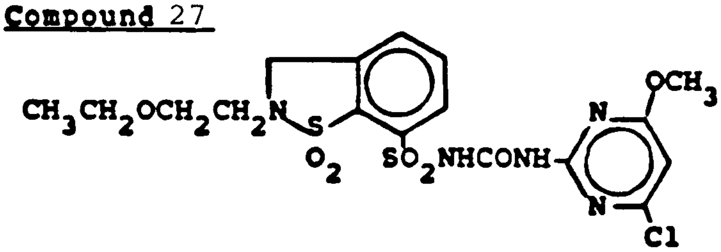

Compounds

Compound 28

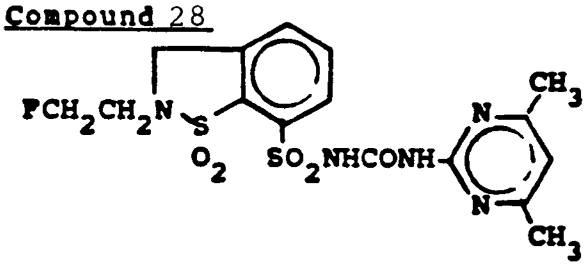

Compound 29

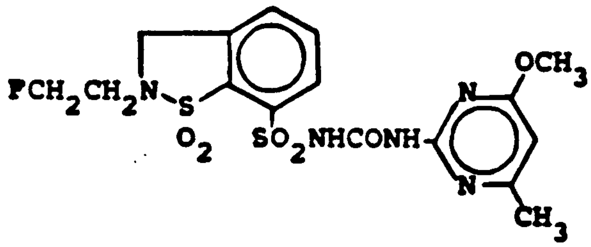

Compound 30

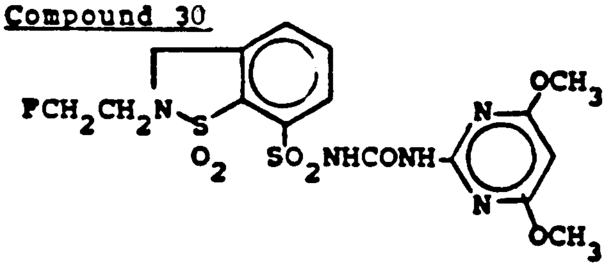

Compound 31

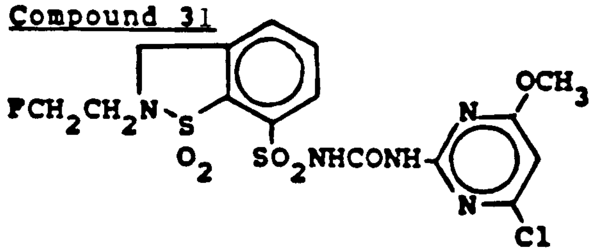

Compound 32

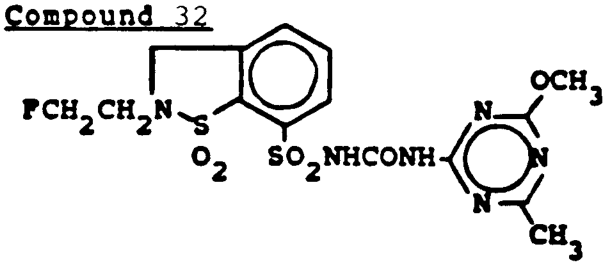

## Compounds

**Compound 33**

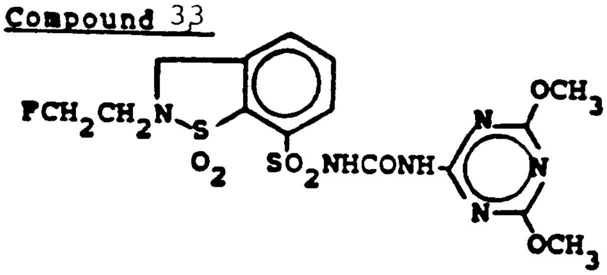

**Compound 34**

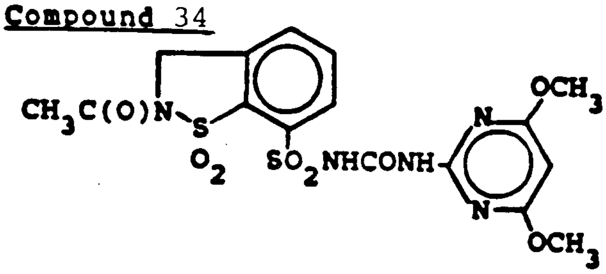

**Compound 35**

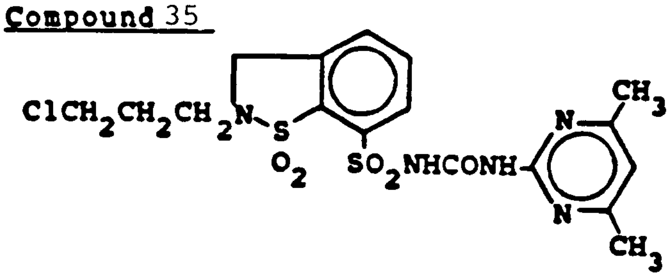

**Compound 36**

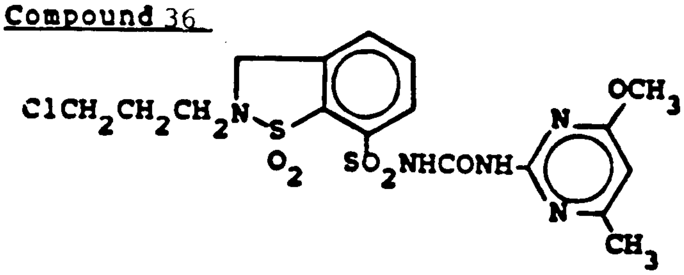

**Compound 37**

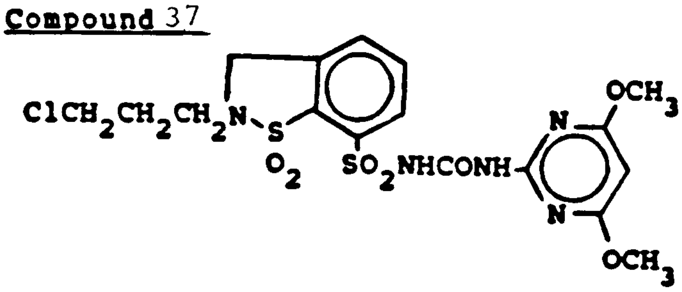

## Compounds

**Compound 38**

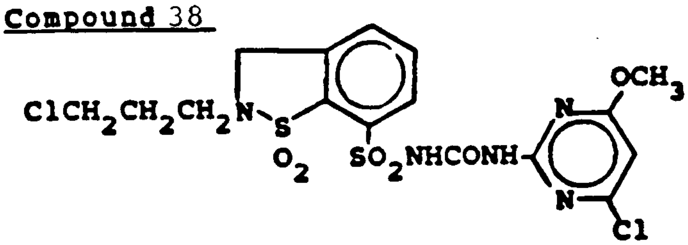

**Compound 39**

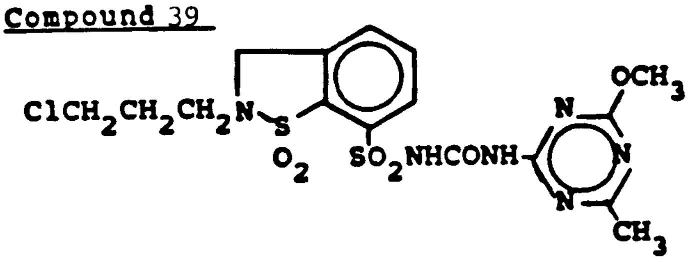

**Compound 40**

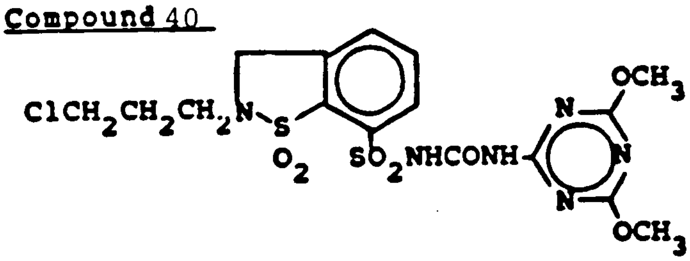

**Compound 41**

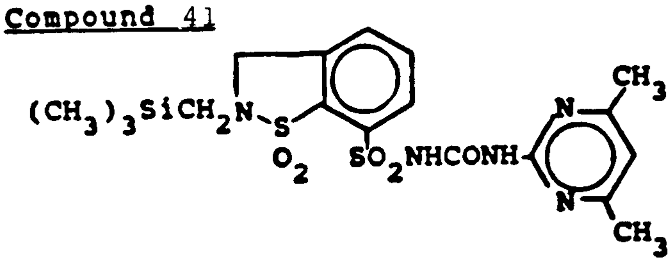

**Compound 42**

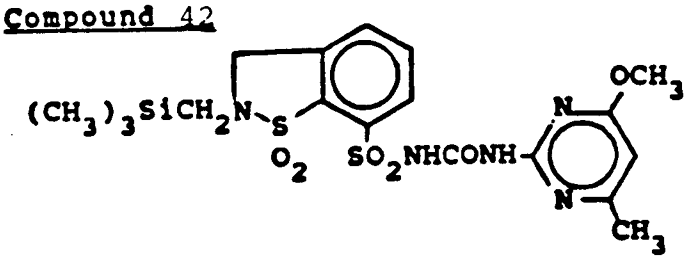

Compounds

Compound 43

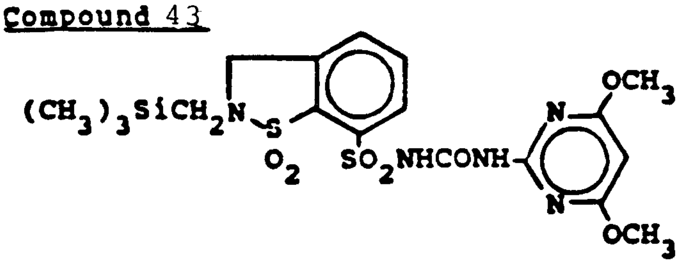

Compound 44

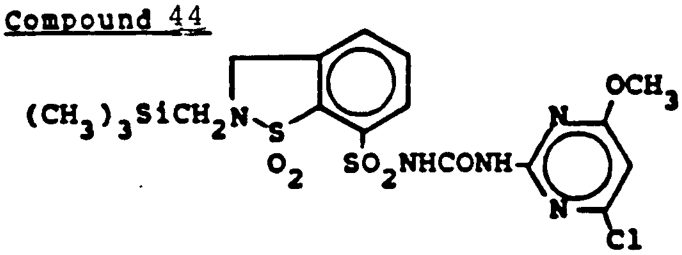

Compound 45

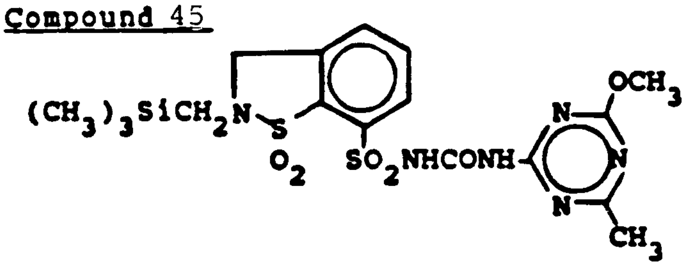

Compound 46

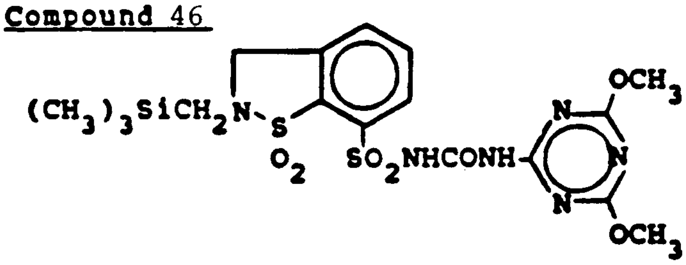

## Table A

| TYPE TEST | RATE G/HA | COTTON | SORGHUM | CORN | SOYBEAN | WHEAT | WILDOATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| | | | | | | | | **Compound 1** | | | | | | | |
| POST | 50 | 2C 4G | 3C 9G | 2C 8H | 2C 7H | 5G | 4G | 3C 9G | 2C 9H | 2G | 2C 3H | 3C 5G | 3C 5G | 2G | 2C 8G |
| PRE | 50 | 0 | 0 | 0 | 1C | 0 | 0 | 1C | 0 | 0 | 3G | 5G | 5G | 0 | 0 |
| | | | | | | | | **Compound 2** | | | | | | | |
| POST | 50 | 3C 8H | 2C 9H | 2C 9G | 4C | 5G | 2C 9G | 5C 9G | 10C | 5c 9G | 3C 9G | 2C 5H | 2G | 8G | 9C |
| PRE | 50 | 2C 8G | 4C 9H | 4C 9G | 4C 7G | 9C | 9C | 10E | 4C 9H | 5C 9G | 9G | 9H | 2C 8G | 10E | 9C |
| | | | | | | | | **Compound 3** | | | | | | | |
| POST | 50 | 4C 8G | 5C 9H | 3C 9G | 5C 9G | 2G | 2C 5G | 9C | 5C 9H | 2C 7G | 9C | 3C 8H | 5C 9G | 4C 9G | 3C 7G |
| PRE | 50 | 8G | 9G | 4U 9H | 3C 7G | 0 | 2C 7G | 10E | 3C 9G | 2C 5G | 9G | 8H | 8G | 10E | 9G |
| | | | | | | | | **Compound 4** | | | | | | | |
| POST | 50 | 9C | 9C | 9C | 9C | 9C | 9C | 9C | 9C | 10C | 10C | 9C | 9C | 9C | 10C |
| PRE | 50 | 2C 9G | 10H | 5C 9H | 4C 9H | 10H | 9C | 10E | 9H | 6C 9G | 9C | 8H | 9G | 10E | 9C |
| | | | | | | | | **Compound 5** | | | | | | | |
| POST | 50 | 0 | 3C 7H | 2C 2H | 0 | 0 | 2C 4G | 2C 4G | 3C 8H | 4G | 1C 2G | 0 | 1C | 0 | 3G |
| PRE | 50 | 0 | 4G | 2C 5G | 0 | 0 | 2C 5G | 3c 8G | 0 | 0 | 2C | 0 | 0 | 0 | 0 |

## Table A

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 6** | | | | | | | | | | | | | | | |
| POST | 50 | 4C<br>7G | 5C<br>9H | 5C<br>9H | 4C<br>8G | 2G | 4C<br>7G | 9C | 5C<br>9H | 6G | 3C<br>8G | 2C<br>8G | 5C<br>9G | 8G | 2C<br>3G |
| PRE | 50 | 2G | 4C<br>9G | 2C<br>7H | 2C<br>5G | 0 | 2C<br>8H | 10E | 4H | 2C<br>8G | 6G | 5G | 2G | 0 | 4G |
| | | | | | | | | | | | | | | | |
| POST | 50 | 4C<br>9G | 4C<br>9H | 5C<br>9G | 4C<br>8G | 2G | 4C<br>8G | 9G | 6C<br>9G | 5C<br>9G | 10C | 3C<br>8G | 9C | 4C<br>9G | 4C<br>9G |
| PRE | 50 | 8G | 3C<br>9G | 2C<br>9G | 3C<br>8G | 0 | 9H | 10E | 9H | 2C<br>9G | 9G | 5G | 2G | 10E | 3C<br>9G |
| **Compound 8** | | | | | | | | | | | | | | | |
| POST | 50 | 1C | 2C<br>6H | 3C<br>8H | 0 | 0 | 0 | 6G | 0 | 0 | 2C<br>3H | 2C<br>5H | 0 | 3G | 2C |
| PRE | 50 | 0 | 2C<br>4G | 2C<br>7G | 1C | 0 | 3G | 8H | 1C | 2G | 2C | 1H | 0 | 0 | 2C |
| **Compound 9** | | | | | | | | | | | | | | | |
| POST | 50 | 4C<br>6G | 3C<br>9G | 3C<br>9H | 3H | 0 | 0 | 4C<br>9G | 9C | 3C<br>6G | 3C<br>7H | 2C | 1C | 2C | 2C |
| PRE | 50 | 2G | 3C<br>9H | 2C<br>9G | 2C | 3G | 3G | 10E | 3C<br>8H | 3G | 2C<br>9G | 3G | 3G | 9G | 3G |

## Table A

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 10** | | | | | | | | | | | | | | | |
| POST | 50 | 4C<br>9H | 3C<br>9G | 2U<br>9G | 5C<br>9G | 6C<br>9G | 9C | 9C | 10C | 9C | 5C<br>8H | 4C<br>8H | 3C<br>3H | 2C<br>5G | 5C<br>9G |
| PRE | 50 | 5G | 3C<br>8G | 2C<br>9G | 3C<br>7G | 0 | 6C<br>9G | 5C<br>9H | 5C<br>6G | 4C<br>9G | 8G | 7H | 2G | 0 | 3C<br>7G |
| **Compound 11** | | | | | | | | | | | | | | | |
| POST | 50 | 2G | 4C<br>9H | 4C<br>9H | 5C<br>9G | 0 | 3C<br>8G | 5C<br>9G | 3C<br>8H | 2G | 5C<br>9H | 3C<br>6H | 5C<br>9H | 2C<br>5G | 0 |
| PRE | 50 | 2G | 3C<br>9G | 2C<br>8G | 2C<br>4G | 0 | 4C<br>9G | 10E | 4C<br>6G | 2G | 2C<br>8G | 6H | 2C<br>4G | 5G | 0 |
| **Compound 12** | | | | | | | | | | | | | | | |
| POST | 50 | 0 | 2G | 2C<br>9H | 3C<br>9G | 7G | 7G | 3C<br>9G | 0 | 0 | 3C<br>7G | 3C<br>9H | 3C<br>5H | 0 | 3H |
| PRE | 50 | 0 | 0 | 2C<br>5G | 1C | 0 | 0 | 2C<br>9H | 0 | 0 | 0 | 7G | 0 | 0 | 8H |
| **Compound 13** | | | | | | | | | | | | | | | |
| POST | 50 | 8H | 5C<br>9G | 4C<br>9H | 5C<br>9G | 8G | 1C | 5C<br>9G | 3C<br>9H | 2C<br>5G | 4C<br>9G | 4C<br>9H | 6C<br>9G | 2C<br>9G | 9C |
| PRE | 50 | 3G | 3C<br>8G | 3C<br>9G | 6H | 8G | 3C<br>8G | 10E | 3C<br>7H | 2C<br>8G | 8G | 9H | 2G | 8G | 5C<br>9G |
| **Compound 14** | | | | | | | | | | | | | | | |
| POST | 50 | 9H | 6C<br>9G | 10C | 5C<br>9G | 7G | 5G | 5C<br>9G | 4C<br>9H | 3C<br>9G | 10C | 10C | 9C | 2C<br>9G | 9C |
| PRE | 50 | 8G | 4C<br>9G | 3C<br>9G | 3C<br>7H | 4C<br>9G | 4C<br>8G | 10E | 5C<br>9H | 2C<br>7G | 8H | 9H | 7G | 10E | 5C<br>9G |

## Table A

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 15** | | | | | | | | | | | | | | | |
| POST | 50 | 0 | 0 | 2C 4H | 2H | 0 | 0 | 5G | 0 | 0 | 2C | 2C 3H | 0 | 0 | 2H |
| PRE | 50 | 0 | 6G | 4G | 1C | 5G | 0 | 9G | 0 | 0 | 0 | 0 | 0 | 0 | 7G |
| **Compound 16** | | | | | | | | | | | | | | | |
| POST | 50 | 8H | 4C 9G | 2C 5G | 3C 9H | 3G | 0 | 4C 8G | 9C | 0 | 4C 8G | 5G | 2C | 0 | 9C |
| PRE | 50 | 0 | 6C 9H | 3C 9H | 1C 3G | 6G | 6G | 10E | 3C 7H | 1C | 5G | 9H | 0 | 10E | 4C 9G |
| **Compound 17** | | | | | | | | | | | | | | | |
| POST | 50 | 3C 8H | 3C 8H | 3C 8H | 5C 9G | 2G | 0 | 6C 9G | 2C 9H | 2G | 5c 9G | 2C 7G | 3C 7G | 2C 7G | 3C 7H |
| PRE | 50 | 0 | 0 | 0 | 0 | 0 | 0 | 0 | 0 | 0 | 0 | 0 | 0 | 0 | 0 |
| **Compound 18** | | | | | | | | | | | | | | | |
| POST | 50 | 3C 9G | 3C 7H | 3C 9H | 4C 9G | 0 | 0 | 5C 9G | 3C 9H | 3C 9G | 9C | 2C 6H | 2C 8G | 2C 8G | 9C |
| PRE | 50 | 0 | 2G | 2C 8G | 2C 7G | 0 | 0 | 8G | 2H | 0 | 9G | 4G | 0 | 0 | 8G |

## Table A

| TYPE TEST | RATE G/HA | COTTON | SORGHUM | CORN | SOYBEAN | WHEAT | WILDOATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 19** | | | | | | | | | | | | | | | |
| POST | 50 | 3G | 0 | 2C 7H | 0 | 0 | 0 | 2G | 0 | 0 | 0 | 0 | 0 | 0 | 3H |
| PRE | 50 | 0 | 0 | 0 | 0 | 0 | 0 | 0 | 0 | 0 | 0 | 0 | 0 | 0 | 0 |
| **Compound 20** | | | | | | | | | | | | | | | |
| POST | 50 | 3C 8H | 2G | 3C 9H | 0 | 3G | 0 | 6C 9G | 3C 9H | 2G | 2C 7H | 3G | 0 | 5G | 0 |
| PRE | 50 | 0 | 3C 9G | 2C 7G | 0 | 0 | 0 | 9H | 0 | 0 | 2G | 0 | 0 | 0 | 0 |
| **Compound 21** | | | | | | | | | | | | | | | |
| POST | 50 | 2C 4G | 3C 8H | 3C 8H | 0 | 0 | 1C | 3C 9G | 3C 9H | 1C | 2C 5H | 0 | -- | 2C 5G | 0 |
| PRE | 50 | 0 | 3C 9H | 3C 8G | 1C | 0 | 0 | 10E | 3C 9H | 3G | 2C 6H | 5G | -- | 5G | 2H |
| **Compound 22** | | | | | | | | | | | | | | | |
| POST | 50 | 0 | 3C 8H | 3C 7H | 1C | 0 | 0 | 3C 6G | 3C 5H | 0 | 3G | 0 | -- | 0 | 0 |
| PRE | 50 | 0 | 0 | 2G | 0 | 0 | 0 | 0 | 0 | 0 | 0 | 0 | -- | 0 | 0 |

## Table A

| TYPE TEST | RATE G/HA | COTTON | SORGHUM | CORN | SOYBEAN | WHEAT | WILDOATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| | | | | | | | Compound 23 | | | | | | | | |
| POST | 50 | 5C<br>9G | 5C<br>9G | 10C | 4C<br>9G | 9C | 9C | 5C<br>9G | 10C | 9C | 10C | 10C | -- | 9C | 10C |
| PRE | 50 | 2C<br>9G | 9G | 9G | 3C<br>8H | 2C<br>8G | 2C<br>8G | 9H | 2C<br>5G | 2C<br>7G | 8G | 5H | -- | 10E | 4C<br>9G |
| | | | | | | | Compound 24 | | | | | | | | |
| POST | 50 | 1C | 2G | 2C<br>7H | 2C<br>2H | 5G | 2C<br>4G | 1C | 2G | 0 | 0 | 0 | -- | 0 | 0 |
| PRE | 50 | 0 | 0 | 0 | 0 | 0 | 0 | 0 | 0 | 0 | 0 | 0 | -- | 0 | 0 |
| | | | | | | | Compound 25 | | | | | | | | |
| POST | 50 | 3C<br>8H | 4C<br>9H | 5C<br>9G | 5C<br>9G | 8G | 5C<br>9G | 5C<br>9G | 5C<br>9H | 2C<br>8G | 3C<br>7H | 3C<br>8H | -- | 2C<br>9G | 3C<br>7H |
| PRE | 50 | 2G | 2C<br>7H | 2C<br>7H | 7G | 2G | 2C<br>7G | 2C<br>6G | 0 | 5G | 3C<br>6G | 9H | -- | 0 | 2C<br>5G |
| | | | | | | | Compound 26 | | | | | | | | |
| POST | 50 | 4C<br>9G | 9C | 10C | 4C<br>9G | 6C<br>9G | 6C<br>9G | 9C | 10C | 9C | 10C | 5C<br>9H | -- | 4C<br>9G | 10C |
| PRE | 50 | 7G | 9H | 4C<br>9G | 4C<br>7G | 9C | 9C | 9H | 3C<br>5H | 3C<br>9G | 9C | 8H | -- | 9G | 4C<br>9G |

**Table A**

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 27** | | | | | | | | | | | | | | | |
| POST | 50 | 2C 5G | 5C 9H | 9C | 1H | 1C 2G | 3C 7G | 5C 9G | 10C | 9C | 2C 3H | 3G | -- | 0 | 3C 7G |
| PRE | 50 | 0 | 3C 9H | 2C 8H | 2C 2G | 2G | 2C 5G | 3C 8H | 3C 8H | 2C 4G | 3C 5G | 0 | -- | 0 | 5H |
| **Compound 28** | | | | | | | | | | | | | | | |
| POST | 50 | 0 | 4C 9H | 2C 9H | 2C 4H | 3C 9G | 9C | 5C 9G | 9C | 3C 8H | 3H | 3G | -- | 0 | 3C 3H |
| PRE | 50 | 0 | 2C | 2G | 0 | 5G | 7G | 0 | 2G | 5G | 0 | 0 | -- | 0 | 0 |
| **Compound 29** | | | | | | | | | | | | | | | |
| POST | 50 | 5C 9H | 9C | 10C | 5C 9G | 9C | 10C | 9C | 10C | 9C | 10C | 5C 9H | -- | 4C 9G | 10C |
| PRE | 50 | 5G | 5C 9H | 5C 9H | 3C 7G | 7C 9H | 9C | 5C 9H | 3C 7H | 10H | 3C 7H | 9H | -- | 10E | 6C 9G |
| **Compound 30** | | | | | | | | | | | | | | | |
| POST | 50 | 5C 9G | 10C | 10C | 9C | 9C | 9C | 9C | 9C | 10C | 10C | 9C | -- | 9C | 10C |
| PRE | 50 | 8G | 5C 9H | 10H | 4C 8H | 9C | 10C | 10H | 9C | 10H | 5C 9G | 4C 9H | -- | 10E | 6C 9G |

**Table A**

| TYPE TEST | RATE G/HA | COTTON | SORGHUM | CORN | SOYBEAN | WHEAT | WILDOATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 31** | | | | | | | | | | | | | | | |
| POST | 50 | 4C<br>8H | 9C | 10C | 3H<br>6G<br>5X | 4C<br>9G | 2C<br>9G | 9C | 10C | 7C<br>9H | 2C<br>7G | 4C<br>8H | -- | 2C<br>5G | 5C<br>9H |
| PRE | 50 | 0 | 9C | 5C<br>9H | 3C<br>4G | 6G | 2C<br>5G | 5C<br>9H | 2C<br>8H | 3C<br>7G | 3C<br>7H | 2H | -- | 4G | 2C<br>5H |
| **Compound 32** | | | | | | | | | | | | | | | |
| POST | 50 | 0 | 4C<br>8H | 6C<br>9H | 1C<br>3G | 5C<br>9G | 4C<br>9G | 4C<br>9G | 4C<br>8H | 0 | 0 | 2G | -- | 3G | 2C<br>5H |
| PRE | 50 | 0 | 2C<br>3G | 2C<br>5G | 0 | 7G | 3G | 2C<br>3G | 1C | 0 | 2C<br>4H | -- | -- | 0 | 3H |
| **Compound 33** | | | | | | | | | | | | | | | |
| POST | 50 | 0 | 4G | 3C<br>8H | 3G | 2C<br>8G | 3C<br>8G | 3C<br>9G | 3C<br>5H | 0 | 0 | 0 | -- | 0 | 0 |
| PRE | 50 | 0 | 0 | 0 | 0 | 0 | 0 | 0 | 0 | 0 | 0 | 0 | -- | 0 | 0 |
| **Compound 34** | | | | | | | | | | | | | | | |
| POST | 50 | 10C | 9C | 10C | 9C | 10C | 10C | 9C | 10C | 10C | 10C | 10C | -- | 10C | 10C |
| PRE | 50 | 9G | 7C<br>9H | 10E | 9H | 9H | 9C | 10E | 4C<br>9H | 10C | 10C | 9H | -- | 10E | 10C |
| **Compound 35** | | | | | | | | | | | | | | | |
| POST | 50 | 0 | 2C<br>4H | 3C<br>8H | 6G | 6C<br>9G | 10C | 9C | 4C<br>9H | 3C<br>8G | 0 | 0 | -- | 0 | 4H |
| PRE | 50 | 0 | 4G | 3G | 0 | 0 | 2C<br>6G | 2C<br>7G | 1H | 0 | 0 | 0 | -- | 0 | 0 |

## Table A

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| **Compound 36** | | | | | | | | | | | | | | | |
| POST | 50 | 3C<br>8H | 9C | 10C | 3C<br>8H | 3C<br>9G | 10C | 9C | 9C | 9C | 4C<br>8H | 4C<br>9H | -- | 3C<br>8G | 3C<br>8H |
| PRE | 50 | 5G | 4C<br>9H | 3C<br>9G | 2C<br>4H | 2G | 9C | 10E | 3C<br>9H | 4C<br>9H | 3C<br>6H | -- | -- | 3C<br>5G | 4C<br>9H |
| **Compound 37** | | | | | | | | | | | | | | | |
| POST | 50 | 5C<br>9G | 9C | 10C | 10C | 9C | 10C | 9C | 9C | 10C | 10C | 10C | -- | 9C | 9C |
| PRE | 50 | 9G | 9H | 9G | 2C<br>7G | 8G | 9C | 10E | 4C<br>9H | 10C | 9G | 8H | -- | 10E | 5C<br>9G |
| **Compound 38** | | | | | | | | | | | | | | | |
| POST | 50 | 0 | 10C | 10C | 7G | 3G | 6C | 9C | 9C | 9C | 2C<br>5H | 2C<br>7G | -- | 2C<br>3G | 4H |
| PRE | 50 | 0 | 3C<br>9H | 2C<br>8G | 0 | 3G | 7G | 10E | 3C<br>9H | 3C<br>9G | 3G | 2G | -- | 10E | 5G |
| **Compound 39** | | | | | | | | | | | | | | | |
| POST | 50 | 0 | 9H | 3C<br>9H | 1H | 2C<br>6G | 9C<br>9H | 4C | 3C | 0 | 0 | 2G | -- | 0 | 3H |
| PRE | 50 | 0 | 0 | 2C<br>4G | 0 | 0 | 2G | 2G | 2H | 0 | 0 | 0 | -- | 0 | 0 |
| **Compound 40** | | | | | | | | | | | | | | | |
| POST | 50 | 0 | 2C<br>5H | 3C<br>7H | 0 | 2G | 5C<br>9G | 8G | 0 | 0 | 0 | 1H | -- | 0 | 0 |
| PRE | 50 | 0 | 0 | 0 | 0 | 0 | 0 | 0 | 0 | 0 | 0 | 0 | -- | 0 | 0 |

**Table A**

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGAR BEETS |
|---|---|---|---|---|---|---|---|---|---|---|---|---|---|---|---|
| **Compound 41** | | | | | | | | | | | | | | | |
| POST | 50 | 4G | 4C 8H | 5C 9H | 2H 5G | 4C 9G | 5C 9G | 5C 9G | 3C 6H | 5G | 3C 8H | 4G | -- | 2c 8G | 3C 7H |
| PRE | 50 | 0 | 2G | 5G | 0 | 7G | 3C 9G | 8H | 0 | 0 | 3C | 0 | -- | 0 | 0 |
| **Compound 42** | | | | | | | | | | | | | | | |
| POST | 50 | 9H | 6C 9G | 9C | 4C 9G | 9C | 9C | 9C | 9C | 5C 9G | 5C 9G | 4C 9H | -- | 4C 9G | 10C |
| PRE | 50 | 3G | 4C 9H | 9G | 3C 7G | 9G | 9C | 5C 9H | 5C 9H | 5C 9G | 9H | -- | -- | 9G | 9G |
| **Compound 43** | | | | | | | | | | | | | | | |
| POST | 50 | 9C | 9C | 10C | 4C 9G | 5C 9G | 9C | 6C 9G | 9C | 10C | 10C | 10C | -- | 9C | 9C |
| PRE | 50 | 9G | 6C 9H | 5C 9H | 3C 8G | 6C 9H | 7C 9H | 10E | 7C 9H | 7C 9H | 9C | 9H | -- | 10E | 9C |
| **Compound 44** | | | | | | | | | | | | | | | |
| POST | 50 | 9H | 9C | 9C | 6H | 2C 6G | 3C 7G | 9C | 10C | 9C | 10C | 8H | -- | 3C 8G | 10C |
| PRE | 50 | 6G | 9C | 3C 9H | 2C 5G | 3C 7G | 3C 8G | 10E | 6C 9H | 3C 7G | 8H | 5H | -- | 3C 9H | 5C 9G |
| **Compound 45** | | | | | | | | | | | | | | | |
| POST | 50 | 0 | 3C 8H | 3C 8H | 0 | 2c 6G | 2C 5G | 2C 9G | 6H | 0 | 0 | 1H | -- | 0 | 1H |
| PRE | 50 | 0 | 2G | 6G | 0 | 5G | 2G | 3G | 0 | 0 | 0 | 0 | -- | 0 | 2G |

**Table A**

| TYPE TEST | RATE G/HA | COTTON | SORGHUM | CORN | SOYBEAN | WHEAT | WILD OATS | RICE | BARNYARDGRASS | CRABGRASS | MORNINGGLORY | COCKLEBUR | SICKLEPOD | NUTSEDGE | SUGARBEETS |
|---|---|---|---|---|---|---|---|---|---|---|---|---|---|---|---|
| | | | | | | | Compound 46 | | | | | | | | |
| POST | 50 | 0 | 2C 3H | 3C 9H | 0 | 2G | 2C 5G | 9G | 4H | 0 | 0 | 0 | -- | 0 | 0 |
| PRE | 50 | 0 | 0 | 2G | 1C | 0 | 2C 3G | 3G | 0 | 0 | 0 | 0 | -- | 0 | 4G |

Test B

Two ten-inch in diameter plastic pans lined with polyethylene liners were filled with prepared Fallsington silt loam soil. One pan was planted with seeds of wheat (Triticum aestivum), barley (Hordeum vulgare), wild oats (Amena fatua), cheatgrass (Bromus secalinus), blackgrass (Alopecurus myosuroides), annual bluegrass (Poa annua), green foxtail (Setaria viridis), rapeseed (Brassica napus),and Italian ryegrass (Lolium multiflorum). The other pan was planted with seeds of Russian thistle (Salsola kali), speedwell (Veronica persica), kochia (Kochia scoparia), shepherd's purse (Capsella bursa-pastoris), Matricaria inodora, black nightshade (Solanum nigrum), and wild buckwheat (Polygonum convolvulus). The above two pans were treated preemergence. At the same time two pans in which the above plant species were growing were treated postemergence. Plant height at the time of treatment ranged from 1-15 cm depending on plant species.

The compounds applied were diluted with a non-phytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data are presented in Table B.

Table B

| TYPE TEST | RATE G/HA | WHEAT | BARLEY | WILD OATS | CHEATGRASS | BLACKGRASS | BLUEGRASS | FOXTAIL | RYEGRASS | CRABGRASS | RAPESEED |
|---|---|---|---|---|---|---|---|---|---|---|---|
| | | | | | Compound 2 | | | | | | |
| POST | 125 | 10C | 10C | 10C | 9G | 10C | 10C | 10C | 10C | 10C | |
| PRE | 125 | 8G | 9G | 9G | 10C | 9G | 8G | 10C | 10C | 9G | |
| POST | 64 | 8G | 10C | 10C | 8G | 10C | 10C | 10C | 10C | 10C | |
| PRE | 64 | 7G | 7G | 9G | 9G | 9G | 9G | 9G | 8G | 5G | |
| POST | 32 | 3G | 10C | 7C | 7G | 9G | 10C | 10C | 7G | 10C | |
| PRE | 32 | 6G | 6G | 8G | 8G | 7G | 7G | 7G | 7G | 0 | |
| POST | 16 | 0 | 10C | 10C | 7G | 9G | 10C | 10C | 6G | 9G | |
| PRE | 16 | 4G | 4G | 6G | 7G | 4G | 7G | 2g | 4G | 0 | |
| POST | 8 | 0 | 9G | 8G | 0 | 3G | 4G | 9G | 4G | 9G | |
| PRE | 8 | 0 | 2G | 4G | 3G | 4G | 4G | 0 | 2G | 0 | |

| TYPE TEST | RATE G/HA | MATRICARIA INODORA | GALIUM | THISTLE | SHEPHERDS PURSE | KOCHIA | NIGHTSHADE | SPEEDWELL | BUCKWHEAT | SUGARBEETS |
|---|---|---|---|---|---|---|---|---|---|---|
| | | | | | Compound 2 | | | | | |
| POST | 125 | 6G | 10C | 10C | 9G | 8G | 10C | 0 | 7G | 9G |
| PRE | 125 | 8G | 10C | 0 | 9G | 9G | 8G | 0 | 9G | 10C |
| POST | 64 | 5G | 10C | 10C | 8G | 8G | 8G | 0 | 7G | 9G |
| PRE | 64 | 7G | 9G | 0 | 9G | 6G | 8G | 0 | 9G | 10C |
| POST | 32 | 2G | 7G | 10C | 9G | 5G | 6G | 0 | 0 | 9G |
| PRE | 32 | 7G | 7G | 0 | 9G | 0 | 7G | 0 | 5G | 9G |
| POST | 16 | 0 | 5G | 10C | 9G | 0 | 0 | 0 | 0 | 9G |
| PRE | 16 | 6G | 6G | 0 | 7G | 0 | 5G | 0 | 0 | 5G |
| POST | 8 | 0 | 4G | 7G | 8g | 0 | 0 | 0 | 0 | 3G |
| PRE | 8 | 3G | 4G | 0 | 6G | 0 | 2G | 0 | 0 | 4G |

Table B

| TYPE TEST | RATE G/HA | WHEAT | BARLEY | WILD OATS | CHEATGRASS | BLACKGRASS | BLUEGRASS | FOXTAIL | RYEGRASS | CRABGRASS | RAPESEED |
|---|---|---|---|---|---|---|---|---|---|---|---|
| | | | | | Compound 3 | | | | | | |
| POST | 125 | 0 | 0 | 0 | 0 | 5G | 3G | 7G | 0 | 10C | |
| PRE | 125 | 0 | 0 | 0 | 0 | 0 | 0 | 0 | 0 | 0 | |
| POST | 64 | 0 | 0 | 0 | 0 | 4G | 0 | 6G | 0 | 8G | |
| PRE | 64 | 0 | 0 | 0 | 0 | 0 | 0 | 0 | 0 | 0 | |
| POST | 32 | 0 | 0 | 0 | 0 | 0 | 0 | 0 | 0 | 7G | |
| PRE | 32 | 0 | 0 | 0 | 0 | 0 | 0 | 0 | 0 | 0 | |
| POST | 16 | 0 | 0 | 0 | 0 | 0 | 0 | 0 | 0 | 4G | |
| PRE | 16 | 0 | 0 | 0 | 0 | 0 | 0 | 0 | 0 | 0 | |
| POST | 8 | 0 | 0 | 0 | 0 | 0 | 0 | 0 | 0 | 2G | |
| PRE | 8 | 0 | 0 | 0 | 0 | 0 | 0 | 0 | 0 | 0 | |

| TYPE TEST | RATE G/HA | MATRICARIA INODORA | GALIUM | THISTLE | SHEPHERDSPURSE | KOCHIA | NIGHTSHADE | SPEEDWELL | BUCKWHEAT | SUGARBEETS |
|---|---|---|---|---|---|---|---|---|---|---|
| | | | | | Compound 3 | | | | | |
| POST | 125 | 5G | 10C | 10C | 7G | 10C | 0 | 0 | 6G | 0 |
| PRE | 125 | 7G | 8G | 4G | 9G | 7G | 8G | 0 | 0 | 4G |
| POST | 64 | 0 | 6G | 9G | 7G | 3G | 0 | 0 | 0 | 0 |
| PRE | 64 | 7G | 2G | 4G | 6G | 7G | 6G | 0 | 0 | 0 |
| POST | 32 | 0 | 4G | 8G | 6G | 0 | 0 | 0 | 0 | 0 |
| PRE | 32 | 6G | 2G | 3G | 4G | 5G | 4G | 0 | 0 | 0 |
| POST | 16 | 0 | 0 | 7G | 6G | 0 | 0 | 0 | 0 | 0 |
| PRE | 16 | 4G | 0 | 0 | 3G | 0 | 0 | 0 | 0 | 0 |
| POST | 8 | 0 | 0 | 3G | 5G | 0 | 0 | 0 | 0 | 0 |
| PRE | 8 | 0 | 0 | 0 | 0 | 0 | 0 | 0 | 0 | 0 |

## Claims

**1.** A compound of the formula:

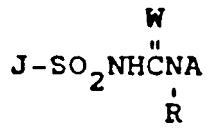

I

wherein

J is

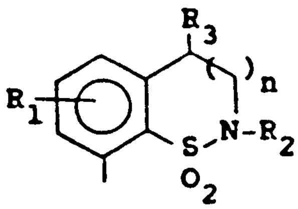

,

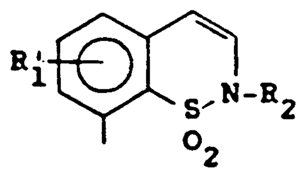

,

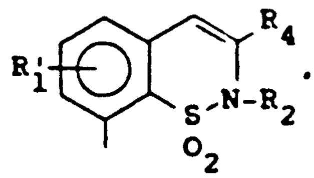

,

J-1

J-2

J-3

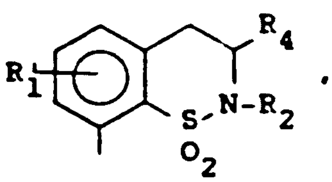

,

J-4

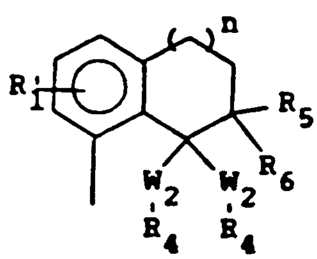

J-11

or

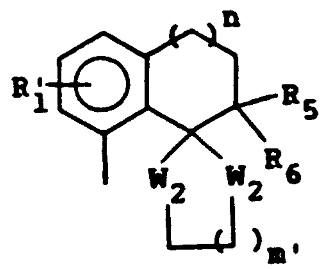

J-12

| | |
|---|---|
| n | is 0 or 1; |
| W | is O or S; |
| $W_1$ | is S; |
| $W_2$ | is O or S; |
| R | is H or $CH_3$; |
| $R_1$ | is H, $C_1$-$C_6$ alkyl, $C_1$-$C_6$ haloalkyl, halogen, nitro, $C_1$-$C_6$ alkoxy, $SO_2NR_aR_b$, $C_1$-$C_6$ alkylthio, $C_1$-$C_6$ alkylsulfinyl, $C_1$-$C_6$ alkylsulfonyl, CN, $CO_2R_c$, $C_1$-$C_6$ haloalkoxy, $C_1$-$C_6$ haloalkylthio, $NH_2$, $C_1$-$C_6$ alkylamino, di($C_1$-$C_6$ alkyl)amino, $Si(CH_3)_2(C_1$-$C_4$ alkyl), Si-$(CH_3)_2$phenyl or $C_1$-$C_3$ alkyl substituted with $C_1$-$C_3$ alkoxy, $C_1$-$C_3$ alkylthio, $C_1$-$C_3$ alkylsulfinyl, $C_1$-$C_3$ alkylsulfonyl, $SO_2NR_dR_e$, $NO_2$, CN, $CO_2R_f$, $C_1$-$C_3$ haloalkoxy or $C_1$-$C_3$ haloalkylthio; |
| $R_a$ | is H, $C_1$-$C_4$ alkyl, $C_1$-$C_3$ cyanoalkyl, methoxy or ethoxy; |
| $R_b$ | is H, $C_1$-$C_4$ alkyl or $C_3$-$C_4$ alkenyl; or |
| $R_a$ and $R_b$ | may be taken together as $-(CH_2)_3-$, $-(CH_2)_4-$, $-(CH_2)_5-$ or $-CH_2CH_2OCH_2CH_2-$; |
| $R_c$ | is $C_1$-$C_4$ alkyl, $C_3$-$C_4$ alkenyl, $C_3$-$C_4$ alkynyl, $C_2$-$C_4$ haloalkyl, $C_2$-$C_3$ cyanoalkyl, $C_5$-$C_6$ cycloalkyl, $C_4$-$C_7$ cycloalkylalkyl or $C_2$-$C_4$ alkoxyalkyl; |
| $R_d$ | is $C_1$-$C_3$ alkyl; |
| $R_e$ | is H or $C_1$-$C_3$ alkyl; |
| $R_f$ | is $C_1$-$C_3$ alkyl; |
| $R_1'$ | is H, $C_1$-$C_3$ alkyl, $C_1$-$C_3$ alkoxy, $C_1$-$C_3$ haloalkoxy, $C_1$-$C_3$ haloalkyl, $C_1$-$C_3$ alkylthio, $C_1$-$C_3$ haloalkylthio, amino, $C_1$-$C_3$ alkylamino, di($C_1$-$C_3$ alkyl)amino, halogen or $NO_2$; |
| $R_2$ | is H, $R_{11}$, $SR_{11}$, $SO_2R_{11}$, $OR_{11}$, $C(O)R_{11}$, $C(O)OR_{11}$, $(C(O))_2OR_{11}$, $(CO)_2R_{11}$, C(O)-$NR_{12}R_{18}$, C(O)NRA, $C(S)SR_{11}$, $NH_2$, $NR_{12}R_{18}$, OH, CN, $P(O)R_{13}R_{14}$, $P(S)R_{13}R_{14}$, Si-$(CH_3)_2R_{15}$, L or C(O)L; |
| R | $_3$ is H or $CH_3$; |
| $R_4$ | is $C_1$-$C_4$ alkyl; |
| $R_5$ | is H or $C_1$-$C_4$ alkyl; |
| $R_6$ | is H or $CH_3$; |

A is 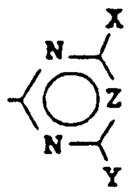 , 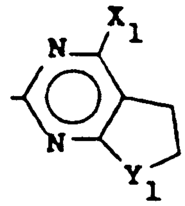 , 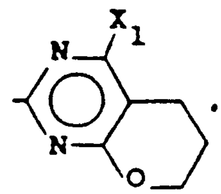 ,

A-1 A-2 A-3

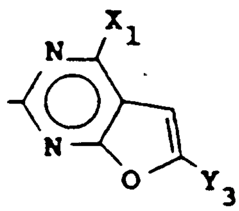 , 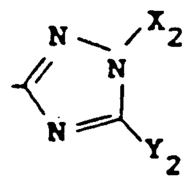 or 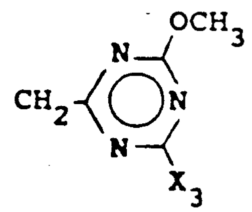 ;

A-4 A-5 A-6

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, F, Cl, Br, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, F, Cl, Br, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_1$-$C_4$ haloalkyl, azido, cyano,

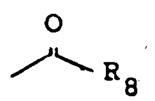 ,

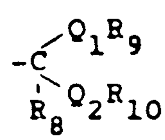 , 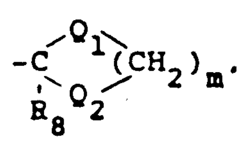 , 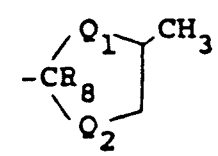 or

$N(OCH_3)CH_3$;

m is 2 or 3;

$Q_1$ and $Q_2$ are independently O or S;

$R_8$ is H or $C_1$-$C_3$ alkyl;

$R_9$ and $R_{10}$ are independently $C_1$-$C_3$ alkyl;

Z is CH, N, $CCH_3$, $CC_2H_5$, CCl or CBr;

$Y_1$ is O or $CH_2$;

$X_1$ is $CH_3$, $OCH_3$, $OC_2H_5$ or $OCF_2H$;

$X_2$ is $CH_3$, $C_2H_5$ or $CH_2CF_3$;

$Y_2$ is $OCH_3$, $OC_2H_5$, $SCH_3$, $SC_2H_5$, $CH_3$ or $CH_2CH_3$;

$X_3$ is $CH_3$ or $OCH_3$;

$Y_3$ is H or $CH_3$;

$R_{11}$ is $C_1$-$C_{10}$ alkyl, $C_1$-$C_{10}$ alkoxyalkoxyalkyl, $C_2$-$C_{10}$ alkenyl, up to $C_{10}$ alkenylalkenyl, $C_2$-$C_{10}$ epoxyalkyl, $C_2$-$C_{10}$ alkynyl, up to $C_{10}$ alkynylalkynyl, up to $C_{10}$ alkynylalkenyl, $C_3$-$C_6$ cycloalkyl, $C_4$-$C_7$ cycloalkylalkyl or

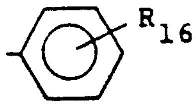 ;

when $R_{11}$ is $C_3$-$C_6$ cycloalkyl or $C_4$-$C_7$ cycloalkylalkyl it may optionally be substituted by $C_1$-$C_4$ alkyl, 1 to 3 atoms of Cl or F or 1 Br; when $R_{11}$ is $C_1$-$C_{10}$ alkyl, $C_2$-$C_{10}$ alkenyl or $C_2$-$C_{10}$ alkynyl it may optionally be substituted by one or more halogens and/or by $(R_{17})_{m'}$, where when m' is 2, the values of $R_{17}$ may be identical or different;

m' is 1 or 2;

$R_{12}$ is H or $C_1$-$C_4$ alkyl;

$R_{13}$ and $R_{14}$ are independently $C_1$-$C_4$ alkyl, $C_1$-$C_4$ alkoxy or $C_1$-$C_4$ alkylthio;

$R_{15}$ is $C_1$-$C_{10}$ alkyl, benzyl or

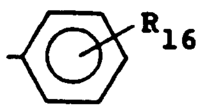 ;

$R_{16}$ is H, F, Cl, Br, $CH_3$, $OCH_3$, $NO_2$, CN, $SCH_3$, $SO_2CH_3$ or $CF_3$;

$R_{17}$ is $OR_{18}$, $OC(O)R_{18}$, $P^+R_9R_{10}R_{15}$, $P^+(C_6H_5)_3$, $OC(O)NR_{12}R_{18}$, $OSO_2R_{18}$, $OP(O)$-$R_{13}R_{14}$, $P(O)R_{13}R_{14}$ $OP(S)R_{13}R_{14}$, $P(S)R_{13}R_{14}$, $OSi(CH_3)_2R_{15}$, $Si(CH_3)_2R_{15}$, $SR_{18}$, $SOR_{18}$, $SO_2R_{18}$, SCN, CN, $SP(O)R_{13}R_{14}$, $SP(S)R_{13}R_{14}$, $N^+R_{12}R_{15}R_{18}$, $NR_{12}R_{18}$, $NR_{12}C(O)R_{18}$, $NR_{12}C(O)OR_{18}$, $NR_{12}C(O)NR_{12}R_{18}$, $NR_{12}SO_2R_{18}$, $NR_{12}P(O)R_{13}R_{14}$, $NR_{12}P(S)R_{13}R_{14}$,$NO_2$, $C(O)R_{18}$, $C(O)OR_{18}$, $C(O)NR_{12}R_{18}$, $SeR_{18}$, naphthyl, L,

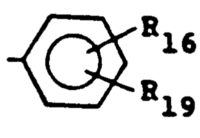 , 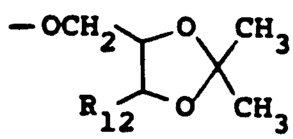 ,

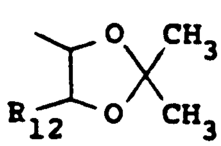 , 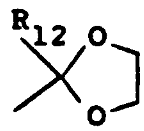 , 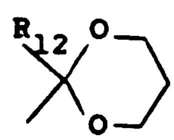

or

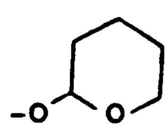

$R_{18}$ is H, $C_1$-$C_{10}$ alkyl, $C_1$-$C_{10}$ haloalkyl, $C_2$-$C_{10}$ alkenyl, $C_2$-$C_{10}$ alkynyl, $C_3$-$C_6$ cycloalkyl or

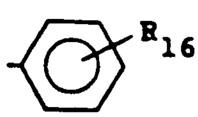 ;

$R_{19}$ is H, F, Cl, Br, $CH_3$,

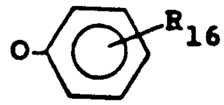

or

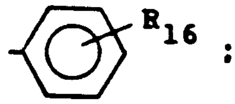

and L is a 5- or 6-membered aromatic heterocycle, a 5- or 6-membered dihydroaromatic heterocycle or a 5- or 6-membered tetrahydroaromatic heterocycle which contains 1-4 heteroatoms selected from 0-1 oxygen atoms, 0-1 sulfur atoms, wherein sulfur may take the form of S, SO or $SO_2$, and/or 0-4 nitrogen atoms, with the proviso that oxygen and sulfur are only linked to each other if the sulfur is in the form of SO or $SO_2$, and these heterocycles may optionally be substituted by 1-4 $CH_3$, 1-2 $OCH_3$, $SCH_3$, Cl, $N(CH_3)_2$ or CN or L is a 5- or 6-membered lactone, lactam or cycloalkanone which may optionally be substituted by 1-4 $CH_3$ groups;

provided that

a) when W is S, then R is H, J is $J_1$, $J_2$, $J_3$ or $J_4$; A is A-1, Z is CH or N, and Y is $CH_3$, $OCH_3$, $OC_2H_5$, $CH_2OCH_3$, $C_2H_5$, $CF_3$, $SCH_3$, $OCH_2CH=CH_2$, $OCH_2C≡CH$, $OCH_2CF_3$, $OCH_2CH_2OCH_3$, $CH(OCH_3)_2$ or

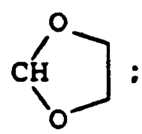

b) when X is F, Cl or Br, then Z is CH and Y is $OCH_3$, $OC_2H_5$, $NH_2$, $NHCH_3$, $N(CH_3)_2$ or $OCF_2H$;
c) when $R_3$ is $CH_3$, then n is O;
d) when J is J-1 or J-2 and $R_2$ is H or $C_1$-$C_4$ alkyl, then $R_1$ and $R_1'$ are other than H, F, Cl, Br, $CH_3$, $OCH_3$, $CF_3$, $OCF_2H$, or $SCH_3$ or X is other than $CH_3$, $OCH_3$, $OCH_2CH_3$, F, Cl, Br, $OCF_2H$, $CH_2Cl$, $CH_2Br$, $CH_2F$, cyclopropyl or $CF_3$ or Y is $C_3$-$C_4$ alkyl, $C_3$-$C_4$ alkoxy, $C_4$ haloalkoxy, $C_4$ haloalkylthio, $C_3$-$C_5$ alkoxyalkyl, $C_4$-$C_5$ alkoxyalkoxy, $C_2$-$C_3$alkylamino, di($C_2$-$C_3$ alkyl)amino, $C_4$ alkenyloxy, $C_4$ alkynyloxy, $C_3$-$C_5$ alkylthioalkyl, $C_2$-$C_4$ haloalkyl, $C_2$-$C_4$ alkynyl, $C(O)R_8$ or $N(OCH_3)CH_3$;
e) the total number of carbon atoms in $R_2$ does not exceed 13;
f) when X is $C_3$-$C_5$ cycloalkyl, then Y is $CH_3$, $CH_2CH_3$, $OCH_3$, $OCH_2CH_3$, $CH_2OCH_3$, $OCF_2H$, $SCF_2H$, $OCH_2CF_3$, $CF_3$, $OCH_2CH=CH_2$, $OCH_2C≡CH$, $NHCH_3$, $N(CH_3)_2$ or $CH(OCH_3)_2$;
g) when $R_{18}$ is H, $R_{17}$ is other than $SOR_{18}$, $SO_2R_{18}$, $OSO_2R_{18}$ or $NR_{12}C(O)OR_{18}$;
h) when $R_1$ or $R_1'$ is para to the sulfonylurea bridge then $R_1$ or $R_1'$ are H, $CH_3$, F, Cl, Br or $OCH_3$; and
i) when X or Y is $OCH_2CH_2F$ or $OCH_2CHF_2$ then $R_2$ is other than H or $C_1$-$C_5$ alkyl, $CH_3OCH_2CH_2$, $C_2H_5OCH_2CH_2$ or $C_1$-$C_4$ alkyl substituted with 1-3 atoms of F, Cl or Br;
j) when X or Y is $OCH_2CH_2F$, $OCH_2CHF_2$ or $OCH_2CF_3$ then the other is not di($C_1$-$C_3$ alkyl)amino $C_1$-$C_3$ alkylamino or $N(OCH_3)CH_3$;
k) when X or Y is $OCF_2H$, then Z is CH; and their agriculturally suitable salts.

**2.** A compound of Claim 1 wherein J is J-1 or J-4.

**3.** A compound of Claim 1 wherein J is J-2 or J-3.

**4.** A compound of Claim 1 wherein J is J-11 or J-12.

**5.** Compounds of Claim 2 wherein W is O; R is H;

X is $CH_3$, $OCH_3$, $OCH_2CH_3$, Cl, F, Br, $OCF_2H$, $CH_2F$, $CF_3$ or cyclopropyl;

Y is H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C≡CH$, $OCH_2CF_3$, CN, $N_3$, $OCH_2CH_2OCH_3$, $CH_2SCH_3$,

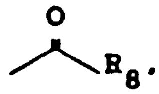

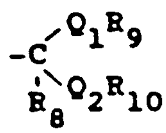 , 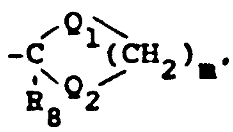 , 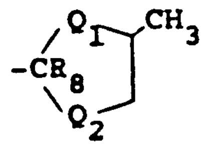

or $QCF_2T$;

Q is O or S;

T is H, CHClF, CHBrF or $CHFCF_3$.

**6.** Compounds of Claim 5 wherein $R_1$ or $R_1'$ is H, Cl, $C_1$-$C_3$ alkyl, $C_1$-$C_3$ alkoxy, $C_1$-$C_3$ haloalkoxy, $C_1$-$C_3$ haloalkyl, $C_1$-$C_3$ alkylthio, $C_1$-$C_3$ haloalkylthio, amino, $C_1$-$C_3$ alkylsulfinyl, $C_1$-$C_3$ alkylsulfonyl, CN, $NH_2$, $C_1$-$C_3$ alkylamino, di($C_1$-$C_3$ alkylamino) or $C_1$-$C_2$ alkyl substituted with $C_1$-$C_2$ alkoxy, $C_1$-$C_2$ alkylthio, $C_1$-$C_2$ haloalkoxy, $C_1$-$C_2$ haloalkylthio, CN or $NO_2$.

**7.** Compounds of Claim 6 wherein $R_2$ is $C_1$-$C_6$ alkyl, $C_1$-$C_6$ alkylcarbonyl, $C_1$-$C_6$ alkoxycarbonyl, $C_1$-$C_6$ alkyl substituted by 1-3 atoms of F, Cl or 1 Br, or by 1 or 2 groups selected from $C_1$-$C_2$ alkoxy, CN, $C_1$-$C_2$ alkoxycarbonyl, $C_1$-$C_2$ alkylcarbonyl, OH, $C_1$-$C_2$ alkylthio $C_1$-$C_2$ alkylsulfonyl, $C_1$-$C_2$ alkylsulfonyloxy or $C_1$-$C_2$ alkylcarbonyloxy, $C_3$-$C_4$ alkenyl, $C_3$-$C_4$ haloalkenyl, $C_3$-$C_4$ alkynyl, $C_4$ alkynyl substituted by 1 atom of F or Cl, phenyl or phenyl substituted with Cl, $CF_3$, $NO_2$, CN or $SO_2CH_3$; and $R_4$ is $CH_3$.

**8.** Compounds of Claim 7 wherein A is A-1 and Y is $CH_3$, $C_2H_5$, $OCH_3$, $CH_2OCH_3$, $OCF_2H$ or $CH(OCH_3)_2$.

**9.** Compounds of Claim 8 wherein $R_1$ and $R_1'$ are H, Cl, $C_1$-$C_2$ alkyl, $C_1$-$C_2$ alkoxy or $C_1$-$C_2$ alkylthio.

**10.** Compounds of Claim 9 wherein J is J-1.

**11.** Compounds of Claim 10 wherein $R_1$ is H and Z is CH.

**12.** Compounds of Claim 11 wherein $R_2$ is $C_1$-$C_4$ haloalkyl, $C_1$-$C_3$ alkyl substituted with $C_1$-$C_2$ alkoxy, $C_1$-$C_2$ alkylthio, $C_1$-$C_2$ alkylsulfonyl or CN, $C_3$-$C_4$ alkenyl, $C_3$-$C_4$ haloalkenyl, $C_3$-$C_4$ alkynyl, $C_1$-$C_3$ alkoxycarbonyl or $C_1$-$C_3$ alkylcarbonyl.

**13.** Compounds of Claim 10 wherein $R_1$ is H; Z is N; and $R_2$ is $C_1$-$C_3$ alkylcarbonyl or $C_1$-$C_3$ alkoxycarbonyl.

**14.** Compounds of Claim 3 wherein W is O; $R_1$ is H; X is $CH_3$, $OCH_3$, $OCH_2CH_3$, Cl, F, Br, $OCF_2H$, $CH_2F$, $CF_3$ or cyclopropyl; Y is H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C≡CH$, $OCH_2CF_3$, CN, $N_3$, $OCH_2CH_2OCH_3$, $CH_2SCH_3$,

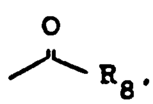

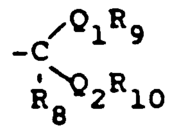 , 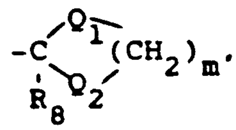 , 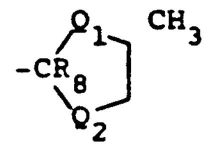

or $QCF_2T$; $R_2$ is $C_1$-$C_6$ alkyl, $C_1$-$C_6$ alkylcarbonyl, $C_1$-$C_6$ alkoxycarbonyl, $C_1$-$C_6$ alkyl substituted by 1-3 atoms of F, Cl or 1 Br, or by 1 or 2 groups selected from $C_1$-$C_2$ alkoxy, CN, $C_1$-$C_2$ alkoxycarbonyl, $C_1$-$C_2$ alkylcarbonyl, OH, $C_1$-$C_2$ alkylthio $C_1$-$C_2$ alkylsulfonyl, $C_1$-$C_2$ alkylsulfonyloxy or $C_1$-$C_2$ alkylcarbonyloxy, $C_3$-$C_4$ alkenyl, $C_3$-$C_4$ haloalkenyl, $C_3$-$C_4$ alkynyl, $C_4$ alkynyl substituted by 1 atom of F or Cl, phenyl or phenyl substituted with Cl, $CF_3$, $NO_2$, CN or $SO_2CH_3$; $R_1'$ is H, Cl, $C_1$-$C_2$ alkyl, $C_1$-$C_2$ alkoxy or $C_1$-$C_2$ alkylthio.

**15.** Compounds of Claim 14 wherein A is A-1; and Y is $CH_3$, $C_2H_5$, $OCH_3$ , $CH_2OCH_3$, $OCF_2H$ or CH-$(OCH_3)_2$.

**16.** Compounds of Claim 4 wherein W is O, $R_1$ is H, $R_1'$ is H, A is A-1, X is $CH_3$, $OCH_3$ , $OCH_2CH_3$, Cl, F, Br, $OCF_2H$, $CH_2F$ or cyclopropyl; and Y is $CH_3$, $C_2H_5$, $OCH_3$ , $CH_2OCH_3$, $OCF_2H$ or $CH(OCH_3)_2$.

**17.** The compound of Claim 1 which is 2,3-dihydro-N-[(4,6-dimethoxypyrimidin-2-yl)aminocarbonyl]-2-(2-methoxyethyl)-1,2-benzisothiazole-7-sulfonamide, 1,1-dioxide.

**18.** The compound of Claim 1 which is 2,3-dihydro-N-[(4,6-dimethoxypyrimidin-2-yl)aminocarbonyl]-2-(3-methylbutyl)-1,2-benzisothiazole-7-sulfonamide, 1,1-dioxide.

**19.** The compound of Claim 1 which is 2,3-dihydro-N-[(4,6-dimethoxypyrimidin-2-yl)aminocarbonyl]-2-(phenylmethyl)-1,2-benzisothiazole-7-sulfonamide, 1,1-dioxide.

**20.** The compound of Claim 1 which is 2,3-dihydro-N-[(4,6-dimethoxypyrimidin-2-yl)aminocarbonyl]-2-(2-oxopropyl)-1,2-benzisothiazole-7-sulfonamide, 1,1-dioxide.

**21.** The compound of Claim 1 which is 2,3-dihydro-N-[(4-chloro-6-methoxypyrimidin-2-yl)aminocarbonyl]-2-(2-ethoxyethyl)-1,2-benzisothiazole-7-sulfonamide, 1,1-dioxide.

**22.** The compound of Claim 1 which is 2,3-dihydro-N-[(4,6-dimethoxypyrimidin-2-yl)aminocarbonyl]-2-(2-ethoxyethyl)-1,2-benzisothiazole-7-sulfonamide, 1-1-dioxide.

**23.** The compound of Claim 1 which is 2,3-dihydro-N-[(4,6-dimethylpyrimidin-2-yl)aminocarbony]]-2-(2-fluoroethyl)-1,2-benzisothiazole-7-sulfonamide, 1,1-dioxide.

**24.** The compound of Claim 1 which is 2,3-dihydro-N-[(4-methoxy-6-methyl-1,3,5-triazin-2-yl)-aminocarbonyl]-2-(2-fluoroethyl)-1,2-benzisothiazole-7-sulfonamide, 1,1-dioxide.

**25.** The compound of Claim 1 which is 2,3-dihydro-N-[(4,6-dimethoxypyrimidin-2-yl)aminocarbonyl]-2-acetyl-1,2-benzoisothiazole-7-sulfonamide, 1,1-dioxide.

**26.** The compound of Claim 1 which is 2,3-dihydro-N-[(4,6-dimethoxypyrimidin-2-yl)aminocarbonyl]-2-(3-chloropropyl)-1,2-benzisothiazole-7-sulfonamide, 1,1-dioxide.

**27.** The compound of Claim 1 which is 2,3-dihydro-N-[(chloro-6-methoxypyrimidin-2-yl)aminocarbonyl]-2-(2-fluoroethyl)-1,2-benzisothiazole-7-sulfonamide, 1,1-dioxide.

**28.** The compound of Claim 1 which is 2,3-dihydro-N-[(4,6-dimethoxypyrimidin-2-yl)aminocarbonyl]-2-(3-fluoropropyl)-1,2-benzisothiazole-7-sulfonamide, 1,1-dioxide.

**29.** The compound of Claim 1 which is 2,3-dihydro-N-[(4-chloro-6-methoxypyrimidin-2-yl)aminocarbonyl]-2-(2-chloroethyl)-1,2-benzisothiazole-7-sulfonamide, 1,1-dioxide.

**30.** The compound of Claim 1 which is N-[(4-chloro-6-methoxypyrimidin-2-yl)aminocarbonyl]-2-(2-fluoroethyl)-2,3-dihydro-1,2-benzoisothiazole-7-sulfonamide, 1,1-dioxide.

**31.** The compound of Claim 1 which is N-[(4-chloro-6-methoxypyrimidin-2-yl)aminocarbonyl]-2-(3-fluoropropyl)-2,3-dihydro-1,2-benzoisothiazole-7-sulfonamide, 1,1-dioxide.

**32.** A compound of Claim 1 wherein:

J is selected from J-1 - J-4;

$W_2$ is O;

$R_1$ and $R_1'$ are H, F, Cl, Br, $CH_3$, $C_2H_5$, $OCH_3$, $OC_2H_5$, $CF_3$, $OCF_2H$ or $SCH_3$;

$R_2$ is H, $C_3$-$C_6$ cycloalkyl, a $C_1$-$C_{10}$ alkyl radical, a $C_2$-$C_{10}$ alkenyl radical and its corresponding epoxide, a $C_2$-$C_{10}$ alkynyl radical, a $C_1$-$C_9$ alkoxyl radical, a $C_1$-$C_9$ alkylcarbonyl radical, a $C_1$-$C_8$ alkylsulfonyl radical or a $C_1$-$C_8$ alkoxycarbonyl radical. Each above named radical is optionally substituted with halogen, $C_3$-$C_6$ cycloalkyl, $C_1$-$C_8$ alkoxy, $C_1$-$C_8$ haloalkoxy, $C_1$-$C_8$ alkylthio, $C_1$-$C_8$ haloalkylthio, $C_1$-$C_8$ alkylsulfinyl, $C_1$-$C_8$ haloalkylsulfinyl, $C_1$-$C_8$ alkylsulfonyl, $C_1$-$C_8$ haloalkylsulfonyl, $C_1$-$C_6$ haloalkylcarbonyl, $C_1$-$C_6$ alkylcarbonyloxy, $C_1$-$C_6$ haloalkylcarbonyloxy, $C_1$-$C_6$ alkylsulfonyloxy, $C_1$-$C_6$ alkylcarbonyl, $C_1$-$C_6$ alkoxycarbonyl, $C_1$-$C_6$ haloalkoxycarbonyl, formyl, amino, $C_1$-$C_6$ alkylamino, $C_1$-$C_8$ dialkylamino, $C_1$-$C_6$ alkylcarbonylamino, OH, CN, $NO_2$, $C_3$-$C_6$ alkylsilyl, $C_2$-$C_6$ alkyl(phenylsilyl) where phenyl is optionally substituted with halogen, $CH_3$, $OCH_3$ or $NO_2$ or phenyl optionally substituted with halogen, $CH_3$, $OCH_3$ or $NO_2$;

X is $CH_3$, $OCH_3$, $OCH_2CH_3$, Cl, F, Br, $OCF_2H$, $CH_2F$, $CF_3$ or cyclopropyl;

Y is H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$,

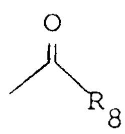,
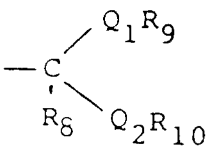,

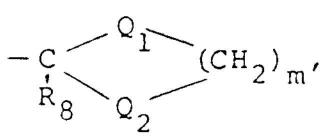,
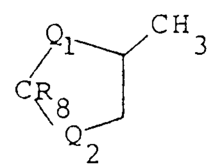
or $QCF_2T$;

m is 2 or 3;

Q is O or S;

$Q_1$ and $Q_2$ are independently O or S;

$R_8$ is H or $C_1$-$C_3$ alkyl,

$R_9$ and $R_{10}$ are independently $C_1$-$C_3$ alkyl; and T is H, CHClF, CHBrF or $CHFCF_3$.

**33.** A composition suitable for controlling the growth of undesired vegetation which comprises an effective amount of a compound of any of claims 1 to 32 and at least one of the following: surfactant, solid or liquid diluent.

**34.** A method for controlling the growth of undesired vegetation which comprises applying to the locus to be protected an effective amount of the compound of any of claims 1 to 32.

**35.** A method for regulating the growth of plants which comprises applying to the locus of such plants an effective but substantially non-phytotoxic amount of a plant growth regulant selected from compounds of any of claims 1 to 32.

**36.** A method for the production of a compound of claim 1 which comprises:

(a) reacting a sulfonylisocyanate or sulfonylisothiocyanate of formula

$J\text{-}SO_2NCW$ (II)

wherein J is $J_1$, $J_2$, $J_3$ or $J_4$ as defined in claim 1 and W is O or S, with an appropriate heterocyclic amine of formula

$$\underset{\displaystyle R}{HN}\text{-}A \qquad (III)$$

wherein R and A are as defined in claim 1; or

(b) reacting a sulfonamide of formula

$J\text{-}SO_2NH_2$ (IV)

wherein J is $J_1$, $J_2$, $J_3$ or $J_4$ as defined in claim 1, with a heterocyclic isothiocyanate of formula

SCN-A

wherein A is as defined in claim 1, to obtain a compound of claim 1 wherein W is S and R is H; or

(c) reacting a sulfonylcarbamate of formula

$$J\text{-}SO_2NH\overset{\displaystyle O}{\overset{\|}{C}}OC_6H_5 \qquad (VI)$$

wherein J is $J_1$, $J_2$, $J_3$ or $J_4$ as defined in claim 1, with an amine of formula (III) as defined above; or

(d) reacting said sulfonamide of formula (IV) with a heterocyclic carbamate of formula

$$PhO\overset{\displaystyle O}{\overset{\|}{C}}\underset{\displaystyle R}{N}\text{-}A \qquad (VII)$$

wherein A and R are as defined in claim 1; or

(e) reacting said sulfonamide of formula (IV) with an appropriate methylcarbamate of formula

$$CH_3O\overset{\displaystyle O}{\overset{\|}{C}}\text{-}\underset{\displaystyle R}{N}\text{-}A \qquad (VIII)$$

wherein A and R are as defined in claim 1.

**37.** Sulfonamides of formula (IV) as defined in claim 36.

**Revendications**

**1.** Un composé de la formule :

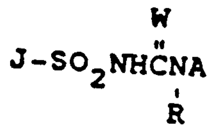

I

dans laquelle
J est

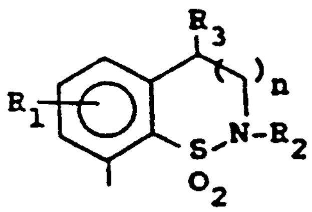, 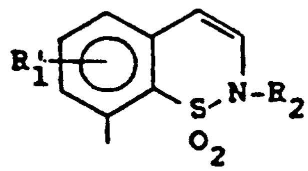, 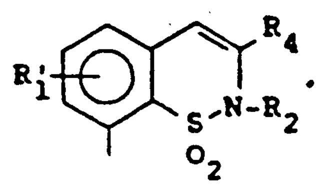,

J-1 J-2 J-3

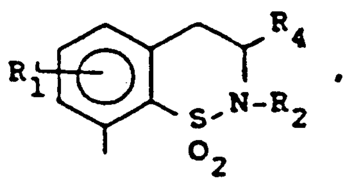,

J-4

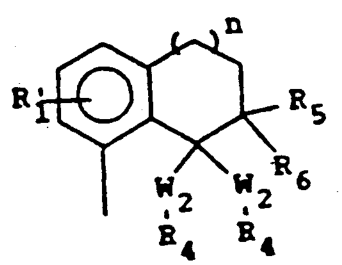

J-11

ou

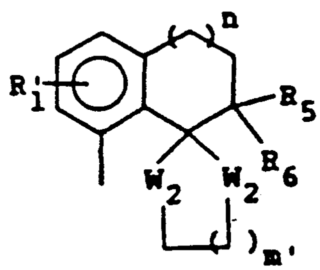

J-12

| | |
|---|---|
| n | est 0 ou 1 ; |
| W | est O ou S ; |
| $W_1$ | est S ; |
| $W_2$ | est O ou S ; |
| R | est H ou $CH_3$; |
| $R_1$ | est H, un groupe alkyle en $C_1$-$C_6$, halogénalkyle en $C_1$-$C_6$, halogéno, nitro, alcoxy en $C_1$-$C_6$, $SO_2NR_aR_b$, alkylthio en $C_1$-$C_6$, alkylsulfinyle en $C_1$-$C_6$, alkylsulfonyle en $C_1$-$C_6$, CN, $CO_2R_c$, halogénalcoxy en $C_1$-$C_6$, halogénalkylthio en $C_1$-$C_6$, $NH_2$, alkylamino en $C_1$-$C_6$, di-(alkyle en $C_1$-$C_6$)amino, $Si(CH_3)_2$(alkyle en $C_1$-$C_4$), $Si(CH_3)_2$phényle ou alkyle en $C_1$-$C_3$ substitué par alcoxy en $C_1$-$C_3$, alkylthio en $C_1$-$C_3$, alkylsulfinyle en $C_1$-$C_3$, alkylsulfonyle en $C_1$-$C_3$, $SO_2NR_dR_e$, $NO_2$, CN, $CO_2$ $R_f$, halogénalcoxy en $C_1$-$C_3$ ou halogénalkylthio en $C_1$-$C_3$ ; |
| $R_a$ | est N, un groupe alkyle en $C_1$-$C_4$, cyanoalkyle en $C_1$-$C_3$, méthoxy ou éthoxy ; |
| $R_b$ | est H, un groupe alkyle en $C_1$-$C_4$ ou alcényle en $C_3$-$C_4$ ; ou bien |
| $R_a$ | et $R_b$ peuvent être pris ensemble sous la forme $-(CH_2)_3-$, $-(CH_2)_4-$, $-(CH_2)_5-$ ou $-CH_2CH_2OCH_2CH_2-$ ; |
| $R_c$ | est un groupe alkyle en $C_1$-$C_4$, alcényle en $C_3$-$C_4$, alcynyle en $C_3$-$C_4$, halogénalkyle en $C_2$-$C_4$, cyanoalkyle en $C_2$-$C_3$, cycloalkyle en $C_5$-$C_6$, cycloalkylalkyle en $C_4$-$C_7$ ou alcoxyalkyle en $C_2$-$C_4$ ; |
| $R_d$ | est un groupe alkyle en $C_1$-$C_3$ ; |
| $R_e$ | est H ou un groupe alkyle en $C_1$-$C_3$ ; |
| $R_f$ | est un groupe alkyle en $C_1$-$C_3$ ; |
| $R_1'$ | est H, un groupe alkyle en $C_1$-$C_3$, alcoxy en $C_1$-$C_3$, halogénalcoxy en $C_1$-$C_3$, halogénalkyle en $C_1$-$C_3$, alkylthio en $C_1$-$C_3$, halogénalkylthio en $C_1$-$C_3$, amino, alkylamino en $C_1$-$C_3$, di(alkyle en $C_1$-$C_3$)amino, halogéno ou $NO_2$ ; |
| $R_2$ | est H, $R_{11}$, $SR_{11}$, $SO_2R_{11}$, $OR_{11}$, $C(O)R_{11}$, $C(O)OR_{11}$, $(C(O))_2OR_{11}$, $(CO)_2R_{11}$, $C(O)NR_{12}R_{18}$, C(O)NRA, $C(S)SR_{11}$, $NH_2$, $NR_{12}R_{18}$, OH, CN, $P(O)R_{13}R_{14}$, $P(S)R_{13}R_{14}$, $Si(CH_3)_2R_{15}$, L ou C(O)L ; |
| $R_3$ | est H ou $CH_3$ ; |
| $R_4$ | est un groupe alkyle en $C_1$-$C_4$ ; |
| $R_5$ | est H ou un groupe alkyle en $C_1$-$C_4$ ; |
| $R_6$ | est H ou $CH_3$ ; |
| A | est |

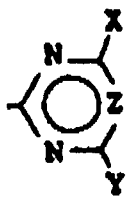 , 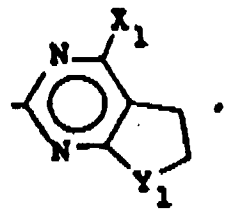 , 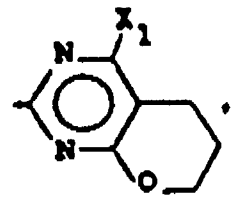 ,

A-1 A-2 A-3

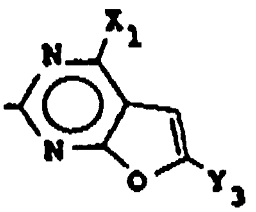 , 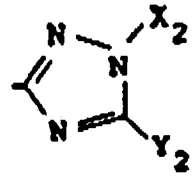 ou 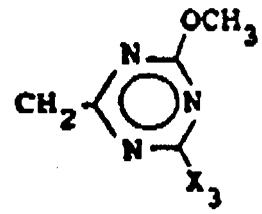 ;

A-4 A-5 A-6

X est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, F, Cl, Br, alcoxyalkyle en $C_2$-$C_5$, alcoxyalcoxy en $C_2$-$C_5$, amino, alkylamino en $C_1$-$C_3$, di(alkyle en $C_1$-$C_3$)amino ou cycloalkyle en $C_3$-$C_5$ ;

Y est H, un groupe alkyle en $C_1$-$C_4$, alcoxy en $C_1$-$C_4$, halogénalcoxy en $C_1$-$C_4$, halogénalkylthio en $C_1$-$C_4$, alkylthio en $C_1$-$C_4$, F, Cl, Br, alcoxyalkyle en $C_2$-$C_5$, alcoxyalcoxy en $C_2$-$C_5$, amino, alkylamino en $C_1$-$C_3$, di(alkyle en $C_1$-$C_3$)amino, alcényloxy en $C_3$-$C_4$, alcynyloxy en $C_3$-$C_4$, alkylthioalkyle en $C_2$-$C_5$,halogénalkyle en $C_1$-$C_4$, azido, cyano,

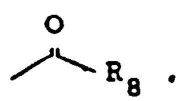 ,

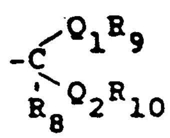 , 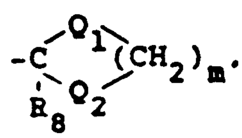 , 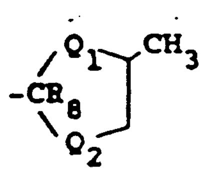 ou

$N(OCH_3)CH_3$ ;

m est 2 ou 3 ;

$Q_1$ et $Q_2$ sont indépendamment O ou S ;

$R_8$ est H ou un groupe alkyle en $C_1$-$C_3$ ;

$R_9$ et $R_{10}$ sont indépendamment un groupe alkyle en $C_1$-$C_3$ ;

Z est CH, N, $CCH_3$, $CC_2H_5$, CCl ou CBr ;

$Y_1$ est O ou $CH_2$ ;

$X_1$ est $CH_3$, $OCH_3$, $OC_2H_5$ ou $OCF_2H$ ;

$X_2$ est $CH_3$, $C_2H_5$ ou $CH_2CF_3$ ;

$Y_2$ est $OCH_3$, $OC_2H_5$, $SCH_3$, $SC_2H_5$, $CH_3$ ou $CH_2CH_3$ ;

$X_3$ est $CH_3$ ou $OCH_3$ ;

$Y_3$ est H ou $CH_3$ ;

$R_{11}$ est un groupe alkyle en $C_1$-$C_{10}$, alcoxyalcoxyalkyle en $C_1$-$C_{10}$, alcényle en $C_2$-$C_{10}$, alcénylalcényle en jusqu'à $C_{10}$, époxyalkyle en $C_2$-$C_{10}$, alcynyle en $C_2$-$C_{10}$, alcynylalcynyle en jusqu'à $C_{10}$, alcynylalcényle en jusqu'à $C_{10}$, cycloalkyle en $C_3$-$C_6$, cycloalkylalkyle en $C_4$-$C_7$ ou

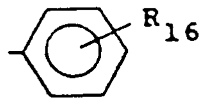 ;

si $R_{11}$ est un groupe cycloalkyle en $C_3$-$C_6$ ou cycloalkylalkyle en $C_4$-$C_7$, il peut être facultativement substitué par un groupe alkyle en $C_1$-$C_4$, 1 à 3 atomes de Cl ou F ou 1 atome de Br ; si $R_{11}$ est un groupe alkyle en $C_1$-$C_{10}$,alcényle en $C_2$-$C_{10}$ ou alcynyle en $C_2$-$C_{10}$, il peut être facultativement substitué par un ou plusieurs halogènes et/ou par $(R_{17})_{m'}$ où, lorsque m' est 2, les valeurs de $R_{17}$ peuvent être identiques ou différentes ; m' est 1 ou 2 ;

$R_{12}$ est H ou un groupe alkyle en $C_1$-$C_4$ ;

$R_{13}$ et $R_{14}$ sont indépendamment un groupe alkyle en $C_1$-$C_4$, alcoxy en $C_1$-$C_4$ ou alkylthio en $C_1$-$C_4$ ;

$R_{15}$ est un groupe alkyle en $C_1$-$C_{10}$, benzyle ou

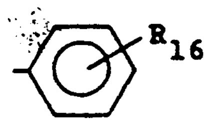 ;

$R_{16}$ est H, F, Cl, Br, $CH_3$, $OCH_3$, $NO_2$, CN, $SCH_3$, $SO_2CH_3$ ou $CF_3$ ;

$R_{17}$ est $OR_{18}$, $OC(O)R_{18}$, $P^+R_9R_{10}R_{15}$, $P^+(C_6H_5)_3$, $OC(O)NR_{12}R_{18}$, $OSO_2R_{18}$, $OP(O)R_{13}R_{14}$, $P(O)R_{13}R_{14}$,$OP(S)R_{13}R_{14}$, $P(S)R_{13}R_{14}$, $OSi(CH_3)_2R_{15}$, $Si(CH_3)_2R_{15}$, $SR_{18}$, $SOR_{18}$, $SO_2R_{18}$, SCN, CN, $SP(O)R_{13}R_{14}$, $SP(S)R_{13}R_{14}$, $N^+R_{12}R_{15}R_{18}$, $NR_{12}R_{18}$, $NR_{12}C(O)R_{18}$, $NR_{12}C(O)OR_{18}$, $NR_{12}C(O)NR_{12}R_{18}$, $NR_{12}SO_2R_{18}$, $NR_{12}P(O)R_{13}R_{14}$, $NR_{12}P(S)$-$R_{13}R_{14}$,$NO_2$, $C(O)R_{18}$, $C(O)OR_{18}$, $C(O)NR_{12}R_{18}$, $SeR_{18}$, un groupe naphtyle, L

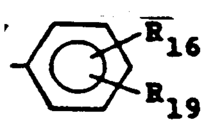 , 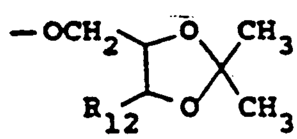 ,

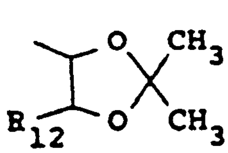 , 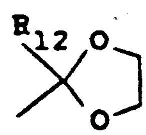 , 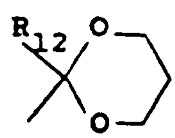

ou

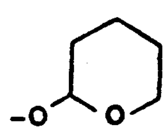

$R_{18}$ est H, un groupe alkyle en $C_1$-$C_{10}$, halogénalkyle en $C_1$-$C_{10}$, alcényle en $C_2$-$C_{10}$, alcynyle en $C_2$-$C_{10}$, cycloalkyle en $C_3$-$C_6$ ou

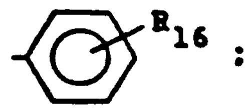

$R_{19}$ est H, F, Cl, Br, $CH_3$,

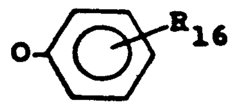

ou

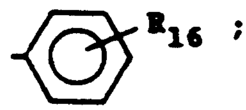

et

L est un hétérocycle aromatique penta- ou hexagonal, un hétérocycle dihydroaromatique penta- ou hexagonal ou un hétérocycle tétrahydroaromatique penta- ou hexagonal qui contient 1 à 4 hétéroatomes choisis parmi 0 ou 1 atome d'oxygène, 0 ou 1 atome de soufre, où le soufre peut être sous la forme de S, SO ou $SO_2$, et/ou 0 à 4 atomes d'azote, avec la condition que l'oxygène et le soufre ne soient liés l'un à l'autre que si le soufre est sous la forme de SO ou $SO_2$ ; et ces hétérocycles peuvent facultativement être substitués par 1 à 4 $CH_3$, 1 ou 2 $OCH_3$, $SCH_3$, Cl, $N(CH_3)_2$ ou CN, ou bien L est une lactone, un lactame ou une cycloalcanone, penta- ou hexagonal, qui peut être facultativement substitué par 1 à 4 groupes $CH_3$ ;

avec les conditions suivantes

a) si W est S, alors R est H, J est $J_1$, $J_2$, $J_3$ ou $J_4$, A est A-1, Z est CH ou N, et Y est $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CF_3$, $OCH_2CH_2OCH_3$,CH-$(OCH_3)_2$ ou

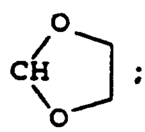

b) si X est F, Cl ou Br, alors Z est CH et Y est $OCH_3$, $OC_2H_5$, $NH_2$, $NHCH_3$, $N(CH_3)_2$ ou $OCF_2H$ ;

c) si $R_3$ est $CH_3$, alors n est 0 ;

d) si J est J-1 ou J-2 et $R_2$ est H ou un groupe alkyle en $C_1$-$C_4$, alors $R_1$ et $R_1'$ sont autre chose que H, F, Cl, Br, $CH_3$, $OCH_3$, $CF_3$, $OCF_2H$ ou $SCH_3$, ou X est autre chose que $CH_3$, $OCH_3$, $OCH_2CH_3$, F, Cl, Br, $OCF_2H$, $CH_2Cl$, $CH_2Br$, $CH_2F$, un groupe cyclopropyle ou $CF_3$, ou Y est un groupe alkyle en $C_3$-$C_4$, alcoxy en $C_3$-$C_4$, halogénalcoxy en $C_4$, halogénalkylthio en $C_4$, alcoxyalkyle en $C_3$-$C_5$, alcoxyalcoxy en $C_4$-$C_5$, alkylamino en $C_2$-$C_3$, di(alkyle en $C_2$-$C_3$)amino, alcényloxy en $C_4$, alcynyloxy en $C_4$, alkylthioalkyle en $C_3$-$C_5$, halogénalkyle en $C_2$-$C_4$, alcynyle en $C_2$-$C_4$, $C(O)R_8$ ou $N(OCH_3)CH_3$ ;

e) le nombre total d'atomes de carbone de $R_2$ ne dépasse pas 13 ;

f) si X est un groupe cycloalkyle en $C_3$-$C_5$, alors Y est $CH_3$, $CH_2CH_3$, $OCH_3$, $OCH_2CH_3$, $CH_2OCH_3$, $OCF_2H$, $SCF_2H$, $OCH_2CF_3$, $CF_3$, $OCH_2CH=CH_2$, $OCH_2C\equiv CH$, $NHCH_3$, $N(CH_3)_2$ ou $CH(OCH_3)_2$ ;

g) si $R_{18}$ est H, $R_{17}$ est autre chose que $SOR_{18}$, $SO_2R_{18}$, $OSO_2R_{18}$ ou $NR_{12}C(O)OR_{18}$ ;

h) si $R_1$ ou $R_1'$ est en para par rapport au pont de sulfonylurée, alors $R_1$ ou $R_1'$ est H, $CH_3$, F, Cl, Br ou $OCH_3$ ; et

i) si X ou Y est $OCH_2CH_2F$ ou $OCH_2CHF_2$, alors $R_2$ est autre chose que H ou un groupe alkyle en $C_1$-$C_5$, $CH_3OCH_2CH_2$, $C_2H_5OCH_2CH_2$ ou alkyle en $C_1$-$C_4$ substitué par 1 à 3 atomes de F, Cl ou Br ;

j) si X ou Y est $OCH_2CH_2F$, $OCH_2CHF_2$ ou $OCH_2CF_3$, alors l'autre n'est pas un groupe di(alkyle en $C_1$-$C_3$)amino, alkylamino en $C_1$-$C_3$ ou $N(OCH_3)CH_3$ ;

k) si X ou Y est $OCF_2H$, alors Z est CH ; et ses sels utilisables en agriculture.

**2.** Un composé de la revendication 1, dans lequel J est J-1 ou J-4.

**3.** Un composé de la revendication 1, dans lequel J est J-2 ou J-3.

**4.** Un composé de la revendication 1, dans lequel J est J-11 ou J-12.

**5.** Composés de la revendication 2, dans lesquels

W est O ;
R est H ;
X est $CH_3$, $OCH_3$, $OCH_2CH_3$, Cl, F, Br, $OCF_2H$, $CH_2F$, $CF_3$ ou un groupe cyclopropyle ;
Y est H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C{\equiv}CH$, $OCH_2CF_3$, CN, $N_3$ , $OCH_2CH_2OCH_3$, $CH_2SCH_3$,

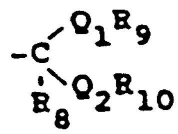 , 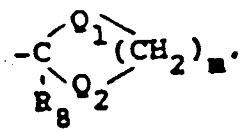 , 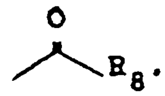 , 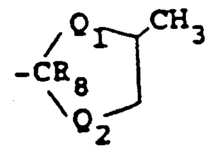

ou $QCF_2T$ ;
Q est O ou S ;
T est H, CHClF, CHBrF ou $CHFCF_3$.

**6.** Composés de la revendication 5, dans lesquels $R_1$ ou $R_1'$ est H, Cl, un groupe alkyle en $C_1$-$C_3$, alcoxy en $C_1$-$C_3$, halogénalcoxy en $C_1$-$C_3$, halogénalkyle en $C_1$-$C_3$, alkylthio en $C_1$-$C_3$, halogénalkylthio en $C_1$-$C_3$, amino, alkylsulfinyle en $C_1$-$C_3$, alkylsulfonyle en $C_1$-$C_3$, CN, $NH_2$, alkylamino en $C_1$-$C_3$, di-(alkylamino en $C_1$-$C_3$) ou alkyle en $C_1$-$C_2$ substitué par alcoxy en $C_1$-$C_2$, alkylthio en $C_1$-$C_2$, halogénalcoxy en $C_1$-$C_2$, halogénalkylthio en $C_1$-$C_2$, CN ou $NO_2$.

**7.** Composés de la revendication 6, dans lesquels $R_2$ est un groupe alkyle en $C_1$-$C_6$, (alkyle en $C_1$-$C_6$)-carbonyle, (alcoxy en $C_1$-$C_6$)carbonyle, alkyle en $C_1$-$C_6$ substitué par 1 à 3 atomes de F, Cl ou 1 atome de Br, ou par 1 ou 2 groupes choisis parmi les groupes alcoxy en $C_1$-$C_2$, CN, (alcoxy en $C_1$-$C_2$)-carbonyles, (alkyle en $C_1$-$C_2$)carbonyles, OH, alkylthio en $C_1$-$C_2$, alkylsulfonyles en $C_1$-$C_2$, alkylsulfonyloxy en $C_1$-$C_2$ et (alkyle en $C_1$-$C_2$)carbonyloxy, alcényle en $C_3$-$C_4$, halogénalcényle en $C_3$-$C_4$, alcynyle en $C_3$-$C_4$, alcynyle en $C_4$ substitué par 1 atome de F ou Cl, phényle ou phényle substitué par Cl, $CF_3$, $NO_2$, CN ou $SO_2CH_3$ ; et $R_4$ est $CH_3$.

**8.** Composés de la revendication 7, dans lesquels A est A-1 et Y est $CH_3$, $C_2H_5$, $OCH_3$, $CH_2OCH_3$, $OCF_2H$ ou $CH(OCH_3)_2$.

**9.** Composés de la revendication 8, dans lesquels $R_1$ et $R_1'$ sont H, Cl, un groupe alkyle en $C_1$-$C_2$, alcoxy en $C_1$-$C_2$ ou alkylthio en $C_1$-$C_2$.

**10.** Composés de la revendication 9, dans lesquels J est J-1.

**11.** Composés de la revendication 10, dans lesquels $R_1$ est H et Z est CH.

**12.** Composés de la revendication 11, dans lesquels $R_2$ est un groupe halogénalkyle en $C_1$-$C_4$, alkyle en $C_1$-$C_3$ substitué par alcoxy en $C_1$-$C_2$, alkylthio en $C_1$-$C_2$,alkylsulfonyle en $C_1$-$C_2$ ou CN, alcényle en $C_3$-$C_4$, halogénalcényle en $C_3$-$C_4$, alcynyle en $C_3$-$C_4$, (alcoxy en $C_1$-$C_3$)carbonyle ou (alkyle en $C_1$-$C_3$)-carbonyle.

**13.** Composés de la revendication 10, dans lesquels $R_1$ est H ; Z est N ; et $R_2$ est un groupe (alkyle en $C_1$-$C_3$)carbonyle ou (alcoxy en $C_1$-$C_3$)carbonyle.

**14.** Composés de la revendication 3, dans lesquels W est O ; $R_1$ est H ; X est $CH_3$, $OCH_3$, $OCH_2CH_3$, Cl,

F, Br, $OCF_2H$, $CH_2F$, $CF_3$ ou un groupe cyclopropyle ; Y est H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$,

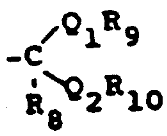 , 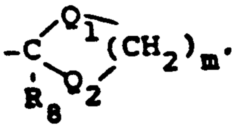 , 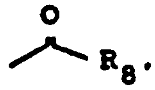 , 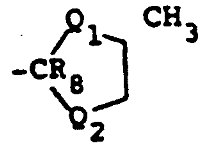

ou $QCF_2T$ ; $R_2$ est un groupe alkyle en $C_1$-$C_6$, (alkyle en $C_1$-$C_6$)carbonyle, (alcoxy en $C_1$-$C_6$)carbonyle, alkyle en $C_1$-$C_6$ substitué par 1 à 3 atomes de F, Cl ou 1 atome de Br, ou par 1 ou 2 groupes choisis parmi les groupes alcoxy en $C_1$-$C_2$, CN, (alcoxy en $C_1$-$C_2$)carbonyles, (alkyle en $C_1$-$C_2$)carbonyles, OH, alkylthio en $C_1$-$C_2$, alkylsulfonyle en $C_1$-$C_2$, alkylsulfonyloxy en $C_1$-$C_2$ ou (alkyle en $C_1$-$C_2$)carbonyloxy, alcényle en $C_3$-$C_4$, halogénalcényle en $C_3$-$C_4$, alcynyle en $C_3$-$C_4$, alcynyle en $C_4$ substitué par 1 atome de F ou Cl, phényle ou phényle substitué par Cl, $CF_3$, $NO_2$, CN ou $SO_2CH_3$ ; $R_1'$ est H, Cl, un groupe alkyle en $C_1$-$C_2$, alcoxy en $C_1$-$C_2$ ou alkylthio en $C_1$-$C_2$.

**15.** Composés de la revendication 14, dans lesquels A est A-1 ; et Y est $CH_3$, $C_2H_5$, $OCH_3$, $CH_2OCH_3$, $OCF_2H$ ou $CH(OCH_3)_2$.

**16.** Composés de la revendication 4, dans lesquels W est O, $R_1$ est H, $R_1'$ est H, A est A-1, X est $CH_3$, $OCH_3$, $OCH_2CH_3$, Cl, F, Br, $OCF_2H$, $CH_2F$ ou un groupe cyclopropyle ; et Y est $CH_3$, $C_2H_5$, $OCH_3$, $CH_2OCH_3$, $OCF_2H$ ou $CH(OCH_3)_2$.

**17.** Le composé de la revendication 1, qui est le 1,1-dioxyde de 2,3-dihydro-N-[(4,6-diméthoxypyrimidine-2-yl)aminocarbonyl]-2-(2-méthoxyéthyl)-1,2-benzisothiazole-7-sulfonamide.

**18.** Le composé de la revendication 1, qui est le 1,1-dioxyde de 2,3-dihydro-N-[(4,6-diméthoxypyrimidine-2-yl)aminocarbonyl]-2-(3-méthylbutyl)-1,2-benzisothiazole-7-sulfonamide.

**19.** Le composé de la revendication 1, qui est le 1,1-dioxyde de 2,3-dihydro-N-[(4,6-diméthoxypyrimidine-2-yl)aminocarbonyl]-2-(phénylméthyl)-1,2-benzisothiazole-7-sulfonamide.

**20.** Le composé de la revendication 1, qui est le 1,1-dioxyde de 2,3-dihydro-N-[(4,6-diméthoxypyrimidine-2-yl)aminocarbonyl]-2-(2-oxopropyl)-1,2-benzisothiazole-7-sulfonamide.

**21.** Le composé de la revendication 1, qui est le 1,1-dioxyde de 2,3-dihydro-N-[(4-chloro-6-méthoxypyrimidine-2-yl)aminocarbonyl]-2-(2-éthoxyéthyl)-1,2-benzisothiazole-7-sulfonamide.

**22.** Le composé de la revendication 1, qui est le 1,1-dioxyde de 2,3-dihydro-N-[(4,6-diméthoxypyrimidine-2-yl)aminocarbonyl]-2-(2-éthoxyéthyl)-1,2-benzisothiazole-7-sulfonamide.

**23.** Le composé de la revendication 1, qui est le 1,1-dioxyde de 2,3-dihydro-N-[(4,6-diméthylpyrimidine-2-yl)aminocarbonyl]-2-(2-fluoréthyl)-1,2-benzisothiazole-7-sulfonamide.

**24.** Le composé de la revendication 1, qui est le 1,1-dioxyde de 2,3-dihydro-N-[(4-méthoxy-6-méthyl-1,3,5-triazine-2-yl)aminocarbonyl]-2-(2-fluoréthyl)-1,2-benzisothiazole-7-sulfonamide.

**25.** Le composé de la revendication 1, qui est le 1,1-dioxyde de 2,3-dihydro-N-[(4,6-diméthoxypyrimidine-2 yl)aminocarbonyl]-2-acétyl-1,2-benzisothiazole-7-sulfonamide.

**26.** Le composé de la revendication 1, qui est le 1,1-dioxyde de 2,3-dihydro-N-[(4,6-diméthoxypyrimidine-2-yl)aminocarbonyl]-2-(3-chloropropyl)-1,2-benzisothiazole-7-sulfonamide.

**27.** Le composé de la revendication 1, qui est le 1,1-dioxyde de 2,3-dihydro-N-[(chloro-6-méthoxypyrimidine-2-yl)aminocarbonyl]-2-(2-fluoréthyl)-1,2-benzisothiazole-7-sulfonamide.

**28.** Le composé de la revendication 1, qui est le 1,1-dioxyde de 2,3-dihydro-N-[(4,6-diméthoxypyrimidine-2-yl)aminocarbonyl]-2-(3-fluoropropyl)-1,2-benzisothiazole-7-sulfonamide,

**29.** Le composé de la revendication 1, qui est le 1,1-dioxyde de 2,3-dihydro-N-[(4-chloro-6-méthoxypyrimidine-2-yl)aminocarbonyl]-2-(2-chloréthyl)-1,2-benzisothiazole-7-sulfonamide.

**30.** Le composé de la revendication 1, qui est le 1,1-dioxyde de N-[(4-chloro-6-méthoxypyrimidine-2-yl)-aminocarbonyl]-2-(2-fluoréthyl)-2,3-dihydro-1,2-benzisothiazole-7-sulfonamide.

**31.** Le composé de la revendication 1, qui est le 1,1-dioxyde de N-[(4-chloro-6-méthoxypyrimidine-2-yl)-aminocarbonyl]-2-(3-fluoropropyl)-2,3-dihydro-1,2-benzisothiazole-7-sulfonamide.

**32.** Un composé de la revendication 1, dans lequel ;

J est choisi entre J-1 à J-4 ;

$W_2$ est O ;

$R_1$ et $R_1'$ sont H, F, Cl, Br, $CH_3$, $C_2H_5$, $OCH_3$, $OC_2H_5$, $CF_3$, $OCF_2H$ ou $SCH_3$ ;

$R_2$ est H, un groupe cycloalkyle en $C_3$-$C_6$, un radical alkyle en $C_1$-$C_{10}$, un radical alcényle en $C_2$-$C_{10}$ et son époxyde correspondant, un radical alcynyle on $C_2$-$C_{10}$, un radical alcoxy en $C_1$-$C_9$, un radical (alkyle en $C_1$-$C_9$)carbonyle, un radical alkylsulfonyle en $C_1$-$C_8$ ou un radical (alcoxy en $C_1$-$C_8$)carbonyle, chaque radical nommé ci-dessus étant facultativement substitué par un halogène, un groupe cycloalkyle en $C_3$-$C_6$, alcoxy en $C_1$-$C_8$, halogénalcoxy en $C_1$-$C_8$, alkylthio en $C_1$-$C_8$, halogénalkylthio en $C_1$-$C_8$, alkylsulfinyle en $C_1$-$C_8$, halogénalkylsulfinyle en $C_1$-$C_8$, alkylsulfonyle en $C_1$-$C_8$, halogénalkylsulfonyle en $C_1$-$C_8$, (halogénalkyle en $C_1$-$C_6$)-carbonyle, (alkyle en $C_1$-$C_6$)carbonyloxy, (halogénalkyle en $C_1$-$C_6$)carbonyloxy, alkylsulfonyloxy en $C_1$-$C_6$, (alkyle en $C_1$-$C_6$)carbonyle, (alcoxy en $C_1$-$C_6$)carbonyle, (halogénalcoxy en $C_1$-$C_6$)carbonyle, formyle, amino, alkylamino en $C_1$-$C_6$, (dialkyle en $C_1$-$C_8$)amino, (alkyle en $C_1$-$C_6$)carbonylamino, OH, CN, $NO_2$, alkylsilyle en $C_3$-$C_6$, (alkyle en $C_2$-$C_6$)(phénylsilyle) où le radical phényle est facultativement substitué par un halogène, $CH_3$, $OCH_3$ ou $NO_2$, ou phényle facultativement substitué par un halogène, $CH_3$, $OCH_3$ ou $NO_2$ ;

X est $CH_3$, $OCH_3$, $OCH_2CH_3$, Cl, F, Br, $OCF_2H$, $CH_2F$, $CF_3$ ou un groupe cyclopropyle ;

Y est H, $CH_3$, $OCH_3$, $OC_2H_5$, $CH_2OCH_3$, $NH_2$, $NHCH_3$, $N(OCH_3)CH_3$, $N(CH_3)_2$, $C_2H_5$, $CF_3$, $SCH_3$, $OCH_2CH=CH_2$, $OCH_2C≡CH$, $OCH_2CF_3$, CN, $N_3$, $OCH_2CH_2OCH_3$, $CH_2SCH_3$,

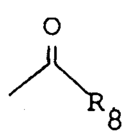,
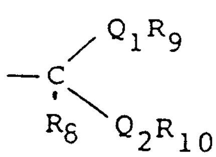,

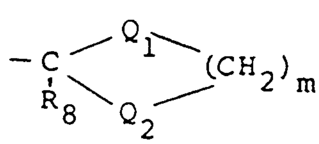,
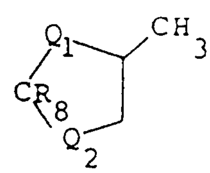

ou $QCF_2T$;

m est 2 ou 3 ;

Q est O ou S ;

$Q_1$ et $Q_2$ sont indépendamment O ou S ;

| | |
|---|---|
| $R_8$ | est H ou un groupe alkyle en $C_1$-$C_3$, |
| $R_9$ et $R_{10}$ | sont indépendamment un groupe alkyle en $C_1$-$C_3$ ; et |
| T | est H, CHClF, CHBrF ou $CHFCF_3$. |

**33.** Une composition convenant pour combattre la croissance de végétation indésirable, qui contient une quantité efficace d'un composé de l'une quelconque des revendications 1 à 32 et au moins l'un des ingrédients suivants : agent tensio-actif, diluant solide ou liquide.

**34.** Un procédé pour combattre la croissance de végétation indésirable, qui consiste à appliquer au lieu à protéger une quantité efficace du composé de l'une quelconque des revendications 1 à 32.

**35.** Un procédé pour réguler la croissance de plantes, qui consiste à appliquer nu lieu où se trouvent ces plantes une quantité efficace, mais sensiblement non phytotoxique, d'un régulateur au croissance de plantes choisi parmi les composés de l'une quelconque des revendications 1 à 32.

**36.** Un procédé pour la production d'un composé de la revendication 1, qui consiste à :
(a) faire réagir un isocyanate de sulfonyle ou isothiocyanate de sulfonyle de formule

$J$-$SO_2NCW$ (II)

où J est $J_1$, $J_2$, $J_3$ ou $J_4$, tels que définis dans le revendication 1, et W est O ou S, avec une amine hétérocyclique appropriée de formule

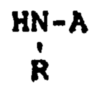 (III)

où R et A sont tels que définis dans la revendication 1 ; ou
(b) faire réagir un sulfonamide de formule

$J$-$SO_2NH_2$ (IV)

où J est $J_1$, $J_2$, $J_3$ ou $J_4$, tels que définis dans la revendication 1, avec un isothiocyanate hétérocyclique de formule

SCN-A

où A est tel que défini dans la revendication 1, pour obtenir un composé de la revendication 1 dans lequel W est S et R est H ; ou
(c) faire réagir un sulfonylcarbamate de formule

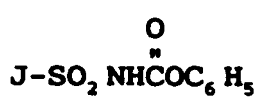 (VI)

où J est $J_1$, $J_2$, $J_3$ ou $J_4$, tels que définis dans la revendication 1, avec une amine de formule (III) telle que définie ci-dessus ; ou
(d) faire réagir ledit sulfonamide de formule (IV) avec un carbamate hétérocyclique de formule

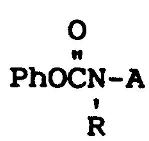 (VII)

où A et R sont tels que définis dans la revendication 1 ; ou

(e) faire réagir ledit sulfonamide de formule (IV) avec un carbamate de méthyle approprié de formule

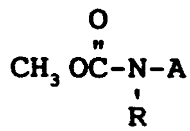

(VIII)

où A et R sont tels que définis dans la revendication 1.

**37.** Sulfonamides de formule (IV) tels que définis dans la revendication 36.

**Patentansprüche**

**1.** Verbindung der Formel

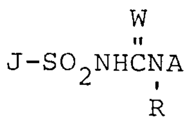

I

worin

J

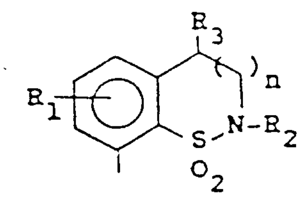

J-1

,

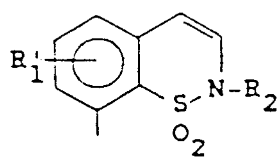

J-2

,

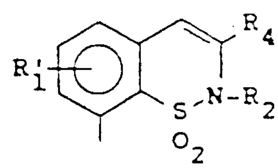

J-3

,

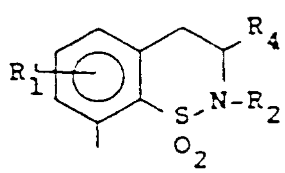

J-4

,

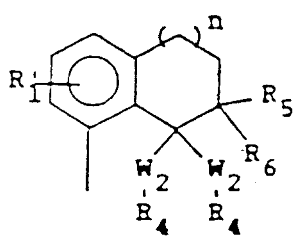

J-11

oder

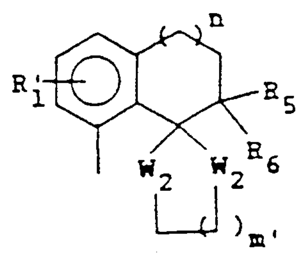

J-12

ist;

| | |
|---|---|
| n | 0 oder 1 ist; |
| W | O oder S ist; |
| $W_1$ | S ist; |
| $W_2$ | O oder S ist; |
| R | H oder $CH_3$ ist; |
| $R_1$ | H, $C_1$-$C_6$-Alkyl, $C_1$-$C_6$-Halogenalkyl, Halogen, Nitro, $C_1$-$C_6$-Alkoxy, $SO_2NR_aR_b$, $C_1$-$C_6$-Alkylthio, $C_1$-$C_6$-Alkylsulfinyl, $C_1$-$C_6$-Alkylsulfonyl, CN, $CO_2R_c$, $C_1$-$C_6$-Halogenalkoxy, $C_1$-$C_6$-Halogenalkylthio, $NH_2$, $C_1$-$C_6$-Alkylamino, Di($C_1$-$C_6$-alkyl)amino, $Si(CH_3)_2$($C_1$-$C_4$-Alkyl), $Si(CH_3)_2$phenyl oder mit $C_1$-$C_3$-Alkoxy, $C_1$-$C_3$-Alkylthio, $C_1$-$C_3$-Alkylsulfinyl, $C_1$-$C_3$-Alkylsulfonyl, $SO_2NR_dR_e$, $NO_2$, CN, $CO_2R_f$, $C_1$-$C_3$-Halogenalkoxy oder $C_1$-$C_3$-Halogenalkylthio substituiertes $C_1$-$C_3$-Alkyl ist; |
| $R_a$ | H, $C_1$-$C_4$-Alkyl, $C_1$-$C_3$-Cyanalkyl, Methoxy oder Ethoxy ist; |

$R_b$ H, $C_1$-$C_4$-Alkyl oder $C_3$-$C_4$-Alkenyl ist, oder

$R_a$ und $R_b$ als $-(CH_2)_3-$, $-(CH_2)_4-$, $-(CH_2)_5-$ oder $-CH_2CH_2OCH_2CH_2-$ zusammengenommen werden können;

$R_c$ $C_1$-$C_4$-Alkyl, $C_3$-$C_4$-Alkenyl, $C_3$-$C_4$-Alkinyl, $C_2$-$C_4$-Halogenalkyl, $C_2$-$C_3$-Cyanalkyl, $C_5$-$C_6$-Cycloalkyl, $C_4$-$C_7$-Cycloalkylalkyl oder $C_2$-$C_4$-Alkoxyalkyl ist;

$R_d$ $C_1$-$C_3$-Alkyl ist;

$R_e$ H oder $C_1$-$C_3$-Alkyl ist;

$R_f$ $C_1$-$C_3$-Alkyl ist;

$R_1'$ H, $C_1$-$C_3$-Alkyl, $C_1$-$C_3$-Alkoxy, $C_1$-$C_3$-Halogenalkoxy, $C_1$-$C_3$-Halogenalkyl, $C_1$-$C_3$-Alkylthio, $C_1$-$C_3$-Halogenalkylthio, Amino, $C_1$-$C_3$-Alkylamino, Di($C_1$-$C_3$-alkyl)-amino, Halogen oder $NO_2$ ist;

$R_2$ H, $R_{11}$, $SR_{11}$, $SO_2R_{11}$, $OR_{11}$, $C(O)R_{11}$, $C(O)OR_{11}$, $(C(O))_2OR_{11}$, $(CO)_2R_{11}$, $V(O)NR_{12}R_{18}$, C(O)NRA, $C(S)SR_{11}$, $NH_2$, $NR_{12}R_{18}$, OH, CN, $P(O)R_{13}R_{14}$, $P(S)R_{13}R_{14}$, $Si(CH_3)_2R_{15}$, L oder C(O)L ist;

$R_3$ H oder $CH_3$ ist;

$R_4$ $C_1$-$C_4$-Alkyl ist;

$R_5$ H oder $C_1$-$C_4$-Alkyl ist;

$R_6$ H oder $CH_3$ ist;

A

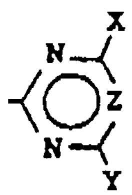 ,

A-1

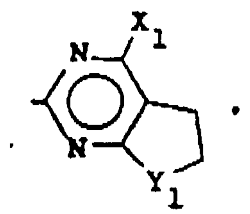 ,

A-2

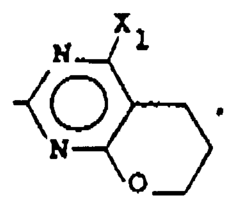 ,

A-3

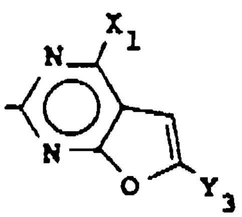 ,

A-4

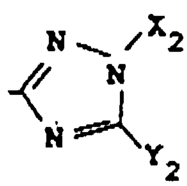 oder

A-5

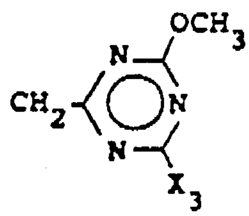 ist;

A-6

X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, F, Cl, Br, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino oder $C_3$-$C_5$-Cycloalkyl ist;

Y H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, F, Cl, Br, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_1$-$C_4$-Halogenalkyl, Azido, Cyano,

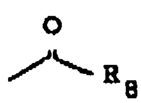 ,

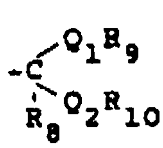, 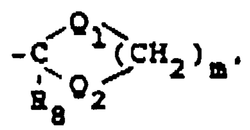, 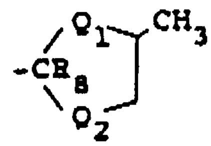

oder $N(OCH_3)CH_3$ ist;

m 2 oder 3 ist;

$Q_1$ und $Q_2$ unabhängig O oder S sind;

$R_8$ H oder $C_1$-$C_3$-Alkyl ist;

$R_9$ und $R_{10}$ unabhängig $C_1$-$C_3$-Alkyl sind:

Z CH, N, $CCH_3$, $CC_2H_5$, CCl oder CBr ist;

$Y_1$ O oder $CH_2$ ist;

$X_1$ $CH_3$, $OCH_3$, $OC_2H_5$ oder $OCF_2H$ ist;

$X_2$ $CH_3$, $C_2H_5$ oder $CH_2CF_3$ ist;

$Y_2$ $OCH_3$, $OC_2H_5$, $SCH_3$, $SC_2H_5$, $CH_3$ oder $CH_2CH_3$ ist;

$X_3$ $CH_3$ oder $OCH_3$ ist;

$Y_3$ H oder $CH_3$ ist;

$R_{11}$ $C_1$-$C_{10}$-Alkyl, $C_1$-$C_{10}$-Alkoxyalkoxyalkyl, $C_2$-$C_{10}$-Alkenyl, bis zu $C_{10}$-Alkenylalkenyl, $C_2$-$C_{10}$-Epoxyalkyl, $C_2$-$C_{10}$-Alkinyl, bis zu $C_{10}$-Alkinylalkinyl bis zu $C_{10}$-Alkinylalkenyl, $C_3$-$C_6$-Cycloalkyl, $C_4$-$C_7$-Cycloalkylalkyl oder

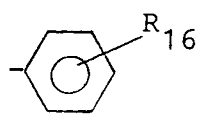

ist; wobei, wenn $R_{11}$ $C_3$-$C_6$-Cycloalkyl oder $C_4$-$C_7$-Cycloalkylalkyl ist, es gegebenenfalls durch $C_1$-$C_4$-Alkyl, 1 bis 3 Atome aus Cl oder F oder durch 1 Br substituiert sein kann; wenn $R_{11}$ $C_1$-$C_{10}$-Alkyl, $C_2$-$C_{10}$-Alkinyl oder $C_2$-$C_{10}$-Alkinyl ist, es gegebenenfalls durch ein oder mehrere Halogene und/oder durch $(R_{17})m'$ substituiert sein kann, worin, wenn m' 2 ist, die Bedeutungen von $R_{17}$ gleich oder verschieden sein können;

m' 1 oder 2 ist;

$R_{12}$ H oder $C_1$-$C_4$-Alkyl ist;

$R_{13}$ und $R_{14}$ unabhängig $C_1$-$C_4$-Alkyl, $C_1$-$C_4$-Alkoxy oder $C_1$-$C_4$-Alkylthio sind;

$R_{15}$ $C_1$-$C_{10}$-Alkyl, Benzyl oder

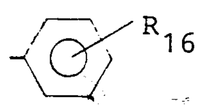

ist;

$R_{16}$ H, F, Cl, Br, $CH_3$, $OCH_3$, $NO_2$, CN, $SCH_3$, $SO_2CH_3$ oder $CF_3$ ist;

$R_{17}$ $OR_{18}$, $OC(O)R_{18}$, $P^+R_9R_{10}R_{15}$, $P^+(C_6H_5)_3$, $OC(O)NR_{12}R_{18}$, $OSO_2R_{18}$, $OP(O)R_{13}R_{14}$, $P(O)R_{13}R_{14}$, $OP(S)R_{13}R_{14}$, $P(S)R_{13}R_{14}$, $OSi(CH_3)_2R_{15}$, $Si(CH_3)_2R_{15}$, $SR_{18}$, $SOR_{18}$, $SO_2R_{18}$, SCN, CN, $SP(O)R_{13}R_{14}$, $SP(S)R_{13}R_{14}$, $N^+R_{12}R_{15}R_{18}$, $NR_{12}R_{18}$, $NR_{12}C(O)R_{18}$, $NR_{12}C(O)OR_{18}$, $NR_{12}C(O)NR_{12}R_{18}$, $NR_{12}SO_2R_{18}$, $NR_{12}P(O)R_{13}R_{14}$, $NR_{12}P(S)R_{13}R_{14}$, $NO_2$, $C(O)R_{18}$, $C(O)OR_{18}$, $C(O)NR_{12}R_{18}$, $SeR_{18}$, Naphthyl, L,

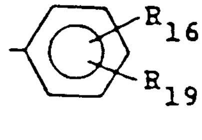, 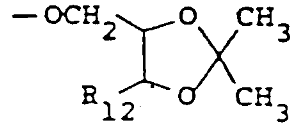,

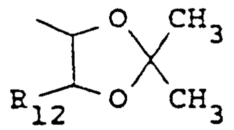 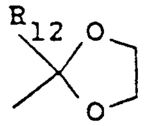 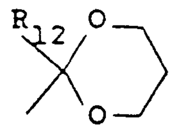

oder

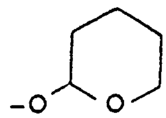

ist;

$R_{18}$ H, $C_1$-$C_{10}$-Alkyl, $C_1$-$C_{10}$-Halogenalkyl, $C_2$-$C_{10}$-Alkenyl, $C_2$-$C_{10}$-Alkinyl, $C_3$-$C_6$-Cycloalkyl oder

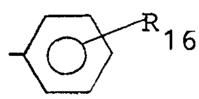

ist;

$R_{19}$ H, F, Cl, Br, $CH_3$,

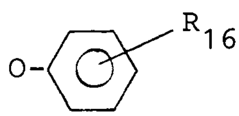

oder

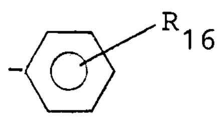

ist; und

L ein 5- oder 6-gliedriger aromatischer Heterozyklus, ein 5- oder 6-gliedriger dihydroaromatischer Heterozyklus oder ein 5- oder 6-gliedriger tetrahydroaromatischer Heterozyklus ist, der 1 bis 4 aus 0-1 Sauerstoffatomen, 0-1 Schwefelatomen, worin Schwefel die Form von S, SO oder $SO_2$ haben kann, und/oder 0-4 Stickstoffatomen ausgewählte Heteroatome enthält, mit der Maßgabe, daß Sauerstoff und Schwefel nur aneinander gebunden sind, wenn der Schwefel in Form von SO oder $SO_2$ vorliegt, und daß diese Heterozyklen gegebenenfalls durch 1-4 $CH_3$, 1-2 $OCH_3$, $SCH_3$, Cl, $N(CH_3)_2$ oder CN substituiert sein können, oder Lein 5- oder 6-gliedriges Lacton, Lactam oder Cycloalkanon ist, das gegebenenfalls durch 1-4 $CH_3$-Gruppen substituiert sein kann;

mit der Maßgabe, daß

a) wenn W S ist, dann R H ist, J $J_1$, $J_2$, $J_3$ oder $J_4$ ist; A A-1 ist, Z CH oder N ist und Y $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CF_3$, $OCH_2CH_2OCH_3$,CH-$(OCH_3)_2$ oder

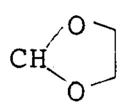

ist;
b) wenn X F, Cl oder Br ist, dann Z CH ist und Y $OCH_3$, $OC_2H_5$, $NH_2$, $NHCH_3$, $N(CH_3)_2$ oder $OCF_2H$ ist;
c) wenn $R_3$ $CH_3$ ist, dann n 0 ist;
d) wenn J J-1 oder J-2 ist und $R_2$ H oder $C_1$-$C_4$-Alkyl ist, dann $R_1$ und $R_1'$ von H, F, Cl, Br, $CH_3$, $OCH_3$, $CF_3$, $OCF_2H$ oder $SCH_3$ verschieden sind oder X von $CH_3$, $OCH_3$, $OCH_2CH_3$, F, Cl, Br, $OCF_2H$, $CH_2Cl$, $CH_2Br$, $CH_2F$, Cyclopropyl oder $CF_3$ verschieden ist oder Y $C_3$-$C_4$-Alkyl, $C_3$-$C_4$-Alkoxy, $C_4$-Halogenalkoxy, $C_4$-Halogenalkylthio, $C_3$-$C_5$-Alkoxyalkyl, $C_4$-$C_5$-Alkoxyalkoxy, $C_2$-$C_3$-Alkylamino, Di($C_2$-$C_3$-Alkyl)amino, $C_4$-Alkenyloxy, $C_4$-Alkinyloxy, $C_3$-$C_5$-Alkylthioalkyl, $C_2$-$C_4$-Halogenalkyl, $C_2$-$C_4$-Alkinyl, $C(O)R_8$ oder $N(OCH_3)CH_3$ ist;
e) die Gesamtzahl der Kohlenstoffatome in $R_2$ 13 nicht übersteigt;
f) wenn X $C_3$-$C_5$-Cycloalkyl ist, dann Y $CH_3$, $CH_2CH_3$, $OCH_3$, $OCH_2CH_3$, $CH_2OCH_3$, $OCF_2H$, $SCF_2H$, $OCH_2CF_3$, $CF_3$, $OCH_2CH=CH_2$, $OCH_2C\equiv CH$, $NHCH_3$, $N(CH_3)_2$ oder $CH(OCH_3)_2$ ist;
g) wenn $R_{18}$ H ist, dann $R_{17}$ von $SOR_{18}$, $SO_2R_{18}$, $OSO_2R_{18}$ oder $NR_{12}C(O)OR_{18}$ verschieden ist;
h) wenn $R_1$ oder $R_1'$ para zur Sulfonylharnstoffbrücke steht, dann $R_1$ oder $R_1'$ H, $CH_3$, F, Cl, Br oder $OCH_3$ ist; und
i) wenn X oder Y $OCH_2CH_2F$ oder $OCH_2CHF_2$ ist, dann $R_2$ von H oder $C_1$-$C_5$-Alkyl, $CH_3OCH_2CH_2$, $C_2H_5OCH_2CH_2$ oder mit 1 bis 3 Atomen aus F, Cl oder Br substituiertem $C_1$-$C_4$-Alkyl verschieden ist;
j) wenn X oder Y $OCH_2CH_2F$, $OCH_2CHF_2$ oder $OCH_2CF_3$ ist, dann das andere nicht Di($C_1$-$C_3$-alkyl)-amino, $C_1$-$C_3$-Alkylamino oder $N(OCH_3)CH_3$ ist;
k) wenn X oder Y $OCF_2H$ ist, dann Z CH ist; sowie deren landwirtschaftlich geeignete Salze.

**2.** Verbindung nach Anspruch 1, worin J J-1 oder J-4 ist.

**3.** Verbindung nach Anspruch 1, worin J J-2 oder J-3 ist.

**4.** Verbindung nach Anspruch 1, worin J J-11 oder J-12 ist.

**5.** Verbindungen nach Anspruch 2,
worin
W O ist;
R H ist;
X $CH_3$, $OCH_3$, $OCH_2CH_3$, Cl, F, Br, $OCF_2H$, $CH_2F$, $CF_3$ oder Cyclopropyl ist;
Y H, $CH_3$, $OCH_3$, $OC_2H_5$, $CH_2OCH_3$, $NH_2$, $NHCH_3$, $N(OCH_3)CH_3$ $N(CH_3)_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$,

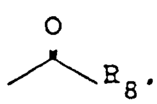 , 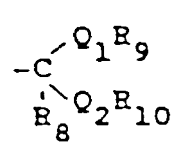 , 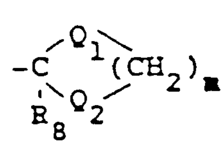 , 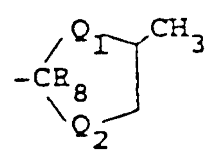

oder $QCF_2T$ ist;
Q O oder S ist;
T H, CHClF, CHBrF oder $CHFCF_3$ ist.

**6.** Verbindungen nach Anspruch 5, worin $R_1$ oder $R_1'$ H, Cl, $C_1$-$C_3$-Alkyl, $C_1$-$C_3$-Alkoxy, $C_1$-$C_3$-Halogenalkoxy, $C_1$-$C_3$-Halogenalkyl, $C_1$-$C_3$-Alkylthio, $C_1$-$C_3$-Halogenalkylthio, Amino, $C_1$-$C_3$-Alkylsulfinyl, $C_1$-$C_3$-Alkylsulfonyl, CN, $NH_2$, $C_1$-$C_3$-Alkylamino, Di($C_1$-$C_3$-alkylamino) oder mit $C_1$-$C_2$-Alkoxy, $C_1$-$C_2$-Alkylthio, $C_1$-$C_2$-Halogenalkoxy, $C_1$-$C_2$-Halogenalkylthio, CN oder $NO_2$ substituiertes $C_1$-$C_2$-Alkyl ist.

**7.** Verbindungen nach Anspruch 6, worin $R_2$ $C_1$-$C_6$-Alkyl, $C_1$-$C_6$-Alkylcarbonyl, $C_1$-$C_6$-Alkoxycarbonyl, mit 1 bis 3 Atomen aus F, Cl oder 1 Br oder durch 1 oder 2 aus $C_1$-$C_2$-Alkoxy, CN, $C_1$-$C_2$-Alkoxycarbonyl, $C_1$-$C_2$-Alkylcarbonyl, OH, $C_1$-$C_2$-Alkylthio, $C_1$-$C_2$-Alkylsulfonyl, $C_1$-$C_2$-Alkylsulfonyloxy oder $C_1$-$C_2$-Alkylcarbonyloxy ausgewählten Gruppen substituiertes $C_1$-$C_6$-Alkyl, $C_3$-$C_4$-Alkenyl, $C_3$-$C_4$-Halogenalkenyl, $C_3$-$C_4$-Alkinyl, durch ein Atom aus F oder Cl substituiertes $C_4$-Alkinyl, Phenyl oder mit Cl, $CF_3$,

$NO_2$, CN oder $SO_2CH_3$ substituiertes Phenyl ist und $R_4$ $CH_3$ ist.

**8.** Verbindungen nach Anspruch 7, worin A A-1 ist und Y $CH_3$, $C_2H_5$, $OCH_3$, $CH_2OCH_3$, $OCF_2H$ oder CH-($OCH_3$)$_2$ ist.

**9.** Verbindungen nach Anspruch 8, worin $R_1$ und $R_1'$ H, Cl, $C_1$-$C_2$-Alkyl, $C_1$-$C_2$-Alkoxy oder $C_1$-$C_2$-Alkylthio sind.

**10.** Verbindungen ach Anspruch 9, worin J J-1 ist.

**11.** Verbindungen nach Anspruch 10, worin $R_1$ H ist und Z CH ist.

**12.** Verbindungen nach Anspruch 11, worin $R_2$ $C_1$-$C_4$-Halogenalkyl, mit $C_1$-$C_2$-Alkoxy, $C_1$-$C_2$-Alkylthio, $C_1$-$C_2$-Alkylsulfonyl oder CN substituiertes $C_1$-$C_3$-Alkyl, $C_3$-$C_4$-Alkenyl, $C_3$-$C_4$-Halogenalkenyl, $C_3$-$C_4$-Alkinyl, $C_1$-$C_3$-Alkoxycarbonyl oder $C_1$-$C_3$-Alkylcarbonyl ist.

**13.** Verbindungen nach Anspruch 10, worin $R_1$ H ist; Z N ist; und $R_2$ $C_1$-$C_3$-Alkylcarbonyl oder $C_1$-$C_3$-Alkoxycarbonyl ist.

**14.** Verbindungen nach Anspruch 3, worin W O ist; $R_1$ H ist; X $CH_3$, $OCH_3$, $OCH_2CH_3$, Cl, F, Br, $OCF_2H$, $CH_2F$, $CF_3$ oder Cyclopropyl ist; Y H, $CH_3$, $OCH_3$, $OC_2H_5$, $CH_2OCH_3$, $NH_2$, $NHCH_3$, $N(OCH_3)CH_3$, N-($CH_3$)$_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$

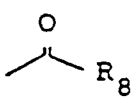, 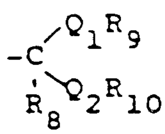, 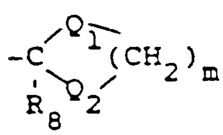, 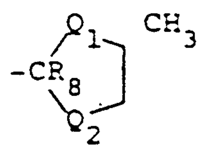

oder $QCF_2T$ ist; $R_2$ $C_1$-$C_6$-Alkyl, $C_1$-$C_6$-Alkylcarbonyl, $C_1$-$C_6$-Alkoxycarbonyl, durch 1-3 Atome aus F, Cl oder 1 Br oder durch 1 oder 2 aus $C_1$-$C_2$-Alkoxy, CN, $C_1$-$C_2$-Alkoxycarbonyl, $C_1$-$C_2$-Alkylcarbonyl, OH, $C_1$-$C_2$-Alkylthio, $C_1$-$C_2$-Alkylsulfonyl, $C_1$-$C_2$-Alkylsulfonyloxy oder $C_1$-$C_2$-Alkylcarbonyloxy ausgewählte Gruppen substituiertes $C_1$-$C_6$-Alkyl, $C_3$-$C_4$-Alkenyl, $C_3$-$C_4$-Halogenalkenyl, $C_3$-$C_4$-Alkinyl, durch 1 Atom aus F oder Cl substituiertes $C_4$-Alkinyl, Phenyl oder mit Cl, $CF_3$, $NO_2$, CN oder $SO_2CH_3$ substituiertes Phenyl ist; $R_1'$ H, Cl, $C_1$-$C_2$-Alkyl, $C_1$-$C_2$-Alkoxy oder $C_1$-$C_2$-Alkylthio ist.

**15.** Verbindungen nach Anspruch 14, worin A A-1 ist und Y $CH_3$, $C_2H_5$, $OCH_3$, $CH_2OCH_3$, $OCF_2H$ oder CH-($OCH_3$)2 ist.

**16.** Verbindungen nach Anspruch 4, worin W O ist, $R_1$ H ist, $R_1'$ H ist, A A-1 ist, X $CH_3$, $OCH_3$, $OCH_2CH_3$, Cl, F, Br, $OCF_2H$, $CH_2F$ oder Cyclopropyl ist und Y $CH_3$, $C_2H_5$, $OCH_3$, $CH_2OCH_3$,$OCF_2H$ oder CH-($OCH_3$)$_2$ ist.

**17.** Verbindung nach Anspruch 1, welche 2,3-Dihydro-N-[(4,6-dimethoxypyrimidin-2-yl)aminocarbon-1]-2-(2-methoxyethyl)-1,2-benzisothiazol-7-sulfonamid-1,1-dioxid ist.

**18.** Verbindung nach Anspruch 1, welche 2,3-Dihydro-N-[(4,6-dimethoxypyrimidin-2-yl)aminocarbonyl]-2-(3-methylbutyl)-1,2-benzisothiazol-7-sulfonamid-1,1-dioxid ist.

**19.** Verbindung nach Anspruch 1, welche 2,3-Dihydro-N-[(4,6-dimethoxypyrimidin-2-yl)aminocarbonyl]-2-(phenylmethyl)-1,2-benzisothiazol-7-sulfonamid-1,1-dioxid ist.

**20.** Verbindung nach Anspruch 1, welche 2,3-Dihydro-N-[(4,6-dimethoxypyrimidin-2-yl)aminocarbonyl]-2-(2-oxopropyl)-1,2-benzisothiazol-7-sulfonamid-1,1-dioxid ist.

**21.** Verbindung nach Anspruch 1, welche 2,3-Dihydro-N-[(4-Chlor-6-methoxypyrimidin-2-yl)aminocarbonyl]-2-(2-ethoxyethyl)-1,2-benzisothiazol-7-sulfonamid-1,1-dioxid ist.

**22.** Verbindung nach Anspruch 1, welche 2,3-Dihydro-N-[(4,6-dimethoxypyrimidin-2-yl)aminocarbonyl]-2-(2-ethoxyethyl)-1,2-benzisothiazol-7-sulfonamid-1,1-dioxid ist.

**23.** Verbindung nach Anspruch 1, welche 2,3-Dihydro-N-[(4,6-dimethylpyrimidin-2-yl)aminocarbonyl]-2-(2-fluorethyl)-1,2-benzisothiazol-7-sulfonamid-1,1-dioxid ist.

**24.** Verbindung nach Anspruch 1, welche 2,3-Dihydro-N-[(4-methoxy-6-methyl-1,3,5-triazin-2-yl)-aminocarbonyl]-2-(2-fluorethyl)-1,2-benzisothiazol-7-sulfonamid-1,1-dioxid ist.

**25.** Verbindung nach Anspruch 1, welche 2,3-Dihydro-N-[(4,6-dimethoxypyrimidin-2-yl)aminocarbonyl]-2-acetyl-1,2-benzisothiazol-7-sulfonamid-1,1-dioxid ist.

**26.** Verbindung nach Anspruch 1, welche 2,3-Dihydro-N-[(4,6-dimethoxypyrimidin-2-yl)aminocarbonyl]-2-(3-chlorpropyl)-1,2-benzisothiazol-7-sulfonamid-1,1-dioxid ist.

**27.** Verbindung nach Anspruch 1, welche 2,3-Dihydro-N-[(chlor-6-methoxypyrimidin-2-yl)aminocarbonyl]-2-(2-fluorethyl)-1,2-benzisothiazol-7-sulfonamid-1,1-dioxid ist.

**28.** Verbindung nach Anspruch 1, welche 2,3-Dihydro-N-[(4,6-dimethoxypyrimidin-2-yl) aminocarbonyl]-2-(3-fluorpropyl)-1,2-benzisothiazol-7-sulfonamid-1,1-dioxid ist.

**29.** Verbindung nach Anspruch 1, welche 2,3-Dihydro-N-[(4-Chlor-6-methoxypyrimidin-2-yl)aminocarbonyl]-2-(2-chlorethyl)-1,2-benzisothiazol-7-sulfonamid-1,1-dioxid ist.

**30.** Verbindung nach Anspruch 1, welche N-[(4-Chlor-6-methoxypyrimidin-2-yl)aminocarbonyl]-2-(2-fluorethyl)-2,3-dihydro-1,2-benzisothiazol-7-sulfonamid-1,1-dioxid ist.

**31.** Verbindung nach Anspruch 1, welche N-[(4-Chlor-6-methoxypyrimidin-2-yl)aminocarbonyl]-2-(3-fluorpropyl)-2,3-dihydro-1,2-benzisothiazol-7-sulfonamid-1,1-dioxid ist.

**32.** Verbindung nach Anspruch 1,
worin

| | |
|---|---|
| J | aus J-1 bis J-4 ausgewählt ist; |
| $W_2$ | O ist; |
| $R_1$ | und $R_1$' H, F, Cl, Br, $CH_3$, $C_2H_5$, $OCH_3$, $OC_2H_5$, $CF_3$, $OCF_2H$ oder $SCH_3$ ist; |
| $R_2$ | H, $C_3$-$C_6$-Cycloalkyl, ein $C_1$-$C_{10}$-Alkylrest, ein $C_2$-$C_{10}$-Alkenylrest und deren entsprechende Epoxide, ein $C_2$-$C_{10}$-Alkinylrest, ein $C_1$-$C_9$-Alkoxylrest, ein $C_1$-$C_9$-Alkylcarbonylrest, ein $C_1$-$C_8$-Alkylsulfonylrest oder ein $C_1$-$C_8$-Alkoxycarbonylrest ist, wobei jeder der oben erwähnten Reste gegebenenfalls mit Halogen, $C_3$-$C_6$-Cycloalkyl, $C_1$-$C_8$-Alkoxy, $C_1$-$C_8$-Halogenalkoxy, $C_1$-$C_8$-Alkylthio, $C_1$-$C_8$-Halogenalkylthio, $C_1$-$C_8$-Alkylsulfinyl, $C_1$-$C_8$-Halogenalkylsulfinyl, $C_1$-$C_8$-Alkylsulfonyl, $C_1$-$C_8$-Halogenalkylsulfonyl, $C_1$-$C_6$-Halogenalkylcarbonyl, $C_1$-$C_6$-Alkylcarbonyloxy, $C_1$-$C_6$-Halogenalkylcarbonyloxy, $C_1$-$C_6$-Alkylsulfonyloxy, $C_1$-$C_6$-Alkylcarbonyl, $C_1$-$C_6$-Alkoxycarbonyl, $C_1$-$C_6$-Halogenalxoxycarbonyl, Formyl, Amino, $C_1$-$C_6$-Alkylamino, $C_1$-$C_8$-Dialkylamino, $C_1$-$C_6$-Alkylcarbonylamino, OH, CN, $NO_2$, $C_3$-$C_6$-Alkylsilyl, $C_2$-$C_6$-Alkyl(phenylsilyl), worin Phenyl gegebenenfalls mit Halogen, $CH_3$, $OCH_3$ oder $NO_2$ substituiert ist, oder gegebenenfalls mit Halogen, $CH_3$, $OCH_3$ oder $NO_2$ substituiertem Phenyl substituiert ist; |
| X | $CH_3$, $OCH_3$, $OCH_2CH_3$, Cl, F, Br, $OCF_2H$, $CH_2F$, $CF_3$ oder Cyclopropyl ist; |
| Y | H, $CH_3$, $OCH_3$, $OC_2H_5$, $CH_2OCH_3$, $NH_2$, $NHCH_3$, $N(OCH_3)$-$CH_3$, $N(CH_3)_2$, $C_2H_5$, $CF_3$, $SCH_3$, $OCH_2CH=CH_2$, $OCH_2C{\equiv}CH$, $OCH_2CF_3$, CN, $N_3$, $OCH_2CH_2OCH_3$, $CH_2SCH_3$, |

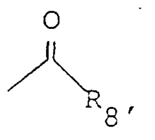, 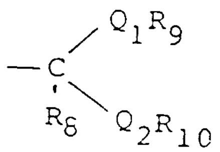, 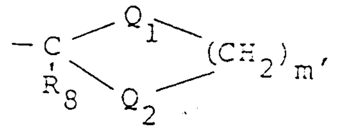, 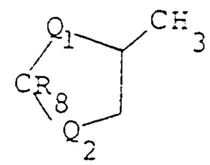

| | |
|---|---|
| | oder $QCF_2T$ ist; |
| m | 2 oder 3 ist; |
| Q | O oder S ist; |
| $Q_1$ und $Q_2$ | unabhängig O oder S sind; |
| $R_8$ | H oder $C_1$-$C_3$-Alkyl ist; |
| $R_9$ und $R_{10}$ | unabhängig $C_1$-$C_3$-Alkyl sind und |
| T H, CHClF, CHBrF oder $CHFCF_3$ | ist. |

**33.** Zur Bekämpfung des Wachstums unerwünschter Vegetation geeignete Zusammensetzung, die eine wirksame Menge einer Verbindung nach einem der Ansprüche 1 bis 32 und wenigstens eines der folgenden: oberflächenaktives Mittel, festes oder flüssiges Verdünnungsmittel, umfaßt.

**34.** Verfahren zur Bekämpfung des Wachstums unerwünschter Vegetation durch Anwenden einer wirksamen Menge einer Verbindung nach einem der Ansprüche 1 bis 32 auf den zu schützenden Ort.

**35.** Verfahren zur Steuerung des Wachstums von Pflanzen durch Anwenden einer wirksamen, jedoch im wesentlichen nicht phytotoxischen Menge eines Pflanzenwachstumsregulans, ausgewählt aus Verbindungen nach einem der Ansprüche 1 bis 32, auf den Ort solcher Pflanzen.

**36.** Verfahren zur Herstellung einer Verbindung nach Anspruch 1 durch

(a) Umsetzen eines Sulfonylisocyanats oder Sulfonylisothiocyanats der Formel

$J\text{-}SO_2NCW$ (II)

worin J $J_1$, $J_2$, $J_3$ oder $J_4$ wie in Anspruch 1 definiert ist und W O oder S ist, mit einem geeigneten heterocyclischen Amin der Formel

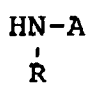 (III)

worin R und A wie in Anspruch 1 definiert sind; oder

(b) Umsetzen eines Sulfonamids der Formel

$J\text{-}SO_2NH_2$ (IV)

worin J $J_1$, $J_2$, $J_3$ oder $J_4$, wie in Anspruch 1 definiert, ist, mit einem heterocyclischen Isothiocyanat der Formel

SCN-A

worin A wie in Anspruch 1 definiert ist, unter Erhalt einer Verbindung nach Anspruch 1, worin W S ist und R H ist; oder

(c) Umsetzen eines Sulfonylcarbamats der Formel

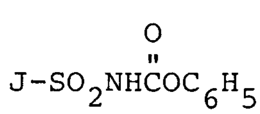 (VI)

worin J $J_1$, $J_2$, $J_3$ oder $J_4$, wie in Anspruch 1 definiert, ist, mit einem Amin der Formel (III), wie vorstehend definiert; oder

(d) Umsetzen des Sulfonamids der Formel (IV) mit einem heterocyclischen Carbamat der Formel

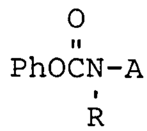

(VII)

worin A und R wie in Anspruch 1 definiert sind; oder

(e) Umsetzen des Sulfonamids der Formel (IV) mit einem geeigneten Methylcarbamat der Formel

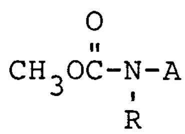

(VIII)

worin A und R wie in Anspruch 1 definiert sind.

**37.** Sulfonamide der Formel (IV), wie in Anspruch 36 definiert.